# EUROPEAN PATENT APPLICATION

(11) **EP 4 516 779 A1**
(43) Date of publication of application: **05.03.2025**
(21) Application number: 23795562.0
(22) Date of filing: 27.04.2023
(51) Int. Cl.: C07D 239/94, C07D 405/12, A61K 31/505, A61P 35/00

(54) **PYRIMIDINE COMPOUND, METHOD FOR PREPARING SAME, AND PHARMACEUTICAL USE THEREOF**

(30) Priority: 29.04.2022 CN 202210472813; 20.01.2023 CN 202310079788
(71) Applicant: Jiangsu Yahong Meditech Co., Ltd., Taizhou, Jiangsu 225316 (CN); Asieris Pharmaceuticals (Shanghai) Co., Ltd., Pudong, Shanghai 201203 (CN)
(72) Inventor: WU, Maojiang, Taizhou, Jiangsu 225316 (CN); WANG, Tielin, Shanghai 201203 (CN); MENG, Zhi, Taizhou, Jiangsu 225316 (CN); SUN, Qiaoling, Taizhou, Jiangsu 225316 (CN); SONG, Xia, Taizhou, Jiangsu 225316 (CN)
(74) Representative: Regimbeau
(86) International application number: PCT/CN2023/091270
(87) International publication number: WO 2023/208130

(57) **Abstract**

Provided are a compound represented by general formula (I'), a method for preparing same, a pharmaceutical composition comprising same, and use thereof as a ubiquitin specific protease 1 (USP1) inhibitor, in particular, use thereof in treating or preventing a cancer.

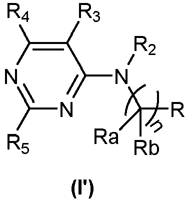

## Description

### FIELD OF THE INVENTION

The present invention relates to compounds having USP1 inhibitory effects and compositions thereof, and a use thereof in the preparation of medicament for treating USP1 enzyme-related disease. In particular, the present invention relates to a compound of formula (I) and a pharmaceutically acceptable salt thereof.

### BACKGROUND OF THE INVENTION

Ubiquitination is a dynamic and reversible process involving the deubiquitinating enzyme (DUB) family. There are about 100 DUBs in the human body, which can be divided into the cysteine protease family and the metalloprotease family. Among them, the cysteine protease family mainly includes ubiquitin-specific proteases (USPs), ubiquitin carboxyl-terminal hydrolases (UCHs), Machado-Josephin domain proteases (MJDs), MINDY proteases (MINDYs) and ovarian tumor domain proteases (OTUs). The USPs family is the largest family of known deubiquitinating enzymes, with more than 50 types encoded by human genes. It plays a role in various physiological functions such as cell cycle, signal transduction, DNA damage repair, chromosome translocation and gene transcription by regulating substrate proteases.

USP1 belongs to the USP subfamily of DUBs. It has no significant activity itself, but acquires full enzymatic activity after binding to UAF1 to form a heterodimeric complex (USP1/UAF1), thereby regulating cellular targets in multiple pathways related to cancer. For example, the USP1/UAF1 complex deubiquitinates the monoubiquitinated proliferating cell nuclear antigen (PCNA), a key protein in the translesion synthesis (TLS) process, to prevent its excessive repair, and deubiquitinates the monoubiquitinated Fanconi anemia complementation group D2 (FAND2), a key protein in the Fanconi anemia (FA) pathway, to block cells from performing improper TLS repair, thereby ensuring genome stability. These two DNA damage response (DDR) pathways are key pathways for repairing DNA damage induced by DNA cross-linking agents such as cisplatin, mitomycin, and ultraviolet radiation. USP1 can also interact with ID proteins and the like, and stabilize the expression of ID proteins in cells through deubiquitination. For example, in osteosarcoma cells, USP1 can deubiquitinate ID1, ID2 and ID3, and promote cell proliferation. Inhibition of USP1 can increase the sensitivity of osteosarcoma cells to chemotherapy. In addition, studies have confirmed that USP1 is closely related to the development of drug resistance in a variety of tumor treatment drugs. For example, in non-small cell lung cancer (NSCLC) cells with cisplatin resistance, USP1 expression is high, and knocking down USP1 can significantly enhance the sensitivity of cells to cisplatin; in breast cancer cells, USP1 is highly expressed, promoting breast cancer cell proliferation and is closely related to the poor prognosis of breast cancer. A document (J. Med. Chem. 2014, 57, 8099-8110, Synthesis and Structure-Activity Relationship Studies of N-Benzyl-2-phenylpyrimidin-4-amine Derivatives as Potent USP1/UAF1 Deubiquitinase Inhibitors with Anticancer Activity against Nonsmall Cell Lung Cancer) reported that USP1 inhibitors such as ML323 compound can be used for treating non-small cell lung cancer. A document (Cui S-Z, Lei Z-Y, Guan T-P, et al. Targeting USP1-dependent KDM4A protein stability as a potential prostate cancer therapy. Cancer Sci. 2020;00:1-15.) reported that USP1 inhibitors are used as potential drugs for prostate cancer. In summary, USP1 is expected to become a hot target for the treatment of various cancers and other diseases.

### SUMMARY OF THE INVENTION

The present invention provides a compound of formula (I'), 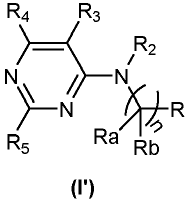 or a pharmaceutically acceptable salt, hydrate, solvate, isotope substitute or stereoisomer thereof,
wherein,
R is selected from the group consisting of C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, C₆₋₁₀ aryl, C₆₋₁₀ aryl fused with 5 to 10 membered heteroaryl, and 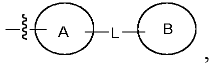 wherein the above groups are each independently optionally substituted by one or more R₁;
ring A and ring B are each independently selected from the group consisting of C₆₋₁₀ aryl, 5 to 10 membered heteroaryl, C₃₋₈ cycloalkyl and 3 to 8 membered heterocyclyl, wherein ring A and ring B are each independently optionally substituted by one or more R₁;
L is selected from the group consisting of a chemical bond, -O-, -S-, -C₁₋₆ alkylene-, -O-C₁₋₆ alkylene-, -C₁₋₆ alkylene-O-, -S-C₁₋₆ alkylene- and -C₁₋₆ alkylene-S-;
Rₐ and R_{b} are each independently selected from the group consisting of H atom, - CN, C₁₋₆ alkyl, -OH, halogen, C₂₋₆ alkenyl, C₂₋₆ alkynyl, C₁₋₆ hydroxyalkyl, C₁₋₆ haloalkyl, C₁₋₆ alkoxy and C₁₋₆ haloalkoxy; or Rₐ and R_{b} together form an oxo, C₃₋₈ cycloalkyl or 3 to 8 membered heterocyclyl;
R₂ is selected from the group consisting of H atom, -OH, -CN, C₁₋₆ alkyl, -C₁₋₆ alkyl-C₆₋₁₀ aryl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, C₆₋₁₀ aryl, 5 to 10 membered heteroaryl, C₃₋₈ cycloalkyl and 3 to 8 membered heterocyclyl, wherein the C₁₋₆ alkyl and -C₁₋₆ alkyl-C₆₋₁₀ aryl are each optionally substituted by one or more R₁;
R₃ is selected from the group consisting of H atom, -OH, -COOH, -NH₂, -CN, halogen, C₁₋₆ alkyl, -S-C₁₋₆ alkyl, -S(O)-C₁₋₆ alkyl, -S(O)₂-C₁₋₆ alkyl, phosphoryl, phosphonyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, C₆₋₁₀ aryl, 5 to 10 membered heteroaryl, C₃₋₈ cycloalkyl, 3 to 8 membered heterocyclyl and -C₁₋₆ alkylene-C(O)-O-C₁₋₆ alkyl, wherein the -NH₂, C₁₋₆ alkyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, C₆₋₁₀ aryl, 5 to 10 membered heteroaryl, C₃₋₈ cycloalkyl and 3 to 8 membered heterocyclyl are each independently optionally substituted by one or more R₁;
R₄ is selected from the group consisting of H atom, -OH, -COOH, -NH₂, -CN, halogen, C₁₋₆ alkyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, C₆₋₁₀ aryl, 5 to 10 membered heteroaryl, C₃₋₈ cycloalkyl and 3 to 8 membered heterocyclyl;
or, R₂ and R₃ together with the atoms to which they are attached form ring C, ring C is a 5 to 7 membered heteroaryl or 5 to 7 membered heterocyclyl, the heteroatom in the 5 to 7 membered heteroaryl or 5 to 7 membered heterocyclyl is O or N, further, the 5 to 7 membered heterocyclyl is selected from the group consisting of morpholinyl, 3-morpholinonyl, pyrrolidinyl, 2-oxazolidinonyl and 2-pyrrolidinonyl, and ring C is optionally substituted by one or more R₁;
or, R₃ and R₄ together with the atoms to which they are attached form ring D, ring D is a 5 to 7 membered heteroaryl or 5 to 7 membered heterocyclyl, and ring D is optionally substituted by one or more R₁;
R₅ is selected from the group consisting of C₁₋₆ alkyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, C₆₋₁₀ aryl, 5 to 10 membered heteroaryl, C₆₋₁₀ aryl fused with 5 to 10 membered heteroaryl, C₆₋₁₀ aryl fused with 3 to 8 membered heterocyclyl, C₃₋₈ cycloalkyl and 3 to 8 membered heterocyclyl, wherein the C₁₋₆ alkyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, C₆₋₁₀ aryl, 5 to 10 membered heteroaryl, C₃₋₈ cycloalkyl and 3 to 8 membered heterocyclyl are each independently optionally substituted by one or more R₁;
R₁ at each occurrence is independently selected from the group consisting of D atom, -OH, -COOH, -NH₂, -CN, oxo, halogen, C₁₋₆ alkyl, C₂₋₆ alkynyl, C₁₋₆ haloalkyl, C₁₋₆ alkoxy, C₁₋₆ haloalkoxy, C₁₋₆ hydroxyalkyl, C₆₋₁₀ aryl, 5 to 10 membered heteroaryl, C₃₋₈ cycloalkyl, 3 to 8 membered heterocyclyl and -O-C₁₋₆ alkylene-O-C₁₋₆ alkyl, wherein the C₆₋₁₀ aryl, 5 to 10 membered heteroaryl, C₃₋₈ cycloalkyl and 3 to 8 membered heterocyclyl are each independently optionally substituted by one or more substituent(s) selected from the group consisting of D atom, -OH, -COOH, -NH₂, -CN, halogen, C₁₋₆ alkyl, C₁₋₆ haloalkyl, C₁₋₆ alkoxy, C₁₋₆ haloalkoxy and C₁₋₆ hydroxyalkyl; and
n is an integer from 0 to 8.

In some embodiments, the compound of formula (I') is a compound of formula (I), 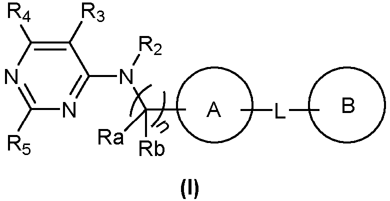 wherein ring A, ring B, L, Rₐ, R_{b}, R₂ to R₅ and n are as defined in formula (I').

In some embodiments, in the compound of formula (I') or (I),
R₂ is selected from the group consisting of H atom, -OH, -CN, C₁₋₆ alkyl, C₂₋₆ alkynyl, -C₁₋₆ alkyl-C₆₋₁₀ aryl, C₁₋₆ alkoxy, C₁₋₆ haloalkyl, C₁₋₆ haloalkoxy, C₁₋₆ hydroxyalkyl, C₃₋₆ cycloalkyl and 5 to 7 membered heterocyclyl, wherein the C₁₋₆ alkyl and -C₁₋₆ alkyl-C₆₋₁₀ aryl are each optionally substituted by one or more R₁, R₁ is as defined in formula (I');
preferably, R₂ is selected from the group consisting of H atom, -CN, methyl, trideuteriomethyl, ethynyl, propynyl, tetrahydrofuranyl, cyclopropyl, methoxy and hydroxy;
R₃ is selected from the group consisting of H atom, -OH, -COOH, -NH₂, -CN, halogen, C₁₋₆ alkyl, C₂₋₆ alkynyl, C₁₋₆ alkoxy, C₁₋₆ haloalkyl, C₁₋₆ haloalkoxy, C₁₋₆ hydroxyalkyl, 5 to 7 membered heterocyclyl and -C₁₋₆ alkylene-C(O)-O-C₁₋₆ alkyl, wherein the C₁₋₆ hydroxyalkyl and 5 to 7 membered heterocyclyl are each independently optionally substituted by one or more C₁₋₆ alkyl;
preferably, R₃ is selected from the group consisting of H atom, methoxy, trifluoromethyl, Cl atom, -CN, isopropoxy, ethynyl, difluoromethoxy, morpholinyl, 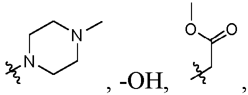 F atom, hydroxymethyl and 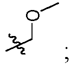 and
R₄ is selected from the group consisting of H atom, -OH, -COOH, -NH₂, -CN, halogen, C₁₋₆ alkyl, C₁₋₆ alkoxy, C₁₋₆ haloalkyl, C₁₋₆ haloalkoxy and C₁₋₆ hydroxyalkyl;
preferably, R₄ is H atom.

In some embodiments, the compound of formula (I') or (I) is a compound of formula (II), 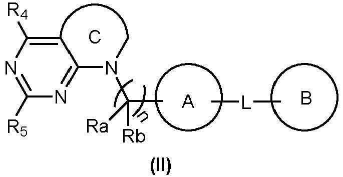
wherein ring C is selected from the group consisting of 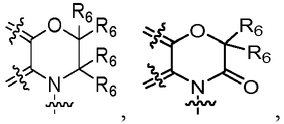 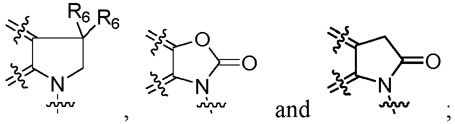
R₆ at each occurrence is independently H or C₁₋₆ alkyl, or two R₆ together with the atom to which they are attached form a C₃₋₈ cycloalkyl or 3 to 8 membered heterocyclyl; and
ring A, ring B, L, Rₐ, R_{b}, R₄, R₅ and n are as defined in formula (I');
particularly,
R₄ is selected from the group consisting of H atom, -OH, -COOH, -NH₂, -CN, halogen, C₁₋₆ alkyl, C₁₋₆ alkoxy, C₁₋₆ haloalkyl, C₁₋₆ haloalkoxy and C₁₋₆ hydroxyalkyl; and preferably, R₄ is H atom.

In some embodiments, the compound of formula (I') is a compound of formula (III), 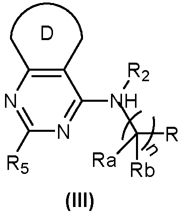
wherein ring D is selected from the group consisting of 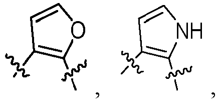 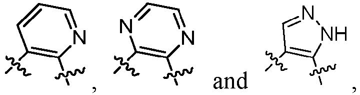 wherein the above groups are each independently optionally substituted by one or more R₁; and
R, Rₐ, R_{b}, R₁, R₂, R₅ and n are as defined in formula (I');
particularly, R₂ is selected from the group consisting of H atom, -OH, C₁₋₆ alkyl, C₂₋₆ alkynyl, -C₁₋₆ alkyl-C₆₋₁₀ aryl, C₁₋₆ alkoxy, C₁₋₆ haloalkyl, C₁₋₆ haloalkoxy and C₁₋₆ hydroxyalkyl, wherein the C₁₋₆ alkyl or -C₁₋₆ alkyl-C₆₋₁₀ aryl is each optionally substituted by one or more R₁, R₁ is as defined in formula (I');
preferably, R₂ is H atom or 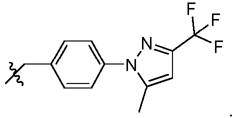
In some embodiments, in the compound of formula (I'), (I), (II) or (III),
ring A and ring B are each independently selected from the group consisting of phenyl, piperidinyl, cyclohexyl, cyclopropyl, cyclobutyl, pyridinyl, pyrimidinyl, imidazolyl, pyrazolyl, bicyclo[2.2.2]octanyl, 2-oxabicyclo[2.2.2]octanyl, pentacyclooctanyl, isoindolinonyl, imidazo[1,2-a]pyrazinyl, piperidine-2,6-dionyl, thienyl, furanyl, cyclopentyl, pyranyl, pyrrolidinyl, piperazinyl, morpholinyl, naphthyl, pyrrolyl, pyrazinyl, pyridazinyl, triazolyl, tetrazolyl, indolyl, isoindolyl, indolinyl, isoindolinyl, indolinonyl, pyrido[3,2-d]pyrimidinyl, pteridinyl, pyrazolo[4,3-c]pyridinyl, pyrazolo[3,4-d]pyrimidinyl and cubanyl, wherein ring A and ring B are each independently optionally substituted by one or more R₁, R₁ is as defined in formula (I');
L is selected from the group consisting of a chemical bond, -O-, -O-C₁₋₆ alkylene- and -C₁₋₆ alkylene-O-;
particularly, 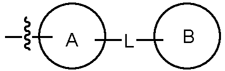 is selected from the group consisting of 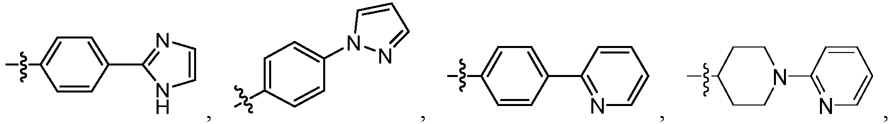 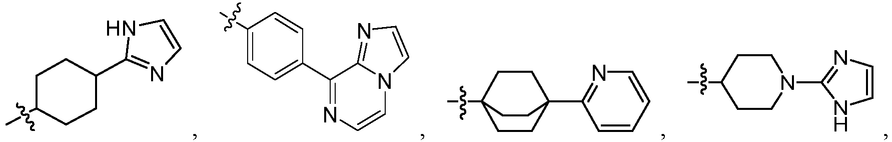 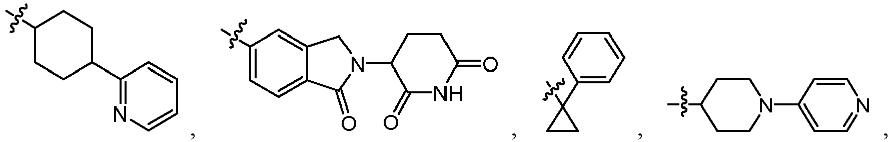 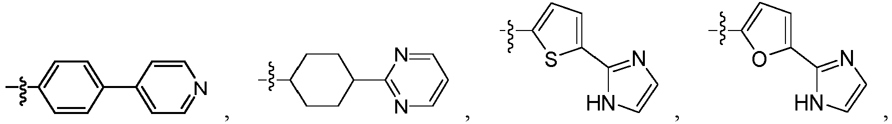 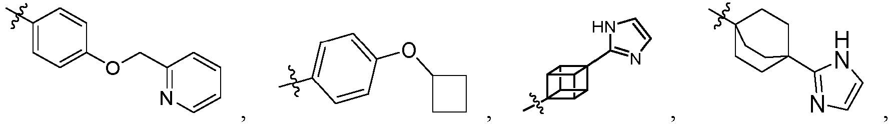 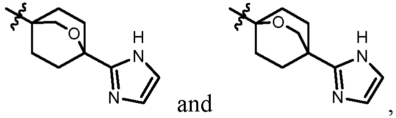 wherein ring A and ring B are each independently optionally substituted by one or more R₁, R₁ is as defined in formula (I');
more particularly, 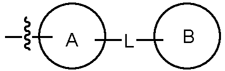 is selected from the group consisting of

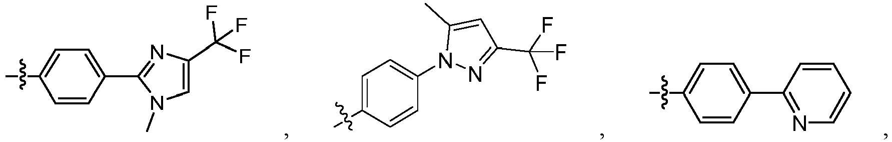

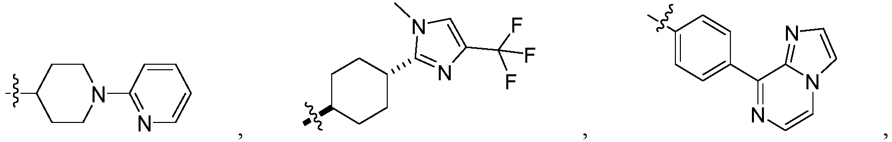

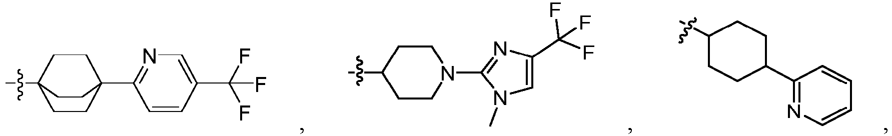

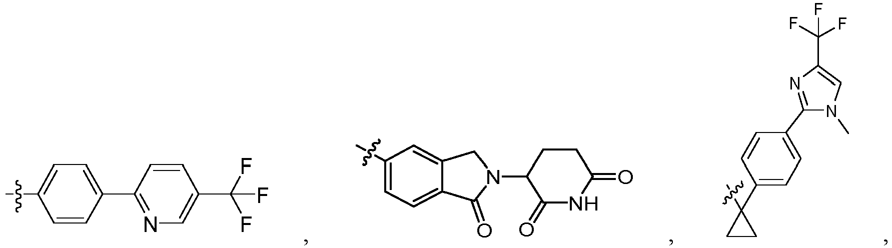

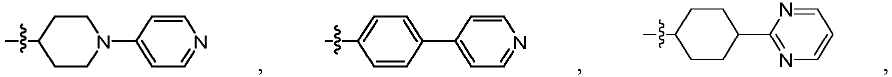

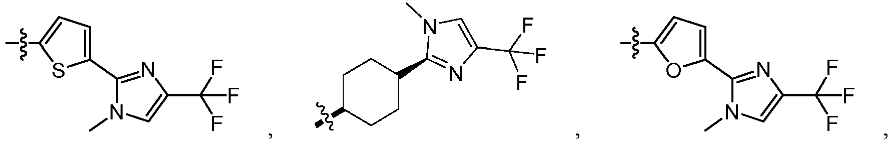

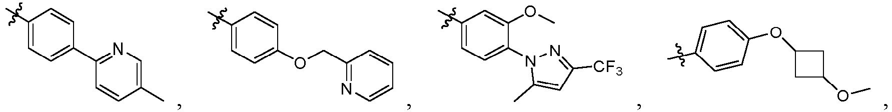

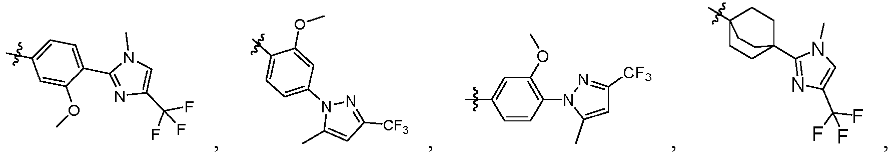

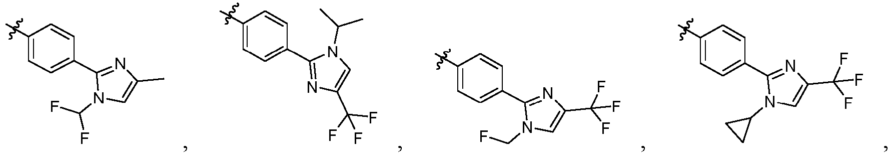

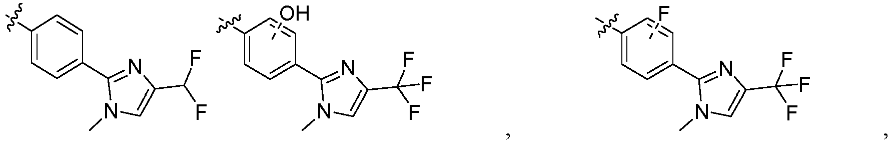

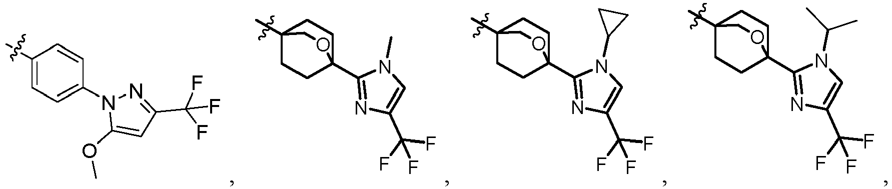

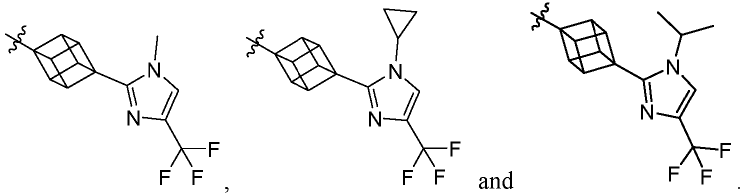

In some embodiments, in the compound of formula (I'),
R is a C₆₋₁₀ aryl or C₆₋₁₀ aryl fused with 5 to 6 membered heteroaryl, wherein the above groups are each independently optionally substituted by one or more substituent(s) selected from the group consisting of oxo, C₁₋₆ alkyl and -O-C₁₋₆ alkylene-O-C₁₋₆ alkyl;
particularly, R is 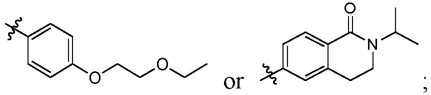
R₂ is selected from the group consisting of H atom, -OH, C₁₋₆ alkyl, C₂₋₆ alkynyl,-C₁₋₆ alkyl-C₆₋₁₀ aryl, C₁₋₆ alkoxy, C₁₋₆ haloalkyl, C₁₋₆ haloalkoxy and C₁₋₆ hydroxyalkyl; and preferably, R₂ is H atom;
R₃ is selected from H atom, -OH, -COOH, -NH₂, -CN, halogen, C₁₋₆ alkyl, C₂₋₆ alkynyl, C₁₋₆ alkoxy, C₁₋₆ haloalkyl, C₁₋₆ haloalkoxy and C₁₋₆ hydroxyalkyl; and preferably, R₃ is methoxy;
R₄ is selected from the group consisting of H atom, -OH, -COOH, -NH₂, -CN, halogen, C₁₋₆ alkyl, C₁₋₆ alkoxy, C₁₋₆ haloalkyl, C₁₋₆ haloalkoxy and C₁₋₆ hydroxyalkyl; and preferably, R₄ is H atom;
or, R₃ and R₄ together with the atom to which they are attached form ring D, ring D is a 5 to 7 membered heteroaryl or 5 to 7 membered heterocyclyl, and preferably furanyl.

In some embodiments, in the compound of formula (I'), (I), (II) or (III),
R₅ is selected from the group consisting of C₆₋₁₀ aryl, 5 to 6 membered heteroaryl, C₆₋₁₀ aryl fused with 5 to 6 membered heterocyclyl, and C₆₋₁₀ aryl fused with 5 to 6 membered heteroaryl, and preferably selected from the group consisting of phenyl, pyridyl, pyrimidinyl, pyrazolyl, imidazolyl, thiazolyl, indolyl, indolinyl and isoxazolyl, wherein the C₆₋₁₀ aryl, 5 to 6 membered heteroaryl, C₆₋₁₀ aryl fused with 5 to 6 membered heterocyclyl, and C₆₋₁₀ aryl fused with 5 to 6 membered heteroaryl are each independently optionally substituted by one or more substituent(s) selected from the group consisting of -OH, -COOH, -NH₂, -CN, halogen, C₁₋₆ alkyl, C₁₋₆ haloalkyl, C₁₋₆ alkoxy, C₁₋₆ haloalkoxy, C₁₋₆ hydroxyalkyl and C₃₋₆ cycloalkyl;
particularly, R₅ is selected from the group consisting of

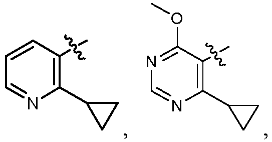

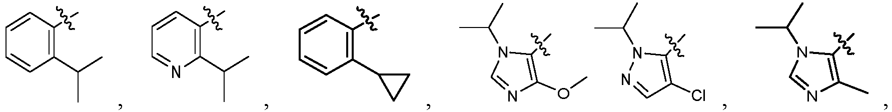

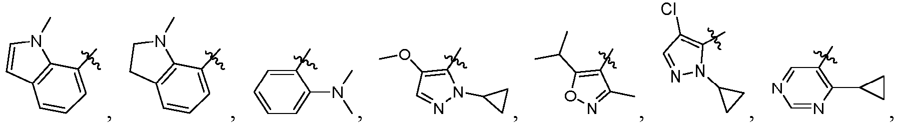

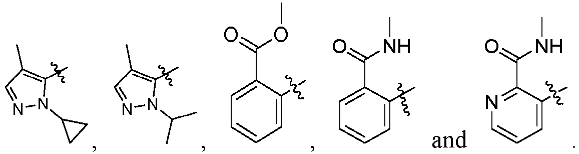

In some embodiments, in the compound of formula (I'), (I), (II) or (III), n is 0 or 1.

In some embodiments, in the compound of formula (I'), (I), (II) or (III), Rₐ and R_{b} are each independently selected from the group consisting of H atom, -CN, C₁₋₆ alkyl,-OH and halogen;
preferably, Rₐ and R_{b} are each independently H atom or -CN.

Typical compounds of formula (I) or (I') of the present invention include, but are not limited to the following compounds:

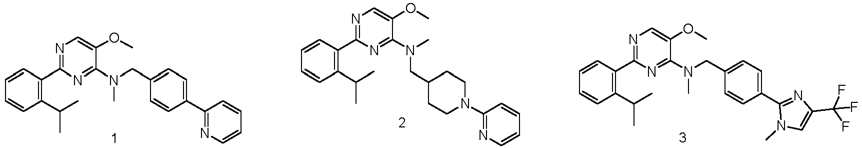

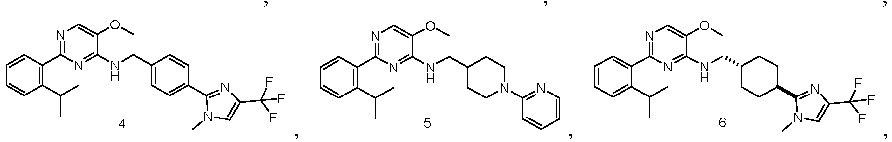

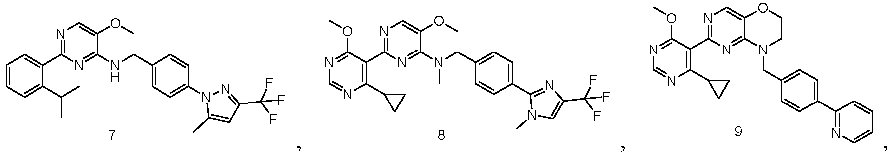

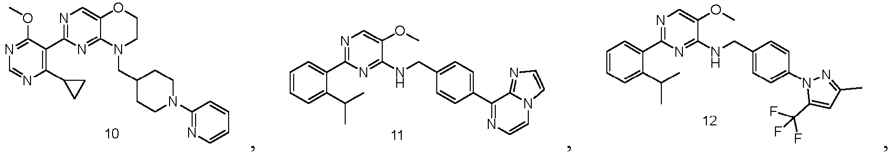

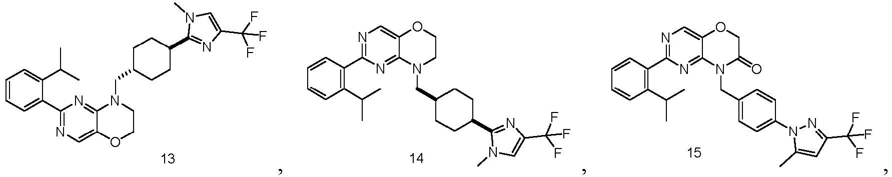

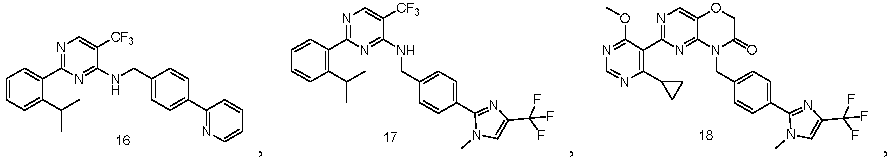

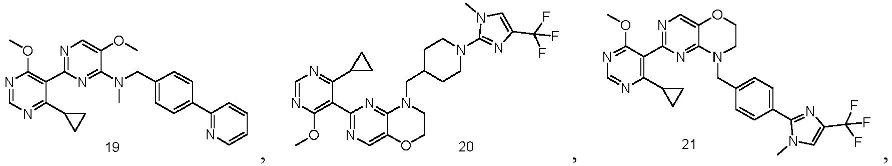

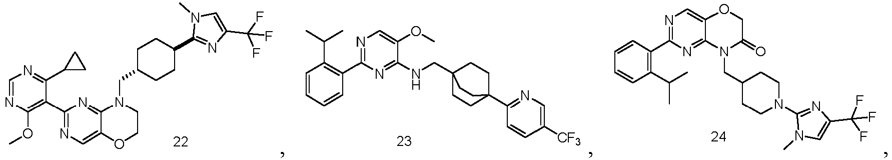

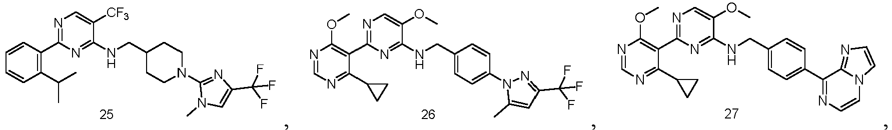

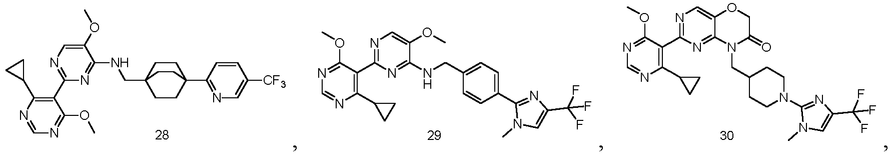

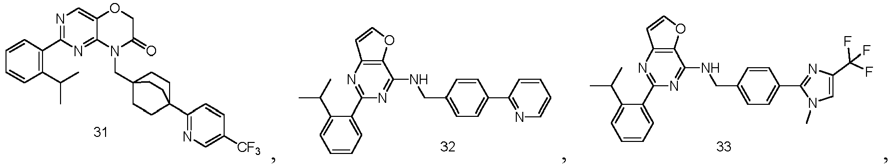

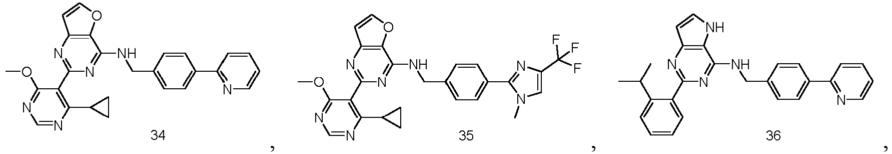

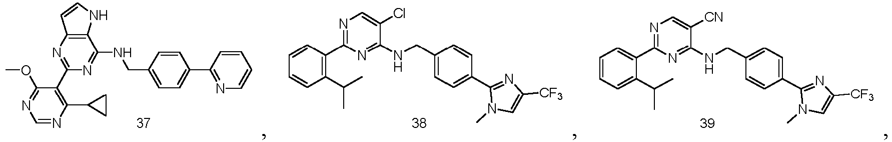

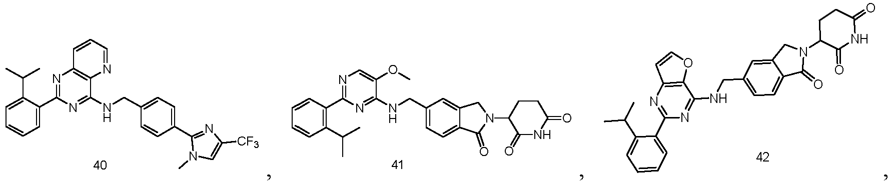

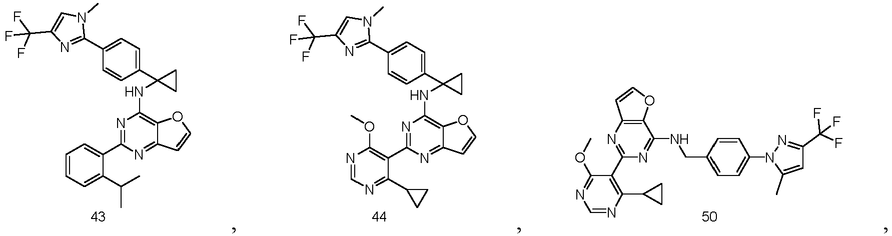

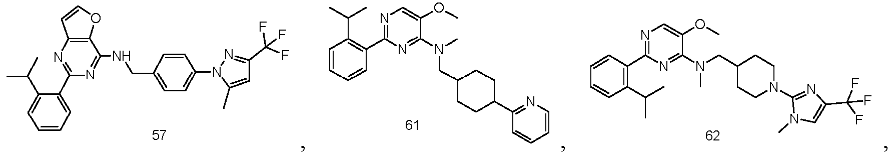

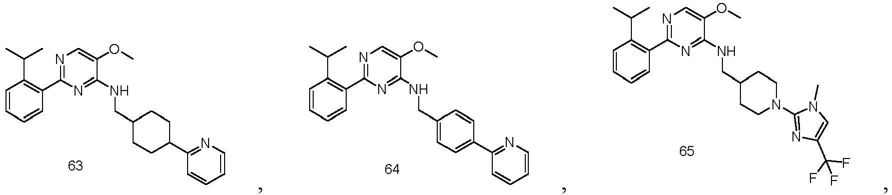

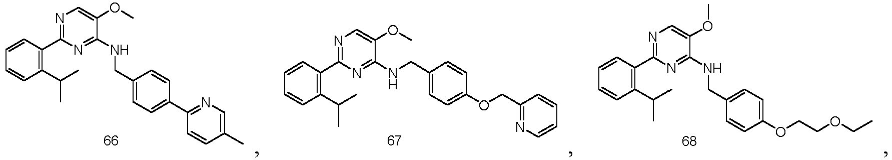

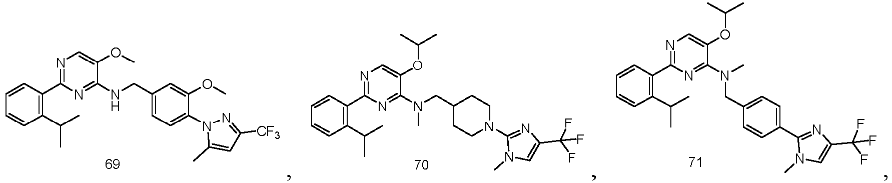

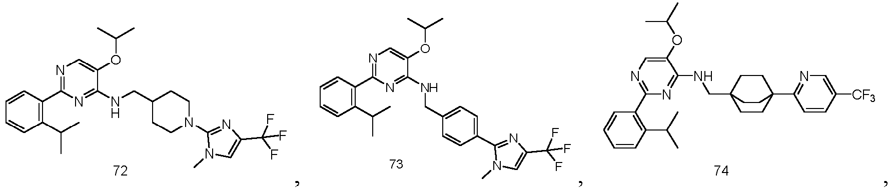

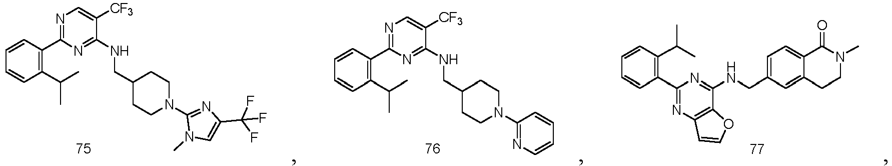

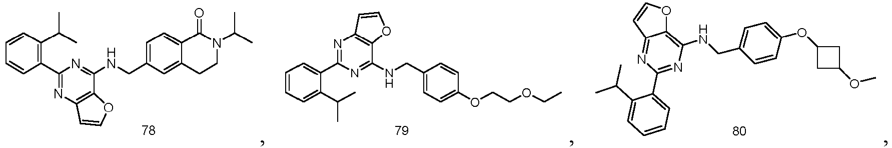

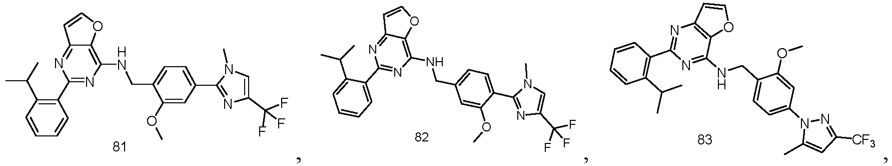

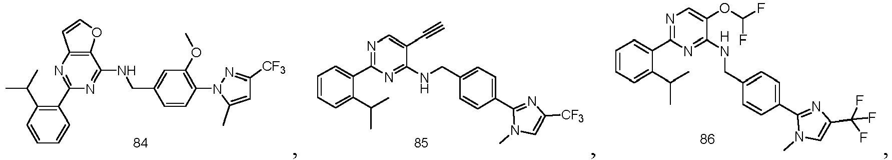

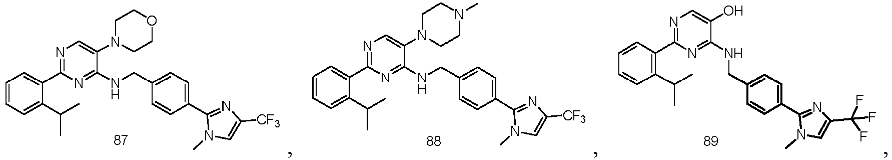

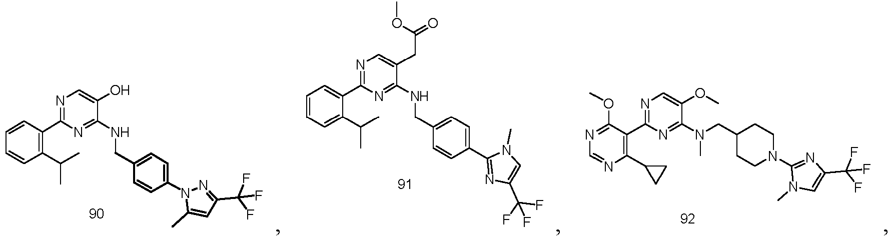

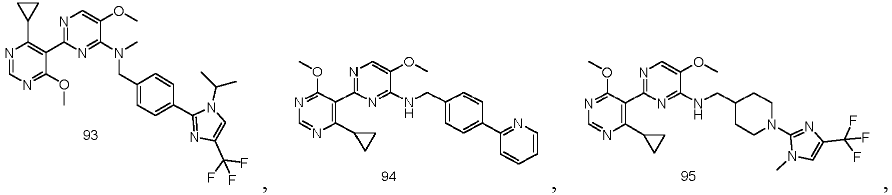

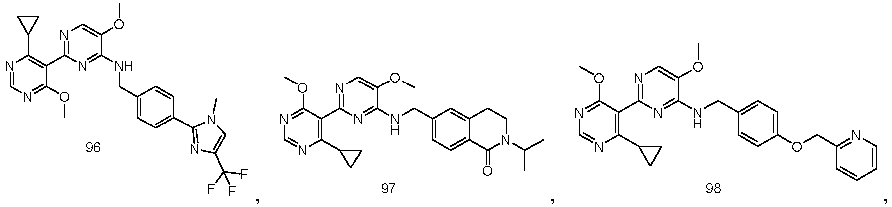

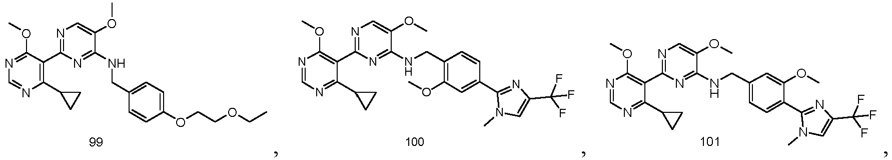

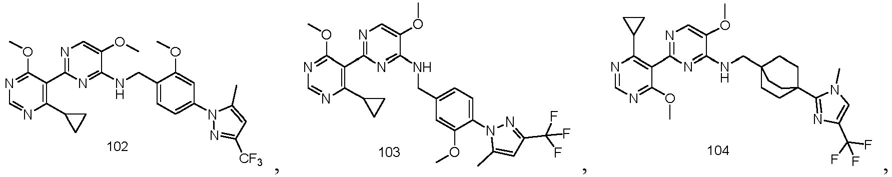

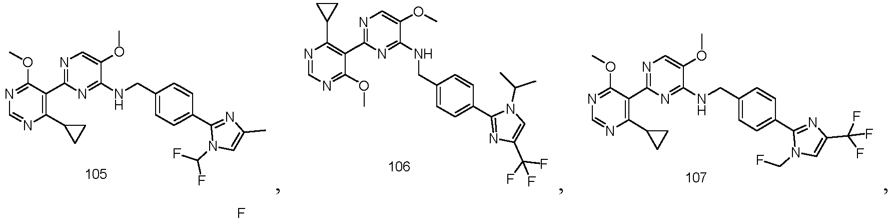

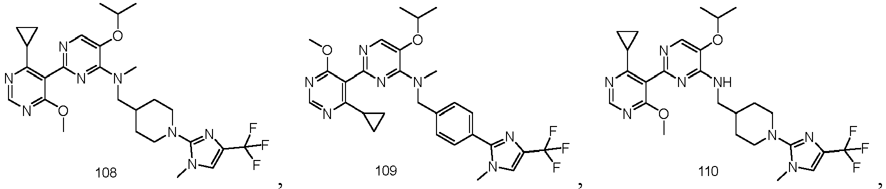

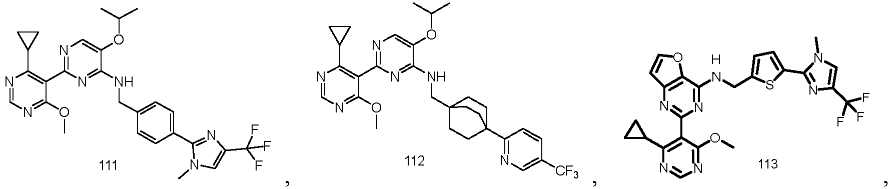

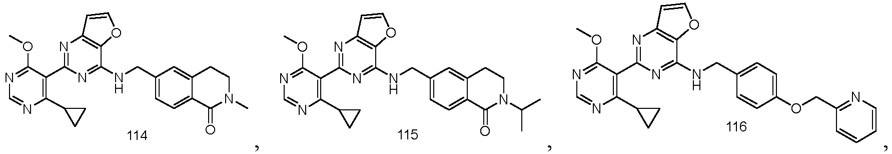

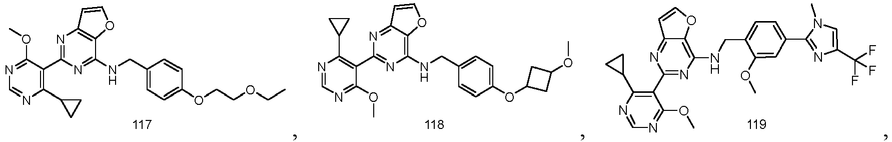

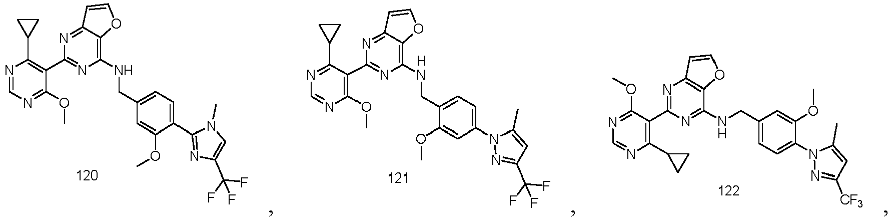

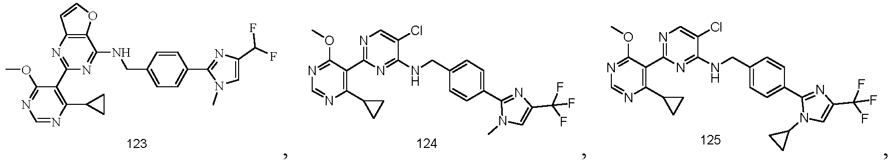

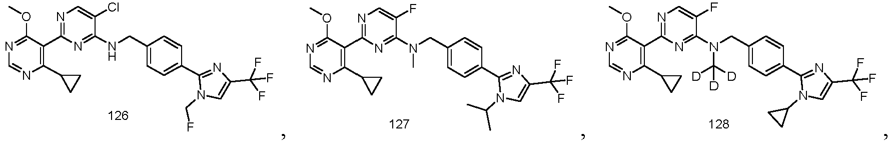

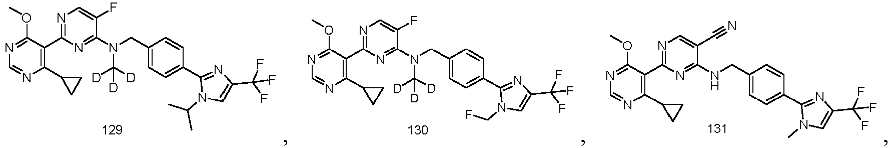

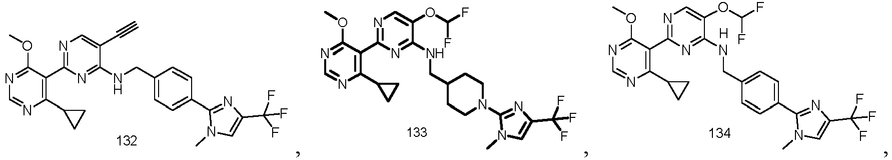

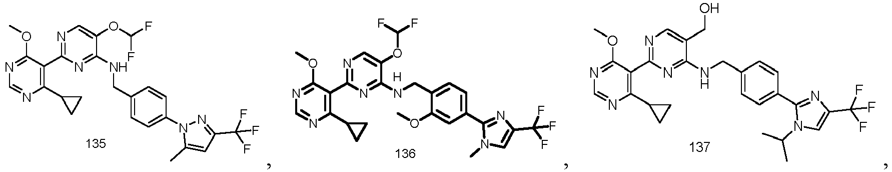

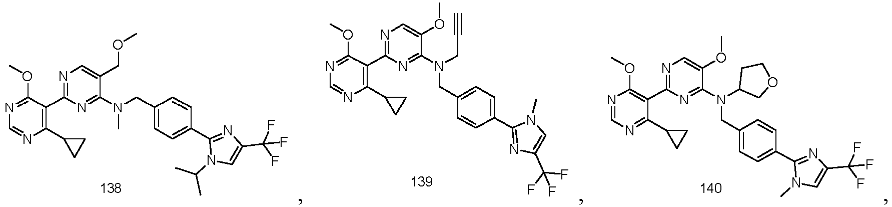

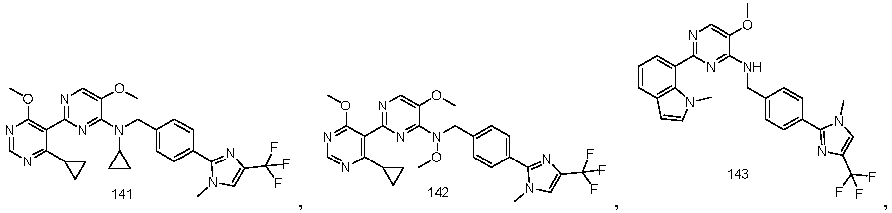

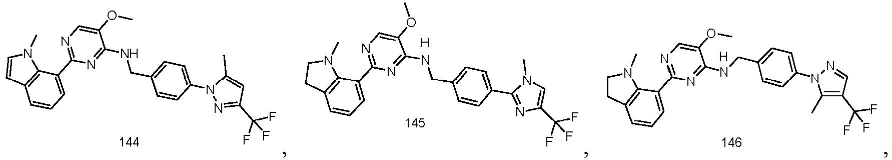

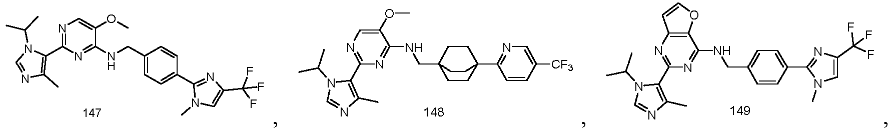

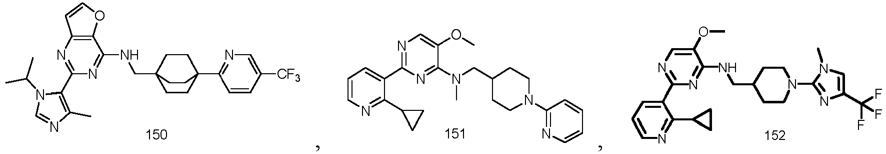

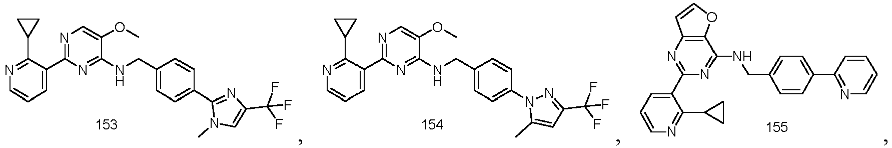

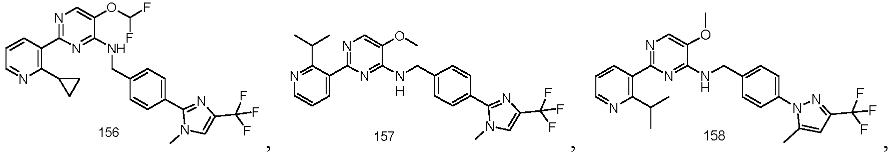

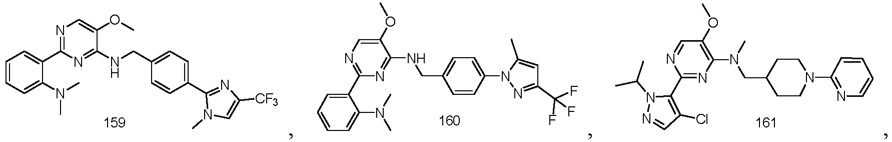

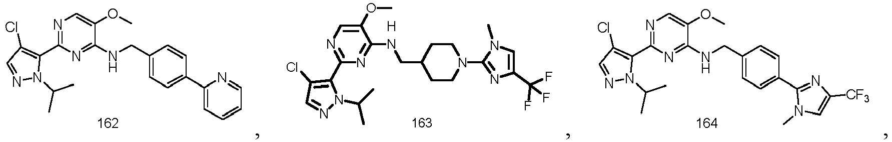

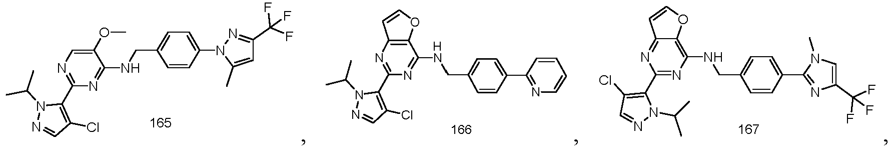

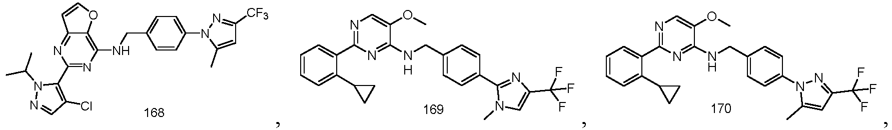

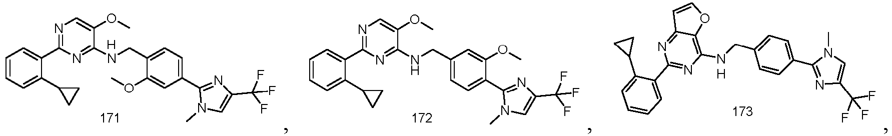

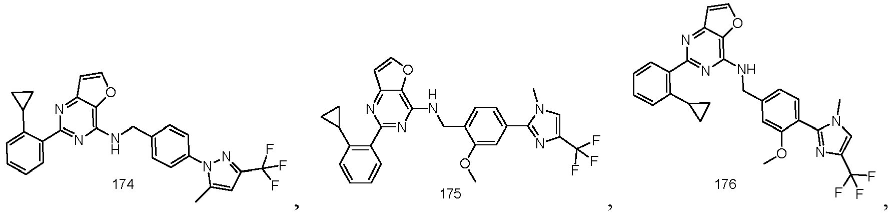

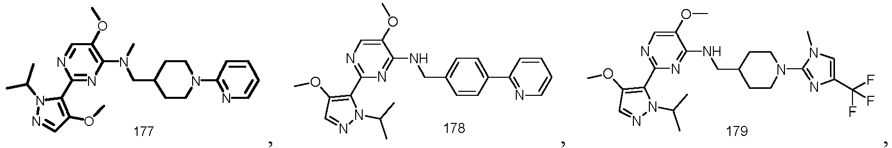

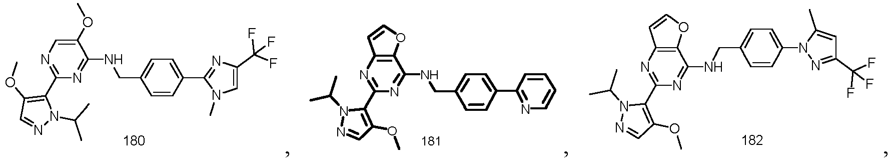

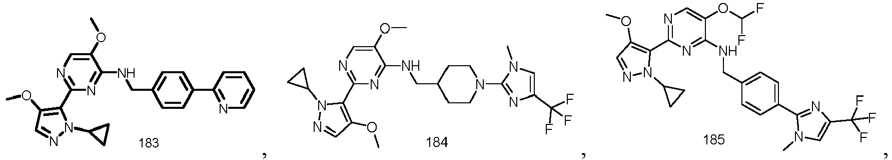

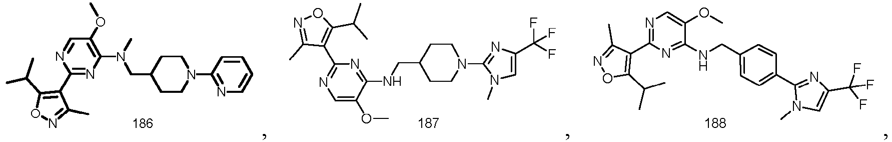

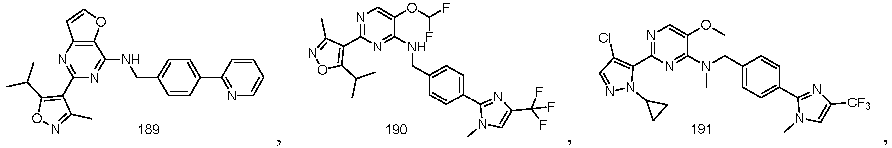

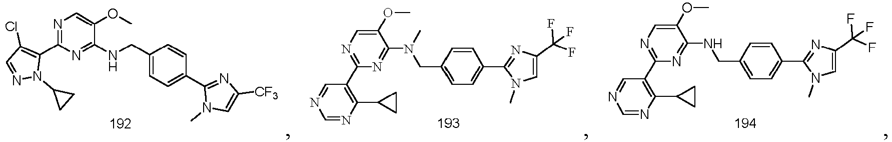

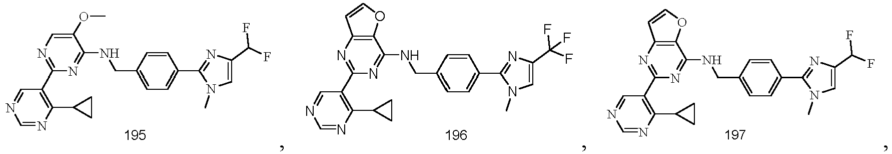

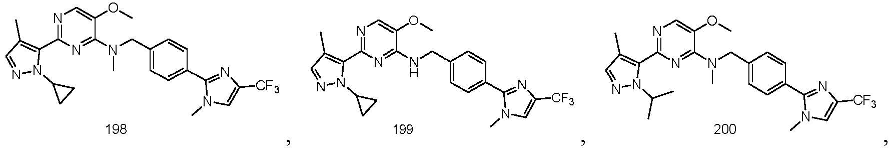

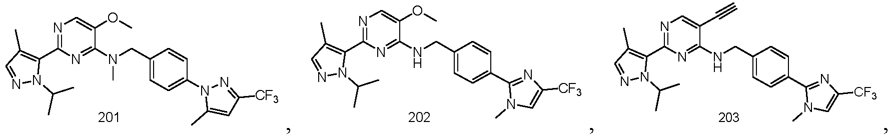

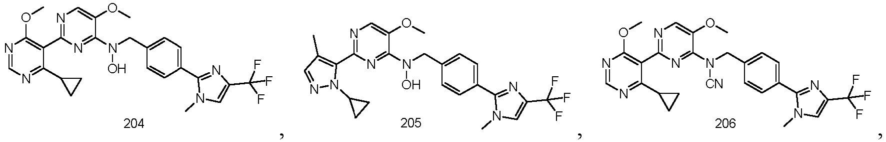

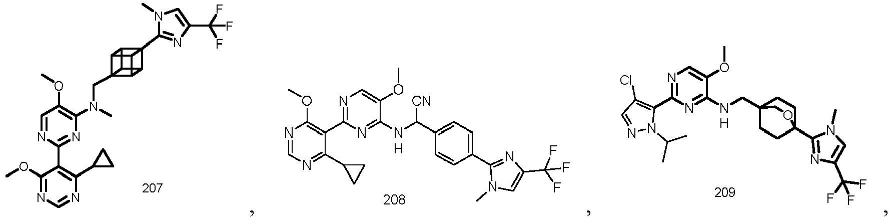

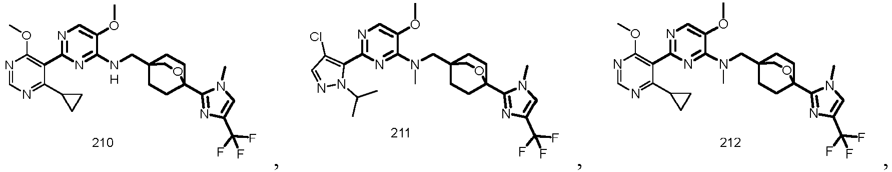

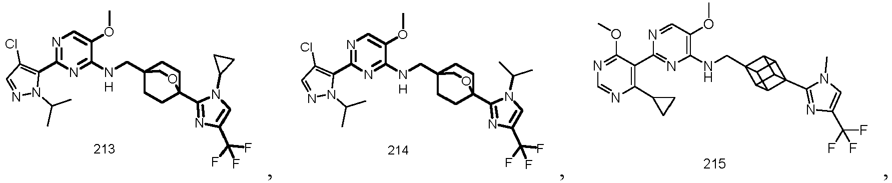

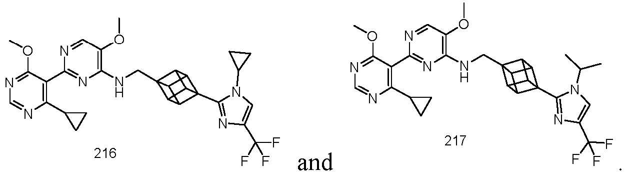

The present invention also provides a method for preparing the compound of formula (I'), comprising the following step of: 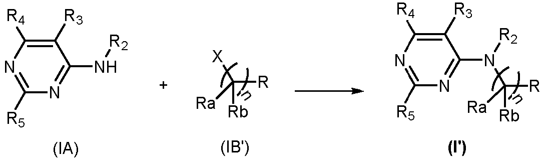
reacting a compound of formula (IA) with a compound of formula (IB') to obtain the compound of formula (I');
X is a leaving group selected from the group consisting of halogen, sulfonate, boronic acid and borate; and
R, Rₐ, R_{b}, R₂ to R₅ and n are as defined in formula (I');
   or 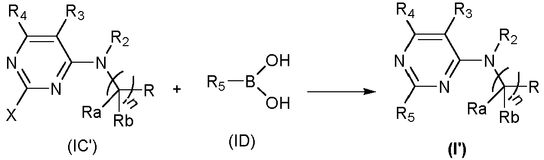
reacting a compound of formula (IC') with a compound of formula (ID) to obtain the compound of formula (I');
X is a leaving group selected from the group consisting of halogen, sulfonate, boronic acid and borate; and
R, Rₐ, R_{b}, R₂ to R₅ and n are as defined in formula (I');
   or 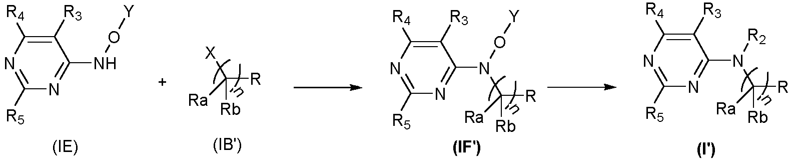
reacting a compound of formula (IE) with a compound of formula (IB') to obtain a compound of formula (IF'), and deprotecting the compound of formula (IF') to obtain the compound of formula (I');
X is a leaving group selected from the group consisting of halogen, sulfonate, boronic acid and borate;
Y is a hydroxy protecting group selected from the group consisting of *tert-*butyldimethylsilyl, trimethylsilyl, triethylsilyl, triisopropylsilyl, tert-butyldiphenylsilyl, benzyl, methoxymethyl and ethoxyethyl;
R₂ is hydroxy;
R, Rₐ, R_{b}, R₃ to R₅ and n are as defined in formula (I');
   or 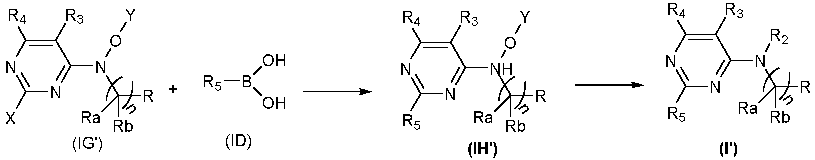
reacting a compound of formula (IG') with a compound of formula (ID) to obtain a compound of formula (IH'), and deprotecting the compound of formula (IH') to obtain the compound of formula (I');
X is a leaving group selected from the group consisting of halogen, sulfonate, boronic acid and borate;
Y is a hydroxy protecting group selected from the group consisting of *tert-*butyldimethylsilyl, trimethylsilyl, triethylsilyl, triisopropylsilyl, tert-butyldiphenylsilyl, benzyl, methoxymethyl and ethoxyethyl;
R₂ is hydroxy;
R, Rₐ, R_{b}, R₃ to R₅ and n are as defined in formula (I');
   or 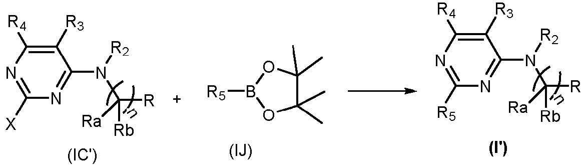
reacting a compound of formula (IC') with a compound of formula (IJ) to obtain the compound of formula (I');
X is a leaving group selected from the group consisting of halogen, sulfonate, boronic acid and borate; and
R, Rₐ, R_{b}, R₂ to R₅ and n are as defined in formula (I').

The present invention also provides a method for preparing the compound of formula (I), comprising the following step of: 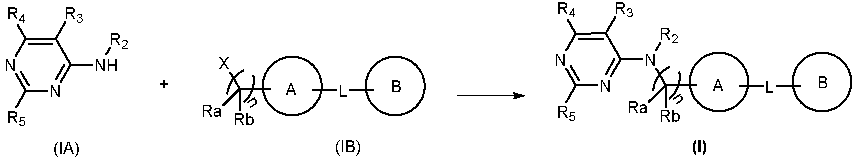
reacting a compound of formula (IA) with a compound of formula (IB) to obtain the compound of formula (I);
X is a leaving group selected from the group consisting of halogen, sulfonate, boronic acid and borate; and
ring A, ring B, L, Rₐ, R_{b}, R₂ to R₅ and n are as defined in formula (I');
   or 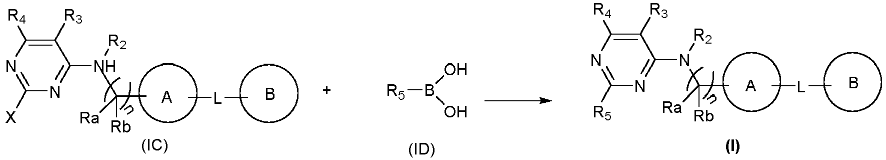
reacting a compound of formula (IC) with a compound of formula (ID) to obtain the compound of formula (I);
X is a leaving group selected from the group consisting of halogen, sulfonate, boronic acid and borate; and
ring A, ring B, L, Rₐ, R_{b}, R₂ to R₅ and n are as defined in formula (I');
   or 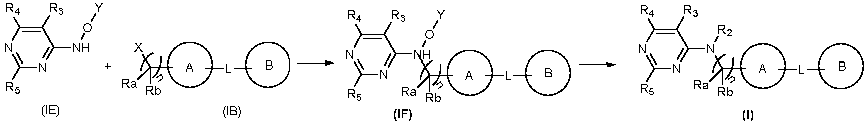
reacting a compound of formula (IE) with a compound of formula (IB) to obtain a compound of formula (IF), and deprotecting the compound of formula (IF) to obtain the compound of formula (I);
X is a leaving group selected from the group consisting of halogen, sulfonate, boronic acid and borate;
Y is a hydroxy protecting group selected from the group consisting of *tert-*butyldimethylsilyl, trimethylsilyl, triethylsilyl, triisopropylsilyl, tert-butyldiphenylsilyl, benzyl, methoxymethyl and ethoxyethyl;
R₂ is a hydroxy;
ring A, ring B, L, Rₐ, R_{b}, R₃ to R₅ and n are as defined in formula (I'); 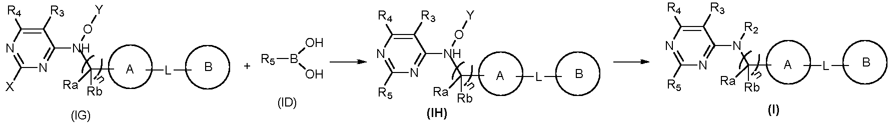
reacting a compound of formula (IG) with a compound of formula (ID) to obtain a compound of formula (IH), and deprotecting the compound of formula (IH) to obtain the compound of formula (I);
X is a leaving group selected from the group consisting of halogen, sulfonate, boronic acid and borate;
Y is a hydroxy protecting group selected from the group consisting of *tert-*butyldimethylsilyl, trimethylsilyl, triethylsilyl, triisopropylsilyl, *tert*-butyldiphenylsilyl, benzyl, methoxymethyl and ethoxyethyl;
R₂ is a hydroxy;
ring A, ring B, L, Rₐ, R_{b}, R₃ to R₅ and n are as defined in formula (I');
   or 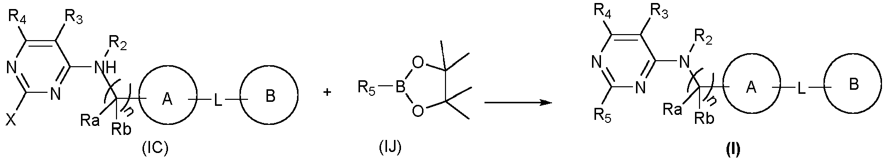
reacting a compound of formula (IC) with a compound of formula (IJ) to obtain the compound of formula (I);
X is a leaving group selected from the group consisting of halogen, sulfonate, boronic acid and borate; and
ring A, ring B, L, Rₐ, R_{b}, R₂ to R₅ and n are as defined in formula (I').

The present invention also provides a pharmaceutical composition comprising at least one compound of formula (I'), (I), (II) or (III), and one or more pharmaceutically acceptable excipient(s).

The present invention relates to a use of the compound of formula (I'), (I), (II) or (III) or the pharmaceutical composition comprising the same in the preparation of a medicament for treating or preventing diseases or conditions associated with inhibition of ubiquitin-specific protease 1 (USP1).

The present invention relates to a use of the compound of formula (I'), (I), (II) or (III) or the pharmaceutical composition comprising the same in the preparation of a medicament for treating or preventing cancer, particularly, the cancer is selected from the group consisting of lung cancer, non-small cell lung cancer (NSCLC), colon cancer, bladder cancer, osteosarcoma, ovarian cancer, skin cancer, and breast cancer.

The present invention relates to the compound of formula (I'), (I), (II) or (III) or the pharmaceutical composition comprising the same, for use as a medicament.

The present invention relates to the compound of formula (I'), (I), (II) or (III) or the pharmaceutical composition comprising the same, for use in treating or preventing diseases or conditions associated with inhibition of ubiquitin-specific protease 1 (USP1).

The present invention relates to the compound of formula (I'), (I), (II) or (III) or the pharmaceutical composition comprising the same, for use in treating or preventing cancer, particularly, the cancer is selected from the group consisting of lung cancer, non-small cell lung cancer (NSCLC), colon cancer, bladder cancer, osteosarcoma, ovarian cancer, skin cancer, and breast cancer.

The present invention relates to a method for treating or preventing diseases or conditions associated with inhibition of ubiquitin-specific protease 1 (USP1), comprising a step of administering a therapeutically effective amount of the compound of formula (I'), (I), (II) or (III) or the pharmaceutical composition comprising the same to a patient in need thereof.

The present invention relates to a method for treating or preventing cancer, comprising a step of administering a therapeutically effective amount of the compound of formula (I'), (I), (II) or (III) or the pharmaceutical composition comprising the same to a patient in need thereof, particularly, the cancer is selected from the group consisting of lung cancer, non-small cell lung cancer (NSCLC), colon cancer, bladder cancer, osteosarcoma, ovarian cancer, skin cancer, and breast cancer.

The pharmaceutical composition of the present invention can be various conventional dosage forms, for example, tablet, aqueous suspension, oily suspension, dispersible powder, dispersible granule, emulsion, hard capsule, soft capsule, sterile aqueous solution for injection, sterile oil-in-water microemulsion for injection, or suppository. Each of the above dosage forms can be prepared by conventional preparation methods.

It is well known to those skilled in the art that the dosage of a drug depends on a variety of factors including, but not limited to the following factors: activity of a specific compound used, age of the patient, weight of the patient, general health condition of the patient, behavior of the patient, diet of the patient, administration time, administration route, excretion rate, drug combination and the like. In addition, the optimal treatment, such as treatment mode, daily dose of the compound or the type of pharmaceutically acceptable salt thereof can be verified according to the traditional therapeutic regimens.

### Definition of terms

Unless otherwise specified, the following terms and phrases used herein are intended to have the following meanings. A particular term or phrase should not be considered ambiguous or unclear without special definition, but should be understood according to the ordinary meaning.

The term "pharmaceutically acceptable" refers to those compounds, materials, compositions and/or dosage forms which are, within the scope of sound medical judgment, suitable for use in contact with the tissues of human beings and animals without excessive toxicity, irritation, allergic response or other problems or complications, commensurate with a reasonable benefit/risk ratio.

The term "pharmaceutically acceptable salt" refers to salts of compounds of the present invention, prepared from compounds of the present invention having specific substituents and relatively nontoxic acids or bases. When the compounds of the present invention contain relatively acidic functional groups, base addition salts can be obtained by contacting such compounds with a sufficient amount of base in a pure solution or a suitable inert solvent. Pharmaceutically acceptable base addition salts include sodium, potassium, calcium, ammonium, organic amine or magnesium salts or similar salts. When the compounds of the present invention contain relatively basic functional groups, acid addition salts can be obtained by contacting such compounds with a sufficient amount of acid in a pure solution or a suitable inert solvent. Examples of pharmaceutically acceptable acid addition salts include inorganic acid salts, the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, etc.; and organic acid salts, the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, *p*-toluenesulfonic acid, citric acid, tartaric acid and methanesulfonic acid, etc.; also include salts of amino acids (such as arginine, etc.), and salts of organic acids such as glucuronic acid. Certain specific compounds of the present invention contain basic and acidic functional groups, and can be converted into any base or acid addition salt.

The term "isomer" refers to compounds having the same composition and molecular weight, but different physical and/or chemical properties. The structural differences may lie in the configuration (geometric isomers) or the ability to rotate the plane of polarized light (stereoisomers). With respect to stereoisomers, the compounds of formula (I) may have one or more asymmetric carbon atoms and may exist as racemates, racemic mixtures and as individual enantiomers or diastereomers.

The term "optional" or "optionally" means that the event or circumstance described subsequently can, but need not, occur, and such a description includes the situation in which the event or circumstance occurs or the situation in which the event or circumstance does not occur.

The term "solvate" refers to a complex of variable stoichiometry formed by a solute and a solvent. Such solvents for the purposes of the present application may not interfere with the biological activity of the solute. Examples of suitable solvents include, but are not limited to, water, MeOH, EtOH, and AcOH. Solvates in which water is a solvent molecule are commonly referred to as hydrates. Hydrates include compositions containing stoichiometric amounts of water, as well as compositions containing variable amounts of water.

As used herein, the term "disease" or "disorder" or "condition" refers to a condition for which treatment is needed and/or desired and represents a disorder and/or abnormality generally regarded as a pathological condition or function, and which may manifest in the form of specific signs, symptoms and/or dysfunction. The compound of the present invention inhibits USP1 protein and is useful in treating diseases and conditions such as proliferative diseases, where inhibition of USP1 protein would provide benefit.

"USP1" and "ubiquitin-specific processing protease 1" refer to any native polypeptide or polynucleotide encoding USP1. The term "USP1" encompasses "full-length" unprocessed USP1 polypeptides as well as any form of USP1 resulting from processing within a cell (e.g., removal of a signal peptide). The term also encompasses naturally occurring variants of USP1, such as those encoded by splice variants and allelic variants. The USP1 polypeptides described herein can be isolated from a variety of sources, such as from a human tissue type or from another source, or prepared by recombinant or synthetic methods.

The terms "cancer" and "tumor" refer to or describe a physiological condition in which a population of cells in a mammal is characterized by unregulated cell growth. The terms encompass solid cancers and hematologic/lymphoid cancers. Examples of cancer include, but are not limited to, cancers with defects in DNA damage repair pathways. Other examples of cancer include, but are not limited to, lung cancer, non-small cell lung cancer (NSCLC), colon cancer, bladder cancer, osteosarcoma, ovarian cancer, skin cancer, and breast cancer (including triple-negative breast cancer). The cancer can be BRCA1 or BRCA2 wild-type. The cancer can also be BRCA1 or BRCA2 mutant. The cancer can also be a PARP inhibitor-resistant or refractory cancer, or a PARP inhibitor-resistant or refractory BRCA1 or BRCA2 mutant cancer.

Where any variable (such as R) appears more than once in the structure of a compound, its definition in each case is independent. For example, if a group is substituted by 0 to 2 R, the group can optionally be substituted by up to two R, and R in each case has independent options.

The term "alkyl" refers to a saturated linear or branched monovalent hydrocarbon group having 1 to 20 (for example, 1, 2, 3, 4, 5, 6, 7, 8, 9, 10, 11, 12, 13, 14, 15, 16, 17, 18, 19 and 20) carbon atoms. The alkyl is preferably a C₁₋₁₀ alkyl, and more preferably C₁₋₆ alkyl. Examples of alkyl include, but are not limited to, methyl, ethyl, *n*-propyl, isopropyl, *n*-butyl, isobutyl, *tert-butyl, sec*-butyl, *n*-pentyl, 2,2-dimethylpropyl, 2-methylbutyl, *n*-hexyl, 2,2-dimethylbutyl, 2-methylpentyl, 3-methylpentyl, 2,3-dimethylbutyl, *n*-heptyl, 2-methylhexyl, 3-methylhexyl, 2,3-dimethylpentyl, 2,4-dimethylpentyl, 2,2-dimethylpentyl, 3,3-dimethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 3-ethylhexyl, 4-ethylhexyl, 2-methyl-3-ethylpentyl, n-nonyl, 2-methyl-3-ethylhexyl, n-decyl and 3,3-diethylhexyl.

The term "alkenyl" refers to a linear or branched monovalent hydrocarbon group having 2 to 6 (for example 2, 3, 4, 5 and 6) carbon atoms and at least one carbon-carbon double bond, wherein the carbon-carbon double bond may be located anywhere within the alkenyl. The alkenyl is preferably a C₂₋₅ alkenyl. Examples of alkenyl include, but are not limited to, -CH=CH₂, -CH=CH-CH₃, -CH₂-CH=CH₂, -CH=CH-CH₂-CH₃, -CH₂-CH=CH-CH₃, -CH=CH-CH=CH₂, -CH=C(CH₃)-CH₃ and -CH₂-C(CH₃)=CH₂.

The term "alkynyl" refers to a linear or branched monovalent hydrocarbon group having 2 to 6 (for example 2, 3, 4, 5 and 6) carbon atoms and at least one carbon-carbon triple bond, wherein the carbon-carbon triple bond may be located anywhere within the alkynyl. The alkynyl is preferably a C₂₋₅ alkynyl. Examples of alkynyl include, but are not limited to, -C≡CH, -C≡C-CH₃, -CH₂-C≡CH, -C≡C-CH₂-CH₃, -CH₂-CH₂-C≡CH,-CH(CH₃)C≡CH and -CH₂-C≡C-CH₃.

The term "cycloalkyl" includes two categories, one is conventional cycloalkyl, and the other is heterostructured cycloalkyl.

The conventional cycloalkyl refers to an aliphatic saturated or partially unsaturated monovalent cyclic hydrocarbon group having 3 to 20 (for example, 3, 4, 5, 6, 7, 8, 9, 10, 11, 12, 13, 14, 15, 16, 17, 18, 19 and 20) carbon atoms. The conventional cycloalkyl is preferably a C₃₋₁₂ conventional cycloalkyl, more preferably a C₃₋₁₀ conventional cycloalkyl, further preferably a C₃₋₈ conventional cycloalkyl, and most preferably a C₃₋₆ conventional cycloalkyl. The conventional cycloalkyl optionally comprises one or more double or triple bonds.

The conventional cycloalkyl can be a monocyclic cycloalkyl. Examples of monocyclic cycloalkyl include, but are not limited to, cyclopropyl, cyclobutyl, cyclopentyl, cyclopentenyl, cyclohexyl, cyclohexenyl, cyclohexadienyl, cycloheptyl, cycloheptatrienyl and cyclooctyl. The conventional cycloalkyl can also be a polycyclic cycloalkyl (for example, bicycloalkyl, tricycloalkyl, tetracycloalkyl and pentacycloalkyl). Polycyclic cycloalkyl includes spiro cycloalkyl, fused cycloalkyl and bridged cycloalkyl.

The term "spiro cycloalkyl" refers to a 5 to 20 membered (for example, 5, 6, 7, 8, 9, 10, 11, 12, 13, 14, 15, 16, 17, 18, 19 and 20 membered) spiro cycloalkyl. The spiro cycloalkyl is preferably a 6 to 14 membered spiro cycloalkyl, and more preferably a 7 to 10 membered spiro cycloalkyl. The spiro cycloalkyl can be a mono-spiro cycloalkyl, a di-spiro cycloalkyl, or a poly-spiro cycloalkyl, and the spiro cycloalkyl is preferably a mono-spiro cycloalkyl, and more preferably a 4-membered/4-membered, 4-membered/5-membered, 4-membered/6-membered, 5-membered/5-membered, or 5-membered/6-membered mono-spiro cycloalkyl. Examples of spiro cycloalkyl include, but are not limited to:

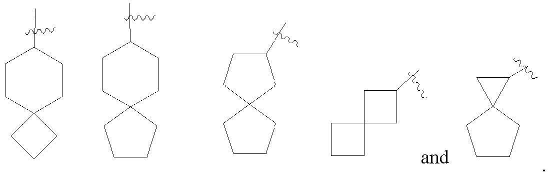

The term "fused cycloalkyl" refers to a 5 to 20 membered (for example, 5, 6, 7, 8, 9, 10, 11, 12, 13, 14, 15, 16, 17, 18, 19 and 20 membered) fused cycloalkyl. The fused cycloalkyl is preferably a 6 to 14 membered fused cycloalkyl, and more preferably a 7 to 10 membered fused cycloalkyl. The fused cycloalkyl can be a bicyclic, tricyclic, tetracyclic, pentacyclic or more fused cycloalkyl, and the fused cycloalkyl is preferably a bicyclic or tricyclic fused cycloalkyl, and more preferably a 5-membered/5-membered, or 5-membered/6-membered fused cycloalkyl. Examples of fused cycloalkyl include, but are not limited to:

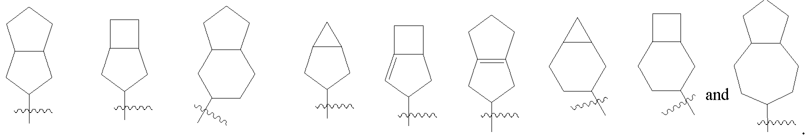

The term "bridged cycloalkyl" refers to a 5 to 20 membered (for example, 5, 6, 7, 8, 9, 10, 11, 12, 13, 14, 15, 16, 17, 18, 19 and 20 membered) bridged cycloalkyl. The bridged cycloalkyl is preferably a 6 to 14 membered bridged cycloalkyl, and more preferably a 7 to 10 membered bridged cycloalkyl. The bridged cycloalkyl can be a bicyclic, tricyclic, tetracyclic, pentacyclic or more bridged cycloalkyl, and the bridged cycloalkyl is preferably a bicyclic, tricyclic or tetracyclic bridged cycloalkyl, and more preferably a bicyclic or tricyclic bridged cycloalkyl. Examples of bridged cycloalkyl include, but are not limited to:

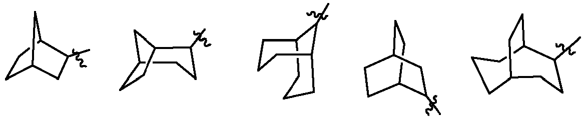

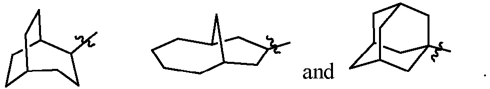

The term "heterostructured cycloalkyl" includes monocyclic cycloalkyl, spiro cycloalkyl, fused cycloalkyl and bridged cycloalkyl fused with any one selected from the group consisting of conventional aryl, conventional heteroaryl and conventional heterocyclyl, and the attachment point is located on the corresponding conventional cycloalkyl (referring to monocyclic cycloalkyl, spiro cycloalkyl, fused cycloalkyl or bridged cycloalkyl). Examples of heterostructured cycloalkyl include, but are not limited to:

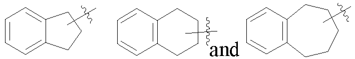

The term "heterocyclyl" includes two categories, one is conventional heterocyclyl, and the other is heterostructured heterocyclyl.

The conventional heterocyclyl refers to an aliphatic saturated or partially unsaturated monovalent cyclic hydrocarbon group having 3 to 20 (for example, 3, 4, 5, 6, 7, 8, 9, 10, 11, 12, 13, 14, 15, 16, 17, 18, 19 and 20) ring atoms, wherein one or more ring atoms are replaced by one or more elements selected from the group consisting of N, O, S, S(O) and S(O)₂, and the replacement does not form -O-O-, -O-S- or -S-S-. The conventional heterocyclyl is preferably a C₃₋₁₂ conventional heterocyclyl, wherein 1 to 4 (for example 1, 2, 3 and 4) atoms are heteroatoms; more preferably a C₃₋₈ conventional heterocyclyl, wherein 1 to 3 (for example 1, 2 and 3) atoms are heteroatoms; and most preferably a C₅₋₇ conventional heterocyclyl, wherein 1 to 2 or 1 to 3 atoms are heteroatoms.

The conventional heterocyclyl can be a monocyclic heterocyclyl. Examples of monocyclic heterocyclyl include, but are not limited to oxetanyl, 3-pyrrolinyl, pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl and pyranyl, and preferably 1,2,5-oxadiazolyl, pyranyl or morpholinyl. The conventional heterocyclyl can also be a polycyclic heterocyclyl. Polycyclic heterocyclyl includes spiro heterocyclyl, fused heterocyclyl and bridged heterocyclyl.

The term "spiro heterocyclyl" refers to a 5 to 20 membered (for example, 5, 6, 7, 8, 9, 10, 11, 12, 13, 14, 15, 16, 17, 18, 19 and 20 membered) spiro heterocyclyl. The spiro heterocyclyl is preferably a 6 to 14 membered spiro heterocyclyl, and more preferably a 7 to 10 membered spiro heterocyclyl. The spiro heterocyclyl can be a mono-spiro heterocyclyl, a di-spiro heterocyclyl, or a poly-spiro heterocyclyl, and the spiro heterocyclyl is preferably a mono-spiro heterocyclyl or a di-spiro heterocyclyl, and more preferably a 3-membered/6-membered, 4-membered/4-membered, 4-membered/5-membered, 4-membered/6-membered, 5-membered/5-membered, or 5-membered/6-membered mono-spiro heterocyclyl. Examples of spiro heterocyclyl include, but are not limited to:

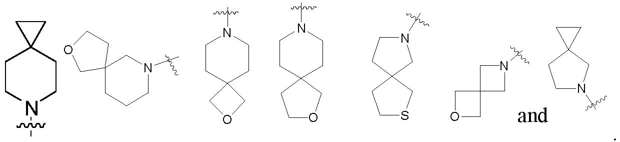

The term "fused heterocyclyl" refers to a 5 to 20 membered (for example, 5, 6, 7, 8, 9, 10, 11, 12, 13, 14, 15, 16, 17, 18, 19 and 20 membered) fused heterocyclyl. The fused heterocyclyl is preferably a 6 to 14 membered fused heterocyclyl, and more preferably a 7 to 10 membered fused heterocyclyl. The fused heterocyclyl can be a bicyclic, tricyclic, tetracyclic, pentacyclic or more fused heterocyclyl, and the fused heterocyclyl is preferably a bicyclic or tricyclic fused heterocyclyl, and more preferably a 5-membered/5-membered, or 5-membered/6-membered bicyclic fused heterocyclyl. Examples of fused heterocyclyl include, but are not limited to:

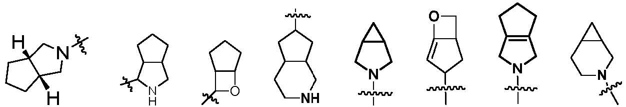

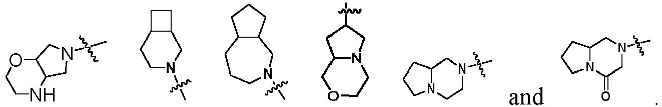

The term "bridged heterocyclyl" refers to a 5 to 14 membered (for example, 5, 6, 7, 8, 9, 10, 11, 12, 13 and 14 membered) bridged heterocyclyl. The bridged heterocyclyl is preferably a 6 to 14 membered bridged heterocyclyl, and more preferably a 7 to 10 membered bridged heterocyclyl. The bridged heterocyclyl can be a bicyclic, tricyclic, tetracyclic, pentacyclic or more bridged heterocyclyl, and the bridged heterocyclyl is preferably a bicyclic, tricyclic or tetracyclic bridged heterocyclyl, and more preferably a bicyclic or tricyclic bridged heterocyclyl. Examples of bridged heterocyclyl include, but are not limited to:

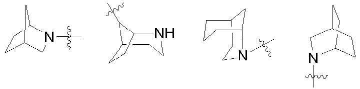

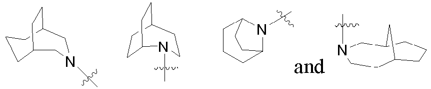

The term "heterostructured heterocyclyl" includes monocyclic heterocyclyl, spiro heterocyclyl, fused heterocyclyl and bridged heterocyclyl fused with any one selected from the group consisting of conventional aryl, conventional heteroaryl and conventional cycloalkyl, and the attachment point is located on the corresponding conventional heterocyclyl (referring to monocyclic heterocyclyl, spiro heterocyclyl, fused heterocyclyl or bridged heterocyclyl). Examples of heterostructured heterocyclyl include, but are not limited to:

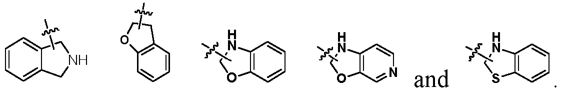

The term "aryl" includes two categories, one is conventional aryl, and the other is heterostructured aryl.

The conventional aryl refers to a 6 to 14 membered (for example, 6, 7, 8, 9, 10, 11, 12, 13 and 14 membered) aromatic hydrocarbon group. The conventional aryl is preferably a C₆₋₁₀ conventional aryl, and more preferably phenyl, naphthyl, phenanthryl or anthracenyl.

The term "heterostructured aryl" includes conventional aryl fused with any one selected from the group consisting of conventional heteroaryl, conventional heterocyclyl and conventional cycloalkyl, and the attachment point is located on the conventional aryl. Examples of heterostructured aryl include, but are not limited to:

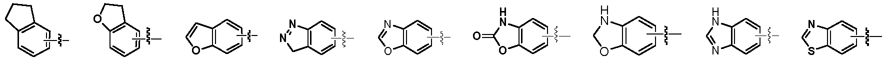

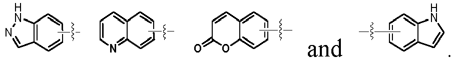

The term "heteroaryl" includes two categories, one is conventional heteroaryl, and the other is heterostructured heteroaryl.

The conventional heteroaryl refers to a 5 to 14 membered (for example, 5, 6, 7, 8, 9, 10, 11, 12, 13 and 14 membered) aromatic hydrocarbon group, wherein 1 to 4 (for example, 1, 2, 3 and 4) carbon atoms are replaced by heteroatoms, the heteroatom is selected from the group consisting of O, S and N. Preferably, the number of ring atoms is 5 to 10, including 1 to 3 (for example, 1, 2 and 3) heteroatoms. More preferably, the number of ring atoms is 5 or 6, including 1 to 2 heteroatoms. Examples of conventional heteroaryl include, but are not limited to imidazolyl, furanyl, thienyl, thiazolyl, pyrazolyl, oxazolyl, pyrrolyl, tetrazolyl, pyridyl, pyrimidinyl, thiadiazolyl and pyrazinyl, preferably imidazolyl, thiazolyl, pyrazolyl, pyrimidinyl or thiazolyl, and more preferably pyrazolyl or thiazolyl.

The term "heterostructured heteroaryl" includes conventional heteroaryl fused with any one selected from the group consisting of conventional aryl, conventional cycloalkyl and conventional heterocyclyl, and the attachment point is located on the conventional heteroaryl. Examples of heterostructured heteroaryl include, but are not limited to:

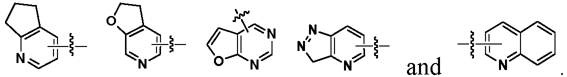

The term "alkoxy" includes -O-alkyl and -O-cycloalkyl, wherein the "alkyl" and "cycloalkyl" are as defined above. Examples of alkoxy include, but are not limited to: methoxy, ethoxy, propoxy, butoxy, cyclopropyloxy, cyclobutyloxy, cyclopentyloxy and cyclohexyloxy.

The term "haloalkyl" refers to an alkyl substituted by one or more halogen(s), wherein the alkyl is as defined above.

The term "haloalkoxy" refers to an alkoxy substituted by one or more halogen(s), wherein the alkoxy is as defined above.

The term "hydroxy" refers to an -OH group.

The term "halogen" refers to a -F, -Cl, -Br or -I group.

The term "amino" refers to a -NH₂ group.

The term "cyano" refers to a -CN group.

The term "nitro" refers to a -NO₂ group.

The term "oxo" refers to a =O group.

The term "carboxy" refers to a -C(=O)OH group.

The term "thiol" refers to a -SH group.

The term "alkoxycarbonyl" refers to a -C(=O)O-alkyl or a -C(=O)O-cycloalkyl, wherein the alkyl and cycloalkyl are as defined above.

The term "acyl" refers to a -C(=O)R group, where R is selected from the group consisting of alkyl, cycloalkyl, heterocyclyl, aryl and heteroaryl.

The term "hydroxy protecting group" refers to a group that is introduced on a hydroxy and is easily removed, and is used to block or protect the hydroxy while reacting on other functional groups of the compound. Non-limiting examples include: trimethylsilyl (TMS), triethylsilyl (TES), triisopropylsilyl (TIPS), *tert*-butyldimethylsilyl (TBS), *tert*-butyldiphenylsilyl (TBDPS), methyl, *tert*-butyl, allyl, benzyl, methoxymethyl (MOM), ethoxyethyl, 2-tetrahydropyranyl (THP), formyl, acetyl, benzoyl,*p*-nitrobenzoyl, etc.

The symbol 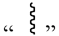 refers to the attachment point.

The term "stereoisomer" refers to isomers with the same structure but different arrangements of atoms in space. It includes cis and trans (or *Z* and *E*) isomers, (-)- and (+)-isomers, (*R*)- and (*S*)-enantiomers, diastereomers, (*D*)- and (*L*)-isomers, tautomers, atropisomers, conformers and mixtures thereof (e.g., racemates, mixtures of diastereomers). Substituents in the compound of the present invention may have additional asymmetric atoms. All of these stereoisomers and mixtures thereof are included within the scope of the present invention. Optically active (-)- and (+)-isomers, (*R*)- and (*S*)-enantiomers and (*D*)- and (*L*)-isomers may be prepared by chiral synthesis, chiral reagents or other conventional techniques. An isomer of a compound of the present invention can be prepared by asymmetric synthesis or chiral auxiliary, or, when the molecule contains a basic functional group (such as amino) or an acidic functional group (such as carboxyl), it can be formed into a diastereoisomer salt with an appropriate optically active acid or base, and then the diastereoisomers can be resolved by conventional methods known in the art to obtain pure isomers. In addition, the resolution of enantiomers and diastereomers is usually achieved by chromatography.

In the chemical structure of the compound described in the present invention, the bond 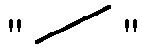 indicates an unspecified configuration, that is, if chiral isomers exist in the chemical structure, the bond 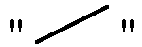 can be 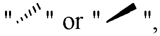 or contain both 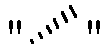 and " 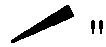 configurations. For all carbon-carbon double bonds, even if only one configuration is named, both *Z* and *E* configurations are included.

The compounds and intermediates of the present invention may also exist in different tautomeric forms, and all such forms are included within the scope of the present invention. The term "tautomer" or "tautomeric form" refers to structural isomers of different energies that can interconvert via a low energy barrier. For example, proton tautomers (also known as prototropic tautomers) include interconversions via proton migration, such as keto-enol and imine-enamine, lactam-lactimide isomerizations.

The compound of the present invention includes all suitable isotope substitutes of the compound. The term "isotope substitute" refers to a compound in which at least one atom is replaced by an atom having the same atomic number but different atomic masses. Examples of isotopes that can be incorporated into the compound of the present invention include stable and radioactive isotopes of hydrogen, carbon, nitrogen, oxygen, phosphorus, sulphur, fluorine, chlorine, bromine and iodine, such as ²H (deuterium, D), ³H (tritium , T), ¹¹C, ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁵1, ¹²⁹I and ¹³¹I, etc., preferably deuterium.

As used herein, the singular form of "a," "an," and "the" include plural references and vice versa unless the context clearly dictates otherwise.

When the term "about" is applied to a parameter such as pH, concentration, temperature, etc., it is intended that the parameter may vary by ±10%, and is sometimes more preferably within ±5%. As those skilled in the art will appreciate, when a parameter is not critical, numbers are generally given for purposes of illustration only and not limitation.

### DETAILED DESCRIPTION OF THE INVENTION

The present invention is described in detail below by way of examples, but it is not intended to limit the present invention in any way. The present invention has been described in detail herein, and specific embodiments thereof are also disclosed. It will be apparent to those skilled in the art that various changes and modifications can be made to the specific embodiments of the present invention without departing from the spirit and scope of the present invention.

The compound of the present invention is prepared by using convenient starting materials and general preparation procedures. The present invention provides typical or preferential reaction conditions, such as reaction temperature, time, solvent, pressure and molar ratio of reactants. However, unless otherwise specified, other reaction conditions can also be adopted. Optimal conditions may vary with the use of specific reactants or solvents, but under normal circumstances, reaction optimization steps and conditions can be determined.

In addition, certain protecting groups may be used in the present invention to protect certain functional groups from unnecessary reactions. The protecting groups suitable for various functional groups and their protection or deprotection conditions are well known to those skilled in the art.

The isolation and purification of compounds and intermediates are carried out by appropriate methods and steps according to specific needs, such as filtration, extraction, distillation, crystallization, column chromatography, preparative thin-layer chromatography, preparative high performance liquid chromatography or a combination of the above methods. The specific method used may be referred to the examples described in the present invention. Of course, other similar isolation and purification methods can also be used. They can be characterized using conventional methods (including physical constants and spectral data).

The purity analysis method is as follows: a Kinetex EVO C18 (50×4.6 mm, 5 µm, 100Å) chromatographic column is used, acetonitrile-water is used as the mobile phase for gradient elution, the flow rate is 1.5 mL/min, and the detection wavelength is 220 nm.

MS is determined by a LC (Agilent 1260 Infinity II)/MS (G6125B single quadrupole) mass spectrograph (manufacturer: Agilent) (Photodiode Array Detector).

The structures of the compounds are identified by hydrogen nuclear magnetic resonance, and the equipment model is WNMR-I-400MHz.

Preparative liquid chromatography is conducted on an Agilent 1260 Infinity II high performance liquid chromatograph (manufacturer: Agilent). The chromatographic column is Daisogel C18 10 µm 100A (30 mm×250 mm), and the mobile phase is acetonitrile/water.

GF254 silica gel plate of Qingdao Haiyang Chemical is used for the thin-layer silica gel chromatography (TLC). The dimension of the silica gel plate used in TLC is 0.20 mm to 0.25 mm, and the dimension of the silica gel plate used in product purification is 0.5 mm.

Silica gel of 100 to 200 mesh, 200 to 300 mesh or 300 to 400 mesh of Qingdao Haiyang Chemical is used as a carrier for gel column chromatography.

The known starting materials of the present invention can be prepared by the known methods in the art, or can be purchased from Wanghua Mall, Beijing Ouhe Technology, Sigma, J&K Scientific, Yishiming, Shanghai Shuya Chemical, Shanghai Innochem Science & Technology, Energy Chemical, Shanghai Bide Pharmatech and the like.

Unless otherwise stated, the reactions are carried out under nitrogen atmosphere.

Nitrogen atmosphere means that a reaction flask is equipped with a nitrogen balloon (about 1 L).

The reaction solvent, organic solvent or inert solvent are each expressed as the solvent used that does not participate in the reaction under the described reaction conditions, which includes for example, benzene, toluene, acetonitrile, tetrahydrofuran (THF), dimethylformamide (DMF), chloroform, dichloromethane, ether, methanol, N-methylpyrrolidone (NMP).

Unless otherwise specified in the examples, a solution means an aqueous solution.

The chemical reactions described in the present invention are generally carried out under normal pressure. The reaction time and conditions are, for example, between -78°C and 200°C at one atmosphere, and completed within about 1 to 24 hours. If the reaction is carried out overnight, the reaction time is generally 16 hours. Unless otherwise specified in the examples, the reaction temperature is room temperature, that is, 20°C to 30°C.

Unless otherwise defined, all professional and scientific terms used herein have the same meaning as those familiar to those skilled in the art. In addition, any methods and materials similar or equivalent to the content described herein can be applied to the method of the present invention.

Unless otherwise specified, the mixing ratios of different solvents are volume ratios.

The following introduces the synthesis of general intermediates, including A series, AA series, B series, BB series, C series, D series, and BBC series and BBD series further synthesized from BB series and C series or D series.

### Synthesis of general intermediate A1 (2-(2-isopropylphenyl)-5-methoxypyrimidin-4-amine)

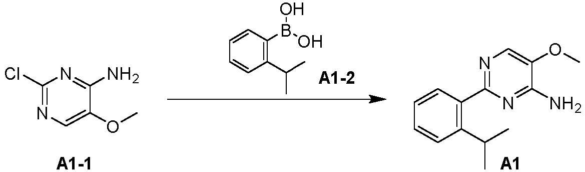

2-Chloro-5-methoxypyrimidin-4-amine (compound **Al-1,** 5.0 g, 31.3 mmol, 1.00 eq), (2-isopropylphenyl)boric acid (compound **A1-2**, 6.7 g, 40.7 mmol, 1.30 eq, purchased from Bide Pharmatech), potassium carbonate (13.0 g, 94.0 mmol, 3.00 eq) and 1,1'-bis(diphenylphosphino)ferrocene palladium dichloride (2.3 g, 3.13 mmol, 0.10 eq) were added to dioxane (50.0 mL) and water (12.5 mL) successively. The resulting mixture was purged with nitrogen three times, and reacted at 100°C under nitrogen atmosphere for 16 hours. Then, ethyl acetate (500.0 mL) was added, and the resulting mixture was stirred for 10 minutes and filtered. The resulting filtrate was concentrated under vacuum to obtain a crude product, which was purified by reverse phase HPLC (0.1% NH₃•H₂O) to obtain compound **A1** (4.9 g, 63.3% yield, 98.2% purity) as a light yellow solid. ¹H NMR (400 MHz, DMSO) δ 7.93 (s, 1H), 7.32-7.36 (m, 3H), 7.16-7.19 (m, 1H), 6.71 (s, 2H), 3.87 (s, 3H), 3.44-3.50 (m, 1H), 1.12 (d, *J =* 7.20 Hz, 6H); LC-MS: m/z = 244.2 (M+H)⁺.

### Synthesis of general intermediate A2 (4'-cyclopropyl-5,6'-dimethoxy-N-methyl-[2,5'-bipyrimidin]-4-amine)

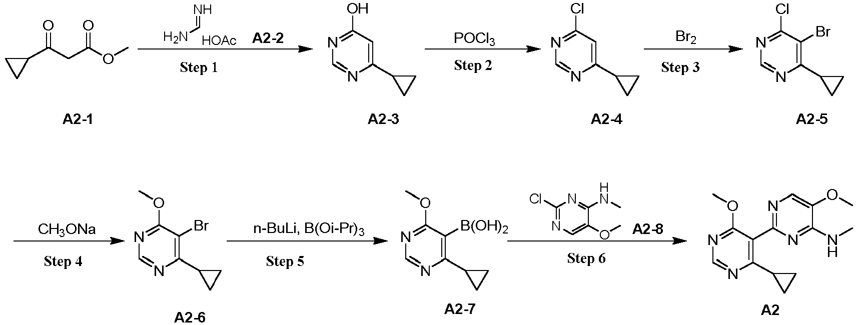

### Step 1: Synthesis of compound A2-3 (6-cyclopropylpyrimidin-4-ol)

Compound **A2-1** (200.0 g, 1.4 mol, 1.00 *eq)* and compound **A2-2** (292.0 g, 2.81 mol, *2.00 eq)* were added to methanol (1.2 L) successively, and a solution of sodium methoxide in methanol (5.4 M, 1.3 L, 5.00 *eq)* was added in portions at 0°C. After the addition was completed, the reaction was heated up to 20°C and stirred for 13 hours. Then, glacial acetic acid was added at 0°C to adjust the pH to 7-8. The resulting mixture was concentrated under vacuum to obtain a crude product, which was purified by column chromatography (eluent: petroleum ether/ethyl acetate = 1/0 - 1/1) to obtain compound **A2-3** (90.3 g). LC-MS: m/z =137.1 (M+H)⁺.

### Step 2: Synthesis of compound A2-4 (4-chloro-6-cyclopropylpyrimidine)

Compound **A2-3** (40.0 g, 293.0 mmol, 1.00 *eq*) was added to phosphorus oxychloride (180.0 mL) in portions. After the addition was completed, the reaction was heated up to 60°C and stirred for 2 hours. The reaction solution was concentrated under vacuum to obtain a crude product. The crude product was dissolved in ethyl acetate (400.0 mL) and water (400.0 mL), and stirred for 5 minutes. The organic layer was separated, and the aqueous layer was extracted twice with ethyl acetate (400.0 mL). The organic layers were combined, dried over anhydrous sodium sulfate, and filtered to obtain the filtrate. The filtrate was concentrated under vacuum to obtain a crude product, which was purified by column chromatography (eluent: petroleum ether/ethyl acetate = 20/1 - 1/1) to obtain compound **A2-4** (11.0 g). LC-MS: m/z = 155.0 (M+H)⁺.

### Step 3: Synthesis of compound A2-5 (5-bromo-4-chloro-6-cyclopropylpyrimidine)

Compound **A2-4** (11.0 g, 71.1 mmol, 1.00 *eq)* was dissolved in methanol (150.0 mL), and bromine (34.1 g, 213.0 mmol, 3.00 *eq)* was slowly added at -60°C. After the addition was completed, the reaction was heated up to 20°C and stirred for 2 hours. Then, saturated sodium bicarbonate solution (200.0 mL) and water (100.0 mL) were added at 0°C, the resulting mixture was stirred for 5 minutes, and extracted with dichloromethane (200.0 mL) three times. The organic layers were combined, washed with saturated brine (200.0 mL) twice, dried over anhydrous sodium sulfate, and filtered to obtain the filtrate. The filtrate was concentrated under vacuum to obtain a crude product, which was purified by column chromatography (eluent: petroleum ether/ethyl acetate = 1/0 - 3/1) to obtain compound **A2-5** (7.8 g). ¹H NMR (400 MHz, CDCl₃) δ 8.61 (s, 1H), 2.56 - 2.62 (m, 1H), 1.23 - 1.26 (m, 2H), 1.16 - 1.21 (m, 2H); LC-MS: m/z = 232.9 (M+H)⁺.

### Step 4: Synthesis of compound A2-6 (5-bromo-4-cyclopropyl-6-methoxypyrimidine)

Compound **A2-5** (7.8 g, 33.4 mmol, 1.00 *eq*) was dissolved in methanol (240.0 mL), and sodium methoxide (18.0 g, 100.0 mmol, 3.00 *eq*) was added at 0°C. After the addition was completed, the reaction was heated up to 30°C and stirred for 1 hour. The reaction solution was concentrated under vacuum to obtain a crude product, which was purified by column chromatography (eluent: petroleum ether/ethyl acetate = 10/1 - 5/1) to obtain compound **A2-6** (7.3 g). LC-MS: m/z =228.9 (M+H)⁺.

### Step 5: Synthesis of compound A2-7 ((4-cyclopropyl-6-methoxypyrimidin-5-yl)boronic acid)

Compound **A2-6** (10.3 g, 44.9 mmol, 1.00 *eq*) and triisopropyl borate (11.8 g, 62.9 mmol, 1.40 *eq)* were dissolved in tetrahydrofuran (30.0 mL) and toluene (90.0 mL), and n-butyl lithium (2.5 M, 25.1 mL, 1.40 *eq*) was added dropwise at -70°C. After the addition was completed, the reaction was stirred for 3 hours at -70°C. Then, 1 N hydrochloric acid solution (50.0 mL) was added dropwise at -70°C. After the addition was completed, the reaction was heated up to 20°C and stirred for 0.5 hours. Saturated aqueous sodium bicarbonate solution was then added to adjust the pH to 7-8, and the resulting mixture was extracted with ethyl acetate (100.0 mL) three times. The organic layers were combined, washed with saturated brine (100.0 mL) three times, dried over anhydrous sodium sulfate, and filtered to obtain the filtrate, which was concentrated under vacuum to obtain compound **A2-7** (6.9 g). ¹H NMR (400 MHz, CDCl₃) δ 8.52 (s, 1H), 8.45 (s, 2H), 3.84 (s, 3H), 1.88 - 1.92 (m, 2H), 0.93 - 1.01 (m, 4H).

### Step 6: Synthesis of intermediate A2

Compound **A2-8** (1.2 g, 6.9 mmol, 1.0 *eq*)*,* 1,1'-bis(diphenylphosphino)ferrocene palladium dichloride (505.0 mg, 0.7 mmol, 0.10 *eq*)*,* and potassium carbonate (2.9 g, 20.7 mmol, 3.00 *eq*) were added to dioxane (100.0 mL) and water (25.0 mL) successively, and the resulting mixture was purged three times with nitrogen. Compound **A2-7** (1.6 g, 8.3 mmol, 1.20 *eq*) dissolved in N,N-dimethylformamide (10.0 mL) was added dropwise at 100°C under nitrogen atmosphere. After the addition was completed, the reaction was reacted at 100°C for 2 hours under nitrogen atmosphere. Then, the reaction solution was concentrated under vacuum, followed by the addition of water (60.0 mL), and the resulting mixture was extracted twice with ethyl acetate (40.0 mL). The organic layers were combined, washed twice with saturated brine (40.0 mL), dried over anhydrous sodium sulfate, and filtered to obtain the filtrate. The filtrate was concentrated under vacuum to obtain a crude product, which was purified by column chromatography (eluent: petroleum ether/ethyl acetate = 1/0 - 10/1) to obtain compound **A2** (100.0 mg, 348 µmol, 5.0% yield, 100% purity) as a white solid. LC-MS: m/z =288.0 (M+H)⁺.

### Synthesis of general intermediate A3 (4-chloro-2-(2-isopropylphenyl)-5-nitropyrimidine)

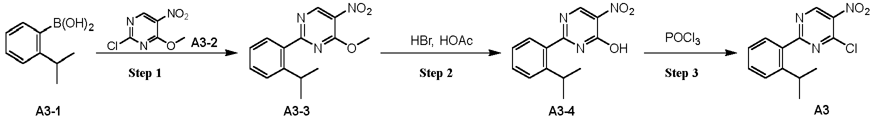

### Step 1: Synthesis of compound A3-3 (2-(2-isopropylphenyl)-4-methoxy-5-nitropyrimidine)

Compound **A3-2** (3.3 g, 17.4 mmol, 1.00 eq), compound **A3-1** (5.7 g, 34.8 mmol, 2.00 eq), 1,1'-bis(diphenylphosphino)ferrocene palladium dichloride (1.3 g, 1.7 mmol, 0.10 eq), and potassium carbonate (4.8 g, 34.8 mmol, 2.00 eq) were dissolved in dioxane (30.0 mL) and water (7.0 mL). The resulting mixture was purged three times with nitrogen, and reacted at 100°C for 12 hours under nitrogen atmosphere. Then, the reaction solution was concentrated under vacuum to obtain a crude product, which was purified by column chromatography (eluent: petroleum ether/ethyl acetate = 10/1 - 5/1) to obtain compound **A3-3** (3.2 g). LC-MS: m/z = 274.1 (M+H)⁺.

### Step 2: Synthesis of compound A3-4 (2-(2-isopropylphenyl)-5-nitropyrimidin-4-ol)

Compound **A3-3** (2.0 g, 7.3 mmol, 1.00 *eq*) was dissolved in dioxane (30.0 mL), and hydrobromic acid and glacial acetic acid (6 M, 15.0 mL, 12.30 *eq*) was added. After the addition was completed, the reaction was heated up to 50°C and stirred for 1 hour. Then, the reaction solution was concentrated under vacuum to obtain crude compound **A3-4** (2.0 g), which was directly used in the next step. LC-MS: m/z = 260.0 (M+H)⁺.

### Step 3: Synthesis of intermediate A3

Compound **A3-4** (2.0 g, 6.8 mmol, 1.00 *eq*) was added to phosphorus oxychloride (18.0 mL) and reacted at 90°C for 2 hours. The reaction solution was concentrated under vacuum to obtain a crude product, which was purified by column chromatography (eluent: petroleum ether/ethyl acetate = 50/1 - 30/1) to obtain compound **A3** (1.0 g, 3.59 mmol, 53.1% yield, 99.8% purity) as a light yellow oil. LC-MS: m/z = 278.1 (M+H)⁺.

### Synthesis of general intermediate A4 (2-(2-isopropylphenyl)-5-(trifluoromethyl)pyrimidin-4-amine)

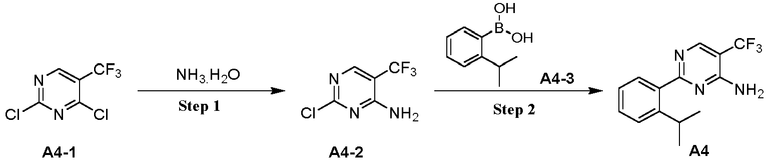

### Step 1: Synthesis of compound A4-2 (2-chloro-5-(trifluoromethyl)pyrimidin-4-amine)

Compound **A4-1** (18.7 g, 86.2 mmol, 1.00 eq) was dissolved in tetrahydrofuran (20.0 mL), and aqueous ammonia (15.1 g, 129 mmol, 30.0% purity, 1.50 eq) was added at 0°C. After the addition was completed, the reaction was heated up to 20°C and stirred for 16 hours. Then, water (30.0 mL) was added, and the resulting mixture was extracted with ethyl acetate (30.0 mL) three times. The organic layers were combined, dried over anhydrous sodium sulfate, and filtered to obtain the filtrate. The filtrate was concentrated under vacuum to obtain a crude product, which was purified by column chromatography (eluent: petroleum ether/ethyl acetate = 1/0 - 0/1) to obtain compound **A4-2** (1.73 g). ¹H NMR (400 MHz, DMSO) δ 8.56 (s, 1H), 7.95 (s, 2H); LC-MS: m/z = 197.9 (M+H)⁺.

### Step 2: Synthesis of intermediate A4

Compound **A4-2** (1.15 g, 5.77 mmol, 1.00 eq), compound **A4-3** (1.14 g, 6.92 mmol, 1.20 eq), potassium carbonate (2.39 g, 17.3 mmol, 3.00 eq), and 1,1'-bis(diphenylphosphino)ferrocene palladium dichloride (422 mg, 577 µmol, 0.10 eq) were added to dimethyl sulfoxide (12.0 mL) and water (2.5 mL) successively. The resulting mixture was purged three times with nitrogen, and reacted at 100°C for 12 hours under nitrogen atmosphere. Then, the reaction solution was concentrated under vacuum to obtain a crude product, which was purified by preparative HPLC to obtain compound **A4** (200 mg, 711 µmol, 12.3% yield, 100% purity) as a light yellow oil. ¹H NMR (400 MHz, DMSO) δ 8.57 (s, 1H), 7.41 - 7.44 (m, 3H), 7.22 - 7.26 (m, 1H), 3.41 - 3.45 (m, 1H), 3.30 (s, 2H), 1.16 (dd, *J₁* = 7.2Hz, *J₂* = 13.2Hz, 6H); LC-MS: m/z = 282.1 (M+H)⁺.

### Synthesis of general intermediate A5 (2-(2-isopropylphenyl)-5-methoxy-N-methylpyrimidin-4-amine)

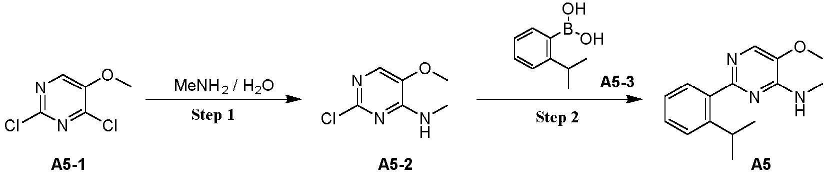

### Step 1: Synthesis of compound A5-2 (2-chloro-5-methoxy-N-methylpyrimidin-4-amine)

Compound **A5-1** (10.0 g, 55.9 mmol, 1.00 eq) was dissolved in tetrahydrofuran (100.0 mL), and methylamine (2.0 M methylamine aqueous solution, 54.5 mL, 1.95 eq) was added at 0°C. After the addition was completed, the reaction was heated up to 20°C and stirred for 2 hours. Water (200.0 mL) was added. and the resulting mixture was stirred for 5 minutes, and extracted with ethyl acetate (100.0 mL) three times. The organic layers were combined, dried over anhydrous sodium sulfate, and filtered to obtain the filtrate, which was concentrated under vacuum to obtain compound **A5-2** (9.0 g). ¹H NMR (400 MHz, CDCl₃) δ 7.50 (s, 1H), 5.48 (s, 1H), 3.86 (s, 3H), 3.05 (d, *J =* 4.00 Hz, 3H); LC-MS: m/z = 173.9 (M+H)⁺.

### Step 2: Synthesis of intermediate A5

Compound **A5-2** (3.0 g, 17.3 mmol, 1.00 *eq),* compound **A5-3** (3.7 g, 22.5 mmol, *1.3 eq*), potassium carbonate (7.2 g, 51.8 mmol, 3.00 *eq*) and 1,1'-bis(diphenylphosphino)ferrocene palladium dichloride (1.3 g, 1.7 mmol, 0.10 *eq*) were added to dioxane (30.0 mL) and water (7.5 mL) successively. The resulting mixture was purged three times with nitrogen, and reacted at 100°C for 16 hours under nitrogen atmosphere, followed by the addition of ethyl acetate (500.0 mL), and stirred for 10 minutes. The resulting mixture was filtered to obtain a filtrate, which was concentrated under vacuum to obtain a crude product. The crude product was purified by reverse phase HPLC (0.1% NH₃•H₂O) to obtain compound A5 (4.3 g, 16.3 mmol, 94.4% yield, 96.9% purity) as a brown oil. ¹H NMR (400 MHz, CDCl₃) δ 7.82 (s, 1H), 7.56 (d, *J* = 7.60 Hz, 1H), 7.33-7.41 (m, 2H), 7.23-7.25 (m, 1H), 5.29 (s, 1H), 3.92 (s, 3H), 3.53-3.60 (m, 1H), 3.07 (d, *J* = 5.20 Hz, 3H), 1.25-1.29 (m, 6H); LC-MS: m/z = 258.0 (M+H)⁺.

### Synthesis of general intermediate A6 (2-(2-isopropylphenyl)-7,8-dihydro-6H-pyrimido[5,4-b][1,4]oxazine)

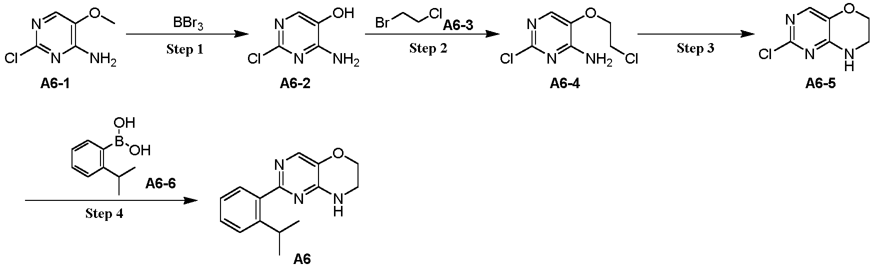

### Step 1: Synthesis of compound A6-2 (4-amino-2-chloropyrimidin-5-ol)

Compound **A6-1** (15.6 g, 97.8 mmol, 1.00 eq) was dissolved in dichloromethane (1.5 L), and boron tribromide (367.0 g, 1.5 mol, 15.0 eq) was slowly added at 0°C. After the addition was completed, the reaction was heated up to 20°C and stirred for 96 hours. Then, methanol (1.0 L) was slowly added at 0°C to quench the reaction. The reaction solution was concentrated under vacuum to obtain a crude product. Dichloromethane (200.0 mL) was added, and the resulting mixture was stirred for 5 minutes, and filtered to obtain a filter cake. The filter cake was dissolved in water (200.0 mL), and saturated aqueous sodium bicarbonate solution was added to adjust the pH to 7. The resulting mixture was filtered to obtain a filter cake, which was washed with water (50.0 mL) twice, and concentrated under vacuum to obtain compound **A6-2** (12.9 g). ¹H NMR (400 MHz, DMSO) δ 10.0 (d, *J =* 10.0 Hz, 1H), 7.47 (d, *J =* 15.6 Hz, 1H), 7.07 (s, 2H); LC-MS: m/z = 145.9 (M+H)⁺.

### Step 2: Synthesis of compound A6-4 (2-chloro-5-(2-chloroethoxy)pyrimidin-4-amine)

Compound **A6-2** (19.0 g, 130 mmol, 1.00 eq), compound **A6-3** (28.1 g, 196 mmol, 16.2 mL, 1.50 eq), and potassium carbonate (54.1 g, 392 mmol, 3.00 eq) were added to N,N-dimethylformamide (200.0 mL) successively, and reacted at 20°C for 16 hours to obtain crude compound **A6-4** (27.2 g), and the reaction solution was directly used in the next step. LC-MS: m/z = 207.8 (M+H)⁺.

### Step 3: Synthesis of compound A6-5 (2-chloro-7,8-dihydro-6H-pyrimido[5,4-b][1,4]oxazine)

The reaction solution of compound **A6-4** (27.2 g) was heated up to 100°C, stirred for 16 hours, and filtered to obtain a filtrate. Water (800.0 mL) was added, the resulting mixture was stirred for 5 minutes, and extracted with ethyl acetate (300.0 mL) three times. The organic layers were combined, washed three times with saturated brine (100.0 mL), dried over anhydrous sodium sulfate, and filtered to obtain a filtrate, which was concentrated under vacuum to obtain a crude product. Methyl *tert*-butyl ether (1000.0 mL) was added, the resulting mixture was stirred for 30 minutes, and filtered to obtain a filter cake, which was concentrated under vacuum to obtain compound **A6-5** (3.4 g). ¹H NMR (400 MHz, CDCl₃) δ 8.33 (d, *J =* 9.60 Hz, 1H), 7.63 (d, *J =* 18.8 Hz, 1H), 4.10-4.34 (m, 2H), 3.43-3.46 (m, 2H); LC-MS: m/z = 171.9 (M+H)⁺.

### Step 4: Synthesis of intermediate A6

Compound **A6-5** (1.5 g, 8.74 mmol, 1.00 eq), compound **A6-6** (1.4 g, 8.74 mmol, 1.00 eq), potassium carbonate (3.6 g, 26.2 mmol, 3.00 eq) and 1,1'-bis(diphenylphosphino)ferrocene palladium dichloride (640.0 mg, 874 µmol, 0.10 eq) were added to N,N-dimethylformamide (15.0 mL) and water (3.8 mL) successively. The resulting mixture was purged three times with nitrogen, and stirred at 100°C for 16 hours under nitrogen atmosphere. The reaction solution was concentrated under vacuum to obtain a crude product, which was purified by preparative HPLC (column: Kromasil Eternity XT 250×80mm×10µm; mobile phase: [water (ammonium hydroxide v/v)-ACN]; B%: 28%-58%, 21 min) to obtain compound **A6** (1.37 g, 5.27 mmol, 60.3% yield, 98.3% purity) as a brown solid. ¹H NMR (400 MHz, DMSO) δ 7.86 (s, 1H), 7.73-7.83 (m, 1H), 7.54-7.73 (m, 1H), 7.31-7.36 (m, 2H), 7.16-7.20 (m, 1H), 4.16-4.18 (m, 2H), 3.43-3.48 (m, 3H), 1.12-1.23 (m, 6H); LC-MS: m/z = 456.0 (M+H)⁺.

### Synthesis of general intermediate A7 (2-(2-isopropylphenyl)-6H-pyrimido[5,4-b][1,4]oxazin-7(8H)-one)

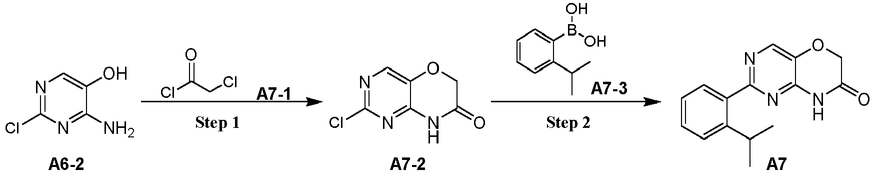

### Step 1: Synthesis of compound A7-2 (2-chloro-6H-pyrimido[5,4-b][1,4]oxazin-7(8H)-one)

Compound **A6-2** (4.00 g, 27.4 mmol, 1.00 *eq*)*,* compound **A7-1** (4.66 g, 41.2 mmol, 3.28 mL, 1.50 *eq*)*,* and potassium carbonate (11.4 g, 82.4 mmol, 3.00 *eq)* were added to N,N-dimethylformamide (50.0 mL) successively, and reacted at 30°C for 3 hours. Water (150.0 mL) was added, and the resulting mixture was extracted with ethyl acetate (50.0 mL) three times. The organic layers were combined, washed with saturated brine (70.0 mL) three times, dried over anhydrous sodium sulfate, and concentrated under vacuum to obtain a crude product, which was purified by column chromatography (eluent: petroleum ether/ethyl acetate = 20/1 - 5/1) to obtain compound **A7-2** (1.1 g). ¹H NMR (400 MHz, CDCl₃) δ 12.00 (s, 1H), 8.18 - 8.23 (m, 1H), 4.77 - 4.78 (m, 2H); LC-MS: m/z = 185.9 (M+H)⁺.

### Step 2: Synthesis of intermediate A7

Compound **A7-2** (500.0 mg, 2.69 mmol, 1.00 *eq),* compound **A7-3** (574.1 mg, 3.50 mmol, 1.30 *eq*), aqueous potassium phosphate solution (1.5 M, 5.39 mL, 3.00 *eq*) and (SP-4-3)-[dicyclohexyl[2',4',6'-tri(isopropyl)[1,1'-biphenyl]-2-yl]phosphino](methanesulfonato)[2'-(methylamino)[1,1'-biphenyl]-2-yl]palladium (231.1 mg, 269 µmol, 0.100 *eq*) were added to tetrahydrofuran (25.0 mL) successively. The resulting mixture was purged three times with nitrogen, and stirred at 60°C for 12 hours under nitrogen atmosphere. The reaction solution was concentrated under vacuum to obtain a crude product, which was purified by preparative HPLC (column: 3_Phenomenex Luna C18 75×30mm×3µm; mobile phase: [water(HCl)-ACN]; B%: 28%-48%, 8 min) to obtain **A7** (90.0 mg, 334 µmol, 12.4% yield) as a white solid. LC-MS: m/z = 270.1 (M+H)⁺.

### Synthesis of general intermediate A8 (2-(4-cyclopropyl-6-methoxypyrimidin-5-yl)-7,8-dihydro-6H-pyrimido[5,4-b][1,4]oxazine)

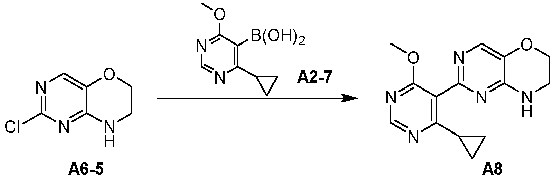

Compound **A6-5** (1.50 g, 8.74 mmol, 1.00 eq), compound **A2-7** (1.70 g, 8.74 mmol, 1.00 eq), potassium carbonate (3.62 g, 26.2 mmol, 3.00 eq), and 1,1'-bis(diphenylphosphino)ferrocene palladium dichloride (640 mg, 874 µmol, 0.10 eq) were added to a mixed solvent of N,N-dimethylformamide (15.0 mL) and water (3.75 mL) successively. The resulting mixture was purged three times with nitrogen, and stirred at 100°C for 16 hours under nitrogen atmosphere. The reaction solution was concentrated under vacuum to obtain a crude product, which was purified by preparative HPLC (column: Waters Xbridge BEH C18 250×50mm×10µm; mobile phase: [water (ammonium hydroxide v/v)-ACN]; B%: 10%-35%, 20 min) to obtain compound **A8** (414 mg, 1.45 mmol, 16.6% yield, 100% purity) as a white solid. ¹H NMR (400 MHz, DMSO) δ 8.59 (s, 1H), 7.94 (s, 1H), 7.87 (s, 1H), 4.17-4.19 (m, 2H), 3.82 (s, 3H), 3.47-3.49 (m, 2H), 1.67-1.71 (m, 1H), 0.99-1.01 (m, 2H), 0.88-0.90 (m, 2H); LC-MS: m/z = 286.0 (M+H)⁺.

### Synthesis of general intermediate A9 (4'-cyclopropyl-5,6'-dimethoxy-[2,5'-bipyrimidin]-4-amine)

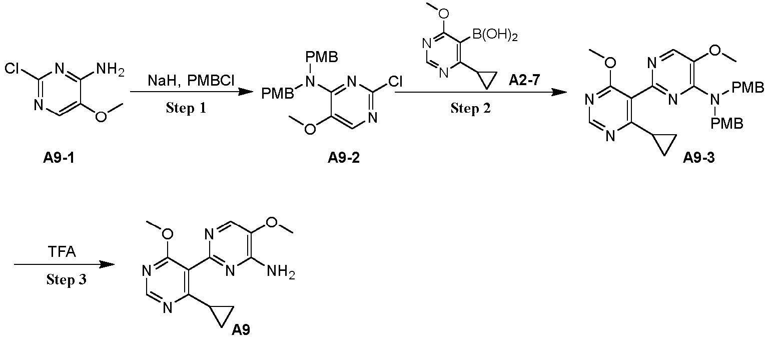

### Step 1: Synthesis of compound A9-2 (2-chloro-5-methoxy-N,N-bis(4-methoxybenzyl)pyrimidin-4-amine)

Compound **A9-1** (6.0 g, 37.6 mmol, 1.00 eq) was dissolved in tetrahydrofuran (60.0 mL), and sodium hydride (3.31 g, 82.7 mmol, 60% purity, 2.20 eq) was added in portions at 0°C. After the addition was completed, the resulting mixture was stirred at 5°C for 30 minutes. 4-Methoxybenzyl chloride (12.9 g, 82.7 mmol, 2.20 eq) was added and stirred at 5°C for 12 hours. Then, saturated aqueous ammonium chloride solution (40.0 mL) was added at 0°C to quench the reaction. Water (150.0 mL) was added, and the resulting mixture was extracted with ethyl acetate (70.0 mL) three times. The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 5/1 - 1/1) to obtain compound **A9-2** (10.3 g). LC-MS: m/z = 400.2 (M+H)⁺.

### Step 2: Synthesis of compound A9-3 (4'-cyclopropyl-5,6'-dimethoxy-N,N-bis(4-methoxybenzyl)-[2,5'-bipyrimidin]-4-amine)

Compound **A9-2** (2.0 g, 5.00 mmol, 1.00 *eq),* compound **A2-7** (1.46 g, 7.50 mmol, *1.50 eq*)*,* aqueous potassium phosphate solution (1.5 M, 10.0 mL, 3.00 *eq*)*,* and (SP-4-3)-[dicyclohexyl[2',4',6'-tri(isopropyl)[1,1'-biphenyl]-2-yl]phosphino](methanesulfonato)[2'-(methylamino)[1,1'-biphenyl]-2-yl]palladium (430 mg, 500 µmol, 0.100 *eq*) were added to tetrahydrofuran (40.0 mL) successively. The resulting mixture was purged three times with nitrogen, and stirred at 80°C for 12 hours under nitrogen atmosphere. The reaction solution was concentrated under vacuum to obtain a crude product, which was purified by preparative HPLC (column: Phenomenex luna C18 (250×70mm,10 µm); mobile phase: [water (FA)-ACN]; B%: 35%-65%,21 min) to obtain **A9-3** (0.9 g). LC-MS: m/z = 514.3 (M+H)⁺.

### Step 3: Synthesis of intermediate A9

Compound **A9-3** (900 mg, 1.75 mmol, 1.00 eq) was added to trifluoroacetic acid (10.0 mL), heated up to 90°C and stirred for 12 hours. The reaction solution was concentrated under vacuum, and saturated aqueous sodium bicarbonate solution (10.0 mL) was added to adjust the pH to 8. The resulting mixture was extracted with ethyl acetate (20.0 mL) three times. The organic layers were combined, dried over anhydrous sodium sulfate, and filtered to obtain a filtrate. The filtrate was concentrated under vacuum to obtain a crude product, which was purified by reverse phase HPLC (0.1% NH₃•H₂O) to obtain **A9** (300.0 mg, 62.5% yield, 100% purity) as a white solid. LC-MS: m/z = 274.1 (M+H)⁺.

### Synthesis of general intermediate A10 (2-(4-cyclopropyl-6-methoxypyrimidin-5-yl)-6H-pyrimido[5,4-b][1,4]oxazin-7(8H)-one)

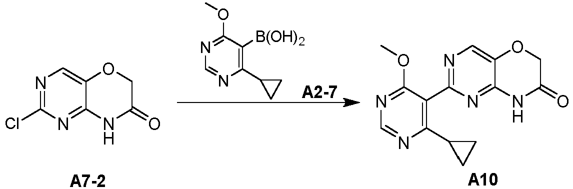

Compound **A7-2** (600 mg, 3.23 mmol, 1.00 *eq*)*,* **A2-7** (940 mg, 4.85 mmol, 1.50 *eq*)*,* aqueous potassium phosphate solution (1.5 M, 6.47 mL, 3.00 *eq*)*,* and (SP-4-3)-[dicyclohexyl[2',4',6'-tri(isopropyl)[1,1'-biphenyl]-2-yl]phosphino](methanesulfonato)[2'-(methylamino)[1,1'-biphenyl]-2-yl]palladium (417 mg, 485 µmol, 0.150 *eq*) were added to tetrahydrofuran (10.0 mL) successively. The resulting mixture was purged three times with nitrogen, and stirred at 80°C for 12 hours under nitrogen atmosphere. The reaction solution was concentrated under vacuum to obtain a crude product, which was purified by preparative HPLC (column: Phenomenex luna C18 150×40mm×15µm; mobile phase: [water (TFA)-ACN]; B%: 10%-40%, 10 min) to obtain intermediate **A10** (60.0 mg, 0.2 mmol, 6.2% yield, 100% purity). LC-MS: m/z =300.1 (M+H)⁺.

### Synthesis of general intermediate AA3 (1-methyl-7-(4,4,5,5-tetramethyl-1,3,2-dioxaborolan-2-yl)-1H-indole)

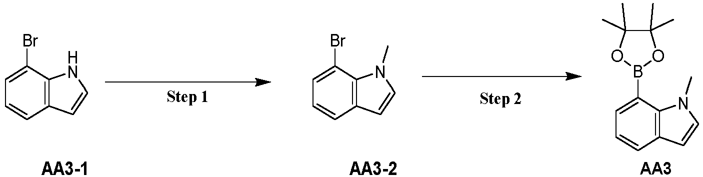

### Step 1: Synthesis of compound AA3-2 (7-bromo-1-methyl-1H-indole)

7-Bromoindole (compound **AA3-1,** 15.0 g, 76.5 mmol, 1.00 eq) was dissolved in THF (150 mL), and NaH (4.59 g, 115 mmol, purity 60%, 1.50 eq) was added in portions at 0°C. After the addition was completed, the resulting mixture was heated up to room temperature (25°C) and stirred for 1.5 hours. Then, potassium iodide (14.1 g, 99.5 mmol, 6.19 mL, 1.30 eq) was added at 0°C. After the addition was completed, the resulting mixture was heated up to room temperature (25°C) and stirred for 12 hours. 60 mL of ice water was added at 0°C to quench the reaction, and the resulting mixture was extracted with ethyl acetate (70.0 mL) three times. The organic layers were combined, washed three times with saturated brine (100.0 mL), dried over anhydrous sodium sulfate, and filtered to obtain a filtrate, which was concentrated under vacuum to obtain compound **AA3-2** (16.5 g, crude) as a yellow solid. ¹H NMR (400 MHz, CDCl₃) δ 7.59 (d, *J =* 7.6Hz, 1H), 7.39 - 7.41 (m, 1H), 7.01 - 7.02 (m, 1H), 6.94 - 6.98 (m, 1H), 6.50 - 6.51 (m, 1H), 4.18 (s, 3H); LC-MS: m/z = 212.0 (M+H)⁺.

### Step 2: Synthesis of intermediate AA3

Compound **AA3-2** (4.00 g, 19.0 mmol, 1.00 eq), bis(pinacolato)diboron (9.67 g, 38.1 mmol, 2.00 eq), [1,1'-bis(diphenylphosphino)ferrocene]palladium dichloride dichloromethane complex (1.39 g, 1.90 mmol, 0.100 eq) and potassium acetate (3.74 g, 38.1 mmol, 2.00 eq) were dissolved in dioxane (40.0 mL), and stirred at an external temperature of 95°C for 12 hours. Then, water (80.0 mL) was added, and the resulting mixture was extracted with ethyl acetate (50.0 mL) three times. The organic layers were combined, washed twice with saturated brine (50.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 3/1) to obtain intermediate **AA3** (2.47 g, 9.45 mmol, 49.6% yield, 98.4% purity) as a white solid. ¹H NMR (400 MHz, CDCl₃) δ 7.75 (d, *J* = 8.0Hz, 1H), 7.70 (d, *J* = 6.0Hz, 1H), 7.11 - 7.15 (m, 1H), 7.03 - 7.04 (m, 1H), 6.51 - 6.52 (m, 1H), 4.00 (s, 3H), 1.43 - 1.50 (m, 12H); LC-MS: m/z = 258.1 (M+H)⁺.

### Synthesis of general intermediate AA4 ((1-methylindolin-7-yl)boronic acid)

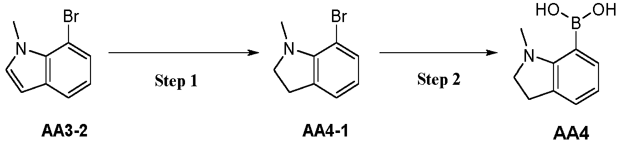

### Step 1: Synthesis of compound AA4-1 (7-bromo-1-methylindole)

Compound **AA3-2** (6.70 g, 31.8 mmol, 1.00 eq) was added to acetic acid (50.0 mL), followed by the addition of sodium cyanoborohydride (16.0 g, 255 mmol, 8.00 eq) at 10°C, and the mixture was stirred at 20°C for 12 hours. 1 M aqueous sodium hydroxide solution was added to adjust the pH to about 8, and the resulting mixture was extracted with dichloromethane (60 mL) three times. The organic layers were combined, washed twice with saturated brine (50 mL), dried over anhydrous sodium sulfate, and filtered to obtain a filtrate, which was concentrated under vacuum to obtain compound **AA4-1** (8.00 g, crude) as a brown oil. LC-MS: m/z = 212.1 (M+H)⁺.

### Step 2: Synthesis of intermediate AA4

Compound **AA4-1** (500 mg, 2.36 mmol, 1.00 eq) and triisopropyl borate (576 mg, 3.06 mmol, 704 µL, 1.30 eq) were added to tetrahydrofuran (10.0 mL), and n-butyl lithium (2.5 M, 1.23 mL, 1.30 eq) was added dropwise at -78°C. After the addition was completed, the resulting mixture was heated up to 25°C and stirred for 4 hours. Then, saturated aqueous ammonium chloride solution (20 mL) was added dropwise at 0°C to quench the reaction. Water (30.0 mL) was added, and the resulting mixture was extracted with ethyl acetate (15 mL) three times. The organic layers were combined, washed twice with saturated brine (20 mL), dried over anhydrous sodium sulfate, and filtered to obtain a filtrate. The filtrate was concentrated under vacuum to obtain a crude product, which was purified by reverse phase HPLC (0.1% FA) to obtain intermediate **AA4** (120 mg, 588.45 µmol, 24.96% yield, 86.8% purity) as a white solid. ¹H NMR (400MHz, DMSO-*d*₆) δ 8.06 (s, 1H), 7.02 (d, *J* = 6.8Hz, 2H), 6.52 - 6.60 (m, 2H), 3.24 (t, *J* = 8.0Hz, 2H), 2.84 (t, *J* = 8.0Hz, 2H), 2.76(s, 3H); LC-MS: m/z = 178.1 (M+H)⁺.

### Synthesis of general intermediate AA5 ((1-isopropyl-4-methyl-1H-imidazol-5-yl)boronic acid)

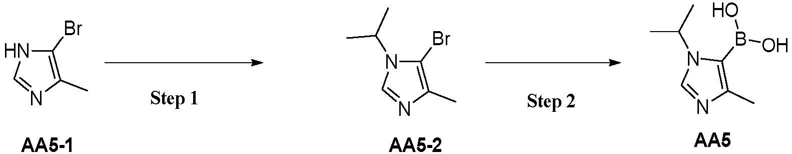

### Step 1: Synthesis of compound AA5-2 (5-bromo-1-isopropyl-4-methyl-1H-imidazole)

4-Methyl-5-bromoimidazole **(AA5-1,** 40.0 g, 248 mmol, 1.00 eq) was dissolved in tetrahydrofuran (800 mL), and sodium hydride (7.15 g, 178 mmol, 60% purity, 0.720 eq) was added in portions at -15°C. After the addition was completed, the resulting mixture was stirred at -15°C for 30 minutes. 2-Iodopropane (42.2 g, 248 mmol, 24.8 mL, 1.00 eq) was added, and the resulting mixture was stirred at 0°C for 3 hours. Saturated aqueous ammonium chloride solution (100 mL) was added to quench the reaction. Water (800.0 mL) was added, and the resulting mixture was extracted with ethyl acetate (500 mL) three times. The organic layers were combined, washed twice with saturated brine (500 mL), dried over anhydrous sodium sulfate, and filtered to obtain a filtrate. The filtrate was concentrated under vacuum to obtain a crude product, which was purified by reverse phase HPLC (0.1% NH₃•H₂O) to obtain compound **AA5-2** (7.00 g, 34.4 mmol, 46.6% yield) as a brown oil. ¹H NMR (400MHz, CDCl₃) δ 7.56 (s, 1H), 4.32 - 4.39 (m, 1H), 2.20 (s, 3H), 1.47 (d, *J* = 4.4 Hz, 6H); LC-MS: m/z = 203.0 (M+H)⁺.

### Step 2: Synthesis of intermediate AA5

Compound **AA5-2** (7.00 g, 34.5 mmol, 1.00 eq) and triisopropyl borate (32.4 g, 172 mmol, 39.6 mL, 5.00 eq) were added to tetrahydrofuran (150.0 mL), and n-butyl lithium (2.50 M, 27.6 mL, 2.00 eq) was added dropwise at -78°C. After the addition was completed, the resulting mixture was heated up to 25°C and stirred for 4 hours. Then, saturated aqueous ammonium chloride solution (200 mL) was added dropwise at 0°C to quench the reaction. Water (300.0 mL) was added, and the resulting mixture was extracted with ethyl acetate (300 mL) three times. The organic layers were combined, washed twice with saturated brine (300 mL), dried over anhydrous sodium sulfate, and filtered to obtain a filtrate, which was concentrated under vacuum to obtain compound **AA5** (6.5 g, crude) as a yellow solid. ¹H NMR (400MHz, DMSO-*d*₆) δ 8.22 - 8.26 (m, 2H), 8.20 (s, 1H), 5.12 (s, 1H),2.30 (s, 3H), 1.37 - 1.39 (m, 6H); LC-MS: m/z = 169.1 (M+H)⁺.

### Synthesis of general intermediate AA6 (2-cyclopropyl-3-(4,4,5,5-tetramethyl-1,3,2-dioxaborolan-2-yl)pyridine)

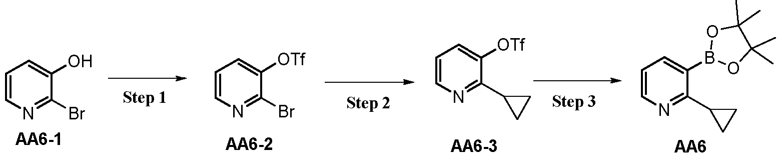

### Step 1: Synthesis of compound AA6-2 (2-bromopyridin-3-yl trifluoromethanesulfonate)

5-Bromo-3-hydroxypyridine (compound **AA6-1,** 1.0 g, 7.72 mmol), N-phenylbis(trifluoromethanesulfonyl)imide (2.76 g, 7.72 mmol, purchased from Bide Pharmatech), and triethylamine (1.1 mL, 8.1 mmol) were added to dichloromethane (20 mL) successively. The resulting mixture was stirred at 0°C for 1 hour under nitrogen atmosphere, and then heated up to 25°C and stirred for 1.5 hours. The reaction solution was washed once with 1 M sodium hydroxide (100 mL), washed twice with saturated brine (100 mL), dried over anhydrous sodium sulfate, and filtered to obtain a filtrate, which was concentrated under vacuum to obtain compound **AA6-2** (2.8 g, crude) as a light yellow oil. LC-MS: m/z = 305.9 (M+H)⁺.

### Step 2: Synthesis of compound AA6-3 (2-cyclopropylpyridin-3-yl trifluoromethanesulfonate)

Compound **AA6-2** (2.5 g), tetrakis(triphenylphosphine)palladium (199 mg), and cyclopropylzinc chloride (0.4 M THF solution, 23 mL) were added to tetrahydrofuran (15 mL) successively. The resulting mixture was stirred at 70°C for 3 hours under nitrogen atmosphere, and cooled to room temperature. Saturated aqueous sodium bicarbonate solution (60 mL) was added, and the resulting mixture was extracted with ethyl acetate (50 mL) three times. The organic layers were combined, dried over anhydrous sodium sulfate, and concentrated under vacuum to obtain a crude product, which was purified by column chromatography (eluent: petroleum ether/ethyl acetate = 1/0 - 1/1) to obtain compound **AA6-3** (1.5 g) as a colorless transparent oil. LC-MS: m/z = 268.1 (M+H)⁺.

### Step 3: Synthesis of intermediate AA6

Compound **AA6-3** (0.5 g), bis(pinacolato)diboron (0.57 g), potassium carbonate (0.525 g) and [1,1'-bis(diphenylphosphino)ferrocene]palladium dichloride dichloromethane complex (0.156 g) were added to dioxane (80 mL). The resulting mixture was stirred at 100°C for 20 hours, and cooled to room temperature. Ethyl acetate (200 mL) was added, the resulting mixture was washed with saturated brine (100 mL) three times, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **AA6** (0.2 g) as a white solid.

### Synthesis of general intermediate AA7 (2-isopropyl-3-(4,4,5,5-tetramethyl-1,3,2-dioxaborolan-2-yl)pyridine)

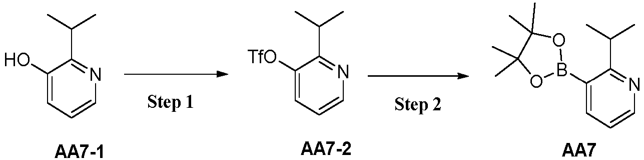

### Step 1: Synthesis of compound AA7-2 (2-isopropylpyridin-3-yl trifluoromethanesulfonate)

2-Isopropylpyridin-3-ol (compound **AA7-1**, 3.00 g, 21.8 mmol, 1.00 eq) was added to pyridine (30.0 mL), followed by the addition of trifluoromethanesulfonic anhydride (6.17 g, 21.8 mmol, 3.61 mL, 1.00 eq) at 0°C, and stirred at 15°C for 2 hours. Water (30 mL) was added, and the resulting mixture was extracted with ethyl acetate (60 mL) three times. The organic layers were combined, washed three times with saturated brine (100 mL), dried over anhydrous sodium sulfate, and filtered to obtain a filtrate, which was concentrated under vacuum to obtain compound **AA7-2** (3.62 g, crude) as a yellow oil. ¹H NMR (400MHz, CDCl₃) δ 8.59 (t, *J* = 3.6 Hz, 1H), 7.55 - 7.58 (m, 1H), 7.21 - 7.24 (m, 1H), 3.39 - 3.46 (m, 1H), 1.30 (d, *J* = 6.8 Hz, 6H); LC-MS: m/z = 270.2 (M+H)⁺.

### Step 2: Synthesis of compound AA7

Compound **AA7-2** (3.62 g, 13.4 mmol, 1.00 eq), bis(pinacolato)diboron (6.83 g, 26.8 mmol, 2.00 eq), potassium acetate (2.64 g, 26.8 mmol, 2.00 eq) and [1,1'-bis(diphenylphosphino)ferrocene]palladium dichloride (983 mg, 1.34 mmol, 0.100 eq) were added to dioxane (60.0 mL) successively. The resulting mixture was stirred at 100°C for 20 hours, and cooled to room temperature. Ethyl acetate (300 mL) was added, the resulting mixture was washed with saturated brine (150 mL) three times, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 0/1) to obtain compound **AA7** (2.00 g, 8.09 mmol, 60.2% yield) as a light yellow oil. ¹H NMR (400MHz, CDCl₃) δ 8.60 (dd, *J₁* = 2.0Hz, *J₂* = 3.2Hz, 1H), 7.99 (dd, *J₁* = 2.0Hz, *J₂* = 5.2Hz, 1H), 7.07 (dd, *J₁* = 4.8Hz, *J₂* = 2.8Hz, 1H), 3.71 - 3.78 (m, 1H), 1.35 (s, 12H), 1.24 - 1.28 (m, 6H).

### General intermediate AA8 ((2-(dimethylamino)phenyl)boronic acid)

Intermediate **AA8** was purchased from Bide Pharmatech.

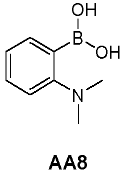

### Synthesis of general intermediate AA9 (4-chloro-1-isopropyl-5-(4,4,5,5-tetramethyl-1,3,2-dioxaborolan-2-yl)-1H-pyrazole)

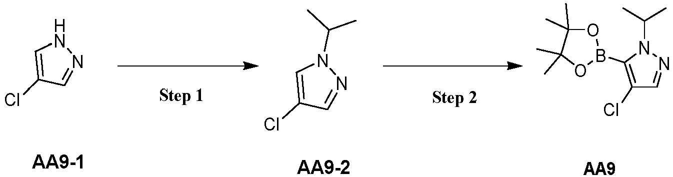

### Step 1: Synthesis of compound AA9-2 (4-chloro-1-isopropyl-1H-pyrazole)

Compound **AA9-1** (50.00 g, 487.70 mmol, 1.00 *eq*)*,* 2-iodopropane (124.36 g, 731.55 mmol, 73.15 mL, 1.50 *eq*) and cesium carbonate (317.80 g, 975.40 mmol, 2.00 *eq)* were added to acetonitrile (500.00 mL), and stirred at 80°C for 2 hours under N₂ atmosphere. Then, the reaction solution was filtered to obtain a filtrate. The filtrate was concentrated under vacuum to obtain a crude product, which was purified by column chromatography (eluent: petroleum ether/ethyl acetate = 1/0 - 5/1) to obtain compound **AA9-2** (45.00 g, 306.53 mmol, 62.85% yield, 98.50% purity) as a light yellow oil. ¹H NMR (400 MHz, CDCl₃) δ 7.41-7.40 (m, 2H), 4.46 - 4.41(m, 1H), 1.48 (d, *J* = 6.8 Hz, 6H); LC-MS: m/z = 144.0 (M+H)⁺.

### Step 2: Synthesis of intermediate AA9

Compound **AA9-2** (10 g, 69.16 mmol, 1 eq) was dissolved in anhydrous tetrahydrofuran (100 mL), and *n*-butyl lithium (2.5 M, 33.19 mL, 1.2 eq) was added dropwise at 0°C. After the addition was completed, the resulting mixture was heated up to 25°C and stirred for 1 hour. Isopropyl pinacolyl borate (15.44 g, 82.99 mmol, 16.93 mL, 1.2 eq) was added at -78°C, and the resulting mixture was heated up to 25°C and stirred for 2 hours. Then, saturated aqueous ammonium chloride solution (100 mL) was added at 0°C to quench the reaction. Water (200 mL) was added, and the resulting mixture was extracted with ethyl acetate (50.0 mL) three times. The organic layers were combined, washed twice with saturated brine (60.0 mL), dried over anhydrous sodium sulfate, and filtered to obtain a filtrate. The filtrate was concentrated under vacuum to obtain a crude product, which was purified by prep-HPLC (column: Welch Ultimate XB-CN 250×70×10µm; mobile phase: [hexane-ethanol]; B%: 1%-1%, 15 min) to obtain compound **AA9** (6.5 g, 18.33 mmol, 26.51% yield, 76.3% purity) as a light yellow oil. ¹H NMR (400MHz, CDCl₃) δ 7.45 (s, 1H), 5.07 - 5.00(m, 1H), 1.46 (d, *J* = 6.8 Hz, 6H), 1.36 (s, 12H); LC-MS: m/z = 271.2 (M+H)⁺.

### General intermediate AA10 ((2-cyclopropylphenyl)boronic acid)

Intermediate **AA10** was purchased from Bide Pharmatech.

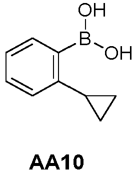

### Synthesis of general intermediate AA11 ((1-isopropyl-4-methoxy-1H-pyrazol-5-yl)boronic acid)

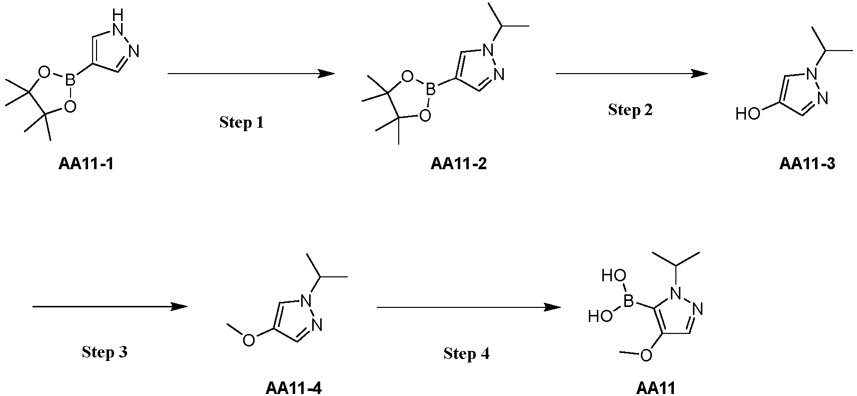

### Step 1: Synthesis of compound AA11-2 (1-isopropyl-4-(4,4,5,5-tetramethyl-1,3,2-dioxaborolan-2-yl)-1H-pyrazole)

4-Pyrazolyl pinacolyl borate (compound **AA11-1,** 130 g, 669 mmol, 1.00 eq) was dissolved in N,N-dimethylformamide (114 g, 669 mmol, 67.0 mL, 1.00 eq). 2-Iodopropane (114 g, 669 mmol, 67.0 mL, 1.00 eq) and cesium carbonate (327 g, 1.00 mol, 1.50 eq) were added, and the mixture was stirred at an external temperature of 90°C for 12 hours. Then, the reaction solution was filtered to obtain a filtrate. The filtrate was concentrated under vacuum to obtain a crude product, which was purified by column chromatography (eluent: petroleum ether/ethyl acetate = 1/0 - 0/1) to obtain compound **AA11-2** (120 g, 508 mmol, 75.9% yield) as a colorless transparent oil. ¹H NMR (400MHz, CDCl₃) δ 7.74 (m, 2H), 4.46 - 4.53 (m, 1H), 1.48 (d, *J =* 6.8Hz, 6H), 1.29 (s, 12H); LC-MS: m/z = 237.1 (M+H)⁺.

### Step 2: Synthesis of compound AA11-3 (1-isopropyl-1H-pyrazol-4-ol)

Compound **AA11-2** (60.0 g, 254 mmol, 1.00 eq) was dissolved in tetrahydrofuran (600 mL), followed by the addition of aqueous sodium hydroxide solution (2.50 M, 203 mL, 2.00 eq) and hydrogen peroxide (72.0 g, 635 mmol, 61.0 mL, 30.0% purity, 2.50 eq). The mixture was stirred at 25°C for 3 hours. 1 M aqueous hydrochloric acid solution was added to adjust the pH to about 2. Then, anhydrous sodium sulfite (50.0 g) was added at 0°C to quench the reaction. The resulting mixture was concentrated under vacuum to remove tetrahydrofuran, and extracted three times with dichloromethane/methanol = 10/1 (1500 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 0/1) to obtain compound **AA11-3** (17.5 g, 138 mmol, 54.6% yield) as a light yellow oil. ¹H NMR (400MHz, CDCl₃) δ7.12 (s, 1H), 7.09 (d, *J =* 0.8 Hz, 1H), 4.32 - 4.39 (m, 1H), 1.43 (s, 3H), 1.41 (s, 3H).

### Step 3: Synthesis of compound AA11-4 (1-isopropyl-4-methoxy-1H-pyrazole)

Compound **AA11-3** (17.5 g, 139 mmol, 1.00 eq) was dissolved in N,N-dimethylformamide (350 mL), followed by the addition of cesium carbonate (67.8 g, 208 mmol, 1.50 eq) and iodomethane (29.5 g, 208 mmol, 13.0 mL, 1.50 eq). The mixture was stirred at room temperature (25°C) for 3 hours. Then, water (1000 mL) was added, and the resulting mixture was extracted three times with ethyl acetate (600 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **AA11-4** (12.3 g, 87.7 mmol, 63.3% yield) as a light yellow solid. ¹H NMR (400MHz, CDCl₃) δ 7.17 (s, 1H), 7.05 (d, *J =* 0.8 Hz, 1H), 4.30 - 4.36 (m, 1H), 3.70 (s, 3H), 1.42 (s, 3H), 1.40 (s, 3H).

### Step 4: Synthesis of compound AA11

Compound **AA11-4** (1.00 g, 7.13 mmol, 1.00 eq) was dissolved in anhydrous tetrahydrofuran (15.0 mL). *n*-Butyl lithium (2.50 M, 4.28 mL, 1.50 eq) was added dropwise at -70°C under nitrogen atmosphere. After the addition was completed, the resulting mixture was stirred at -70°C for 1 hour. Isopropyl pinacolyl borate (2.01 g, 10.8 mmol, 2.2 mL, 1.50 eq) was then added at -70°C. The resulting mixture was stirred at - 70°C for 1 hour, heated up to 25°C and stirrred for 12 hours. Then, saturated aqueous ammonium chloride solution (20.0 mL) was added at 0°C, and the resulting mixture was extracted with ethyl acetate (100 mL) three times. The organic layers were combined, dried over anhydrous sodium sulfate, and filtered to obtain a filtrate. The filtrate was concentrated under vacuum to obtain a crude product, which was triturated with petroleum ether (20.00 mL) for 15 minutes to obtain intermediate **AA11** (200 mg, 1.09 mmol, 15.2% yield) as an off-white solid. LC-MS: m/z = 185.2 (M+H)⁺.

### Synthesis of general intermediate AA12 ((1-cyclopropyl-4-methoxy-1H-pyrazol-5-yl)boronic acid)

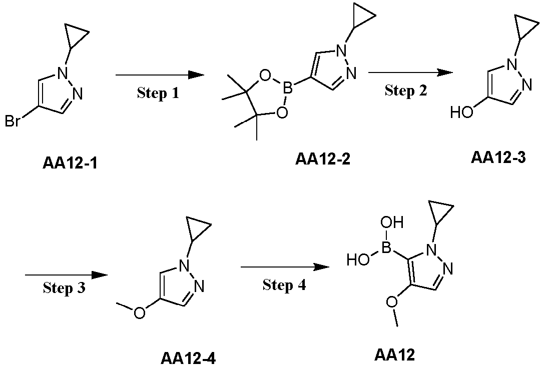

### Step 1: Synthesis of compound AA12-2 (1-cyclopropyl-4-(4,4,5,5-tetramethyl-1,3,2-dioxaborolan-2-yl)-1H-pyrazole)

Compound **AA12-1** (19.0 g, 102 mmol, 1.00 *eq)* was dissolved in dioxane (250 mL), followed by the addition of bis(pinacolato)diboron (36.1 g, 142 mmol, 1.40 *eq),* potassium acetate (39.9 g, 406 mmol, 4.00 *eq)* and [1,1'-bis(diphenylphosphino)ferrocene]palladium dichloride dichloromethane complex (4.15 g, 5.08 mmol, 0.05 *eq)* successively. The mixture was stirred at 85°C for 12 hours under nitrogen atmosphere. Then, water (800 mL) and ethyl acetate (500 mL) were added, and the resulting mixture was filtered to obtain a filtrate, which was extracted with ethyl acetate (500 mL) three times. The organic layers were combined, washed twice with saturated brine (500 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/0) to obtain compound **AA12-2** (4.40 g) as a light yellow oil. ¹H NMR (400MHz, CDCl₃) δ 7.75 (d, *J* = 3.2Hz, 2H), 3.50 - 3.60 (m, 1H), 1.32 (s, 12H), 1.09 - 1.12 (m, 2H), 1.01 - 1.04 (m, 2H); LC-MS: m/z = 235.1 (M+H)⁺.

### Step 2: Synthesis of compound AA12-3 (1-cyclopropyl-1H-pyrazol-4-ol)

Compound **AA12-2** (3.30 g, 14.1 mmol, 1.00 *eq)* was dissolved in tetrahydrofuran (43.0 mL), followed by the addition of aqueous sodium hydroxide solution (2.50 M, 11.3 mL, 2.00 *eq*) and hydrogen peroxide (5.84 g, 51.5 mmol, 4.95 mL, 30.0% purity, 3.65 *eq)* were added. The mixture was stirred at 25°C for 3 hours. 1 M aqueous hydrochloric acid solution was added to adjust the pH to about 2. Then, anhydrous sodium sulfite (50.0 g) was added at 0°C to quench the reaction. The resulting mixture was concentrated under vacuum to remove tetrahydrofuran, and extracted three times with dichloromethane/methanol = 10/1 (150 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 0/1) to obtain compound **AA12-3** (0.980 g, crude) as a light yellow oil. ¹H NMR (400MHz, CDCl₃) δ 7.12 (d, *J* = 3.6Hz, 2H), 3.46 - 3.48 (m, 1H), 0.95 - 0.96 (m, 2H), 0.94 - 0.95 (m, 2H).

### Step 3: Synthesis of compound AA12-4 (1-cyclopropyl-4-methoxy-1H-pyrazole)

Compound **AA12-3** (0.980 g, 7.89 mmol, 1.00 *eq*) was dissolved in N,N-dimethylformamide (19.5 mL), followed by the addition of cesium carbonate (3.86 g, 11.8 mmol, 1.50 *eq*) and iodomethane (1.68 g, 11.8 mmol, 737 µL, 1.50 *eq*)*.* The mixture was stirred at room temperature (25°C) for 3 hours. Then, water (10.0 mL) was added, and the resulting mixture was extracted three times with ethyl acetate (10.0 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/2) to obtain compound **AA12-4** (0.460 g, 3.33 mmol, 42.2% yield) as a light yellow solid. ¹H NMR (400MHz, CDCl₃) δ 7.21 (s, 1H), 7.13 (s, 1H), 3.74 (s, 3H), 3.49 - 3.53 (m, 1H), 1.08 - 1.09 (m, 2H), 0.96 - 0.99 (m, 2H); LC-MS: m/z = 139.2 (M+H)⁺.

### Step 4: Synthesis of intermediate AA12

Compound **AA12-4** (0.300 g, 2.17 mmol, 1.00 *eq*) was dissolved in anhydrous tetrahydrofuran (8.50 mL), and n-butyl lithium (2.50 M, 1.74 mL, 2.00 *eq*) was added dropwise at -70°C under nitrogen atmosphere. After the addition was completed, the resulting mixture was stirred at -70°C for 1 hour. Isopropyl pinacolyl borate (808 mg, 4.34 mmol, 886 µL, 2.00 *eq*) was then added at -70°C, and the resulting mixture was stirred at -70°C for 1 hour, heated up to 25°C and stirred for 12 hours. Then, saturated aqueous ammonium chloride solution (30.0 mL) was added at 0°C, and the resulting mixture was extracted three times with ethyl acetate (10.0 mL). The organic layers were combined, dried over anhydrous sodium sulfate, and filtered to obtain a filtrate. The filtrate was concentrated under vacuum to obtain a crude product, which was purified by prep-HPLC (chromatographic column: Waters Xbridge C18 150×50mm× 10µm; mobile phase: [water (NH₄HCO₃)-ACN]; B%: 3%-33%, 10 min) to obtain compound **AA12** (0.400 g, crude) as a light yellow oil. LC-MS: m/z = 183.1 (M+H)⁺.

### Synthesis of general intermediate AA13 ((5-isopropyl-3-methylisoxazol-4-yl)boronic acid)

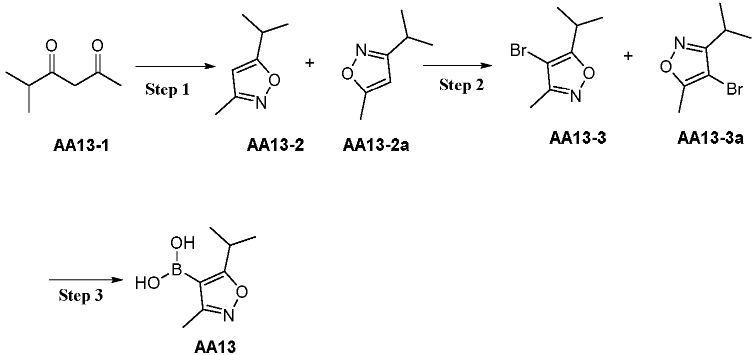

### Step 1: Synthesis of compound AA13-2 (5-isopropyl-3-methylisoxazole)

Isobutyrylacetone (compound **AA13-1**, 2.00 g, 15.6 mmol, 1.00 *eq*) and hydroxylamine hydrochloride (1.36 g, 19.6 mmol, 1.25 *eq*) were added to ethanol (10.0 mL) successively, and stirred at 130°C for 10 minutes. Dichloromethane (100 mL) was added, and the resulting mixture was washed twice with saturated brine (50.0 mL). The organic layer was separated, dried over anhydrous sodium sulfate, and filtered to obtain a filtrate, which was concentrated under vacuum to obtain compound **AA13-2** and compound **AA13-2a** (4.80 g in total, crude) as a colorless transparent liquid. Compound **AA13-2:** ¹H NMR (400MHz, CDCl₃) δ 5.76 (s, 1H), 3.04 - 2.97 (m,1H), 2.35 (s, 3H), 1.28 - 1.23 (m, 6H).

### Step 2: Synthesis of compound AA13-3 (4-bromo-5-isopropyl-3-methylisoxazole)

Compound **AA13-2** and compound **AA13-2a** (3.50 g, 28.0 mmol, 1.00 *eq*) were dissolved in N,N-dimethylformamide (15 mL), followed by the addition of N-bromosuccinimide (7.94 g, 44.6 mmol, 1.59 *eq*)*.* The mixture was stirred at 25°C for 15 hours. Ethyl acetate (250 mL) was added, and the resulting mixture was washed once with saturated sodium thiosulfate (100 mL) and three times with saturated brine (200 mL). The organic layer was separated, dried over anhydrous sodium sulfate, and filtered to obtain a filtrate, which was concentrated under vacuum to obtain compound **AA13-3** and compound **AA13-3a** (5.24 g, crude) as a light yellow oil. Compound **AA13-3**: ¹H NMR (400MHz, CDCl₃) δ 3.20 - 3.13 (m, 1H), 2.24 (s, 3H), 1.33 - 1.30 (m, 6H); LC-MS: m/z = 203.9 (M+H)⁺.

### Step 3: Synthesis of compound AA13

Compound **AA13-3**, compound **AA13-3a** (3.00 g, 14.7 mmol, 1.00 *eq*) and triisopropyl borate (3.59 g, 19.1 mmol, 4.39 mL, 1.30 *eq*) were dissolved in tetrahydrofuran (30.0 mL), and *n*-butyl lithium (2.5 M, 7.64 mL, 1.30 eq) was added dropwise at -70°C. After the addition was completed, the resulting mixture was heated up to 25°C and stirred for 4 hours. Water (10.0 mL) was added at 0°C to quench the reaction, and the resulting mixture was extracted three times with ethyl acetate (30.0 mL). The organic layers were combined, dried over anhydrous sodium sulfate, and filtered to obtain a filtrate. The filtrate was concentrated under vacuum to obtain a crude product, which was purified by prep-HPLC (chromatographic column: Phenomenex luna C18 250×80mm× 10 µm; mobile phase: [water (FA)-ACN]; B%: 20%-50%, 20 min) to obtain compound **AA13** (470 mg, 2.73 mmol, 18.5% yield, 98.0% purity) as a white solid. ¹H NMR (400MHz, CDCl₃) δ 3.83 - 3.76 (m, 1H), 2.49 (s, 3H), 1.38 (s, 3H), 1.36 (s, 3H); LC-MS: m/z = 170.1 (M+H)⁺.

### Synthesis of general intermediate AA14 (4-chloro-1-cyclopropyl-5-(4,4,5,5-tetramethyl-1,3,2-dioxaborolan-2-yl)-1H-pyrazole)

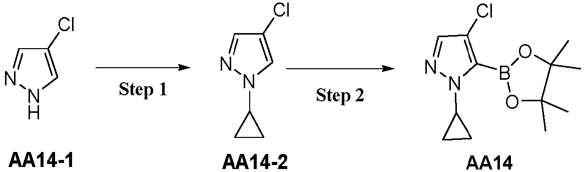

### Step 1: Synthesis of compound AA14-2 (4-chloro-1-cyclopropyl-1H-pyrazole)

4-Chloropyrazole (compound **AA14-1,** 10.0 g, 97.54 mmol, 1.0 eq), cyclopropyl bromide (21.2 g, 175.57 mmol, 1.8 eq) and cesium carbonate (63 g, 195.08 mmol, 2.0 eq) were added to dioxane (50 mL) successively, and stirred at an external temperature of 140°C for 16 hours. The reaction solution was cooled to room temperature, and concentrated under vacuum to obtain a crude product, which was purified by column chromatography (eluent: petroleum ether/ethyl acetate = 1/0 - 1/2) to obtain compound **AA14-2** (6.4 g) as a light yellow oil. ¹H NMR (400MHz, CDCl₃) δ 7.42 (s, 1H), 7.37 (s, 1H), 3.54 (tt, J = 7.3, 3.8 Hz, 1H), 1.13 - 1.05 (m, 2H), 1.05 - 0.95 (m, 2H). LC-MS: m/z = 143.1 (M+H)+.

### Step 2: Synthesis of intermediate AA14

Compound **AA14-2** (8.3 g, 58.21 mmol, 1.0 eq) was dissolved in tetrahydrofuran (100 mL), and lithium diisopropylamide (58 mL, 116.42 mmol, 2.0 eq) was added dropwise under nitrogen atmosphere at -70°C. After the addition was completed, the resulting mixture was stirred at -70°C for 1 hour. Isopropyl pinacolyl borate (17.3 g, 93.14 mmol, 1.6 eq) was added, and the resulting mixture was stirred at -70°C for 1 hour, heated up to 25°C and stirred for 2 hours. Then, water (100 mL) was added to quench the reaction, and the resulting mixture was extracted three times with dichloromethane (200 mL). The organic layers were combined, dried over anhydrous sodium sulfate, and concentrated under vacuum to obtain a crude product, which was purified by column chromatography (eluent: petroleum ether/ethyl acetate = 1/0 - 1/1) to obtain intermediate **AA14** (4.2 g) as a colorless and transparent oil. ¹H NMR (400MHz, CDCl₃) δ 7.42 (s, 1H), 7.37 (s, 1H), 3.54 (tt, J = 7.3, 3.8 Hz, 1H), 1.13 - 1.05 (m, 2H), 1.05 - 0.95 (m, 2H).

### General intermediate AA15 ((4-cyclopropylpyrimidin-5-yl)boronic acid)

Intermediate **AA15** was purchased from Leyan Reagents.

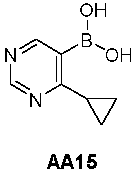

### Synthesis of general intermediate AA16 (1-cyclopropyl-4-methyl-5-(4,4,5,5-tetramethyl-1,3,2-dioxaborolan-2-yl)-1H-pyrazole)

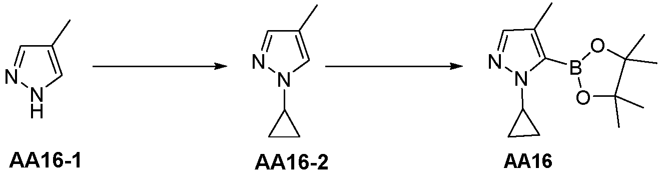

In accordance with the same steps of intermediate **AA14,** intermediate **AA16** (2.9 g) was obtained using 4-methylpyrazole (compound **AA16-1**) as starting material. ¹H NMR (400 MHz, DMSO-*d*₆) δ 7.24 (s, 1H), 4.03 (td, *J* = 7.5, 3.8 Hz, 1H), 2.15 (s, 3H), 1.35 (s, 12H), 1.12 - 1.01 (m, 2H), 0.99 - 0.89 (m, 2H).

### Synthesis of general intermediate AA17 (1-isopropyl-4-methyl-5-(4,4,5,5-tetramethyl-1,3,2-dioxaborolan-2-yl)-1H-pyrazole)

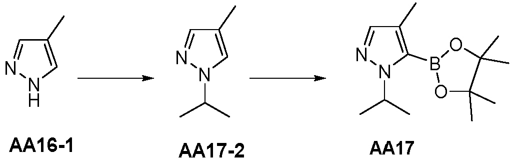

In accordance with the same steps of intermediate **AA14,** intermediate **AA17** (4.1 g) was obtained using 4-methylpyrazole (compound **AA16-1**) as starting material. ¹H NMR (400 MHz, DMSO-*d*₆) δ 7.27 (s, 1H), 4.94-4.98 (m, 1H), 2.12 (s, 3H), 1.34 (d, *J =* 6.6 Hz, 6H), 1.29 (s, 12H).

### Synthesis of general intermediate B1 (3-(4-(chloromethyl)piperidin-1-yl)pyridine)

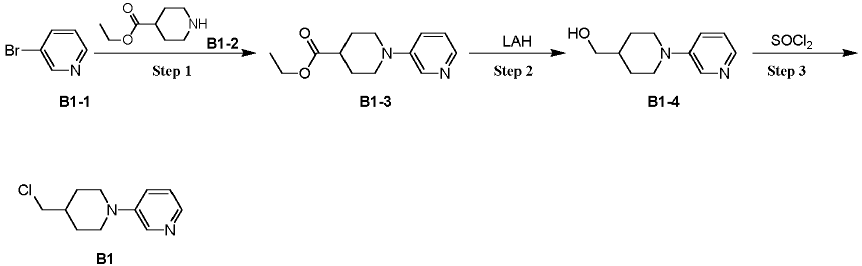

### Step 1: Synthesis of compound B1-3 (ethyl 1-(pyridin-3-yl)piperidine-4-carboxylate)

Compound **B1-1** (6.4 g, 40.7 mmol, 1.28 eq), compound **B1-2** (5.0 g, 31.8 mmol, 1.00 eq), 4,5-bis(diphenylphosphino)-9,9-dimethylxanthene (1.1 g, 1.91 mmol, 0.06 eq), cesium carbonate (13.9 g, 42.90 mmol, 1.35 eq), and tris(dibenzylideneacetone)dipalladium (582.0 mg, 0.6 mmol, 0.02 eq) were added to anhydrous dioxane (50.0 mL) successively. The resulting mixture was purged three times with nitrogen, and reacted at 100°C for 12 hours under nitrogen atmosphere. The reaction solution was concentrated under vacuum to obtain a crude product, which was purified by reverse phase HPLC (0.1% FA) to obtain compound **B1-3** (3.2 g). ¹H NMR (400 MHz, CDCl₃) δ 8.29 (d, *J =* 1.6 Hz, 1H), 8.05 (d, *J* = 3.2 Hz, 1H), 7.12 - 7.19 (m, 2H), 4.11 - 4.17 (m, 2H), 3.60 - 3.65 (m, 2H), 2.80 - 2.86 (m, 2H), 2.39 - 2.48 (m, 2H), 2.00 - 2.04 (m, 2H), 1.83 - 1.87 (m, 2H), 1.25 (t, J = 7.2 Hz, 3H); LC-MS: m/z =235.1 (M+H)⁺.

### Step 2: Synthesis of compound B1-4 ((1-(pyridin-3-yl)piperidin-4-yl)methanol)

Compound **B1-3** (2.0 g, 8.5 mmol, 1.00 eq) was dissolved in tetrahydrofuran (20.0 mL), and lithium aluminum tetrahydride (356.0 mg, 9.4 mmol, 1.10 eq) was added at 0°C. After the addition was completed, the resulting mixture was heated up to 25°C and stirred for 4 hours. Then, water (1.6 mL) and aqueous sodium hydroxide solution (1 M, 0.4 mL) were added at 0°C. After the addition was completed, the resulting mixture was stirred at 0°C for 0.5 hours. Tetrahydrofuran (10.0 mL) and anhydrous sodium sulfate (1.0 g) were added, and the resulting mixture was stirred for 5 minutes, and filtered to obtain a filtrate, which was concentrated under vacuum to obtain crude compound **B1-4** (1.2 g), which was directly used in the next step. ¹H NMR (400 MHz, CDCl₃) δ 8.29 (d, *J* = 2.8 Hz, 1H), 8.03 (d, *J* = 4.4 Hz, 1H), 7.19 - 7.21 (m, 1H), 7.12 - 7.16 (m, 1H), 3.71 - 3.74 (m, 2H), 3.53 - 3.55 (m, 2H), 2.73 - 2.80 (m, 2H), 2.60 (s, 1H), 1.86 - 1.89 (m, 2H), 1.67 - 1.70 (m, 1H), 1.25 - 1.44 (m, 2H); LC-MS: m/z = 193.2 (M+H)⁺.

### Step 3: Synthesis of intermediate B1

Compound **B1-4** (0.5 g, 2.6 mmol, 1.00 eq) was dissolved in dichloromethane (5.0 mL), and thionyl chloride (3.1 g, 26.0 mmol, 10.0 eq) was added at 0°C. After the addition was completed, the resulting mixture was heated up to 20°C and stirred for 15 hours. The reaction solution was concentrated under vacuum to obtain compound **B1** (520 mg, 2.31 mmol, 88.9% yield, 93.7% purity) as a light yellow solid. LC-MS: m/z =211.2 (M+H)⁺.

### Synthesis of general intermediate B2 (4-(4-(chloromethyl)piperidin-1-yl)pyridine)

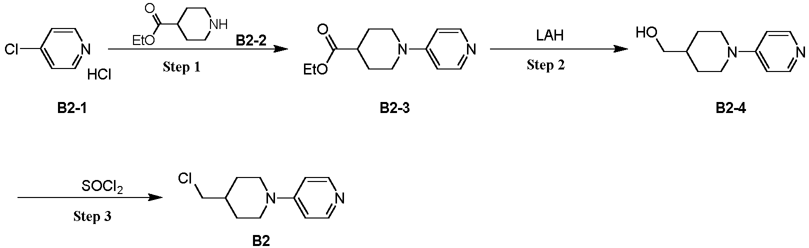

### Step 1: Synthesis of compound B2-3 (ethyl 1-(pyridin-4-yl)piperidine-4-carboxylate)

Compound **B2-1** (5.0 g, 33.3 mmol, 1.00 eq), compound **B2-2** (5.2 g, 33.3 mmol, 1.00 eq), and triethylamine (10.1 g, 100.0 mmol, 3.00 eq) were added to ethanol (50.0 mL), the mixture was heated up to 100°C and stirred for 16 hours. Then, water (50.0 mL) was added at 25°C, and the resulting mixture was extracted three times with ethyl acetate (50.0 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **B2-3** (2.7 g). ¹H NMR (400 MHz, CDCl₃) δ 8.25 (d, *J* = 5.2 Hz, 2H), 6.65 (d, *J* = 5.6 Hz, 2H), 4.15 (q, *J =* 7.2 Hz, 2H), 3.82 (d, *J* = 13.2 Hz, 2H), 2.96 (d, *J* = 11.6 Hz, 2H), 2.51 - 2.58 (m, 1H), 1.99 - 2.02 (m, 2H), 1.77 - 1.84 (m, 2H), 1.27 (d, *J* = 7.2 Hz, 3H); LC-MS: m/z =235.2 (M+H)⁺.

### Step 2: Synthesis of compound B2-4 ((1-(pyridin-4-yl)piperidin-4-yl)methanol)

Compound **B2-3** (1.7 g, 7.3 mmol, 1.00 eq) was dissolved in tetrahydrofuran (20.0 mL), and lithium aluminum hydride (302.0 mg, 8.0 mmol, 1.10 eq) was added at 0°C. After the addition was completed, the resulting mixture was heated up to 25°C and stirred for 4 hours. Aqueous sodium hydroxide solution (0.25 M, 2 mL) was added at 0°C, and the resulting mixture was stirred at 0°C for 0.5 hours. Tetrahydrofuran (10.0 mL) and anhydrous sodium sulfate (1.0 g) were added, and the resulting mixture was stirred for 5 minutes, and filtered to obtain a filtrate, which was concentrated under vacuum to obtain crude compound **B2-4** (1.1 g), which was directly used in the next step. ¹H NMR (400 MHz, CDCl₃) δ 8.25 (t, *J* = 1.6 Hz, 2H), 6.67 (t, *J* = 1.2 Hz, 2H), 3.92 (d, *J* = 16.0 Hz, 2H), 3.55 (d, *J* = 2.4 Hz, 2H), 2.84 - 2.90 (m, 2H), 1.78 - 1.87 (m, 3H), 1.30 - 1.37 (m, 2H); LC-MS: m/z = 193.0 (M+H)⁺.

### Step 3: Synthesis of compound B2

Compound **B2-4** (500.0 mg, 2.60 mmol, 1.00 eq) was dissolved in dichloromethane (5.0 mL), and thionyl chloride (2.5 g, 20.8 mmol, 8.00 eq) was added at 0°C. After the addition was completed, the resulting mixture was heated up to 20°C and stirred for 15 hours. The reaction solution was concentrated under vacuum to obtain compound **B2** (530 mg, 2.52 mmol, 96.7% yield) as a white solid. LC-MS: m/z =193.0 (M+H)⁺.

### Synthesis of general intermediate B3 (3-(4-(chloromethyl)phenyl)pyridine)

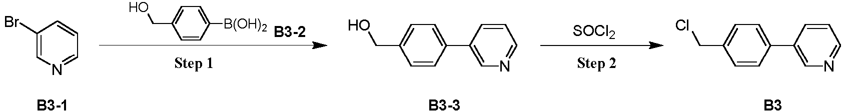

### Step 1: Synthesis of compound B3-3 ((4-(pyridin-3-yl)phenyl)methanol)

Compound **B3-1** (3.0 g, 18.9 mmol, 1.00 eq), compound **B3-2** (3.8 g, 24.6 mmol, 1.30 eq), sodium carbonate (14.8 g, 140.0 mmol, 7.40 eq), tetrakis(triphenylphosphine)palladium (2.2 g, 1.89 mmol, 0.10 eq) were added to a mixed solvent of toluene (15.0 mL), water (15.0 mL) and ethanol (3.0 mL) successively. The resulting mixture was purged three times with nitrogen, and reacted at 100°C for 12 hours under nitrogen atmosphere. The reaction solution was concentrated under vacuum to obtain a crude product, which was purified by reverse phase HPLC (0.1% NH₃•H₂O) to obtain compound **B3-3** (2.3 g). ¹H NMR (400 MHz, CDCl₃) δ 8.80 (s, 1H), 8.59 (d, *J* = 4.8 Hz, 1H), 7.87 - 7.90 (m, 1H), 7.59 (d, *J* = 8.0 Hz, 2H), 7.50 (d, *J* = 8.0 Hz, 2H), 7.36 - 7.39 (m, 1H), 4.78 (s, 2H), 1.96 - 2.06 (m, 1H); LC-MS: m/z = 186.1 (M+H)⁺.

### Step 2: Synthesis of intermediate B3

Compound **B3-3** (1.0 g, 5.4 mmol, 1.00 eq) was dissolved in dichloromethane (5.0 mL), and thionyl chloride (6.4 g, 53.9 mmol, 10.0 eq) was added at 0°C. After the addition was completed, the resulting mixture was heated up to 20°C and stirred for 15 hours. The reaction solution was concentrated under vacuum to obtain compound **B3** (1.1 g, 5.35 mmol, 99.1% yield) as a white solid. LC-MS: m/z = 204.0 (M+H)⁺.

### Synthesis of general intermediate B4 (4-(4-(chloromethyl)phenyl)pyridine)

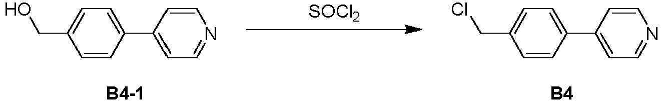

Compound **B4-1** (250.0 mg, 1.4 mmol, 1.00 eq) was dissolved in dichloromethane (5.0 mL), and thionyl chloride (802.0 mg, 6.8 mmol, 5.00 eq) was added at 0°C. After the addition was completed, the resulting mixture was heated up to 20°C and stirred for 15 hours. The reaction solution was concentrated under vacuum to obtain compound **B4** (260 mg, 1.28 mmol, 94.5% yield) as a white solid. LC-MS: m/z = 204.0 (M+H)⁺.

### Synthesis of general intermediate B5 (5-(4-(chloromethyl)phenyl)-2-methylpyridine)

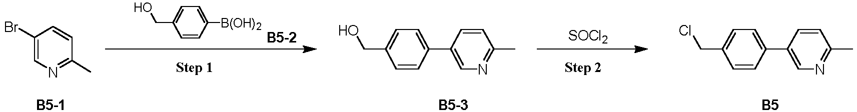

### Step 1: Synthesis of compound B5-3 ((4-(6-methylpyridin-3-yl)phenyl)methanol)

Compound **B5-1** (3.0 g, 17.4 mmol, 1.00 eq), compound **B5-2** (3.5 g, 22.6 mmol, 1.30 eq), sodium carbonate (13.6 g, 129.0 mmol, 7.40 eq), and tetrakis(triphenylphosphine)palladium (2.0 g, 1.74 mmol, 0.10 eq) were added to a mixed solvent of toluene (15.0 mL), water (15.0 mL) and ethanol (3.00 mL) successively. The resulting mixture was purged three times with nitrogen, and reacted at 100°C for 12 hours under nitrogen atmosphere. The reaction solution was concentrated under vacuum to obtain a crude product, which was purified by reverse phase HPLC (0.1% NH₃•H₂O) to obtain compound **B5-3** (3.0 g). ¹H NMR (400 MHz, CDCl₃) δ 8.59 - 8.63 (m, 1H), 7.76 - 7.78 (m, 1H), 7.54 (d, *J* = 8.0 Hz, 2H), 7.47 (t, *J* = 4.0 Hz, 2H), 7.22 (d, *J* = 8.0 Hz, 1H), 4.76 (s, 2H), 2.61 (s, 3H), 2.33 - 2.37 (m, 1H); LC-MS: m/z = 200.1 (M+H)⁺.

### Step 2: Synthesis of intermediate B5

Compound **B5-3** (0.5 g, 2.5 mmol, 1.00 eq) was dissolved in dichloromethane (5.0 mL), and thionyl chloride (1.5 g, 12.5 mmol, 5.00 eq) was added at 0°C. After the addition was completed, the resulting mixture was heated up to 20°C and stirred for 15 hours. The reaction solution was concentrated under vacuum to obtain compound **B5** (530 mg, 2.43 mmol, 97.0% yield) as a white solid. LC-MS: m/z = 218.1 (M+H)⁺.

### Synthesis of general intermediate B6 (2-(4-(chloromethyl)phenyl)-1-methyl-4-(trifluoromethyl)-1H-imidazole)

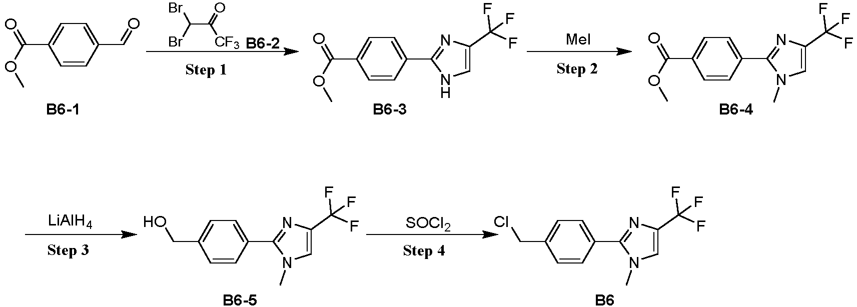

### Step 1: Synthesis of compound B6-3 (methyl 4-(4-(trifluoromethyl)-1H-imidazol-2-yl)benzoate)

Compound **B6-2** (7.2 g, 26.8 mmol, 1.10 eq) and sodium acetate (2.2 g, 27.3 mmol, 1.12 eq) were dissolved in water (8.0 mL), and stirred at 100°C for 1 hour. Compound **B6-1** (4.0 g, 24.4 mmol, 1.00 eq) dissolved in methanol (80.0 mL) and ammonia water (22.0 mL) was added at 25°C. After the addition was completed, the resulting mixture was stirred at 25°C for 40 minutes, heated up to 100°C and stirred for 2 hours. Then, water (60.0 mL) was added to quench the reaction, and the resulting mixture was extracted three times with ethyl acetate (80.0 mL). The organic layers were combined, washed twice with saturated brine (80.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 3/1) to obtain compound **B6-3** (4.5 g). ¹HNMR (400 MHz, CDCl₃) δ 13.4 (s, 1H), 8.10 - 8.12 (m, 2H), 8.04 - 8.06 (m, 2H), 7.99 (s, 1H), 3.87 (s, 3H); LC-MS: m/z = 271.0 (M+H)⁺.

### Step 2: Synthesis of compound B6-4 (methyl 4-(1-methyl-4-(trifluoromethyl)-1H-imidazol-2-yl)benzoate)

Compound **B6-3** (2.5 g, 9.3 mmol, 1.00 eq) was dissolved in tetrahydrofuran (20.0 mL), and sodium hydride (444.1 mg, 11.1 mmol, 1.20 eq) was added in portions at 0°C. After the addition was completed, the resulting mixture was reacted at 0°C for 30 minutes. Then, iodomethane (6.8 g, 48.1 mmol, 5.20 eq) was added. After the addition was completed, the resulting mixture was heated up to 20°C and stirred for 15 hours. Then, ice water (20.0 mL) was added at 0°C to quench the reaction, and the resulting mixture was extracted three times with ethyl acetate (30.0 mL). The organic layers were combined, washed twice with saturated brine (30.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 3/1) to obtain compound **B6-4** (1.1 g). ¹H NMR (400 MHz, CDCl₃) δ 8.07 (d, *J* = 8.4Hz, 2H), 7.99 (s, 1H), 7.90 (d, *J* = 8.4Hz, 2H), 3.89 (s, 3H), 3.84 (s, 3H); LC-MS: m/z =285.1 (M+H)⁺.

### Step 3: Synthesis of compound B6-5 ((4-(1-methyl-4-(trifluoromethyl)-1H-imidazol-2-yl)phenyl)methanol)

Compound **B6-4** (0.6 g, 2.1 mmol, 1.00 eq) was dissolved in tetrahydrofuran (6.0 mL), and lithium aluminum hydride (88.1 mg, 2.32 mmol, 1.10 eq) was added at 0°C. After the addition was completed, the resulting mixture was heated up to 25°C and stirred for 4 hours. Water (1.6 mL) and aqueous sodium hydroxide solution (1 M, 0.4 mL) were added at 0°C, and the resulting mixture was stirred at 0°C for 0.5 hours. Tetrahydrofuran (10.0 mL) and anhydrous sodium sulfate (1.0 g) were added, and the resulting mixture was filtered to obtain a filtrate. The filtrate was concentrated under vacuum to obtain crude product **B6-5** (505.4 mg), which was directly used in the next step. ¹H NMR (400 MHz, CDCl₃) δ 7.92 (s, 1H), 7.67 (d, *J =* 8.4 Hz, 2H), 7.45 (d, *J* = 8.0 Hz, 2H), 5.30 - 5.33 (m, 1H), 4.58 (d, *J* = 5.6 Hz, 2H), 3.78 (s, 3H); LC-MS: m/z = 257.1 (M+H)⁺.

### Step 4: Synthesis of intermediate B6

Compound **B6-5** (500.0 mg, 2.0 mmol, 1.00 eq) was dissolved in dichloromethane (5.0 mL), and thionyl chloride (1.9 g, 15.6 mmol, 8.00 eq) was added at 0°C. After the addition was completed, the resulting mixture was heated up to 20°C and stirred for 15 hours. The reaction solution was concentrated under vacuum to obtain compound **B6** (523 mg, 1.90 mmol, 97.6% yield) as a brown solid. LC-MS: m/z = 275.1 (M+H)⁺.

### Synthesis of general intermediate B7 (2-(4-(chloromethyl)cyclohexyl)pyrimidine)

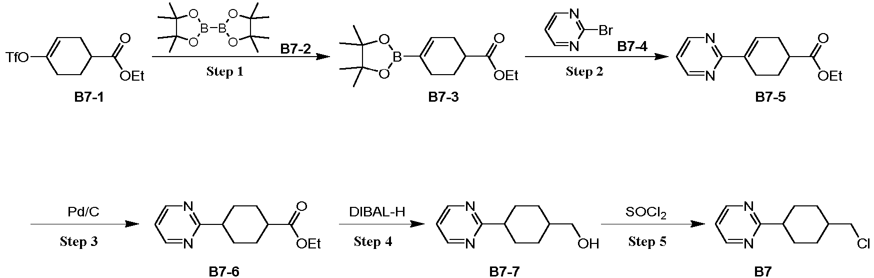

### Step 1: Synthesis of compound B7-3 (ethyl 4-(4,4,5,5-tetramethyl-1,3,2-dioxaborolan-2-yl)cyclohex-3-ene-1-carboxylate)

Compound **B7-1** (10.0 g, 33.1 mmol, 1.00 eq), compound **B7-2** (9.2 g, 36.4 mmol, 1.10 eq), [1,1'-bis(diphenylphosphino)ferrocene]palladium dichloride dichloromethane complex (1.4 g, 1.7 mmol, 0.05 eq), and potassium acetate (9.8 g, 99.3 mmol, 3.00 eq) were added to dioxane (80.0 mL) successively. The resulting mixture was purged three times with nitrogen, and stirred at 80°C for 2 hours under nitrogen atmosphere. Then, water (200.0 mL) was added, and the resulting mixture was extracted three times with ethyl acetate (70.0 mL). The organic layers were combined, washed twice with saturated brine (100.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 30/1) to obtain compound **B7-3** (4.5 g). ¹H NMR (400 MHz, CDCl₃) δ 6.55 (s, 1H), 4.12-4.17 (m, 2H), 2.50-2.51 (m, 1H), 2.26-2.35 (m, 3H), 2.01-2.05 (m, 2H), 1.58-1.63 (m, 1H), 1.27-1.28 (m, 15H); LC-MS: m/z = 281.1 (M+H)⁺.

### Step 2: Synthesis of compound B7-5 (ethyl 4-(pyrimidin-2-yl)cyclohex-3-ene-1-carboxylate)

Compound **B7-3** (4.9 g, 17.3 mmol, 1.10 *eq*)*,* compound **B7-4** (2.5 g, 15.7 mmol, 1.00 *eq*)*,* sodium carbonate (5.0 g, 47.2 mmol, 3.00 *eq*)*,* and tetrakis(triphenylphosphine)palladium (1.8 g, 1.6 mmol, 0.10 *eq)* were added to dioxane (50.0 mL) and water (12.5 mL) successively. The resulting mixture was purged three times with nitrogen, and stirred at 90°C for 20 hours under nitrogen atmosphere. Then, water (100.0 mL) was added, and the resulting mixture was extracted three times with ethyl acetate (100.0 mL). The organic layers were combined, washed three times with saturated brine (100.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 0/1) to obtain compound **B7-5** (3.1 g). ¹H NMR (400 MHz, CDCl₃) δ 8.67 (d, *J =* 4.80 Hz, 2H), 7.28-7.29 (m, 1H), 7.07-7.10 (m, 1H), 4.12-4.20 (m, 2H), 2.82-2.83 (m, 1H), 2.58-2.63 (m, 1H), 2.55-2.58 (m, 3H), 2.04-2.21 (m, 1H), 1.81-1.84 (m, 1H), 1.23-1.30 (m, 3H); LC-MS: m/z = 233.1 (M+H)⁺.

### Step 3: Synthesis of compound B7-6 (ethyl 4-(pyrimidin-2-yl)cyclohexane-1-carboxylate)

Compound **B7-5** (3.0 g, 13.1 mmol, 1.00 *eq*) and wet palladium on carbon (1.4 g, 1.3 mmol, 10.0% purity, 0.10 *eq*) were added to ethanol (90.0 mL) successively, and stirred at 25°C under hydrogen atmosphere for 16 hours. The reaction solution was filtered to obtain a filtrate. The filtrate was concentrated under vacuum to obtain a crude product, which was purified by column chromatography (eluent: petroleum ether/ethyl acetate = 1/0 - 0/1) to obtain compound **B7-6** (2.1 g). ¹H NMR (400 MHz, CDCl₃) δ 8.64-8.66 (m, 2H), 7.07-7.12 (m, 1H), 4.10-4.15 (m, 2H), 2.95-2.97 (m, 1H), 2.59-2.61 (m, 1H), 2.10-2.15 (m, 3H), 1.98-2.02 (m, 1H), 1.90-1.98 (m, 1H), 1.67-1.68 (m, 2H), 1.26-1.68 (m, 1H), 1.21-1.26 (m, 3H); LC-MS: m/z = 235.1 (M+H)⁺.

### Step 4: Synthesis of compound B7-7 ((4-(pyrimidin-2-yl)cyclohexyl)methanol)

Compound **B7-6** (500.0 mg, 2.1 mmol, 1.00 eq) was dissolved in tetrahydrofuran (20.0 mL), and diisobutylaluminum hydride (1.0 M, 3.20 eq) was added at -65°C. After the addition was completed, the resulting mixture was reacted at -65°C for 0.5 hours, heated up to 20°C and stirred for 1 hour. Then, saturated aqueous ammonium chloride solution (20.0 mL) was added at 0°C. After the addition was completed, the resulting mixture was heated to 20°C and stirred for 1 hour. The reaction solution was filtered to obtain a filtrate. The organic layer was separated, dried over anhydrous sodium sulfate, and filtered to obtain a filtrate. The filtrate was concentrated under vacuum to obtain crude compound **B7-7** (263.5 mg), which was directly used in the next step. ¹H NMR (400 MHz, CDCl₃) δ 8.69 (d, *J* = 4.80 Hz, 2H), 7.11-7.14 (m, 1H), 3.64 (d, *J* = 7.20 Hz, 2H), 3.04-3.08 (m, 1H), 2.05-2.08 (m, 2H), 1.83-1.85 (m, 3H), 1.64-1.69 (m, 4H), 1.14-1.26 (m, 1H); LC-MS: m/z = 193.0 (M+H)⁺.

### Step 5: Synthesis of intermediate B7

Compound **B7-7** (263.0 mg, 1.4 mmol, 1.00 eq) was dissolved in dichloromethane (5.0 mL), and thionyl chloride (814.0 mg, 6.8 mmol, 5.00 eq) was added at 0°C. After the addition was completed, the resulting mixture was heated up to 20°C and stirred for 15 hours. The reaction solution was concentrated under vacuum to obtain compound **B7** (300 mg, crude) as a light yellow oil. ¹H NMR (400 MHz, CDCl₃) δ 8.79-8.98 (m, 2H), 7.17-7.25 (m, 1H), 3.59-3.76 (m, 1H), 3.26-3.47 (m, 2H), 2.05-2.37 (m, 2H), 1.73-1.88 (m, 4H), 1.47-1.56 (m, 1H), 1.45-1.47 (m, 1H), 1.27-1.45 (m, 1H); LC-MS: m/z = 211.2 (M+H)⁺.

### Synthesis of general intermediate B8 ((4-(1-methyl-4-(trifluoromethyl)-1H-imidazol-2-yl)phenyl)methanamine)

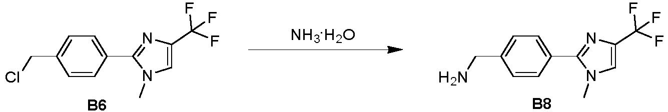

Intermediate **B6** (700.0 mg, 2.55 mmol, 1.00 *eq)* and aqueous ammonia (27.3 g, 233.0 mmol, 30% purity, 91.70 *eq)* were added to dioxane (15.0 mL), and the resulting mixture was reacted at 50°C for 12 hours in a sealed tube. The reaction solution was concentrated under vacuum to obtain a crude product, which was purified by reverse phase HPLC (0.1% NH₃•H₂O) to obtain **B8** (580 mg, 1.99 mmol, 78.0% yield, HCl) as a white solid. ¹H NMR (400 MHz, CDCl₃) δ 7.73 - 7.76 (m, 3H), 7.63 - 7.65 (m, 2H), 4.22 (s, 2H), 3.80 (s, 3H); LC-MS: m/z = 256.0 (M+H)⁺.

### Synthesis of general intermediate B9 (2-(5-(chloromethyl)thiophen-2-yl)-1-methyl-4-(trifluoromethyl)-1H-imidazole)

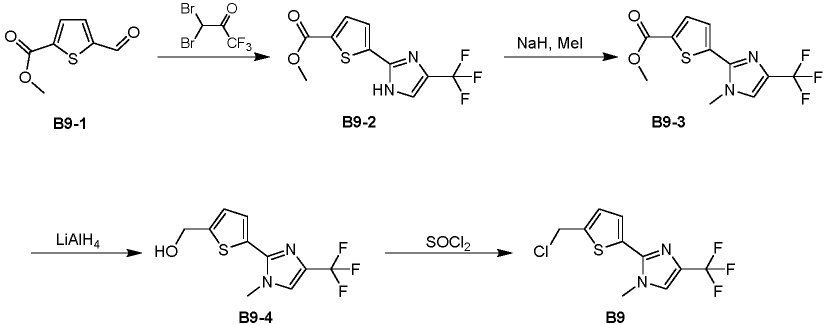

In accordance with the synthesis of intermediate **B6,** general intermediate **B9** was obtained by replacing **B6-1** with an equimolar amount of **B9-1,** while other experimental conditions remained unchanged. Compound **B9** (330.0 mg, crude) was finally obtained as a white solid. LC-MS: m/z = 281.0 (M+H)⁺.

### Synthesis of general intermediate B10 (2-(4-(chloromethyl)phenyl)pyridine)

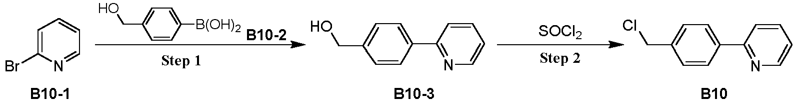

### Step 1: Synthesis of compound B10-3 ((4-(pyridin-2-yl)phenyl)methanol)

Compound **B10-1** (10.0 g, 63.3 mmol, 6.02 mL, 1.00 eq), compound **B10-2** (12.5 g, 82.3 mmol, 1.30 eq), tetrakis(triphenylphosphine)palladium (7.31 g, 6.33 mmol, 0.100 eq), and sodium carbonate (49.6 g, 468 mmol, 7.40 eq) were added to a mixed solvent of toluene (50.0 mL), water (50.0 mL) and ethanol (10.0 mL) successively. The resulting mixture was purged three times with nitrogen, and stirred at 100°C for 12 hours under nitrogen atmosphere. The reaction solution was filtered to obtain a filtrate. The filtrate was concentrated under vacuum to obtain a crude product, which was purified by reverse phase HPLC (0.1% NH₃•H₂O) to obtain compound **B10-3** (9.63 g). ¹H NMR (400 MHz, CDCl₃) δ 8.67 (d, J=4.4Hz, 1H), 7.75 - 7.95 (m, 2H), 7.70 - 7.74 (m, 2H), 7.42 - 7.44 (m, 2H), 7.22 - 7.25 (m, 1H), 4.73 (s, 2H), 2.70 (s, 1H); LC-MS: m/z = 186.2 (M+H)⁺.

### Step 2: Synthesis of intermediate B10

Compound **B10-3** (1.0 g, 5.40 mmol, 1.00 eq) was dissolved in dichloromethane (10.0 mL), and thionyl chloride (3.2 g, 27.0 mmol, 1.96 mL, 5.00 eq) was added at 0°C. After the addition was completed, the resulting mixture was heated up to 20°C and stirred for 12 hours. The reaction solution was concentrated under vacuum to obtain **B10** (1.1 g, crude) as a white solid. LC-MS: m/z = 204.1 (M+H)⁺.

### Synthesis of general intermediate B11 (2-(4-(chloromethyl)piperidin-1-yl)pyridine)

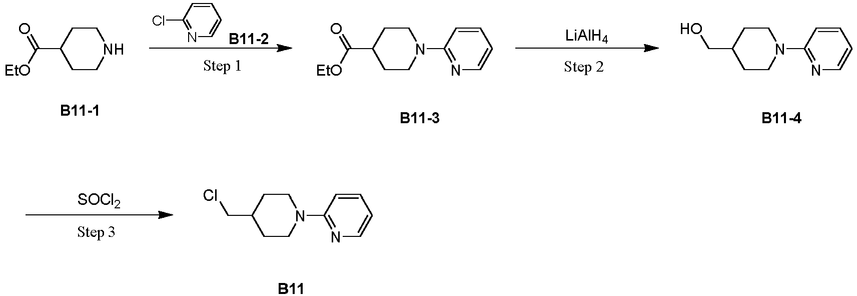

### Step 1: Synthesis of compound B11-3 (ethyl 1-(pyridin-2-yl)piperidine-4-carboxylate)

Compound **B11-1** (20.8 g, 132 mmol, 20.4 mL, 1.00 eq), compound **B12-2** (15.0 g, 132.0 mmol, 1.00 eq) and triethylamine (26.7 g, 264 mmol, 36.8 mL, 2.00 eq) were dissolved in DMSO (50.0 mL) and the resulting mixture was reacted at 120 to 150°C for 16 hours. The reaction solution was cooled down to quench the reaction. Water (50 ml) was added at room temperature to quench the reaction, and the resulting mixture was extracted with ethyl acetate (60.0 mL) three times. The organic layers were combined, washed twice with saturated brine (30.0 mL), dried over anhydrous sodium sulfate, and filtered to obtain a filtrate. The filtrate was concentrated under vacuum to obtain a crude product, which was purified by silica gel column chromatography (petroleum ether: ethyl acetate 2:1) to obtain compound B12-2 (6.0 g, 19.4% yield, 100% purity) as a yellow oil. ¹H NMR (400 MHz, CDCl₃) δ 8.17 - 8.19 (m, 1H), 7.44 - 7.48 (m, 1H), 6.66 (d, J=8.4Hz, 1H), 6.59 - 6.61 (m, 1H), 4.21 - 4.24 (m, 2H), 4.13 - 4.18 (m, 2H), 2.92 - 2.99 (m, 2H), 2.52 - 2.53 (m, 1H), 1.98 - 2.02 (m, 2H), 1.75 - 1.79 (m, 2H), 1.25 - 1.28 (m, 3H). **LC-MS:** m/z =235.2(M+H)⁺.

### Step 2: Synthesis of compound B11-4 ((1-(pyridin-2-yl)piperidin-4-yl)methanol)

Compound **B11-3** (6.00 g, 25.6 mmol) was dissolved in THF (60.0 mL), and lithium aluminum hydride (LiAlH₄, 1.07 g, 28.2 mmol, 1.10 eq) was carefully added at 0°C, and then the resulting mixture was stirred at room temperature for 16 hours. Additional lithium aluminum hydride (LiAlH₄, 486 mg, 12.8 mmol, 0.500 eq) was added and the resulting mixture was further stirred for 6 hours. H₂O (20.0 mL) was carefully added at 0°C to quench the reaction. The resulting mixture was extracted with ethyl acetate (60.0 mL × 3). The organic layers were combined, washed twice with saturated brine (20.0 mL), dried over anhydrous sodium sulfate, and filtered to obtain a filtrate. The filtrate was concentrated under vacuum to obtain a crude product, which was purified by silica gel column chromatography (petroleum ether: ethyl acetate 1:1) to obtain compound **B11-4** (1.20 g, yield 23.8%, purity 97.8%) as a yellow oil. **¹H NMR** (400 MHz, CDCl₃) δ 8.18 (d, J=3.6Hz, 1H), 7.43 - 7.48 (m, 1H), 6.67 (d, J=8.8Hz, 1H), 6.58 - 6.59 (m, 1H),4.33 (d, J=12.8Hz, 2H), 3.54 (d, J=6.0Hz, 2H), 2.81 - 2.88 (m, 2H), 1.77 - 1.86 (m, 2H), 1.73 - 1.76 (m, 1H), 1.55 (s, 1H), 1.28 - 1.35 (m, 2H). **LC-MS:** m/z =193.2(M+H)⁺.

### Step 3: Synthesis of intermediate B11

Compound **B11-4** (1.20 g, 6.24 mmol, 1.00 eq) was dissolved in dichloromethane (12.0 mL), and thionyl chloride (3.71 g, 31.2 mmol, 2.26 mL, 5.00 eq) was added at 0°C. After the addition was completed, the resulting mixture was heated up to 20°C and stirred for 12 hours. The reaction solution was concentrated under vacuum to obtain compound **B11** (1.57 g, crude) as a white solid. LC-MS: m/z = 211.1.

### Synthesis of general intermediate B12 (2-((1r,4r)-4-(chloromethyl)cyclohexyl)-1-methyl-4-(trifluoromethyl)-1H-imidazole)

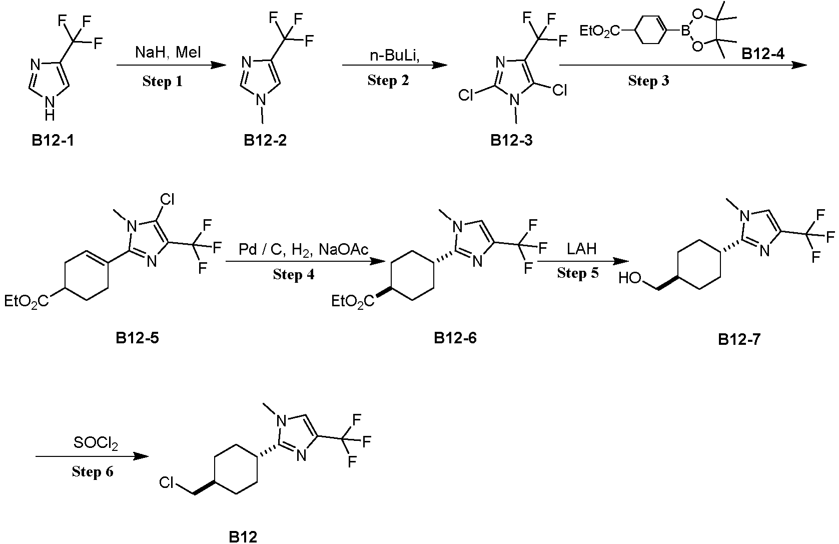

### Step 1: Synthesis of compound B12-2 (1-methyl-4-(trifluoromethyl)-1H-imidazole)

Compound **B12-1** (33.0 g, 242.0 mmol, 1.00 eq) was dissolved in tetrahydrofuran (330.0 mL), and sodium hydride (9.7 g, 242.0 mmol, 60.0% purity, 1.00 eq) was added in portions at 0°C. After the addition was completed, the resulting mixture was reacted at 0°C for 30 minutes. Then, iodomethane (34.4 g, 242.0 mmol, 15.1 mL, 1.00 eq) was added. After the addition was completed, the resulting mixture was heated up to 25°C and stirred for 5 hours. Saturated aqueous ammonium chloride solution (30.0 mL) was added at 0°C to quench the reaction. Water (30.0 mL) was added, and the resulting mixture was extracted three times with ethyl acetate (30.0 mL). The organic layers were combined, washed twice with saturated brine (30.0 mL), dried over anhydrous sodium sulfate, and filtered to obtain a filtrate. The filtrate was concentrated under vacuum to obtain a crude product, which was purified by reverse phase HPLC (0.1% NH₃•H₂O) to obtain compound **B12-2** (21.0 g). ¹H NMR (400 MHz, CDCl₃) δ 7.48 (s, 1H), 7.24 (s, 1H), 3.75 (s, 3H); LC-MS: m/z = 151.1 (M+H)⁺.

### Step 2: Synthesis of compound B12-3 (2,5-dichloro-1-methyl-4-(trifluoromethyl)-1H-imidazole)

Compound **B12-2** (16.8 g, 111.0 mmol, 1.00 eq) was dissolved in anhydrous tetrahydrofuran (370.0 mL), and n-butyl lithium (2.5 M, 44.7 mL, 1.00 eq) was added dropwise at -70°C. After the addition was completed, the resulting mixture was stirred at -70°C for 30 minutes. Then, hexachloroethane (15.9 g, 67.0 mmol, 0.60 eq) dissolved in anhydrous tetrahydrofuran (60.0 mL) was added dropwise to the reaction solution. After the addition was completed, the resulting mixture was reacted at -70°C for 1 hour, heated up to 20°C and stirred for 3 hours. Saturated aqueous ammonium chloride solution (200.0 mL) was added at 0°C to quench the reaction. Water (300.0 mL) was added, and the resulting mixture was extracted three times with ethyl acetate (150.0 mL). The organic layers were combined, washed twice with saturated brine (200.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 20/1) to obtain compound **B12-3** (12.0 g). LC-MS: m/z = 219.0 (M+H)⁺.

### Step 3: Synthesis of compound B12-5 (ethyl 4-(5-chloro-1-methyl-4-(trifluoromethyl)-1H-imidazol-2-yl)cyclohex-3-ene-1-carboxylate)

Compound **B12-3** (7.0 g, 31.9 mmol, 1.00 eq), compound **B12-4** (9.0 g, 31.9 mmol, 1.00 eq), potassium phosphate (20.3 g, 95.8 mmol, 3.00 eq), chloro(2-dicyclohexylphosphino-2',4',6'-triisopropyl-1,1'-biphenyl)[2-(2'-amino-1,1'-biphenyl)]palladium (II) (1.3 g, 1.6 mmol, 0.05 eq) and 2-dicyclohexylphosphino-2',4',6'-triisopropylbiphenyl (5.9 g, 12.4 mmol, 0.39 eq) were added to a mixed solvent of dioxane (70.0 mL) and water (10.0 mL) successively. The resulting mixture was purged three times with nitrogen, and stirred at 100°C for 5 hours under nitrogen atmosphere. The reaction solution was filtered to obtain a filtrate. The filtrate was concentrated under vacuum to obtain a crude product, which was purified by reverse phase HPLC (0.1% FA) to obtain compound **B12-5** (1.9 g). ¹H NMR (400 MHz, CDCl₃) δ 6.05 (d, *J =* 2.0 Hz, 1H), 4.18 (q, *J =* 7.2 Hz, 2H), 3.61 (s, 3H), 2.50 - 2.67 (m, 5H), 2.13 - 2.14 (m, 1H), 1.86 - 1.89 (m, 1H), 1.28 (t, *J =* 7.2 Hz, 3H); LC-MS: m/z = 337.2 (M+H)⁺.

### Step 4: Synthesis of compound B12-6 (ethyl (1R,4R)-4-(1-methyl-4-(trifluoromethyl)-1H-imidazol-2-yl)cyclohexane-1-formate)

Compound **B12-5** (1.9 g, 5.6 mmol, 1.00 eq), sodium acetate (925.0 mg, 11.2 mmol, 2.00 eq) and wet palladium on carbon (0.5 g, 1.4 mmol, 10.0% purity, 0.25 eq) were added to ethanol (30.0 mL) successively, and the resulting mixture was reacted at 50°C under hydrogen atmosphere (15 psi) for 5 hours. The reaction solution was filtered to obtain a filtrate, which was concentrated under vacuum to obtain a crude product. The crude product was purified by preparative HPLC (column: Waters Xbridge C18 150×50mm×10µm; mobile phase: [water (NH₄HCO₃)-ACN]; B%: 34%-64%, 10 min) to obtain compound **B12-6** (0.3 g). ¹H NMR (400 MHz, CDCl₃) δ 7.13 (s, 1H), 4.15(q, *J=* 6.8 Hz, 2H), 3.64(s, 3H), 2.60 - 2.65 (m, 1H), 2.41 - 2.44 (m, 1H), 2.12 - 2.15 (m, 2H), 1.97 - 1.98 (m, 2H), 1.77 - 1.80 (m, 2H), 1.54 - 1.59 (m, 2H), 1.27 (t, *J =* 8.8 Hz, 3H); LC-MS: m/z = 305.0 (M+H)⁺.

### Step 5: Synthesis of compound B12-7 (((1R,4R)-4-(1-methyl-4-(trifluoromethyl)-1H-imidazol-2-yl)cyclohexyl)methanol)

Compound **B12-6** (0.6 g, 2.0 mmol, 1.00 eq) was dissolved in tetrahydrofuran (10.0 mL), and lithium aluminum hydride (224.0 mg, 5.9 mmol, 3.00 eq) was added at 0°C. After the addition was completed, the resulting mixture was reacted at 0°C for 0.5 hours, heated up to 20°C and stirred for 12 hours. Then, sodium sulfate decahydrate (0.5 g) was added at 0°C. After the addition was completed, the resulting mixture was heated up to 20°C and stirred for 30 minutes, followed by the addition of tetrahydrofuran (10.0 mL), and filtered to obtain a filtrate. The organic layer was separated, dried over anhydrous sodium sulfate, and filtered to obtain a filtrate. The filtrate was concentrated under vacuum to obtain crude compound **B12-7** (0.5 g), which was directly used in the next step. ¹H NMR (400 MHz, CDCl₃) δ 7.12 (s, 1H), 3.63 (s, 3H), 3.52 (d, *J =* 5.6 Hz, 2H), 2.59 - 2.65 (m, 1H), 1.95 - 1.98 (m, 4H), 1.77 - 1.80 (m, 3H), 1.07 - 1.17 (m, 2H); LC-MS: m/z = 263.1 (M+H)⁺.

### Step 6: Synthesis of intermediate B12

Compound **B12-7** (500.0 mg, 1.9 mmol, 1.00 eq) was dissolved in dichloromethane (2.0 mL), and thionyl chloride (4.5 g, 38.1 mmol, 20.0 eq) was added at 0°C. After the addition was completed, the resulting mixture was heated up to 20°C and stirred for 24 hours. The reaction solution was concentrated under vacuum to obtain compound **B12** (550 mg, 1.36 mmol, 71.5% yield, HCl) as a light yellow solid. LC-MS: m/z = 281.0 (M+H)⁺.

### Synthesis of general intermediate B13 (4-(chloromethyl)-1-(1-methyl-4-(trifluoromethyl)-1H-imidazol-2-yl)piperidine hydrochloride)

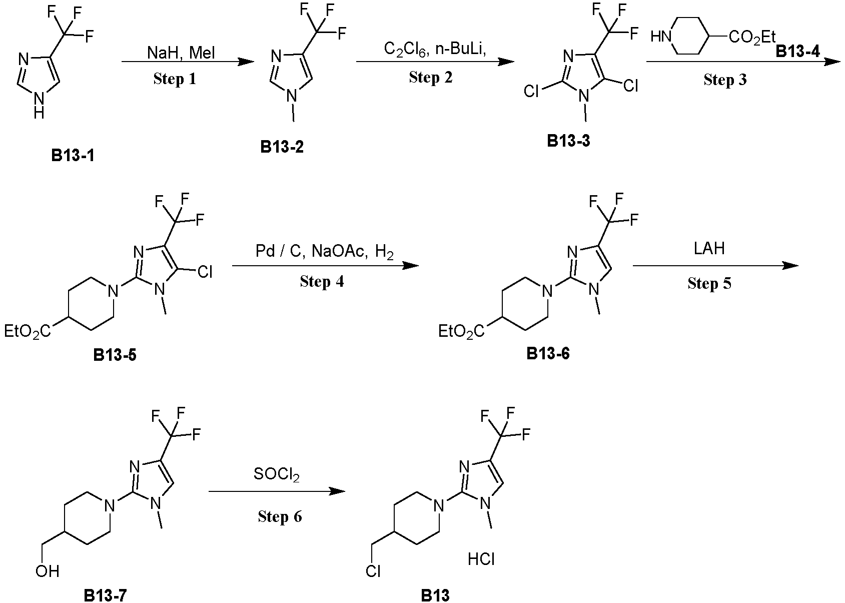

### Step 1: Synthesis of compound B13-2 (1-methyl-4-(trifluoromethyl)-1H-imidazole)

Compound **B13-1** (33.0 g, 242 mmol, 1.00 eq) was dissolved in tetrahydrofuran (330.0 mL), and sodium hydride (9.70 g, 242 mmol, 60.0% purity, 1.00 eq) was added in portions at 0°C. After the addition was completed, the resulting mixture was reacted at 0°C for 30 minutes. Then, iodomethane (34.4 g, 242 mmol, 15.1 mL, 1.00 eq) was added. After the addition was completed, the resulting mixture was heated up to 25°C and stirred for 4 hours. Then, ice water (200.0 mL) was added at 0°C to quench the reaction, and the resulting mixture was extracted three times with ethyl acetate (200.0 mL). The organic layers were combined, dried over anhydrous sodium sulfate, and filtered to obtain a filtrate. The filtrate was concentrated under vacuum to obtain a crude product, which was purified by reverse phase HPLC (0.1% NH₃•H₂O) to obtain compound **B13-2** (21.0 g). ¹H NMR (400 MHz, CDCl₃) δ 7.48 (s, 1H), 7.24 (s, 1H), 3.75 (s, 3H); LC-MS: m/z = 151.1 (M+H)⁺.

### Step 2: Synthesis of compound B13-3 (2,5-dichloro-1-methyl-4-(trifluoromethyl)-1H-imidazole)

Compound **B13-2** (16.8 g, 111 mmol, 1.00 eq) was dissolved in anhydrous tetrahydrofuran (370.0 mL), and n-butyl lithium (2.5 M, 44.7 mL, 1.00 eq) was added dropwise at -70°C. After the addition was completed, the resulting mixture was stirred at -70°C for 30 minutes. Then, hexachloroethane (15.9 g, 67.0 mmol, 0.60 eq) dissolved in anhydrous tetrahydrofuran (60.0 mL) was added dropwise to the reaction solution. After the addition was completed, the resulting mixture was reacted at -70°C for 1 hour, heated up to 20°C and stirred for 3 hours. Saturated aqueous ammonium chloride solution (200.0 mL) was added at 0°C to quench the reaction. Water (300.0 mL) was added, and the resulting mixture was extracted three times with ethyl acetate (150.0 mL). The organic layers were combined, washed twice with saturated brine (200.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 20/1) to obtain compound **B13-3** (12.0 g). LC-MS: m/z = 219.0 (M+H)⁺.

### Step 3: Synthesis of compound B13-5 (ethyl 1-(5-chloro-1-methyl-4-(trifluoromethyl)-1H-imidazol-2-yl)piperidine-4-carboxylate)

Compound **B13-3** (7.0 g, 31.9 mmol, 1.00 eq), compound **B13-4** (7.54 g, 47.9 mmol, 7.39 mL, 1.50 eq), N,N-diisopropylethylamine (16.5 g, 127 mmol, 22.2 mL, 4.00 eq) and sodium iodide (479 mg, 3.20 mmol, 0.100 eq) were added to N,N-dimethylformamide (70.0 mL) successively, and the resulting mixture was reacted at 130°C for 72 hours. Water (350 mL) was added, the resulting mixture was stirred for 5 minutes, and extracted three times with ethyl acetate (100 mL). The organic layers were combined, dried over anhydrous sodium sulfate, and filtered to obtain a filtrate. The filtrate was concentrated under vacuum to obtain a crude product, which was purified by reverse phase HPLC (0.1% FA) to obtain compound **B13-5** (2.2 g). ¹H NMR (400 MHz, CDCl₃) δ 4.17 (q, *J* = 7.2Hz, 2H), 3.45 (s, 3H), 3.24 - 3.28 (m, 2H), 2.90 - 2.97 (m, 2H), 2.40 - 2.45 (m, 1H), 2.02 - 2.06 (m, 2H), 1.85 - 1.88 (m, 2H), 1.28 (t, *J =* 8.0Hz, 2H); LC-MS: m/z = 340.1 (M+H)⁺.

### Step 4: Synthesis of compound B13-6 (ethyl 1-(1-methyl-4-(trifluoromethyl)-1H-imidazol-2-yl)piperidine-4-carboxylate)

Compound **B13-5** (2.2 g, 6.48 mmol, 1.00 eq), sodium acetate (1.06 g, 12.9 mmol, 2.00 eq) and wet palladium on carbon (0.5 g, 1.94 mmol, 10.0% purity, 0.300 eq) were added to ethanol (40.0 mL) successively, and the resulting mixture was reacted at 50°C under hydrogen atmosphere (15 psi) for 36 hours. The reaction solution was filtered to obtain a filtrate, which was concentrated under vacuum to obtain compound **B13-6** (1.9 g). ¹H NMR (400 MHz, CDCl₃) δ 7.02 (s, 1H), 4.15 (q, *J* = 7.2Hz, 2H), 3.52 (s, 3H), 3.25 - 3.29 (m, 2H), 2.92 - 2.98 (m, 2H), 2.40 - 2.45 (m, 1H), 2.01 - 2.05 (m, 2H), 1.85 - 1.89 (m, 2H), 1.28 (t, *J =* 6.8Hz, 3H); LC-MS: m/z = 306.1 (M+H)⁺.

### Step 5: Synthesis of compound B13-7 ((1-(1-methyl-4-(trifluoromethyl)-1H-imidazol-2-yl)piperidin-4-yl)methanol)

Compound **B13-6** (1.2 g, 3.93 mmol, 1.00 eq) was dissolved in tetrahydrofuran (20.0 mL), and lithium aluminum hydride (447 mg, 11.8 mmol, 3.00 eq) was added at 0°C. After the addition was completed, the resulting mixture was reacted at 0°C for 0.5 hours, heated up to 20°C and stirred for 12 hours. Then, sodium sulfate decahydrate (0.5 g) was added at 0°C. After the addition was completed, the resulting mixture was heated up to 20°C and stirred for 30 minutes, followed by the addition of tetrahydrofuran (10.0 mL), and filtered to obtain a filtrate. The organic layer was separated, dried over anhydrous sodium sulfate, and filtered to obtain a filtrate. The filtrate was concentrated under vacuum to obtain crude compound **B13-7** (1.0 g), which was directly used in the next step. ¹H NMR (400 MHz, CDCl₃) δ 7.01 (s, 1H), 3.57 (d, *J =* 6.4Hz, 2H), 3.51 (s, 3H), 3.27 - 3.30 (m, 2H), 2.90 - 2.96 (m, 2H), 1.84 - 1.87 (m, 2H), 1.65 - 1.70 (m, 1H), 1.39 - 1.42(m, 2H); LC-MS: m/z = 264.1 (M+H)⁺.

### Step 6: Synthesis of intermediate B13

Compound **B13-7** (1.0 g, 3.80 mmol, 1.00 eq) was dissolved in dichloromethane (2.0 mL), and thionyl chloride (4.5 g, 38.1 mmol, 20.0 eq) was added at 0°C. After the addition was completed, the resulting mixture was heated up to 20°C and stirred for 24 hours. The reaction solution was concentrated under vacuum to obtain compound **B13** (1.1 g, 3.46 mmol, 91.0% yield, HCl) as a brown solid. LC-MS: m/z = 282.1 (M+H)⁺.

### Synthesis of general intermediate B14 (2-(5-(chloromethyl)furan-2-yl)-1-methyl-4-(trifluoromethyl)-1H-imidazole)

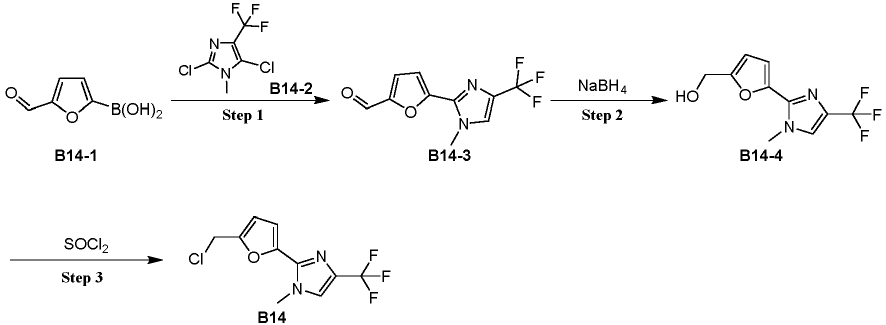

### Step 1: Synthesis of compound B14-3 (5-(1-methyl-4-(trifluoromethyl)-1H-imidazol-2-yl)furan-2-carbaldehyde)

Compound **B14-2** (400 mg, 1.83 mmol, 1.00 eq), **B14-1** (255 mg, 1.83 mmol, 1.00 eq), methanesulfonato (2-dicyclohexylphosphino-2',6'-dimethoxy-1,1'-biphenyl)(2'-amino-1,1'-biphenyl-2-yl)palladium (II) (71.2 mg, 91.3 µmol, 0.0500 eq), and potassium phosphate (1.16 g, 5.48 mmol, 3.00 eq) were added to n-butanol (3.0 mL) and water (0.5 mL) successively. The resulting mixture was purged three times with nitrogen, and reacted at 60°C for 12 hours under nitrogen atmosphere. The reaction solution was concentrated under vacuum to obtain a crude product, which was purified by preparative HPLC (column: Phenomenex C18 75×30mm×3µm; mobile phase: [water (FA)-ACN]; B%: 22%-52%, 7 min) to obtain compound **B14-3** (60.0 mg). ¹H NMR (400 MHz, CDCl₃) δ 9.70 (s, 1H), 7.37 (d, *J=* 3.6Hz, 1H), 7.32 (s, 1H), 7.22 (d, *J =* 3.6 Hz, 1H), 4.07 (s, 3H); LC-MS: m/z = 245.2 (M+H)+.

### Step 2: Synthesis of compound B14-4 ((5-(1-methyl-4-(trifluoromethyl)-1H-imidazol-2-yl)furan-2-yl)methanol)

Compound **B14-3** (60.0 mg, 245 µmol, 1.00 eq) was dissolved in methanol (1.0 mL), followed by the addition of sodium borohydride (90.0 mg, 2.38 mmol, 9.68 eq), the resulting mixture was stirred at 15°C for 30 minutes. Then saturated aqueous ammonium chloride solution (10.0 mL) was added at 0°C and water (20.0 mL) was added, and the resulting mixture was extracted three times with ethyl acetate (10.0 mL). The organic layers were combined, dried over anhydrous sodium sulfate, and filtered to obtain a filtrate, which was concentrated under vacuum to obtain compound **B14-4** (50.0 mg). ¹H NMR (400 MHz, CDCl₃) δ 7.25 (s, 1H), 6.92 (d, *J=* 3.2Hz, 1H), 6.42 (d, *J =* 3.2Hz, 1H), 4.70 (s, 2H), 3.93 (s, 3H); LC-MS: m/z = 247.1 (M+H)⁺.

### Step 3: Synthesis of intermediate B14

Compound **B14-4** (50.0 mg, 203 µmol, 1.00 eq) was dissolved in dichloromethane (2.0 mL), and thionyl chloride (1.2 g, 10.1 mmol, 50.0 eq) was added at 0°C. After the addition was completed, the resulting mixture was heated up to 25°C and reacted for 24 hours. The reaction solution was concentrated under vacuum to obtain compound **B14** (45 mg, crude) as a light yellow oil. LC-MS: m/z = 265.1 (M+H)⁺.

### Synthesis of general intermediate B15 (8-(4-(chloromethyl)phenyl)imidazo[1,2-a]pyrazine)

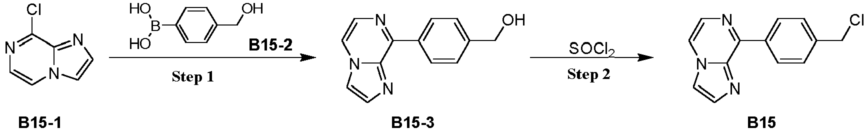

### Step 1: Synthesis of compound B15-3 ((4-(imidazo[1,2-a]pyrazin-8-yl)phenyl)methanol)

Compound **B15-1** (9.6 g, 62.5 mmol, 1.00 *eq),* compound **B15-2** (12.4 g, 81.3 mmol, *1.30 eq),* tetrakis(triphenylphosphine)palladium (7.2 g, 6.25 mmol, 0.10 *eq),* and sodium carbonate (49.0 g, 463 mmol, 7.40 *eq)* were added to a mixed solvent of toluene (48.0 mL), water (48.0 mL) and ethanol (9.6 mL) successively. The resulting mixture was purged three times with nitrogen, and stirred at 100°C for 12 hours under nitrogen atmosphere. The reaction solution was filtered to obtain a filtrate, which was concentrated under vacuum to obtain a crude product. The crude product was purified by reverse phase HPLC (0.1% NH₃•H₂O) to obtain compound **B15-3** (12.7 g). LC-MS: m/z = 226.2 (M+H)⁺.

### Step 2: Synthesis of intermediate B15

Compound **B15-3** (6.0 g, 26.6 mmol, 1.00 *eq)* was dissolved in dichloromethane (10.0 mL), and thionyl chloride (15.9 g, 133 mmol, 5.00 *eq)* was added at 0°C. After the addition was completed, the resulting mixture was heated up to 20°C and stirred for 12 hours. The reaction solution was concentrated under vacuum to obtain compound B15 (7.3 g, 26.2 mmol, 98.2% yield, HCl) as an off-white solid. LC-MS: m/z = 244.1 (M+H)⁺.

### Synthesis of general intermediate B16 ((4-(5-(trifluoromethyl)pyridin-2-yl)bicyclo[2.2.2]octan-1-yl)methyl-4-methylbenzenesulfonate)

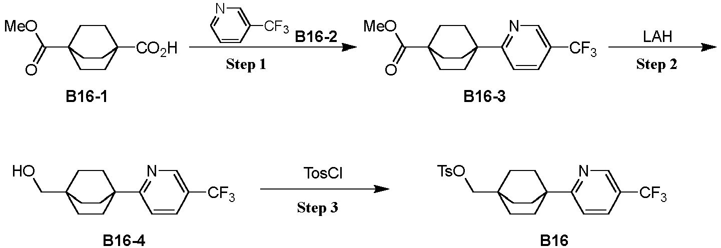

### Step 1: Synthesis of compound B16-3 (methyl 4-(5-(trifluoromethyl)pyridin-2-yl)bicyclo[2.2.2]octane-1-carboxylate)

Compound **B16-1** (25.0 g, 118 mmol, 1.00 eq), compound **B16-2** (20.8 g, 141 mmol, 1.20 eq), ammonium persulfate (26.9 g, 118 mmol, 25.6 mL, 1.00 eq) and silver nitrate (4.01 g, 23.6 mmol, 0.20 eq) were added to a mixed solvent of dichloromethane (750.0 mL) and water (750.0 mL) successively. The resulting mixture was reacted at 20°C for 16 hours, followed by the addition of dichloromethane (250.0 mL), and filtered to obtain a filtrate, which was washed with water (100.0 mL) three times. The organic layer was dried over anhydrous sodium sulfate, and filtered to obtain a filtrate, which was concentrated under vacuum to obtain a crude product. The crude product was purified by preparative HPLC (alkaline conditions, NH₃•H₂O/MeCN/H₂O) to obtain compound **B16-3** (7.5 g). ¹H NMR (400 MHz, CDCl₃) δ 8.81 (s, , 1H), 7.85 (dd, *J*₁ = 4.0Hz, *J*₂ = 4.0Hz, 1H), 7.37 (d, *J =* 4.0Hz, 1H), 3.69 (s, 3H), 1.96 (s, 12H).

### Step 2: Synthesis of compound B16-4 ((4-(5-(trifluoromethyl)pyridin-2-yl)bicyclo[2.2.2]octan-1-yl)methanol)

Compound **B16-3** (2.5 g, 7.98 mmol, 1.00 eq) was dissolved in tetrahydrofuran (50.0 mL), and lithium aluminum hydride (908 mg, 23.9 mmol, 3.00 eq) was slowly added at 0°C. After the addition was completed, the resulting mixture was heated up to 20°C and stirred for 3 hours. Then, ice water (50.0 mL) was added at 0°C and the resulting mixture was stirred for 10 minutes, and extracted three times with ethyl acetate (50.0 mL). The organic layers were combined, dried over anhydrous sodium sulfate, and filtered to obtain a filtrate, which was concentrated under vacuum to obtain compound **B16-4** (2.3 g). LC-MS: m/z = 286.1 (M+H)⁺.

### Step 3: Synthesis of intermediate B16

Compound **B16-4** (1.6 g, 5.61 mmol, 1.00 eq) and *p*-toluenesulfonyl chloride (1.28 g, 6.73 mmol, 1.20 eq) were added to pyridine (25.0 mL), and the resulting mixture was reacted at 20°C for 12 hours. The reaction solution was concentrated under vacuum. Water (10.0 mL) was added, and the resulting mixture was stirred for 5 minutes, and extracted three times with dichloromethane (5.0 mL). The organic layers were combined, dried over anhydrous sodium sulfate, and filtered to obtain a filtrate, which was concentrated under vacuum to obtain a crude product. The crude product was purified by column chromatography (eluent: petroleum ether/ethyl acetate = 1/0 - 3/1) to obtain compound **B16** (1.5 g, 3.38 mmol, 60.2% yield, 98.9% purity) as a white solid. ¹H NMR (400 MHz, CDCl₃) δ 8.79 (s, 1H), 7.84-7.79 (m, 3H), 7.38 (m, 3H), 3.72 (s, 2H), 2.47 (s, 3H), 1.93-1.89 (m, 6H), 1.57-1.54 (m, 6H); LC-MS: m/z = 440.2 (M+H)⁺.

### Synthesis of general intermediate B17 (1-(4-(chloromethyl)phenyl)-5-methyl-3-(trifluoromethyl)-1H-pyrazole)

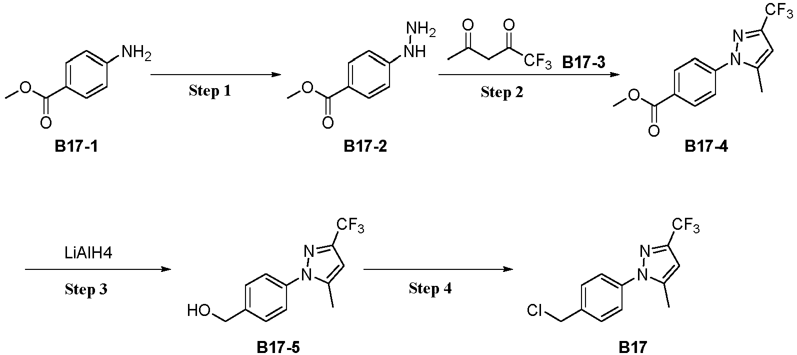

### Step 1: Synthesis of compound B17-2 (methyl 4-hydrazinylbenzoate)

Compound **B17-1** (15.0 g, 90.32 mmol, 1.0 eq) was dissolved in 37% aqueous hydrochloric acid solution (25.0 mL), followed by the addition of sodium nitrite (6.2 g, 90.32 mmol, 1.0 eq) dissolved in water (10.0 mL) at 0°C. After the addition was completed, the resulting mixture was stirred for 10 minutes. Then, tin chloride (85.6 g, 451.62 mmol, 5.0 eq) dissolved in 37% aqueous hydrochloric acid solution (75.0 mL) was added dropwise at 0°C. After the addition was completed, the resulting mixture was heated up to 25°C and stirred for 2 hours. The reaction solution was filtered to obtain a filter cake, which was washed three times with ethyl acetate (50.0 mL), and concentrated under vacuum to obtain compound **B17-2** (16.5 g). LC-MS: m/z = 167.1 (M+H)⁺.

### Step 2: Synthesis of compound B17-4 (methyl 4-(5-methyl-3-(trifluoromethyl)-1H-pyrazol-1-yl)benzoate)

Compound **B17-2** (5 g, 24.75 mmol, 1.0 eq) and compound **B17-3** (3.8 g, 24.75 mmol, 1.0 eq) were added to hexafluoroisopropanol (25.0 mL). Triethylamine (5.0 g, 49.50 mmol, 2.0 *eq)* dissolved in hexafluoroisopropanol (25.0 mL) was added dropwise at 0°C. After the addition was completed, the resulting mixture was heated up to 25°C and stirred for 1 hour. Then, water (10 mL) was added, and the resulting mixture was extracted three times with dichloromethane (200.0 mL). The organic layers were combined, washed twice with saturated brine (200.0 mL), dried over anhydrous sodium sulfate, and filtered to obtain a filtrate, which was concentrated under vacuum to obtain a crude product. The crude product was purified by column chromatography (eluent: petroleum ether/ethyl acetate = 1/0 - 1/1) to obtain compound **B17-4** (5.3 g). LC-MS: m/z = 285.0 (M+H)⁺.

### Step 3: Synthesis of compound B17-5 ((4-(5-methyl-3-(trifluoromethyl)-1H-pyrazol-1-yl)phenyl)methanol)

Compound **B17-4** (5.3 g, 18.65 mmol, 1.0 *eq)* was dissolved in tetrahydrofuran (100.0 mL), and lithium aluminum hydride (1.9 g, 46.64 mmol, 2.5 *eq)* was slowly added at 0°C. After the addition was completed, the resulting mixture was heated up to 20°C and stirred for 6 hours. Then, ice water (50.0 mL) was added at 0°C, and the resulting mixture was stirred for 10 minutes, and extracted three times with ethyl acetate (50.0 mL). The organic layers were combined, dried over anhydrous sodium sulfate, and filtered to obtain a filtrate, which was concentrated under vacuum to obtain a crude product. The crude product was purified by column chromatography (eluent: petroleum ether/ethyl acetate = 1/0 - 1/2) to obtain compound **B17-5** (3.6 g). LC-MS: m/z = 257.1 (M+H)⁺.

### Step 4: Synthesis of intermediate B17

Compound **B17-5** (1.0 g, 3.91 mmol, 1.0 *eq)* was dissolved in dichloroethane (25.0 mL), and thionyl chloride (1.4 g, 11.73 mmol, 5.2 *eq)* was added at 0°C. After the addition was completed, the resulting mixture was heated up to 50°C and stirred for 2 hours. The reaction solution was concentrated under vacuum to obtain compound **B17** (1.0 g, 3.64 mmol, 93.1% yield, 96.9% purity) as a white solid. ¹H NMR (400 MHz, CDCl₃) δ 7.53 (d, *J =* 8.7Hz, 2H), 7.46 (d, *J =* 8.7Hz, 2H), 6.47 (s, 1H), 4.64 (s, 2H), 2.37 (s, 3H); LC-MS: m/z = 275.1 (M+H)⁺.

### General intermediate BB1 (2-chloro-5-methoxy-N-methylpyrimidin-4-amine)

Intermediate **BB1** was purchased from Leyan Reagents.

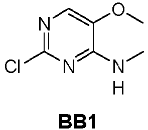

### General intermediate BB2 (2-chloro-5-methoxypyrimidin-4-amine)

Intermediate **BB2** was purchased from Leyan Reagents.

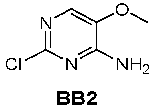

### General intermediate BB3 (2-chloro-5-isopropoxy-N-methylpyrimidin-4-amine)

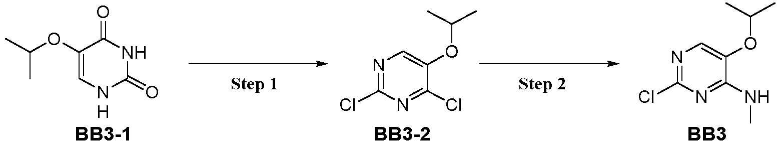

### Step 1: Synthesis of compound BB3-2 (2,4-dichloro-5-isopropoxypyrimidine)

2, 4-Dichloro-5-isopropylpyrimidine (compound **BB3-1,** 0.5 g, 2.41 mmol, 1.00 eq) was added to phosphorus oxychloride (5.0 mL), and refluxed under nitrogen atmosphere for 4 hours. The reaction solution was cooled to room temperature, and concentrated under vacuum to remove phosphorus oxychloride. Ice water (100.0 mL) was added, and the resulting mixture was extracted three times with ethyl acetate (100.0 mL). The organic layers were combined, dried over anhydrous sodium sulfate, and concentrated under vacuum to obtain compound **BB3-2** (551.0 mg, crude) as a light yellow oil. LC-MS: m/z = 207.1 (M+H)⁺.

### Step 2: Synthesis of intermediate BB3

Compound **BB3-2** (100 mg, 0.48 mmol, 1.00 eq), methylamine hydrochloride (97.8 mg, 1.45 mmol, 3.00 eq) and cesium carbonate (472.4 mg, 1.45 mmol, 3.00 eq) were added to N,N-dimethylformamide (2.0 mL) successively, and the resulting mixture was stirred at 60°C for 6 hours. Ethyl acetate (15.0 mL) was added, the resulting mixture was stirred for 5 minutes, and filtered to obtain a filtrate. The filtrate was concentrated under vacuum to obtain a crude product, which was purified by column chromatography (eluent: petroleum ether/ethyl acetate = 1/0 - 0/1) to obtain intermediate **BB3** (38.8 mg, 0.19 mmol, 40.1% yield). LC-MS: m/z = 202.1 (M+H)⁺.

### General intermediate BB4 (2-chloro-5-isopropoxypyrimidin-4-amine)

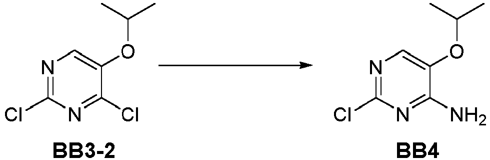

Compound **BB3-2** (100 mg, 0.48 mmol, 1.00 eq) and aqueous ammonia (1.68 g, 48.0 mmol, 30% purity, 100.0 *eq)* were added to dioxane (3.0 mL), and the resulting mixture was reacted at 50°C in a sealed tube for 12 hours. The reaction solution was concentrated under vacuum to obtain a crude product, which was purified by reverse phase HPLC (0.1% NH₃•H₂O) to obtain intermediate **BB4** (27.3 mg, 0.15 mmol, 30.3% yield) as a white solid. LC-MS: m/z = 188.2 (M+H)⁺.

### General intermediate BB12 (2-chloro-5-(trifluoromethyl)pyrimidin-4-amine)

Intermediate **BB12** was purchased from Bide Pharmatech.

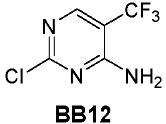

### Synthesis of general intermediate BB13 (2-chlorofuro[3,2-d]pyrimidin-4-amine)

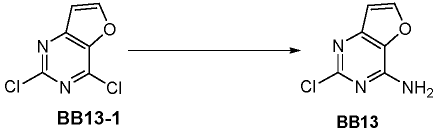

2,4-Dichlorofuro[3,2-d]pyrimidine (compound **BB13-1,** 5.0 g, 26.46 mmol, 1.00 *eq)* and aqueous ammonia (30.9 g, 264.6 mmol, 30% purity, 100.0 *eq)* were added to dioxane (100.0 mL), and the resulting mixture was reacted at 50°C in a sealed tube for 12 hours. The reaction solution was concentrated under vacuum to obtain a crude product, which was purified by reverse phase HPLC (0.1% NH₃•H₂O) to obtain compound **BB13** (3.2 g, 18.87 mmol, 71.3% yield) as a white solid. LC-MS: m/z = 170.1 (M+H)⁺.

### Synthesis of general intermediate BB14 (2-chloropyrido[3,2-d]pyrimidin-4-amine)

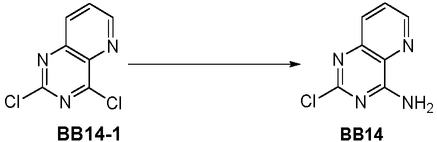

In accordance with the same steps of intermediate **BB13,** intermediate **BB14** (1.1 g, 6.09 mmol, 40.6% yield) was obtained using 2,4-dichloropyrido[3,2-d]pyrimidine **(BB14-1,** 3.0 g, 15.0 mmol, 1.00 *eq)* as starting material. LC-MS: m/z = 181.0 (M+H)⁺.

### General intermediate BB15 (2,5-dichloropyrimidin-4-amine)

Intermediate BB15 was purchased from Bide Pharmatech.

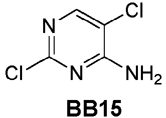

### Synthesis of general intermediate BB16 (2-chloro-5-fluoro-N-methylpyrimidin-4-amine)

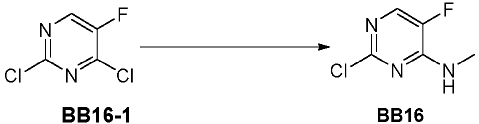

2,4-Dichloro-5-fluoropyrimidine (compound **BB16-1,** 3.0 g, 18.07 mmol, 1.00 *eq),* methylamine hydrochloride (3.66 g, 54.23 mmol, 3.00 *eq)* and cesium carbonate (23.55 g, 72.28 mmol, 4.00 *eq)* were added to N,N-dimethylformamide (500.0 mL) successively, and the resulting mixture was stirred at 60°C for 6 hours. Ethyl acetate (1000.0 mL) was added, the resulting mixture was stirred for 5 minutes, and filtered to obtain a filtrate. The filtrate was concentrated under vacuum to obtain a crude product, which was purified by column chromatography (eluent: petroleum ether/ethyl acetate = 1/0 - 0/1) to obtain compound **BB16** (1.1 g, 6.81 mmol, 37.7% yield). LC-MS: m/z = 162.0 (M+H)⁺.

### Synthesis of general intermediate BB16-D3 (2-chloro-5-fluoro-N-(methyl-D3)pyrimidin-4-amine)

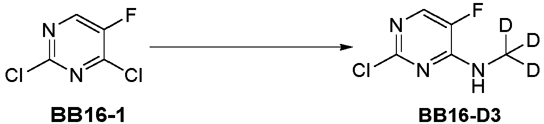

In accordance with the same steps of intermediate **BB16,** intermediate **BB16-D3** (1.47 g, 8.93 mmol, 49.4% yield) was obtained using 2,4-dichloro-5-fluoropyrimidine **(BB16-1,** 3.0 g, 18.07 mmol, 1.00 *eq)* as starting material. LC-MS: m/z = 165.1 (M+H)⁺.

### General intermediate BB17 (4-amino-2-chloropyrimidine-5-carbonitrile)

Intermediate **BB17** was purchased from Bide Pharmatech.

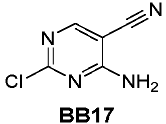

### Synthesis of general intermediate BB18 (2,4-dichloro-5-((trimethylsilyl)ethynyl)pyrimidine)

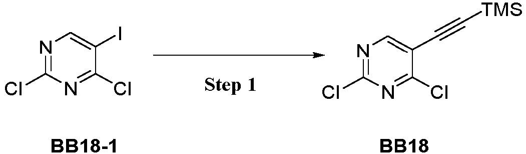

Compound 2,4-dichloro-5-iodopyrimidine **(BB18-1,** 1.0 g, 3.6 mmol, 1.00 eq), bis(triphenylphosphine)palladium dichloride (256 mg, 0.3 mmol, 0.1 eq), cuprous iodide (139 mg, 0.7 mmol, 0.2 eq), trimethylethynylsilane (700 mg, 7.2 mmol, 2.0 eq) and triethylamine (1.1 g, 10.9 mmol, 3.0 eq) were added to tetrahydrofuran (15 mL) successively. The reaction mixture was stirred at 40°C for 3 hours, and concentrated under vacuum to obtain a crude product, which was purified by column chromatography (eluent: petroleum ether/ethyl acetate = 1/0 - 1/1) to obtain compound **BB18** (590 mg, 2.4 mmol) as a white solid. ¹HNMR (400MHz, DMSO) δ 8.97 - 8.89 (m, 1H), 0.28 (s, 9H).

### Synthesis of general intermediate BB19 (2-chloro-5-(difluoromethoxy)pyrimidin-4-amine)

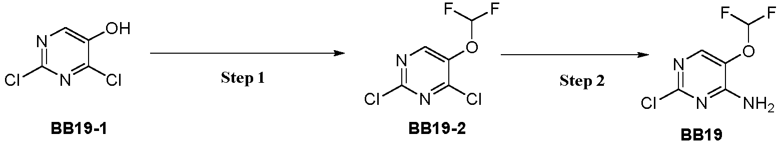

### Step 1: Synthesis of compound BB19-2 (2,4-dichloro-5-(difluoromethoxy)pyrimidine)

2,4-Dichloropyrimidine-5-ol (compound **BB19-1,** 3.40 g, 20.6 mmol, 1.00 eq) and potassium hydroxide (13.3 g, 237 mmol, 11.5 *eq)* were added to acetonitrile (60.0 mL) and water (60.0 mL) successively, and diethyl bromofluoromethylphosphonate (9.35 g, 35.0 mmol, 1.70 *eq)* was added dropwise. After the addition was completed, the resulting mixture was stirred at 25°C for 1.5 hours. Saturated aqueous citric acid solution (10.0 mL) was added to quench the reaction, and water (10.0 mL) was added. The resulting mixture was extracted three times with ethyl acetate (40 mL). The organic layers were combined, washed twice with saturated brine (20.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 2/1) to obtain intermediate **BB19-2** (3.70 g, 17.2 mmol, 83.5% yield) as a light yellow oil. ¹H NMR (400MHz, CDCl₃) δ 8.53 (s, 1H), 6.84 - 6.49 (t, *J* =70.8 Hz, 1H); LC-MS: m/z = 215.0 (M+H)⁺.

### Step 2: Synthesis of intermediate BB19

In accordance with the same steps of intermediate **BB13,** intermediate **BB19** (298.5 mg, 1.53 mmol, 65.5% yield) was obtained using compound **BB19-2** (500.0 mg, 2.33 mmol, 1.00 *eq)* as starting material. LC-MS: m/z = 196.1 (M+H)⁺.

### General intermediate BB21 (4-(2,4-dichloropyrimidin-5-yl)morpholine)

Intermediate **BB21** was purchased from Bide Pharmatech.

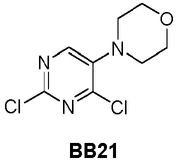

### Synthesis of general intermediate BB22 (2,4-dichloro-5-(4-methylpiperazin-1-yl)pyrimidine)

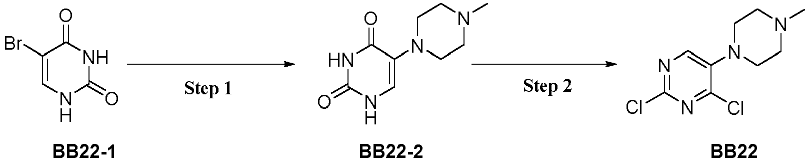

### Step 1: Synthesis of compound BB22-2 (5-(4-methylpiperazin-1-yl)pyrimidine-2,4(1H,3H)-dione)

5-Bromouracil **(BB22-1,** 5.0 g, 26.18 mmol, 1.00 eq) was dissolved in pyridine (25 mL), and N-methylpiperazine (3.9 g, 39.2 mmol, 1.5 eq) was added. The resulting mixture was heated up to 110°C and stirred for 4 hours, then cooled to room temperature. The resulting mixture was concentrated under vacuum to obtain a crude product, which was triturated with ethyl acetate (50 mL). The resulting mixture was filtered to obtain a solid, which was concentrated under vacuum to obtain compound **BB22-2** (5.1 g, 24.26 mmol, 92.7% yield) as a brown solid. LC-MS: m/z = 211.1 (M+H)⁺.

### Step 2: Synthesis of compound BB22

**BB22-2** (5.0 g, 23.8 mmol, 1.0 *eq)* was added to phosphorus oxychloride (200 mL), and the mixture was stirred at 90°C for 16 hours. The resulting mixture was cooled to room temperature, and concentrated under vacuum. Saturated aqueous sodium bicarbonate solution was added to adjust pH to about 7, and the resulting mixture was extracted with dichloromethane (300 mL) three times. The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 0/1) to obtain intermediate **BB22** (1.3 g, 5.26 mmol, 22.1% yield, 97.8% purity) as a yellow solid. ¹H NMR (400 MHz, DMSO-d6) δ 8.51 (s, 1H), 3.16 - 3.05 (m, 4H), 2.50 - 2.42 (m, 4H), 2.24 (s, 3H); LC-MS: m/z =247.1 (M+H)⁺.

### General intermediate BB24 (2,4-dichloropyrimidin-5-ol)

Intermediate **BB24** was purchased from Bide Pharmatech.

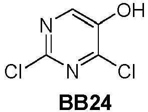

### General intermediate BB35 ((2,4-dichloropyrimidin-5-yl)methanol)

Intermediate **BB35** was purchased from Bide Pharmatech.

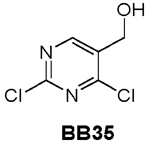

### Synthesis of general intermediate BB36 (2-chloro-5-(methoxymethyl)-N-methylpyrimidin-4-amine)

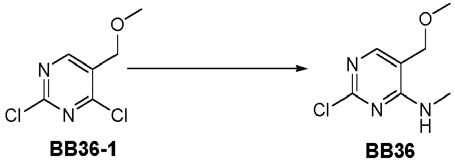

In accordance with the same steps of intermediate BB16, intermediate BB36 (1.89 g, 10.07 mmol, 67.2% yield) was obtained using 2,4-dichloro-5-(methoxymethyl)pyrimidine **(BB36-1,** 2.9 g, 15.0 mmol, 1.00 *eq)* as starting material. LC-MS: m/z = 188.1 (M+H)⁺.

### Synthesis of general intermediate BB41 (methyl 2-(2,4-dichloropyrimidin-5-yl)acetate)

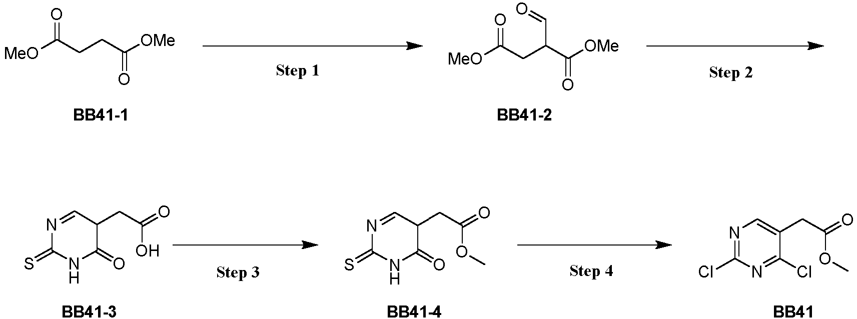

### Step 1: Synthesis of compound BB41-2 (dimethyl 2-formylsuccinate)

Dimethyl succinate (compound **BB41-1,** 200 g, 1.37 mol, 179 mL, 1.00 *eq),* ethyl formate (203 g, 2.74 mol, 220 mL, 2.00 *eq)* and sodium methoxide (259 g, 1.44 mol, 30.0% purity, 1.05 *eq)* were added to tetrahydrofuran (850 mL) successively, and the resulting mixture was stirred at 25°C for 12 hours. Then, the reaction solution was concentrated under vacuum to obtain a crude product, which was washed twice with petroleum ether (400 mL). 1 M hydrochloric acid was added to adjusted the pH to about 6, and the resulting mixture was extracted three times with methyl tert-butyl ether (300 mL). The organic layers were combined, dried over anhydrous sodium sulfate, and filtered to obtain a filtrate, which was concentrated under vacuum to obtain compound **BB41-2** (70.0 g, 402 mmol, 29.4% yield) as a yellow oil. ¹H NMR (400MHz, CDCl₃) δ 7.69 (m, 1H), 4.03-3.58 (m, 1H), 3.57-3.56 (m, 6H), 1.17-1.10 (m, 1H), 1.07-1.03, (m, 1H).

### Step 2: Synthesis of compound BB41-3 (2-(6-oxo-2-thioxo-1,2,5,6-tetrahydropyrimidin-5-yl)acetic acid)

Compound **BB41-2** (60.0 g, 345 mmol, 1.00 eq) was dissolved in methanol (525 mL), sodium methoxide (124 g, 689 mmol, 30.0% purity, 2.00 eq) and thiourea (26.2 g, 345 mmol, 1.00 eq) were added. The resulting mixture was stirred at 100°C for 12 hours, and filtered to obtain a filter cake. 1 M aqueous hydrochloric acid solution (600 mL) was added, and the resulting mixture was stirred at 0°C for 30 minutes. The resulting mixture was filtered to obtain a filter cake, which was washed with water (100 mL) twice to obtain compound **BB41-3** (30.0 g, 161 mmol, 46.8% yield, 100% purity) as a white solid. ¹H NMR (400MHz, CDCl₃) δ 12.5 (s, 1H), 12.3-12.2 (m, 2H), 7.42 (s, 1H), 3.20 (s, 2H).

### Step 3: Synthesis of compound BB41-4 (methyl 2-(6-oxo-2-thioxo-1,2,5,6-tetrahydropyrimidin-5-yl)acetate)

Compound **BB41-3** (15.0 g, 80.6 mmol, 1.00 eq) was dissolved in methanol (100 mL), and concentrated sulfuric acid (23.7 g, 242 mmol, 12.9 mL, 3.00 eq) was added. The resulting mixture was stirred at 80°C for 12 hours, and filtered to obtain a filtrate, which was concentrated under vacuum to obtain compound **BB41-4** (13.0 g, 64.9 mmol, 80.6% yield) as a white solid. ¹H NMR (400MHz, CDCl₃) δ 12.5 (s, 1H), 12.3(s, 1H), 7.40-7.60 (m, 1H), 3.59 (s, 3H), 3.30 (s, 2H).

### Step 4: Synthesis of intermediate BB41

Compound **BB41-3** (12.0 g, 59.9 mmol, 1.00 eq) was dissolved in phosphorus oxychloride (158 g, 1.03 mol, 96.0 mL, 17.2 eq), and stirred at 110°C for 12 hours under nitrogen atmosphere. The reaction solution was cooled to 20°C, and ice water (50.0 mL) was added. Saturated aqueous sodium bicarbonate solution was added to adjust the pH to about 7, and the resulting mixture was extracted three times with ethyl acetate (30 mL). The organic layers were combined, washed three times with saturated brine (35.0 mL), and filtered to obtain a filtrate. The filtrate was concentrated under vacuum to obtain a crude product, which was purified by column chromatography (eluent: petroleum ether/ethyl acetate = 1/0 - 1/1) to obtain compound **BB41** (3.60 g, 15.7 mmol, 26.2% yield, 96.4% purity) as a light yellow oil. LC-MS: m/z =220.9 (M+H)⁺.

### Synthesis of general intermediate BB42 (2-chloro-5-methoxy-N-(prop-2-yn-1-yl)pyrimidin-4-amine)

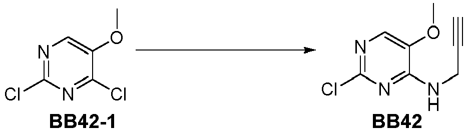

In accordance with the same steps of intermediate **BB16,** intermediate **BB42** (140.8 mg, 0.71 mmol, 25.6% yield) was obtained using 2,4-dichloro-5-methoxypyrimidine **(BB42-1,** 500 mg, 2.79 mmol, 1.00 *eq)* and propargylamine (153.7 mg, 2.79 mmol, 1.00 *eq)* as starting materials. LC-MS: m/z = 198.2 (M+H)⁺.

### Synthesis of general intermediate BB43 (2-chloro-5-methoxy-N-(tetrahydrofuran-3-yl)pyrimidin-4-amine)

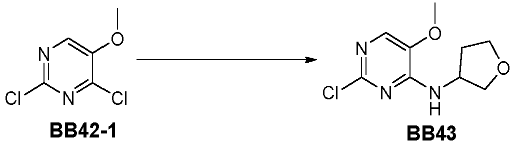

In accordance with the same steps of intermediate **BB16,** intermediate **BB43** (910.5 mg, 3.96 mmol, 59.2% yield) was obtained using 2,4-dichloro-5-methoxypyrimidine **(BB42-1,** 1.2 g, 6.7 mmol, 1.00 *eq)* and 3-aminotetrahydrofuran (584.0 mg, 6.7 mmol, *1.00 eq)* as starting materials. LC-MS: m/z = 230.2 (M+H)⁺.

### Synthesis of general intermediate BB44 (2-chloro-N-cyclopropyl-5-methoxypyrimidin-4-amine)

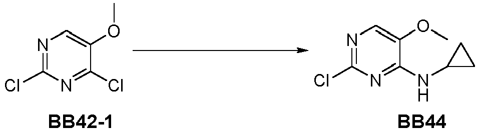

In accordance with the same steps of intermediate **BB16,** intermediate **BB44** (556.3 mg, 2.79 mmol, 62.3% yield) was obtained using 2,4-dichloro-5-methoxypyrimidine **(BB42-1,** 0.8 g, 4.47 mmol, 1.00 *eq)* and cyclopropylamine (255.2 mg, 4.47 mmol, 1.00 *eq)* as starting materials. LC-MS: m/z = 200.1 (M+H)⁺.

### Synthesis of general intermediate BB45 (N-(2-chloro-5-methoxypyrimidin-4-yl)-O-methylhydroxylamine)

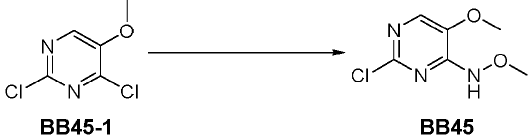

In accordance with the same steps of intermediate **BB16,** intermediate **BB45** (490.0 mg, 2.58 mmol, 92.3% yield) was obtained using 2,4-dichloro-5-methoxypyrimidine (compound **BB45-1,** 500 mg, 2.8 mmol, 1.0 eq) and methoxyamine hydrochloride (352 mg, 4.2 mmol, 1.5 eq) as starting materials. LC-MS: m/z = 190.1 (M+H)⁺.

### Synthesis of general intermediate C2 (2-(4-(chloromethyl)cyclohexyl)pyridine)

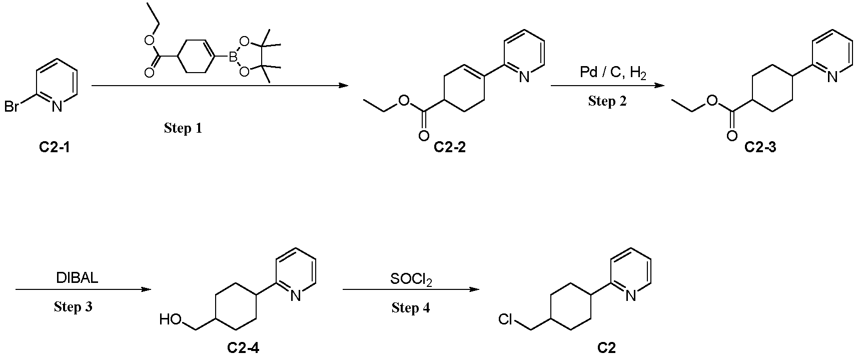

### Step 1: Synthesis of compound C2-2 (ethyl 4-(pyridin-2-yl)cyclohex-3-ene-1-carboxylate)

2-Bromopyridine **(C2-1,** 15.0 g, 94.9 mmol, 9.04 mL, 1.00 eq), 1-ethoxycarbonylcyclohex-3-ene-4-pinacolyl borate (26.9 g, 94.9 mmol, 99.0% purity, 1.00 eq), potassium carbonate (39.4 g, 284 mmol, 3.00 eq) and [1,1'-bis(diphenylphosphino)ferrocene]palladium dichloride (6.95 g, 9.49 mmol, 0.10 eq) were added to water (50.0 mL) and dioxane (200 mL) successively, and the resulting mixture was stirred at 100°C for 16 hours under nitrogen atmosphere. Then, water (30.0 mL) was added, and the resulting mixture was extracted three times with ethyl acetate (100 mL). The organic layers were combined, washed twice with saturated brine (30.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **C2-2** (9.00 g, 38.9 mmol, 41.0% yield) as a colorless transparent oil. ¹H NMR (400 MHz, CDCl₃) δ 8.54 - 8.55 (m, 1H), 7.60 - 7.64 (m, 1H), 7.37 (d, J = 8.8Hz, 1H), 7.10 - 7.13 (m, 1H), 6.66 - 6.68 (m, 1H), 4.11 - 4.20 (m, 2H), 2.62 - 2.72 (m, 5H), 2.50 - 2.53 (m, 1H), 1.79 - 1.87 (m, 1H), 1.25 - 1.29 (m, 3H); LC-MS: m/z = 232.0 (M+H)⁺.

### Step 2: Synthesis of compound C2-3 (ethyl 4-(pyridin-2-yl)cyclohexane-1-carboxylate)

Compound **C2-2** (9.00 g, 38.9 mmol, 1.00 eq) was dissolved in methanol (288.0 mL), and palladium on carbon (4.14 g, 3.89 mmol, 10% purity, 0.10 eq) was added under nitrogen atmosphere. The resulting mixture was purged with hydrogen three times, and stirred at 25°C for 16 hours under hydrogen atmosphere (15 Psi). The reaction mixture was filtered to obtain a filtrate, which was concentrated under vacuum to obtain compound **C2-3** (8.13 g, 34.9 mmol, 89.6% yield) as a colorless transparent oil. ¹H NMR (400 MHz, CDCl₃) δ 8.51 - 8.54 (m, 1H), 7.58 - 7.62 (m, 1H), 7.09 - 7.14 (m, 2H), 4.14 - 4.20 (m, 2H), 2.67 - 2.78 (m, 1H), 2.22 - 2.25 (m, 3H), 1.62 - 1.87 (m, 6H), 1.26 - 1.29 (m, 3H); LC-MS: m/z = 234.0 (M+H)⁺.

### Step 3: Synthesis of compound C2-4 ((4-(pyridin-2-yl)cyclohexyl)methanol)

Compound **C2-3** (8.13 g, 34.8 mmol, 1.00 eq) was dissolved in tetrahydrofuran (300.0 mL), and diisobutylaluminum hydride (1 M, 112 mL, 3.20 eq) was added dropwise at -70°C. After the addition was completed, the resulting mixture was stirred at -70°C for 30 minutes, heated up to 25°C and stirred for 1 hour. Water (100 mL) was added at 0°C to quench the reaction, and the resulting mixture was extracted three times with ethyl acetate (300 mL). The organic layers were combined, washed twice with saturated brine (200 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **C2-4** (4.80 g, 25.1 mmol, 72.0% yield) as a colorless transparent oil. ¹H NMR (400 MHz, CDCl₃) δ 8.54 - 8.55 (m, 1H), 7.59 - 7.63 (m, 1H), 7.10 - 7.20 (m, 2H), 3.52 - 3.71 (m, 2H), 2.80 - 2.85(m, 1H), 1.67 - 1.84 (m, 8H), 1.15 - 1.23 (m, 1H); LC-MS: m/z = 192.0 (M+H)⁺.

### Step 4: Synthesis of compound C2

Compound **C2-4** (800 mg, 4.18 mmol, 1.00 eq) was dissolved in dichloromethane (16.0 mL), and thionyl chloride (2.49 g, 20.9 mmol, 1.52 mL, 5.00 eq) was added at 0°C. After the addition was completed, the resulting mixture was heated up to 20°C and stirred for 12 hours. The reaction solution was concentrated under vacuum to obtain intermediate **C2** (900 mg, crude) as a white solid. LC-MS: m/z = 210.0 (M+H)⁺.

### Synthesis of general intermediate C3 (2-(4-(chloromethyl)phenyl)pyridine)

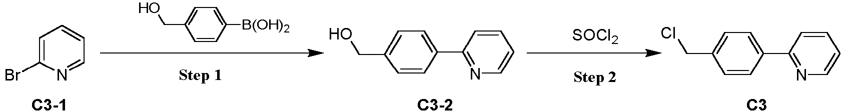

### Step 1: Synthesis of compound C3-2 ((4-(pyridin-2-yl)phenyl)methanol)

Compound 2-bromopyridine **(C3-1,** 10.0 g, 63.3 mmol, 6.02 mL, 1.00 eq), 2-(4-hydroxymethylphenyl)pyridine (12.5 g, 82.3 mmol, 1.30 eq), tetrakis(triphenylphosphine)palladium (7.31 g, 6.33 mmol, 0.100 eq), and sodium carbonate (49.6 g, 468 mmol, 7.40 eq) were added to a mixed solvent of toluene (50.0 mL), water (50.0 mL) and ethanol (10.0 mL) successively. The resulting mixture was purged three times with nitrogen, and stirred at 100°C for 12 hours under nitrogen atmosphere. The reaction solution was filtered to obtain a filtrate. The filtrate was concentrated under vacuum to obtain a crude product, which was purified by reverse phase HPLC (0.1% NH₃•H₂O) to obtain compound C3-2 (9.63 g) as a light yellow oil. ¹H NMR (400 MHz, CDCl₃) δ 8.67 (d, J=4.4Hz, 1H), 7.75 - 7.95 (m, 2H), 7.70 - 7.74 (m, 2H), 7.42 - 7.44 (m, 2H), 7.22 - 7.25 (m, 1H), 4.73 (s, 2H), 2.70 (s, 1H); LC-MS: m/z = 186.2 (M+H)⁺.

### Step 2: Synthesis of intermediate C3

Compound **C3-2** (1.0 g, 5.40 mmol, 1.00 eq) was dissolved in dichloromethane (10.0 mL), and thionyl chloride (3.2 g, 27.0 mmol, 1.96 mL, 5.00 eq) was added at 0°C. After the addition was completed, the resulting mixture was heated up to 20°C and stirred for 12 hours. The reaction solution was concentrated under vacuum to obtain intermediate **C3** (1.1 g, crude) as a white solid. LC-MS: m/z = 204.1 (M+H)⁺.

### Synthesis of general intermediate C4 (4-(chloromethyl)-1-(1-methyl-4-(trifluoromethyl)-1H-imidazol-2-yl)piperidine)

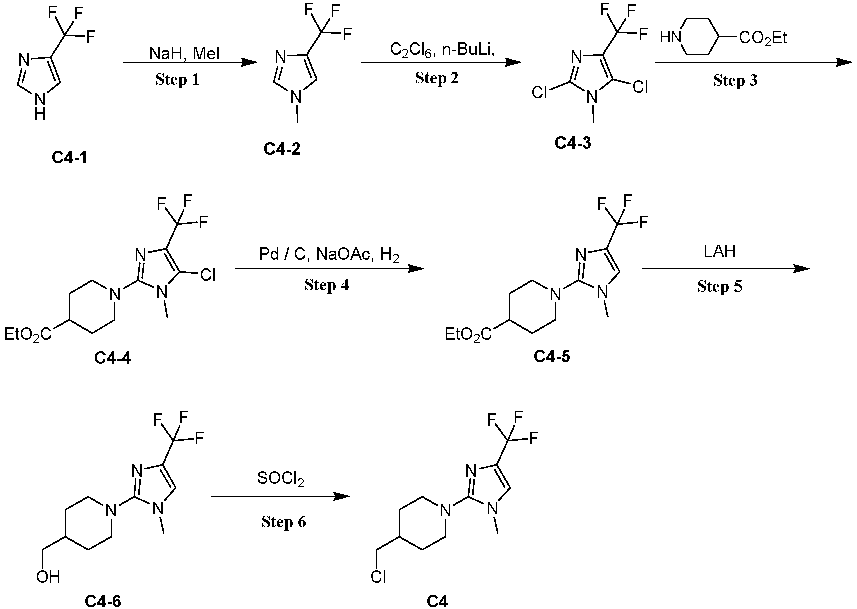

### Step 1: Synthesis of compound C4-2 (1-methyl-4-(trifluoromethyl)-1H-imidazole)

Compound 4-(trifluoromethyl)-1H-imidazole (compound **C4-1,** 33.0 g, 242 mmol, 1.00 eq) was dissolved in tetrahydrofuran (330.0 mL), and sodium hydride (9.70 g, 242 mmol, 60.0% purity, 1.00 eq) was added in portions at 0°C. After the addition was completed, the resulting mixture was reacted at 0°C for 30 minutes. Then, iodomethane (34.4 g, 242 mmol, 15.1 mL, 1.00 eq) was added. After the addition was completed, the resulting mixture was heated up to 25°C and stirred for 4 hours. Then, ice water (200.0 mL) was added at 0°C to quench the reaction, and the resulting mixture was extracted three times with ethyl acetate (200.0 mL). The organic layers were combined, dried over anhydrous sodium sulfate, and filtered to obtain a filtrate. The filtrate was concentrated under vacuum to obtain a crude product, which was purified by reverse phase HPLC (0.1% NH₃•H₂O) to obtain compound **C4-2** (21.0 g). ¹H NMR (400 MHz, CDCl₃) δ 7.48 (s, 1H), 7.24 (s, 1H), 3.75 (s, 3H); LC-MS: m/z = 151.1 (M+H)⁺.

### Step 2: Synthesis of compound C4-3 (2,5-dichloro-1-methyl-4-(trifluoromethyl)-1H-imidazole)

Compound **C4-2** (16.8 g, 111 mmol, 1.00 eq) was dissolved in anhydrous tetrahydrofuran (370.0 mL), and n-butyl lithium (2.5 M, 44.7 mL, 1.00 eq) was added dropwise at -70°C. After the addition was completed, the resulting mixture was stirred at -70°C for 30 minutes. Then, hexachloroethane (15.9 g, 67.0 mmol, 0.60 eq) dissolved in anhydrous tetrahydrofuran (60.0 mL) was added dropwise to the reaction solution. After the addition was completed, the resulting mixture was reacted at -70°C for 1 hour, heated up to 20°C and stirred for 3 hours. Saturated aqueous ammonium chloride solution (200.0 mL) was added at 0°C to quench the reaction. Water (300.0 mL) was added, and the resulting mixture was extracted three times with ethyl acetate (150.0 mL). The organic layers were combined, washed twice with saturated brine (200.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 20/1) to obtain compound **C4-3** (12.0 g). LC-MS: m/z = 219.0 (M+H)⁺.

### Step 3: Synthesis of compound C4-4 (ethyl 1-(5-chloro-1-methyl-4-(trifluoromethyl)-1H-imidazol-2-yl)piperidine-4-carboxylate)

Compound **C4-3** (7.0 g, 31.9 mmol, 1.00 eq), ethyl 4-piperidinylcarboxylate (7.54 g, 47.9 mmol, 7.39 mL, 1.50 eq), N,N-diisopropylethylamine (16.5 g, 127 mmol, 22.2 mL, 4.00 eq) and sodium iodide (479 mg, 3.20 mmol, 0.100 eq) were added to N,N-dimethylformamide (70.0 mL) successively, and the resulting mixture was reacted at 130°C for 72 hours. Water (350 mL) was added, the resulting mixture was stirred for 5 minutes, and extracted three times with ethyl acetate (100 mL). The organic layers were combined, dried over anhydrous sodium sulfate, and filtered to obtain a filtrate. The filtrate was concentrated under vacuum to obtain a crude product, which was purified by reverse phase HPLC (0.1% FA) to obtain compound **C4-4** (2.2 g). ¹H NMR (400 MHz, CDCl₃) δ 4.17 (q, *J =* 7.2Hz, 2H), 3.45 (s, 3H), 3.24 - 3.28 (m, 2H), 2.90 - 2.97 (m, 2H), 2.40 - 2.45 (m, 1H), 2.02 - 2.06 (m, 2H), 1.85 - 1.88 (m, 2H), 1.28 (t, *J =* 8.0Hz, 2H); LC-MS: m/z = 340.1 (M+H)⁺.

### Step 4: Synthesis of compound C4-5 (ethyl 1-(1-methyl-4-(trifluoromethyl)-1H-imidazol-2-yl)piperidine-4-carboxylate)

Compound **C4-4** (2.2 g, 6.48 mmol, 1.00 eq), sodium acetate (1.06 g, 12.9 mmol, 2.00 eq) and wet palladium on carbon (0.5 g, 1.94 mmol, 10.0% purity, 0.300 eq) were added to ethanol (40.0 mL) successively, and the resulting mixture was reacted at 50°C under hydrogen atmosphere (15 psi) for 36 hours. The reaction solution was filtered to obtain a filtrate, which was concentrated under vacuum to obtain compound **C4-5** (1.9 g). ¹H NMR (400 MHz, CDCl₃) δ 7.02 (s, 1H), 4.15 (q, *J =* 7.2Hz, 2H), 3.52 (s, 3H), 3.25 - 3.29 (m, 2H), 2.92 - 2.98 (m, 2H), 2.40 - 2.45 (m, 1H), 2.01 - 2.05 (m, 2H), 1.85 - 1.89 (m, 2H), 1.28 (t, *J =* 6.8Hz, 3H); LC-MS: m/z = 306.1 (M+H)⁺.

### Step 5: Synthesis of compound C4-6 ((1-(1-methyl-4-(trifluoromethyl)-1H-imidazol-2-yl)piperidin-4-yl)methanol)

Compound **C4-5** (1.2 g, 3.93 mmol, 1.00 eq) was dissolved in tetrahydrofuran (20.0 mL), and lithium aluminum hydride (447 mg, 11.8 mmol, 3.00 eq) was added at 0°C. After the addition was completed, the resulting mixture was reacted at 0°C for 0.5 hours, heated up to 20°C and stirred for 12 hours. Then, sodium sulfate decahydrate (0.5 g) was added at 0°C. After the addition was completed, the resulting mixture was heated up to 20°C and stirred for 30 minutes, followed by the addition of tetrahydrofuran (10.0 mL), and filtered to obtain a filtrate. The organic layer was separated, dried over anhydrous sodium sulfate, and filtered to obtain a filtrate. The filtrate was concentrated under vacuum to obtain compound **C4-6** (1.0 g, crude), which was directly used in the next step. ¹H NMR (400 MHz, CDCl₃) δ 7.01 (s, 1H), 3.57 (d, *J =* 6.4Hz, 2H), 3.51 (s, 3H), 3.27 - 3.30 (m, 2H), 2.90 - 2.96 (m, 2H), 1.84 - 1.87 (m, 2H), 1.65 - 1.70 (m, 1H), 1.39 - 1.42(m, 2H); LC-MS: m/z = 264.1 (M+H)⁺.

### Step 6: Synthesis of intermediate C4

Compound **C4-6** (1.0 g, 3.80 mmol, 1.00 eq) was dissolved in dichloromethane (2.0 mL), and thionyl chloride (4.5 g, 38.1 mmol, 20.0 eq) was added at 0°C. After the addition was completed, the resulting mixture was heated up to 20°C and stirred for 24 hours. The reaction solution was concentrated under vacuum to obtain intermediate **C4** (1.1 g, 3.46 mmol, 91.0% yield, HCl) as a brown solid. LC-MS: m/z = 282.1 (M+H)⁺.

### Synthesis of general intermediate C5 (2-(4-(chloromethyl)phenyl)-1-methyl-4-(trifluoromethyl)-1H-imidazole)

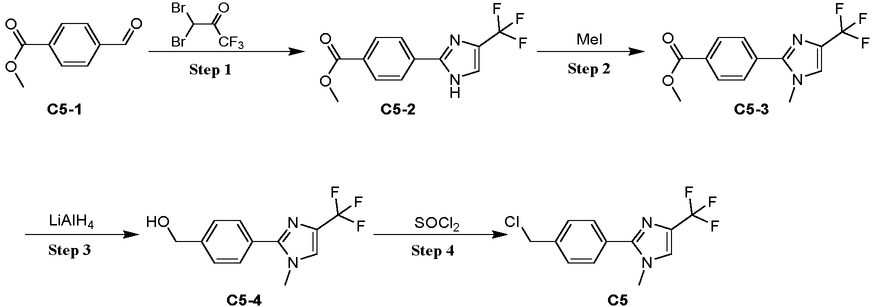

### Step 1: Synthesis of compound C5-2 (methyl 4-(4-(trifluoromethyl)-1H-imidazol-2-yl)benzoate)

1,1-Dibromo-3,3,3-trifluoroacetone (7.2 g, 26.8 mmol, 1.10 eq) and sodium acetate (2.2 g, 27.3 mmol, 1.12 eq) were dissolved in water (8.0 mL), and the resulting mixture was stirred at 100°C for 1 hour. Compound methyl p-formylbenzoate **(C5-1,** 4.0 g, 24.4 mmol, 1.00 eq) dissolved in methanol (80.0 mL) and ammonia water (22.0 mL) was added at 25°C. After the addition was completed, the resulting mixture was reacted at 25°C for 40 minutes, heated up to 100°C and stirred for 2 hours. Then, water (60.0 mL) was added to quench the reaction, and the resulting mixture was extracted three times with ethyl acetate (80.0 mL). The organic layers were combined, washed twice with saturated brine (80.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 3/1) to obtain compound **C5-2** (4.5 g). ¹H NMR (400 MHz, CDCl₃) δ 13.4 (s, 1H), 8.10 - 8.12 (m, 2H), 8.04 - 8.06 (m, 2H), 7.99 (s, 1H), 3.87 (s, 3H); LC-MS: m/z = 271.0 (M+H)⁺.

### Step 2: Synthesis of compound C5-3 (methyl 4-(1-methyl-4-(trifluoromethyl)-1H-imidazol-2-yl)benzoate)

Compound **C5-2** (2.5 g, 9.3 mmol, 1.00 eq) was dissolved in tetrahydrofuran (20.0 mL), and sodium hydride (444.1 mg, 11.1 mmol, 1.20 eq) was added in portions at 0°C. After the addition was completed, the resulting mixture was reacted at 0°C for 30 minutes. Then, iodomethane (6.8 g, 48.1 mmol, 5.20 eq) was added. After the addition was completed, the resulting mixture was heated up to 20°C and stirred for 15 hours. Then, ice water (20.0 mL) was added at 0°C to quench the reaction, and the resulting mixture was extracted three times with ethyl acetate (30.0 mL). The organic layers were combined, washed twice with saturated brine (30.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 3/1) to obtain compound **C5-3** (1.1 g). ¹H NMR (400 MHz, CDCl₃) δ 8.07 (d, *J =* 8.4Hz, 2H), 7.99 (s, 1H), 7.90 (d, *J =* 8.4Hz, 2H), 3.89 (s, 3H), 3.84 (s, 3H); LC-MS: m/z =285.1 (M+H)⁺.

### Step 3: Synthesis of compound C5-4 ((4-(1-methyl-4-(trifluoromethyl)-1H-imidazol-2-yl)phenyl)methanol)

Compound **C5-3** (0.6 g, 2.1 mmol, 1.00 eq) was dissolved in tetrahydrofuran (6.0 mL), and lithium aluminum hydride (88.1 mg, 2.32 mmol, 1.10 eq) was added at 0°C. After the addition was completed, the resulting mixture was heated up to 25°C and stirred for 4 hours. Water (1.6 mL) and aqueous sodium hydroxide solution (1 M, 0.4 mL) were added at 0°C, and the resulting mixture was stirred at 0°C for 0.5 hours. Tetrahydrofuran (10.0 mL) and anhydrous sodium sulfate (1.0 g) were added, and the resulting mixture was filtered to obtain a filtrate. The filtrate was concentrated under vacuum to obtain a crude product **C5-4** (505.4 mg), which was directly used in the next step. ¹H NMR (400 MHz, CDCl₃) δ 7.92 (s, 1H), 7.67 (d, *J =* 8.4 Hz, 2H), 7.45 (d, *J =* 8.0 Hz, 2H), 5.30 - 5.33 (m, 1H), 4.58 (d, *J =* 5.6 Hz, 2H), 3.78 (s, 3H); LC-MS: m/z = 257.1 (M+H)+.

### Step 4: Synthesis of intermediate C5

Compound **C5-4** (500.0 mg, 2.0 mmol, 1.00 eq) was dissolved in dichloromethane (5.0 mL), and thionyl chloride (1.9 g, 15.6 mmol, 8.00 eq) was added at 0°C. After the addition was completed, the resulting mixture was heated up to 20°C and stirred for 15 hours. The reaction solution was concentrated under vacuum to obtain compound C5 (523 mg, 1.90 mmol, 97.6% yield) as a brown solid. ¹H NMR (400 MHz, DMSO-d6) δ 7.94 (d, J = 1.4 Hz, 1H), 7.74 (d, J = 8.2 Hz, 2H), 7.58 (d, J = 8.1 Hz, 2H), 4.84 (s, 2H), 3.80 (s, 3H); LC-MS: m/z = 275.1 (M+H)⁺.

### Synthesis of general intermediate C6 (2-(4-(chloromethyl)piperidin-1-yl)pyridine)

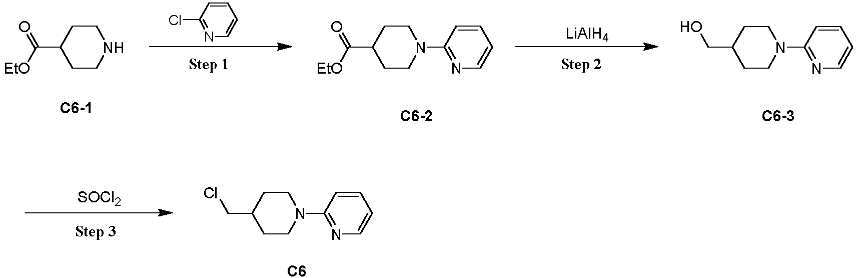

### Step 1: Synthesis of compound C6-2 (ethyl 1-(pyridin-2-yl)piperidine-4-carboxylate)

Ethyl piperidine-4-carboxylate (compound **C6-1,** 20.8 g, 132 mmol, 20.4 mL, 1.00 eq), 2-chloropyridine (15.0 g, 132.0 mmol, 1.00 eq) and triethylamine (26.7 g, 264 mmol, 36.8 mL, 2.00 eq) were dissolved in dimethyl sulfoxide (50.0 mL), and the resulting mixture was reacted at 130°C for 16 hours. The reaction was cooled down and stopped. Water (50 ml) was added at room temperature to quench the reaction, and the resulting mixture was extracted three times with ethyl acetate (60.0 mL). The organic layers were combined, washed twice with saturated brine (30.0 mL), dried over anhydrous sodium sulfate, and filtered to obtain a filtrate. The filtrate was concentrated under vacuum to obtain a crude product, which was purified by silica gel column chromatography (eluent: petroleum ether/ethyl acetate = 1/0 - 1/1) to obtain compound **C6-2** (6.0 g, yield 19.4%, purity 100%) as a yellow oil. ¹H NMR (400 MHz, CDCl₃) δ 8.17 - 8.19 (m, 1H), 7.44 - 7.48 (m, 1H), 6.66 (d, J=8.4Hz, 1H), 6.59 - 6.61 (m, 1H), 4.21 - 4.24 (m, 2H), 4.13 - 4.18 (m, 2H), 2.92 - 2.99 (m, 2H), 2.52 - 2.53 (m, 1H), 1.98 - 2.02 (m, 2H), 1.75 - 1.79 (m, 2H), 1.25 - 1.28 (m, 3H). LC-MS: m/z = 235.2(M+H)⁺.

### Step 2: Synthesis of compound C6-3 ((1-(pyridin-2-yl)piperidin-4-yl)methanol)

Compound **C6-2** (6.00 g, 25.6 mmol) was dissolved in THF (60.0 mL), and lithium aluminum hydride (1.07 g, 28.2 mmol, 1.10 eq) was added in portions at 0°C. The resulting mixture was heated up to room temperature, and stirred for 16 hours. Additional lithium aluminum hydride (486 mg, 12.8 mmol, 0.500 eq) was added, and the resulting mixture was further stirred for 6 hours. Water (20.0 mL) was added at 0°C to quench the reaction, and the resulting mixture was extracted three times with ethyl acetate (60.0 mL). The organic layers were combined, washed twice with saturated brine (20.0 mL), dried over anhydrous sodium sulfate, and filtered to obtain a filtrate. The filtrate was concentrated under vacuum to obtain a crude product, which was purified by silica gel column chromatography (eluent: petroleum ether/ethyl acetate = 1/0 - 1/1) to obtain compound **C6-3** (1.20 g, yield 23.8%, purity 97.8%) as a yellow oil. ¹H NMR (400 MHz, CDCl₃) δ 8.18 (d, J=3.6Hz, 1H), 7.43 - 7.48 (m, 1H), 6.67 (d, J=8.8Hz, 1H), 6.58 - 6.59 (m, 1H),4.33 (d, J=12.8Hz, 2H), 3.54 (d, J=6.0Hz, 2H), 2.81 - 2.88 (m, 2H), 1.77 - 1.86 (m, 2H), 1.73 - 1.76 (m, 1H), 1.55 (s, 1H), 1.28 - 1.35 (m, 2H). LC-MS: m/z =193.2(M+H)⁺.

### Step 3: Synthesis of intermediate C6

Compound **C6-3** (1.20 g, 6.24 mmol, 1.00 eq) was dissolved in dichloromethane (12.0 mL), and thionyl chloride (3.71 g, 31.2 mmol, 2.26 mL, 5.00 eq) was added at 0°C. After the addition was completed, the resulting mixture was heated up to 20°C and stirred for 12 hours. The reaction solution was concentrated under vacuum to obtain intermediate **C6** (1.57 g, crude) as a white solid. LC-MS: m/z = 211.1.

### Synthesis of general intermediate C7 (2-(5-(chloromethyl)thiophen-2-yl)-1-methyl-4-(trifluoromethyl)-1H-imidazole)

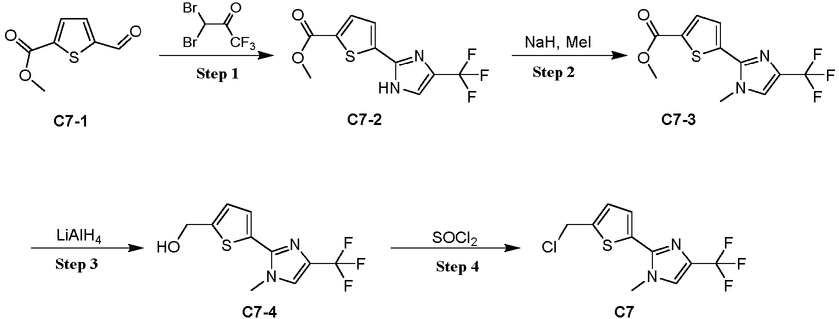

In accordance with the synthesis of intermediate **C5,** general intermediate **C7** was obtained by replacing compound **C5-1** with an equimolar amount of methyl 5-formylthiophene-2-carboxylate (compound **C7-1),** while other experimental conditions remained unchanged. Intermediate **C7** (330.0 mg, crude) was finally obtained as a white solid. LC-MS: m/z = 281.0 (M+H)⁺.

### Synthesis of general intermediate C9 (2-((1R,4R)-4-(chloromethyl)cyclohexyl)-1-methyl-4-(trifluoromethyl)-1H-imidazole)

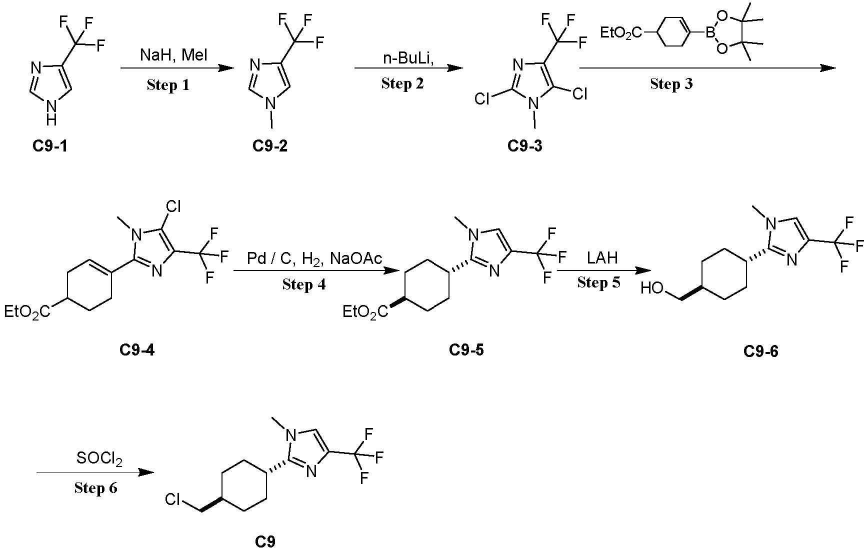

### Step 1: Synthesis of compound C9-2 (1-methyl-4-(trifluoromethyl)-1H-imidazole)

4-(Trifluoromethyl)-1H-imidazole (compound **C9-1,** 33.0 g, 242.0 mmol, 1.00 eq) was dissolved in tetrahydrofuran (330.0 mL), and sodium hydride (9.7 g, 242.0 mmol, 60.0% purity, 1.00 eq) was added in portions at 0°C. After the addition was completed, the resulting mixture was reacted at 0°C for 30 minutes. Then, iodomethane (34.4 g, 242.0 mmol, 15.1 mL, 1.00 eq) was added. After the addition was completed, the resulting mixture was heated up to 25°C and stirred for 5 hours. Saturated aqueous ammonium chloride solution (30.0 mL) was added at 0°C to quench the reaction. Water (30.0 mL) was added, and the resulting mixture was extracted three times with ethyl acetate (30.0 mL). The organic layers were combined, washed twice with saturated brine (30.0 mL), dried over anhydrous sodium sulfate, and filtered to obtain a filtrate. The filtrate was concentrated under vacuum to obtain a crude product, which was purified by reverse phase HPLC (0.1% NH₃•H₂O) to obtain compound **C9-2** (21.0 g). ¹H NMR (400 MHz, CDCl₃) δ 7.48 (s, 1H), 7.24 (s, 1H), 3.75 (s, 3H); LC-MS: m/z = 151.1 (M+H)⁺.

### Step 2: Synthesis of compound C9-3 (2,5-dichloro-1-methyl-4-(trifluoromethyl)-1H-imidazole)

Compound **C9-2** (16.8 g, 111.0 mmol, 1.00 eq) was dissolved in anhydrous tetrahydrofuran (370.0 mL), and n-butyl lithium (2.5 M, 44.7 mL, 1.00 eq) was added dropwise at -70°C. After the addition was completed, the resulting mixture was stirred at -70°C for 30 minutes. Then, hexachloroethane (15.9 g, 67.0 mmol, 0.60 eq) dissolved in anhydrous tetrahydrofuran (60.0 mL) was added dropwise to the reaction solution. After the addition was completed, the resulting mixture was reacted at -70°C for 1 hour, heated up to 20°C and stirred for 3 hours. Saturated aqueous ammonium chloride solution (200.0 mL) was added at 0°C to quench the reaction. Water (300.0 mL) was added, and the resulting mixture was extracted three times with ethyl acetate (150.0 mL). The organic layers were combined, washed twice with saturated brine (200.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 20/1) to obtain compound **C9-3** (12.0 g). LC-MS: m/z = 219.0 (M+H)⁺.

### Step 3: Synthesis of compound C9-4 (ethyl 4-(5-chloro-1-methyl-4-(trifluoromethyl)-1H-imidazol-2-yl)cyclohex-3-ene-1-carboxylate)

Compound **C9-3** (7.0 g, 31.9 mmol, 1.00 eq), 1-ethoxycarbonylcyclohex-3-ene-4-pinacolyl borate (8.94 g, 31.9 mmol, 1.00 eq), potassium phosphate (20.3 g, 95.8 mmol, 3.00 eq), chloro(2-dicyclohexylphosphino-2',4',6'-triisopropyl-1,1'-biphenyl)[2-(2'-amino-1,1'-biphenyl)]palladium(II) (1.3 g, 1.6 mmol, 0.05 eq) and 2-dicyclohexylphosphino-2',4',6'-triisopropylbiphenyl (5.9 g, 12.4 mmol, 0.39 eq) were added to a mixed solvent of dioxane (70.0 mL) and water (10.0 mL) successively. The resulting mixture was purged three times with nitrogen, and stirred at 100°C for 5 hours under nitrogen atmosphere. The reaction solution was filtered to obtain a filtrate, which was concentrated under vacuum to obtain a crude product. The crude product was purified by reverse phase HPLC (0.1% FA) to obtain compound **C9-4** (1.9 g). ¹H NMR (400 MHz, CDCl₃) δ 6.05 (d, *J =* 2.0 Hz, 1H), 4.18 (q, *J =* 7.2 Hz, 2H).3.61 (s, 3H), 2.50 - 2.67 (m, 5H), 2.13 - 2.14 (m, 1H), 1.86 - 1.89 (m, 1H), 1.28 (t, *J =* 7.2 Hz, 3H); LC-MS: m/z = 337.2 (M+H)⁺.

### Step 4: Synthesis of compound C9-5 (ethyl (1R,4R)-4-(1-methyl-4-(trifluoromethyl)-1H-imidazol-2-yl)cyclohexane-1-carboxylate)

Compound **C9-4** (1.9 g, 5.6 mmol, 1.00 eq), sodium acetate (925.0 mg, 11.2 mmol, 2.00 eq) and wet palladium on carbon (0.5 g, 1.4 mmol, 10.0% purity, 0.25 eq) were added to ethanol (30.0 mL) successively, and the resulting mixture was reacted at 50°C under hydrogen atmosphere (15 psi) for 5 hours. The reaction solution was filtered to obtain a filtrate, which was concentrated under vacuum to obtain a crude product. The crude product was purified by preparative HPLC (column: water Xbridge C18 150×50mm×10µm; mobile phase: [water (NH₄HCO₃)-ACN]; B%: 34%-64%, 10 min) to obtain compound **C9-5** (0.3 g). ¹H NMR (400 MHz, CDCl₃) δ 7.13 (s, 1H), 4.15(q, *J =* 6.8 Hz, 2H), 3.64(s, 3H), 2.60 - 2.65 (m, 1H), 2.41 - 2.44 (m, 1H), 2.12 - 2.15 (m, 2H), 1.97 - 1.98 (m, 2H), 1.77 - 1.80 (m, 2H), 1.54 - 1.59 (m, 2H), 1.27 (t, *J =* 8.8 Hz, 3H); LC-MS: m/z = 305.0 (M+H)⁺.

### Step 5: Synthesis of compound C9-6 (((1R,4R)-4-(1-methyl-4-(trifluoromethyl)-1H-imidazol-2-yl)cyclohexyl)methanol)

Compound **C9-5** (0.6 g, 2.0 mmol, 1.00 eq) was dissolved in tetrahydrofuran (10.0 mL), and lithium aluminum hydride (224.0 mg, 5.9 mmol, 3.00 eq) was added at 0°C. After the addition was completed, the resulting mixture was reacted at 0°C for 0.5 hours, heated up to 20°C and stirred for 12 hours. Then, sodium sulfate decahydrate (0.5 g) was added at 0°C. After the addition was completed, the resulting mixture was heated up to 20°C and stirred for 30 minutes, followed by the addition of tetrahydrofuran (10.0 mL), and filtered to obtain a filtrate. The organic layer was separated, dried over anhydrous sodium sulfate, and filtered to obtain a filtrate. The filtrate was concentrated under vacuum to obtain crude compound **C9-6** (0.5 g), which was directly used in the next step. ¹H NMR (400 MHz, CDCl₃) δ 7.12 (s, 1H), 3.63 (s, 3H), 3.52 (d, *J =* 5.6 Hz, 2H), 2.59 - 2.65 (m, 1H), 1.95 - 1.98 (m, 4H), 1.77 - 1.80 (m, 3H), 1.07 - 1.17 (m, 2H); LC-MS: m/z = 263.1 (M+H)+.

### Step 6: Synthesis of intermediate C9

Compound **C9-6** (500.0 mg, 1.9 mmol, 1.00 eq) was dissolved in dichloromethane (2.0 mL), and thionyl chloride (4.5 g, 38.1 mmol, 20.0 eq) was added at 0°C. After the addition was completed, the resulting mixture was heated up to 20°C and stirred for 24 hours. The reaction solution was concentrated under vacuum to obtain intermediate **C9** (550 mg, 1.36 mmol, 71.5% yield) as a light yellow solid. LC-MS: m/z = 281.0 (M+H)⁺.

### Synthesis of general intermediate C11 (1-(4-(chloromethyl)phenyl)-5-methyl-3-(trifluoromethyl)-1H-pyrazole)

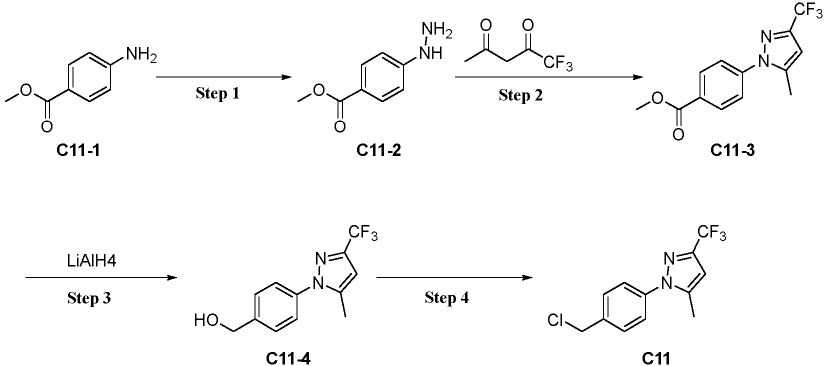

### Step 1: Synthesis of compound C11-2 (methyl 4-hydrazinylbenzoate)

Compound **C11-1** (15.0 g, 90.32 mmol, 1.0 eq) was dissolved in 37% aqueous hydrochloric acid solution (25.0 mL), and sodium nitrite (6.2 g, 90.32 mmol, 1.0 eq) dissolved in water (10.0 mL) was added at 0°C. After the addition was completed, the resulting mixture was stirred for 10 minutes. Then, tin chloride (85.6 g, 451.62 mmol, 5.0 eq) dissolved in 37% aqueous hydrochloric acid solution (75.0 mL) was added dropwise at 0°C. After the addition was completed, the resulting mixture was heated up to 25°C and stirred for 2 hours. The reaction mixture was filtered to obtain a filter cake, which was washed three times with ethyl acetate (50.0 mL), and concentrated under vacuum to obtain compound **C11-2** (16.5 g). LC-MS: m/z = 167.1 (M+H)⁺.

### Step 2: Synthesis of compound C11-3 (methyl 4-(5-methyl-3-(trifluoromethyl)-1H-pyrazol-1-yl)benzoate)

Compound **C11-2** (5 g, 24.75 mmol, 1.0 eq) and 1,1,1-trifluoro-2,4-pentanedione (3.8 g, 24.75 mmol, 1.0 eq) were added to hexafluoroisopropanol (25.0 mL), and triethylamine (5.0 g, 49.50 mmol, 2.0 *eq)* dissolved in hexafluoroisopropanol (25.0 mL) was added dropwise at 0°C. After the addition was completed, the resulting mixture was heated up to 25°C and stirred for 1 hour. Then, water (10 mL) was added, and the resulting mixture was extracted three times with dichloromethane (200.0 mL). The organic layers were combined, washed twice with saturated brine (200.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **C11-3** (5.3 g). LC-MS: m/z = 285.0 (M+H)⁺.

### Step 3: Synthesis of compound C11-4 ((4-(5-methyl-3-(trifluoromethyl)-1H-pyrazol-1-yl)phenyl)methanol)

Compound **C11-3** (5.3 g, 18.65 mmol, 1.0 *eq)* was dissolved in tetrahydrofuran (100.0 mL), and lithium aluminum hydride (1.9 g, 46.64 mmol, 2.5 *eq)* was slowly added at 0°C. After the addition was completed, the resulting mixture was heated up to 20°C and stirred for 6 hours. Then, ice water (50.0 mL) was added at 0°C, the resulting mixture was stirred for 10 minutes, and extracted three times with ethyl acetate (50.0 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/2) to obtain compound **C11-4** (3.6 g). LC-MS: m/z = 257.1 (M+H)⁺.

### Step 4: Synthesis of intermediate C11

Compound **C11-4** (1.0 g, 3.91 mmol, 1.0 *eq)* was dissolved in dichloroethane (25.0 mL), and thionyl chloride (1.4 g, 11.73 mmol, 5.2 *eq)* was added at 0°C. After the addition was completed, the resulting mixture was heated up to 50°C and stirred for 2 hours. The reaction solution was concentrated under vacuum to obtain intermediate **C11** (1.0 g, 3.64 mmol, 93.1% yield, 96.9% purity) as a white solid. ¹H NMR (400 MHz, CDCl₃) δ 7.53 (d, *J =* 8.7Hz, 2H), 7.46 (d, *J =* 8.7Hz, 2H), 6.47 (s, 1H), 4.64 (s, 2H), 2.37 (s, 3H); LC-MS: m/z = 275.1 (M+H)⁺.

### Synthesis of general intermediate C12 (8-(4-(chloromethyl)phenyl)imidazo[1,2-a]pyrazine)

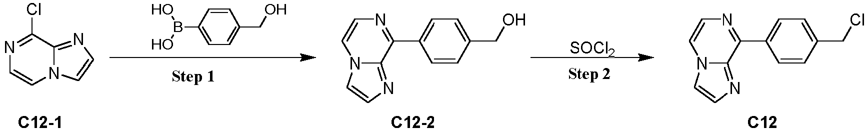

### Step 1: Synthesis of compound C12-2 ((4-(imidazo[1,2-a]pyrazin-8-yl)phenyl)methanol)

8-Chloroimidazo[1,2-A]pyrazine (compound **C12-1,** 9.6 g, 62.5 mmol, 1.00 *eq*), 4-hydroxymethylphenylboronic acid (12.4 g, 81.3 mmol, 1.30 *eq),* tetrakis(triphenylphosphine)palladium (7.2 g, 6.25 mmol, 0.10 *eq),* and sodium carbonate (49.0 g, 463 mmol, 7.40 *eq)* were added to a mixed solvent of toluene (48.0 mL), water (48.0 mL) and ethanol (9.6 mL) successively. The resulting mixture was purged three times with nitrogen, and stirred at 100°C for 12 hours under nitrogen atmosphere. The reaction mixture was filtered to obtain a filtrate, which was concentrated under vacuum to obtain a crude product. The crude product was purified by reverse phase HPLC (0.1% NH₃•H₂O) to obtain compound **C12-2** (12.7 g). LC-MS: m/z = 226.2 (M+H)⁺.

### Step 2: Synthesis of intermediate C12

Compound **C12-2** (6.0 g, 26.6 mmol, 1.00 *eq)* was dissolved in dichloromethane (10.0 mL), and thionyl chloride (15.9 g, 133 mmol, 5.00 *eq)* was added at 0°C. After the addition was completed, the resulting mixture was heated up to 20°C and stirred for 12 hours. The reaction solution was concentrated under vacuum to obtain intermediate **C12** (7.3 g, 26.2 mmol, 98.2% yield, HCl) as an off-white solid. LC-MS: m/z = 244.1 (M+H)+.

### Synthesis of general intermediate C13 (2-(4-(chloromethyl)phenyl)-5-(trifluoromethyl)pyridine)

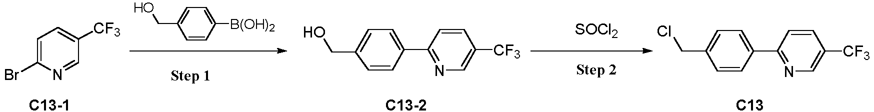

In accordance with the same steps of intermediate **C3,** intermediate **C13** (2.0 g) was obtained using 2-bromo-5-(trifluoromethyl)pyridine (compound **C13-1)** as starting material. LC-MS: m/z = 272.0 (M+H)⁺.

### Synthesis of general intermediate C14 (2-(4-(chloromethyl)phenyl)-5-methylpyridine)

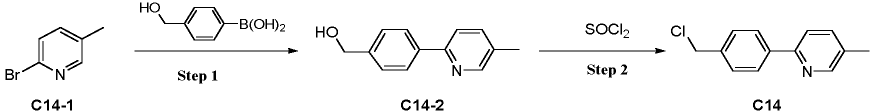

In accordance with the same steps of intermediate **C3,** intermediate **C14** (10.0 g) was obtained using 2-bromo-5-methylpyridine (compound **C14-1)** as starting material. LC-MS: m/z = 218.0 (M+H)⁺.

### Synthesis of general intermediate C19 ((4-(5-(trifluoromethyl)pyridin-2-yl)bicyclo[2.2.2]octan-1-yl)methyl-4-methylbenzenesulfonate)

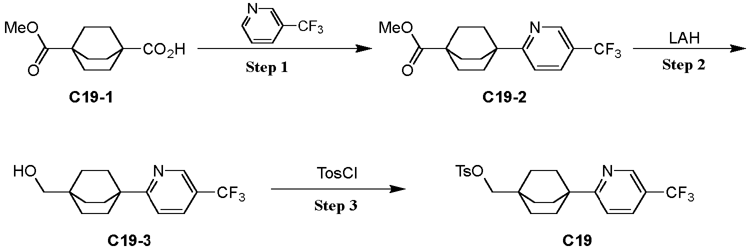

### Step 1: Synthesis of compound C19-2 (methyl 4-(5-(trifluoromethyl)pyridin-2-yl)bicyclo[2.2.2]octane-1-carboxylate)

Monomethyl hydrogen bicyclo[2.2.2]octane-1,4-dicarboxylate (compound **C19-1,** 25.0 g, 118 mmol, 1.00 eq), 3-(trifluoromethyl)pyridine (20.8 g, 141 mmol, 1.20 eq), ammonium persulfate (26.9 g, 118 mmol, 25.6 mL, 1.00 eq) and silver nitrate (4.01 g, 23.6 mmol, 0.20 eq) were added to a mixed solvent of dichloromethane (750.0 mL) and water (750.0 mL) successively, and the resulting mixture was reacted at 20°C for 16 hours. Dichloromethane (250.0 mL) was added, the resulting mixture was filtered to obtain a filtrate, and washed three times with water (100.0 mL). The organic layer was dried over anhydrous sodium sulfate, and filtered to obtain a filtrate. The filtrate was concentrated under vacuum to obtain a crude product, which was purified by preparative HPLC (purification conditions, NH₃.H₂O/MeCN/H₂O) to obtain compound **C19-2** (7.5 g). ¹H NMR (400 MHz, CDCl₃) δ 8.81 (s, , 1H), 7.85 (dd, *J*₁ = 4.0Hz, *J*₂ = 4.0Hz, 1H), 7.37 (d, *J =* 4.0Hz, 1H), 3.69 (s, 3H), 1.96 (s, 12H).

### Step 2: Synthesis of compound C19-3 ((4-(5-(trifluoromethyl)pyridin-2-yl)bicyclo[2.2.2]octan-1-yl)methanol)

Compound **C19-2** (2.5 g, 7.98 mmol, 1.00 eq) was dissolved in tetrahydrofuran (50.0 mL), and lithium aluminum hydride (908 mg, 23.9 mmol, 3.00 eq) was slowly added at 0°C. After the addition was completed, the resulting mixture was heated up to 20°C and stirred for 3 hours. Then, ice water (50.0 mL) was added at 0°C, the resulting mixture was stirred for 10 minutes, and extracted three times with ethyl acetate (50.0 mL). The organic layers were combined, dried over anhydrous sodium sulfate, and filtered to obtain a filtrate, which was concentrated under vacuum to obtain compound **C19-3** (2.3 g) as a white solid. LC-MS: m/z = 286.1 (M+H)⁺.

### Step 3: Synthesis of intermediate C19

Compound **C19-3** (1.6 g, 5.61 mmol, 1.00 eq) and *p*-toluenesulfonyl chloride (1.28 g, 6.73 mmol, 1.20 eq) were added to pyridine (25.0 mL), and the resulting mixture was reacted at 20°C for 12 hours. The reaction solution was concentrated under vacuum. Water (10.0 mL) was added, the resulting mixture was stirred for 5 minutes, and extracted three times with dichloromethane (5.0 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 3/1) to obtain intermediate **C19** (1.5 g, 3.38 mmol, 60.2% yield, 98.9% purity) as a white solid. ¹H NMR (400 MHz, CDCl₃) δ 8.79 (s, 1H), 7.84-7.79 (m, 3H), 7.38 (m, 3H), 3.72 (s, 2H), 2.47 (s, 3H), 1.93-1.89 (m, 6H), 1.57-1.54 (m, 6H); LC-MS: m/z = 440.2 (M+H)⁺.

### Synthesis of general intermediate C25 (6-(chloromethyl)-2-methyl-3,4-dihydroisoquinolin-1(2H)-one)

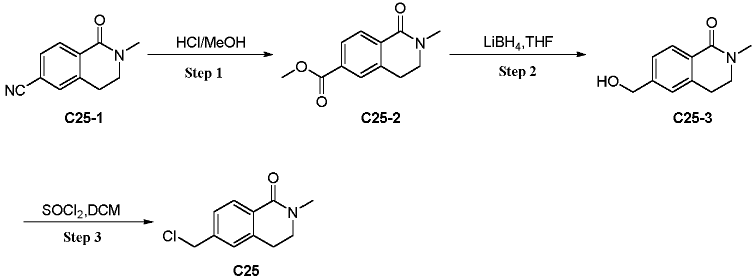

### Step 1: Synthesis of compound C25-2 (methyl 2-methyl-1-oxo-1,2,3,4-tetrahydroisoquinoline-6-carboxylate)

2-Methyl-1-oxo-3,4-dihydroisoquinoline-6-carbonitrile (compound **C25-1,** 500 mg, 2.69 mmol, 1 eq) was dissolved in hydrochloric acid in methanol (4 M, 6.71 mL, 10 eq), and the mixture was stirred at 70°C for 3 hours. The reaction solution was concentrated under vacuum to obtain a crude product, which was purified by prep-HPLC (FA) to obtain compound **C25-2** (0.16 g, 715.21 µmol, 26.64% yield, 98% purity) as a white solid. LC-MS: m/z = 220.2 (M+H)⁺.

### Step 2: Synthesis of compound C25-3 (6-(hydroxymethyl)-2-methyl-3,4-dihydroisoquinolin-1(2H)-one)

Compound **C25-2** (75 mg, 342.10 µmol, 1 eq) was dissolved in tetrahydrofuran (4 mL), and lithium borohydride (2 M, 1 mL, 5.85 eq) was added at 0°C. After the addition was completed, the resulting mixture was stirred at 25°C for 3 hours. Then, hydrochloric acid (2 mL, 1M) was added for quenching three times, and the resulting mixture was extracted three times with ethyl acetate (15.0 mL). The organic layers were combined, dried over anhydrous sodium sulfate, and concentrated under vacuum to obtain compound **C25-3** (50 mg, crude) as a white solid. LC-MS: m/z = 192.2 (M+H)⁺.

### Step 3: Synthesis of intermediate C25

Compound **C25-3** (50 mg, 261.47 µmol, 1 eq) was dissolved in dichloromethane (10.0 mL), and thionyl chloride (155.54 mg, 1.31 mmol, 94.84 µL, 5 eq) was added at 0°C. After the addition was completed, the resulting mixture was heated up to 20°C and stirred for 12 hours. The reaction solution was concentrated under vacuum to obtain intermediate **C25** (50 mg, 238.47 µmol, 91.20% yield) as an off-white solid. LC-MS: m/z = 210.0 (M+H)⁺.

### Synthesis of general intermediate C26 (6-(chloromethyl)-2-isopropyl-3,4-dihydroisoquinolin-1(2H)-one)

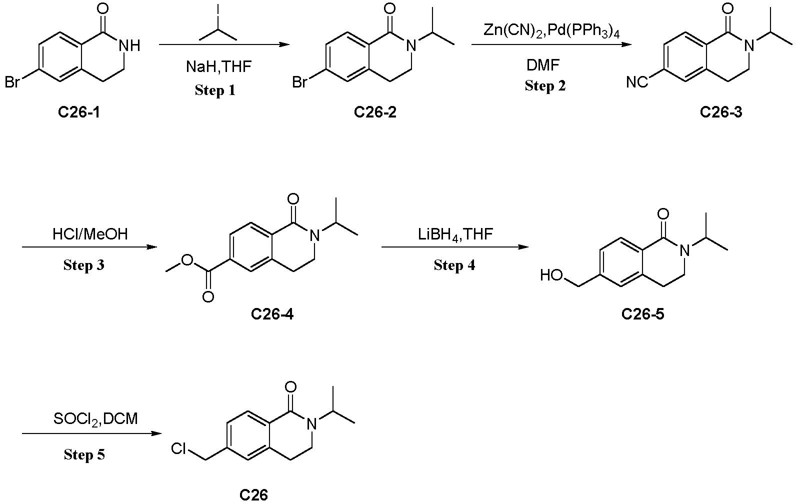

### Step 1: Synthesis of compound C26-2 (6-bromo-2-isopropyl-3,4-dihydroisoquinolin-1(2H)-one)

6-Bromo-3,4-dihydro-2H-isoquinolin-1-one (compound **C26-1,** 500 mg, 2.69 mmol, 1 eq) was dissolved in tetrahydrofuran (40 mL), followed by the addition of sodium hydride (1.06 g, 26.54 mmol, 60% purity, 1.5 eq) at 0°C, and the resulting mixture was stirred for 30 minutes. 2-Iodopropane (6.02 g, 35.39 mmol, 3.54 mL, 2 eq) was added dropwise at 0°C. After the addition was completed, the resulting mixture was stirred at 25°C for 2 hours under nitrogen atmosphere. Water (30 mL) was added at 0°C to quench the reaction, and the resulting mixture was extracted three times with ethyl acetate (50 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 -1/2) to obtain compound **C26-2** (1.7 g, 6.19 mmol, 35.01% yield, 97.7% purity) as a light yellow oil. ¹H NMR (400 MHz, CDCl₃) δ 7.94 (d, J=8.4Hz, 1H), 7.47-7.45 (m, 1H), 7.33 (s, 1H), 5.09-5.03(m, 1H), 3.42 (t, *J₁*=6.4Hz, *J₂*=6.8Hz, 2H), 2.91 (t, *J₁*=6.8Hz, *J₂*=6.4Hz, 2H), 1.19 (d, J=6.8Hz, 6H); LC-MS: m/z = 268.0 (M+H)+.

### Step 2: Synthesis of compound C26-3 (2-isopropyl-1-oxo-1,2,3,4-tetrahydroisoquinoline-6-carbonitrile)

Compound **C26-2** (2.6 g, 9.70 mmol, 1 eq) was dissolved in N,N-dimethylformamide (30 mL), followed by the addition of zinc cyanide (797.00 mg, 6.79 mmol, 430.81 µL, 0.7 eq) and tetrakis(triphenylphosphine)palladium (1.12 g, 969.61 µmol, 0.1 eq), and the resulting mixture was stirred at 80°C for 12 hours under nitrogen atmosphere. Water (30 mL) was added, and the resulting mixture was extracted three times with ethyl acetate (20 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 -0/1) to obtain compound **C26-3** (1.9 g, 8.84 mmol, 91.18% yield, 99.7% purity) as a white solid. ¹H NMR (400 MHz, CDCl₃) δ 8.19 (d, J=8.4Hz, 1H), 7.64-7.62 (m, 1H), 7.50 (s,1H), 5.11-5.04(m, 1H), 3.48(t, *J₁*=6.4Hz, *J₂*=6.4Hz, 2H), 2.99(t, *J₁*=6.4Hz, *J₂*=6.4Hz, 2H), 1.22 (d, J=6.8Hz, 6H); LC-MS: m/z = 215.1 (M+H)⁺.

### Steps 3 to 5: Synthesis of intermediate C26

In accordance with the same steps of intermediate **C25,** intermediate **C26** (433.4 mg, 1.82 mmol, 45.1% yield) was obtained using compound **C26-3** as starting material. LC-MS: m/z = 238.2 (M+H)⁺.

### Synthesis of general intermediate C27 (2-((4-(chloromethyl)phenoxy)methyl)pyridine)

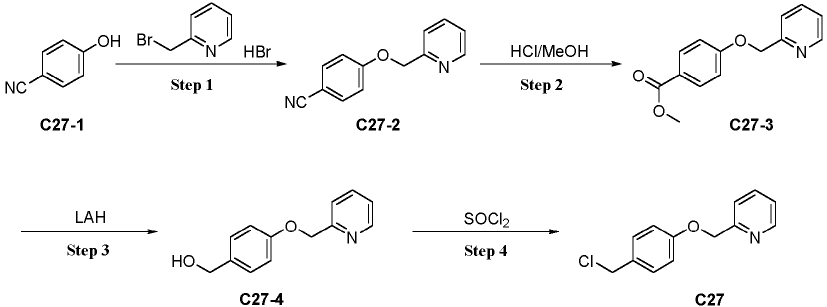

In accordance with the same steps of intermediate **C28,** intermediate **C27** (800.7 mg, crude, a light yellow oil) was obtained using 4-hydroxybenzonitrile (compound **C27-1)** and 2-(bromomethyl)pyridine hydrobromide as starting materials. LC-MS: m/z = 234.0 (M+H)⁺.

### Synthesis of general intermediate C28 (1-(chloromethyl)-4-(2-ethoxyethoxy)benzene)

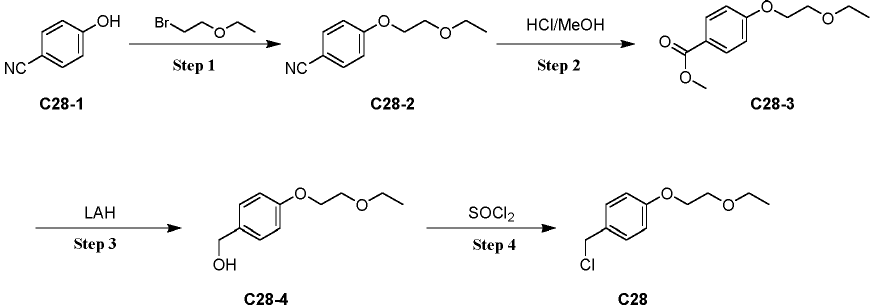

### Step 1: Synthesis of compound C28-2 (4-(2-ethoxyethoxy)benzonitrile)

4-Hydroxybenzonitrile (compound **C28-1,** 4.00 g, 33.6 mmol, 1.00 eq), 2-bromoethyl ethyl ether (10.0 g, 65.4 mmol, 7.35 mL, 1.95 eq) and potassium carbonate (9.28 g, 67.2 mmol, 2.00 eq) were added to acetonitrile (142 mL) successively, and the resulting mixture was stirred at 80°C for 12 hours. The reaction solution was concentrated under vacuum to obtain a crude product. Water (30.0 mL) was added, and the pH was adjusted to about 5 with 1 N HCl aqueous solution. The resulting mixture was extracted three times with dichloromethane (50.0 mL). The organic layers were combined, washed twice with saturated brine (30.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 2/1) to obtain compound **C28-2** (6.69 g, crude) as a light yellow oil. ¹H NMR (400MHz, DMSO) δ 7.75 (dd, *J₁ =* 1.6Hz, *J₂* = 6.8Hz, 2H), 7.11 (d, *J* = 8.8Hz, 2H), 4.16 - 4.19 (m, 2H), 3.69 - 3.71 (m, 2H), 3.46 - 3.51 (m, 2H), 1.11 (t, *J =* 7.0Hz, 3H).

### Step 2: Synthesis of compound C28-3 (methyl 4-(2-ethoxyethoxy)benzoate)

Compound **C28-2** (2.00 g, 10.5 mmol, 1.00 eq) was dissolved in HCl/MeOH (4 M, 105 mL, 40.0 eq), the resulting mixture was stirred at 80°C for 36 hours, and concentrated under vacuum to obtain a crude product. Water (20.0 mL) was added, and the pH was adjusted to about 7 with saturated aqueous sodium bicarbonate solution. The resulting mixture was extracted three times with ethyl acetate (50.0 mL). The organic layers were combined, washed twice with saturated brine (30.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **C28-3** (1.46 g, 6.51 mmol, 62.3% yield) as a light yellow oil. ¹H NMR (400MHz, CDCl₃) δ 7.99 (d, *J =* 8.4Hz, 2H), 6.95 (d, *J* = 8.8Hz, 2H), 4.17 - 4.19 (m, 2H), 3.89 (s, 3H), 3.81 - 3.83 (m, 2H), 3.59 - 3.64 (m, 2H), 1.26 (t, *J =* 7.0Hz, 3H); LC-MS: m/z = 225.3 (M+H)⁺.

### Step 3: Synthesis of compound C28-4 ((4-(2-ethoxyethoxy)phenyl)methanol)

Compound **C28-3** (0.700 g, 3.12 mmol, 1.00 *eq)* was dissolved in tetrahydrofuran (7.00 mL), and lithium aluminum hydride (237 mg, 6.24 mmol, 2.00 *eq)* was slowly added at 0°C. After the addition was completed, the resulting mixture was heated up to 20°C and stirred for 3 hours. Then, ice water (50.0 mL) was added at 0°C, the resulting mixture was stirred for 10 minutes, and extracted three times with ethyl acetate (50.0 mL). The organic layers were combined, dried over anhydrous sodium sulfate, and filtered to obtain a filtrate, which was concentrated under vacuum to obtain compound **C28-4** (0.62 g, crude) as a colorless and transparent oil. ¹H NMR (400MHz, CDCl₃) δ 7.28 - 7.29 (m, 2H), 6.90 - 6.94 (m, 2H), 4.61 (s, 2H), 4.11 - 4.14 (m, 2H), 3.78 - 3.81 (m, 2H), 3.59 - 3.64 (m, 2H), 1.25 (t, *J =* 7.0Hz, 3H).

### Step 4: Synthesis of intermediate C28

Compound **C28-4** (0.560 g, 2.85 mmol, 1.00 *eq)* was dissolved in dichloromethane (10.0 mL), and thionyl chloride (2.46 g, 20.7 mmol, 1.50 mL, 7.25 *eq)* was added at 0°C. After the addition was completed, the resulting mixture was heated up to 20°C and stirred for 12 hours. The reaction solution was concentrated under vacuum to obtain compound **C28** (0.60 g, crude) as a light yellow oil. ¹H NMR (400MHz, DMSO) δ 7.35 - 7.37 (m, 2H), 6.93 - 6.95 (m, 2H), 4.73 (s, 2H), 4.08 - 4.10 (m, 2H), 3.68 - 3.70 (m, 2H), 3.48 - 3.53 (m, 2H), 1.13 (t, *J =* 7.0Hz, 3H); LC-MS: m/z = 215.0 (M+H)⁺.

### Synthesis of general intermediate C29 (1-(chloromethyl)-4-(3-methoxycyclobutoxy)benzene)

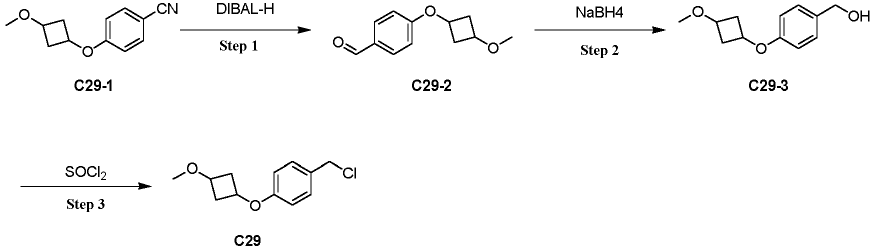

### Step 1: Synthesis of compound C29-2 (4-(3-methoxycyclobutoxy)benzaldehyde)

4-(3-Methoxycyclobutoxy)benzonitrile (compound **C29-1,** 0.560 g, 2.85 mmol, 1.00 *eq)* was dissolved in tetrahydrofuran (6.50 mL), and diisobutylaluminum hydride (1 M, 7.75 mL, 2.50 *eq)* was added dropwise at 0°C. After the addition was completed, the resulting mixture was stirred at 25°C for 2 hours. Saturated aqueous ammonium chloride solution (20.0 mL) was added dropwise at 0°C to quench the reaction, and the resulting mixture was extracted three times with ethyl acetate (50.0 mL). The organic layers were combined, dried over anhydrous sodium sulfate, and filtered to obtain a filtrate, which was concentrated under vacuum to obtain compound **C29-2** (0.65 g, crude) as a light yellow oil. ¹H NMR (400MHz, DMSO) δ 9.87 (s, 1H), 7.85 - 7.87 (m, 2H), 7.04 - 7.06 (m, 2H), 4.50 - 4.54 (m, 1H), 4.07 - 4.10 (m, 1H), 3.61 - 3.67 (m, 3H), 2.90 - 2.92 (m, 2H), 1.91 - 1.96 (m, 2H).

### Step 2: Synthesis of compound C29-3 ((4-(3-methoxycyclobutoxy)phenyl)methanol)

Compound **C29-2** (0.65 g, 3.15 mmol, 1.00 *eq)* was dissolved in methanol (6.00 mL), and sodium borohydride (0.210 g, 5.55 mmol, 1.76 *eq)* was added in portions at 0°C. After the addition was completed, the resulting mixture was heated up to 25°C and stirred for 2 hours. Water (10.0 mL) was added, and the resulting mixture was extracted three times with ethyl acetate (50.0 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/2) to obtain compound **C29-3** (0.12 g, crude) as a light yellow oil. ¹H NMR (400MHz, DMSO) δ 7.22 - 7.24 (m, 2H), 6.77 - 6.79 (m, 2H), 4.28 - 4.32 (m, 1H), 3.73 (s, 2H), 3.65 - 3.68 (m, 1H), 3.28 (s, 3H), 2.86 - 2.91 (m, 2H), 2.05 - 2.16 (m, 2H).

### Step 3: Synthesis of compound C29

Compound **C29-3** (0.12 g, 576 µmol, 1.00 *eq)* was dissolved in dichloromethane (3.0 mL), and thionyl chloride (686 mg, 5.76 mmol, 418 µL, 10.0 *eq)* was added at 0°C. After the addition was completed, the resulting mixture was heated up to 20°C and stirred for 12 hours. The reaction solution was concentrated under vacuum to obtain compound **C29** (0.120 g, 529 µmol, 91.9% yield) as a light yellow oil. ¹H NMR (400MHz, CDCl₃) δ 7.28 - 7.29 (m, 1H), 7.25 - 7.27 (m, 1H), 6.80 - 6.83 (m, 2H), 4.62 (s, 2H), 4.30 - 4.33 (m, 1H), 3.65 - 3.69 (m, 1H), 3.28 (s, 3H), 2.87 - 2.91 (m, 2H), 2.05 - 2.16 (m, 2H); LC-MS: m/z = 227.1 (M+H)⁺.

### Synthesis of general intermediate C30 (2-(4-(chloromethyl)-3-methoxyphenyl)-1-methyl-4-(trifluoromethyl)-1H-imidazole)

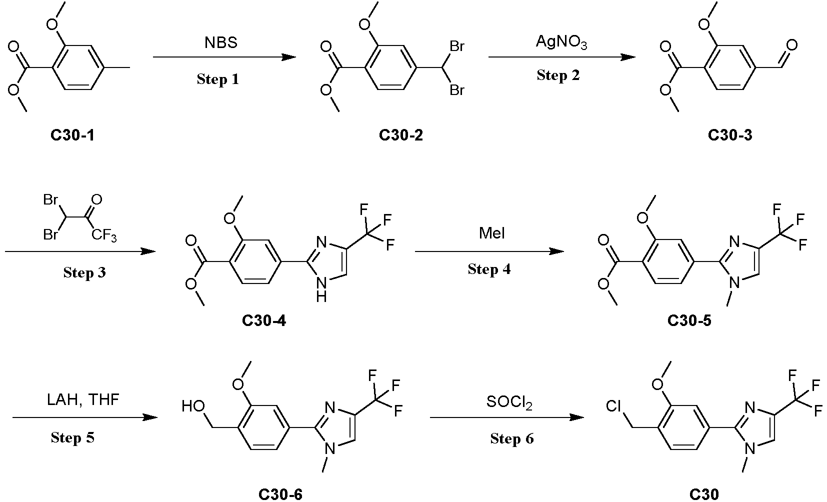

### Step 1: Synthesis of compound C30-2 (methyl 4-(dibromomethyl)-2-methoxybenzoate)

Methyl 2-methoxy-4-methylbenzoate (compound **C30-1,** 5.00 g, 27.8 mmol, 1.00 eq) was added to tetrachloromethane (75.0 mL), and N-bromosuccinimide (10.9 g, 61.0 mmol, 2.20 eq) was added in portions at room temperature. After the addition was completed, the resulting mixture was heated up to 85°C and stirred for 12 hours. The reaction solution was filtered to obtain a filtrate, which was concentrated under vacuum to obtain a crude product. The crude product was purified by column chromatography (eluent: petroleum ether/ethyl acetate = 1/0 - 1/1) to obtain compound **C30-2** (6.50 g, 19.2 mmol, 69.3% yield) as a colorless transparent oil. ¹H NMR (400MHz, CDCl₃) δ 7.77 (d, *J =* 8.0Hz, 1H), 7.21 (d, *J =* 1.6Hz, 1H), 7.13 (dd, *J₁ =* 1.8Hz, *J₂* = 8.2Hz, 1H), 6.62 (s, 1H), 3.96 (s, 3H), 3.90 (s, 3H).

### Step 2: Synthesis of compound C30-3 (methyl 4-formyl-2-methoxybenzoate)

Compound **C30-2** (5.00 g, 14.8 mmol, 1.00 eq) was dissolved in acetone (60.0 mL), and silver nitrate (7.57 g, 44.6 mmol, 3.01 eq) and water (15.0 mL) were added. The resulting mixture was stirred at 20°C for 3 hours, and filtered to obtain a filtrate. The filtrate was concentrated under vacuum to obtain a crude product, which was purified by column chromatography (eluent: petroleum ether/ethyl acetate = 1/0 - 1/1) to obtain compound **C30-3** (2.60 g, 13.4 mmol, 90.5% yield) as a colorless transparent oil. ¹H NMR δ 10.0 (s, 1H), 7.89 - 7.91 (m, 1H), 7.48 - 7.50 (m, 2H), 3.98 (s, 3H), 3.93 (s, 3H).

### Steps 3 to 6: Synthesis of intermediate C30

Regarding Steps 3 to 6, in accordance with the same steps of intermediate **C5,** intermediate **C30** (320 mg, 938 µmol, 75.0% yield, a white solid) was obtained using **C30-3** as starting material. LC-MS: m/z = 305.1 (M+H)⁺.

### Synthesis of general intermediate C31 (2-(4-(chloromethyl)-2-methoxyphenyl)-1-methyl-4-(trifluoromethyl)-1H-imidazole)

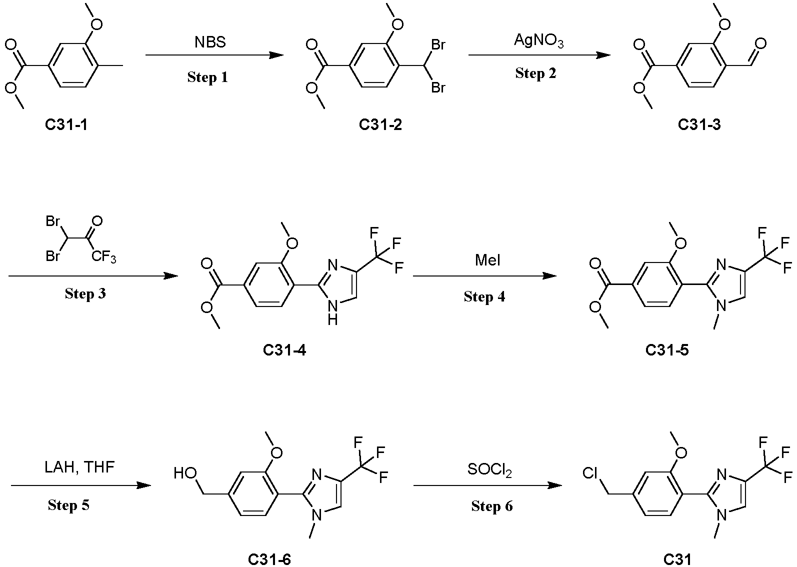

In accordance with the same steps of intermediate **C30,** intermediate C31 (450 mg, a white solid) was obtained using methyl 3-methoxy-4-methylbenzoate (compound **C31-1)** as starting material. LC-MS: m/z = 305.1 (M+H)⁺.

### Synthesis of general intermediate C32 (1-(4-(chloromethyl)-3-methoxyphenyl)-5-methyl-3-(trifluoromethyl)-1H-pyrazole)

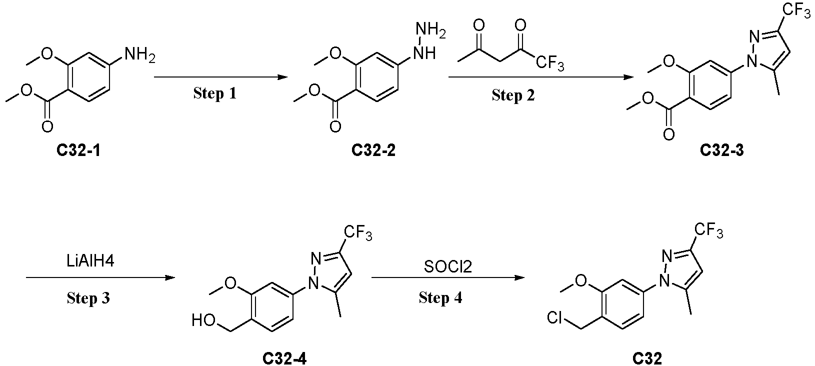

In accordance with the same steps of intermediate **C11,** intermediate **C32** (1.18 g, 3.87 mmol, 88.5% yield, a white solid) was obtained using methyl 2-methoxy-4-aminobenzoate (compound C32-1) as starting material. LC-MS: m/z = 305.1 (M+H)⁺.

### Synthesis of general intermediate C33 (1-(4-(chloromethyl)-2-methoxyphenyl)-5-methyl-3-(trifluoromethyl)-1H-pyrazole)

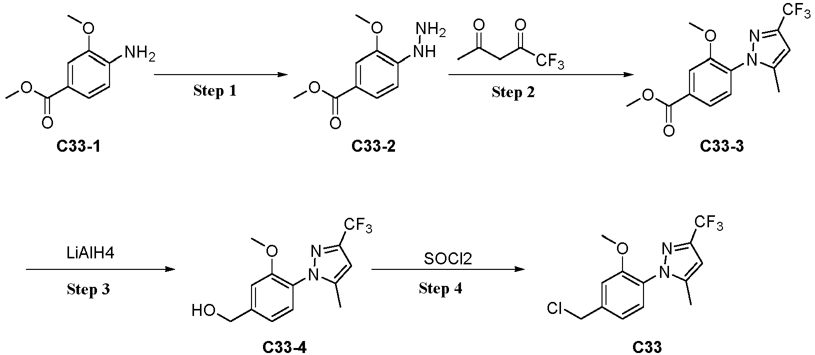

In accordance with the same steps of intermediate **C11,** intermediate **C33** (796.0 mg, 2.61 mmol, 79.6% yield, a white solid) was obtained using methyl 4-amino-3-methoxybenzoate (compound C33-1) as starting material. LC-MS: m/z = 305.0 (M+H)⁺.

### Synthesis of general intermediate C35 ((4-(1-methyl-4-(trifluoromethyl)-1H-imidazol-2-yl)bicyclo[2.2.2]octan-1-yl)methyl-4-methylbenzenesulfonate)

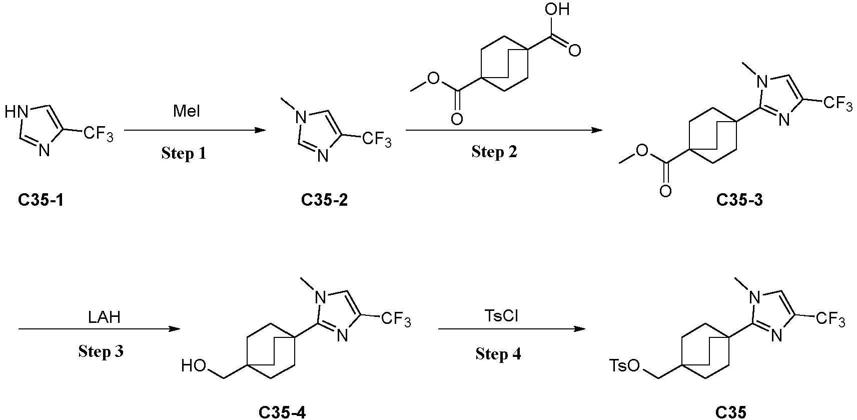

### Step 1: Synthesis of compound C35-2 (1-methyl-4-(trifluoromethyl)-1H-imidazole)

Compound 4-(trifluoromethyl)-1H-imidazole (compound **C35-1,** 26 g, 0.19 mol) and potassium carbonate (105.5 g, 0.76 mol, 4.0 eq) were added to acetonitrile (390 mL), and the resulting mixture was stirred at 0°C for 30 minutes. Iodomethane (32.6 g, 0.23 mol, 1.2 eq) was added dropwise. After the addition was completed, the resulting mixture was stirred at room temperature for 16 hours. Then, the reaction solution was concentrated under vacuum to obtain a crude product. Water (260 mL) was added, the resulting mixture was stirred for 10 minutes, and extracted three times with ethyl acetate (200 mL). The organic layers were combined, washed twice with saturated brine (200 mL), dried over anhydrous sodium sulfate, and filtered to obtain a filtrate, which was concentrated under vacuum to obtain compound **C35-2** (26 g, crude). LC-MS: m/z = 151.0 (M+H)⁺.

### Step 2: Synthesis of compound C35-3 (methyl 4-(1-methyl-4-(trifluoromethyl)-1H-imidazol-2-yl)bicyclo[2.2.2]octane-1-carboxylate)

Compound **C35-2** (14.75 g, 98.3 mmol) and monomethyl hydrogen bicyclo[2.2.2]octane-1,4-dicarboxylate (25 g, 117.9 mmol, 1.2 eq) were dissolved in dichloromethane (295 mL) and water (295 mL), followed by the addition of silver nitrate (6.0 g, 35.4 mmol, 0.3 eq.) and ammonium persulfate (44.9 g, 78.7 mmol, 2.0 eq.), and the resulting mixture was stirred at 25°C for 16 hours. The reaction solution was filtered through diatomaceous earth, and washed twice with dichloromethane (100 mL). The organic layer was separated, and the aqueous layer was extracted three times with dichloromethane (200 mL). The organic layers were combined, washed twice with saturated brine (200 mL), dried over anhydrous sodium sulfate, and filtered to obtain a filtrate, which was concentrated under vacuum to obtain compound **C35-3** (35.2 g, crude) as a yellow oil. ¹H NMR (400MHz, CDCl₃) 7.09 - 7.08 (m, 1H), 3.77 (s, 3H), 3.67 (s, 3H), 2.07 - 2.03 (m, 6H), 1.93 - 1.89 (m, 6H); LC-MS: m/z = 317.2 (M+H)⁺.

### Step 3: Synthesis of compound C35-4 ((4-(1-methyl-4-(trifluoromethyl)-1H-imidazol-2-yl)bicyclo[2.2.2]octan-1-yl)methanol)

Compound **C35-3** (35.2 g, 111.4 mmol) was dissolved in tetrahydrofuran (180.0 mL), and lithium aluminum hydride (2.5 M, 111.4 mL, 278.5 mmol, 2.5 eq) was slowly added at 0°C. After the addition was completed, the resulting mixture was heated up to 20°C and stirred for 3 hours. Then, ice water (50.0 mL) was added at 0°C, the resulting mixture was stirred for 10 minutes, and extracted three times with ethyl acetate (500.0 mL). The organic layers were combined, dried over anhydrous sodium sulfate, and filtered to obtain a filtrate, which was concentrated under vacuum to obtain compound **C35-4** (25.1 g, crude) as a yellow oil. LC-MS: m/z = 289.1 (M+H)⁺.

### Step 4: Synthesis of compound C35

Compound **C35-4** (25.1 g, 87.2 mmol), p-toluenesulfonyl chloride (33.2 g, 174.4 mmol, 2.0 eq) and 4-dimethylaminopyridine (32.0 g, 261.6 mmol, 3.0 eq) were added to dichloromethane (251 mL) successively, and the resulting mixture was stirred at 25°C for 16 hours. The reaction solution was washed twice with water (100 mL), and the organic layer was separated. The organic layer was dried over anhydrous sodium sulfate, and filtered to obtain a filtrate. The filtrate was concentrated under vacuum to obtain a crude product, which was purified by column chromatography (eluent: petroleum ether/dichloromethane = 1/0 - 0/1) to obtain intermediate **C35** (10.0 g) as a white solid. ¹H NMR (400 MHz, DMSO-d6) δ 7.82 (d, J = 8.2 Hz, 2H), 7.67 (s, 1H), 7.52 (d, J = 8.0 Hz, 2H), 3.77 (s, 3H), 3.72 (s, 2H), 2.46 (s, 3H), 1.95 - 1.86 (m, 6H), 1.49 - 1.37 (m, 6H); LC-MS: m/z = 443.3 (M+H)⁺.

### Synthesis of general intermediate C48 (2-(4-(chloromethyl)phenyl)-4-(difluoromethyl)-1-methyl-1H-imidazole)

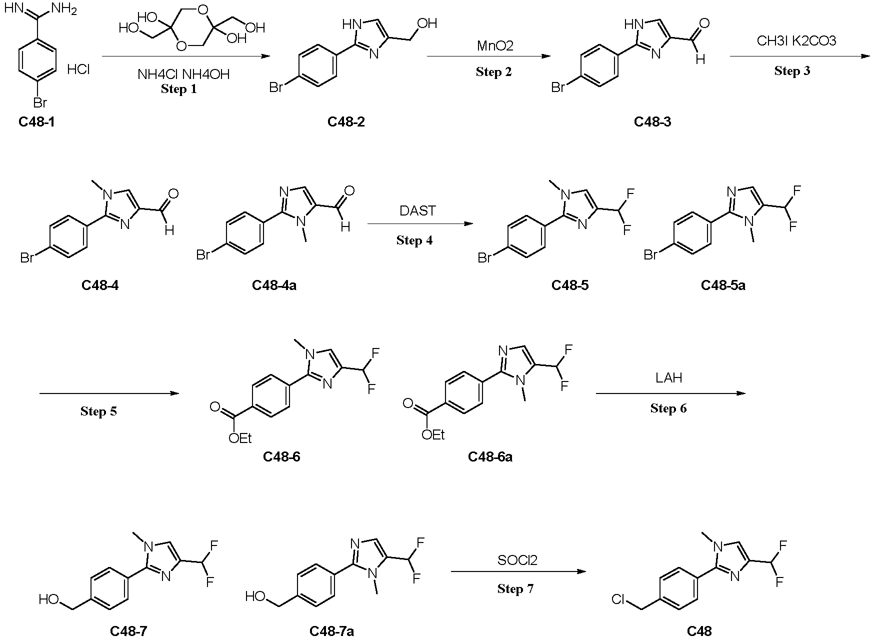

### Step 1: Synthesis of compound C48-2 ((2-(4-bromophenyl)-1H-imidazol-4-yl)methanol)

4-Bromobenzimidamide hydrochloride (compound **C48-1,** 17.8 g, 75.36 mmol), 1,3-dihydroxyacetone dimer (15 g, 83.26 mmol, 1.1 eq), ammonium chloride (20 g, 374 mmol, 5 eq) and sodium hydroxide (3 g, 75.36 mmol, 1 eq) were added to aqueous ammonia (500 mL) successively, and the resulting mixture was stirred at an external temperature of 80°C for 2 hours. The reaction solution was cooled to room temperature, and filtered to obtain a solid, which was concentrated under vacuum to obtain compound **C48-2** (15 g, crude) as a white solid. LC-MS: m/z = 252.6 (M+H)⁺.

### Step 2: Synthesis of compound C48-3 (2-(4-bromophenyl)-1H-imidazole-4-carbaldehyde)

Compound **C48-2** (14 g, 55.56 mmol) was dissolved in tetrahydrofuran (300 mL), and manganese dioxide (48 g, 555.6 mmol, 10 eq) was added and stirred at an external temperature of 60°C for 16 hours. The reaction solution was cooled to room temperature, and filtered to obtain a filtrate, which was concentrated under vacuum to obtain compound **C48-3** (11.1 g, crude) as a white solid. LC-MS: m/z = 250.8 (M+H)⁺.

### Step 3: Synthesis of compound C48-4 (2-(4-bromophenyl)-1-methyl-1H-imidazole-4-carbaldehyde)

Compound **C48-3** (11.1 g, 44.4 mmol), iodomethane (8.2 g, 57.7 mmol, 1.3 eq), and potassium carbonate (24.6 g, 177.6 mmol, 4 eq) were added to N,N-dimethylformamide (120 mL). The reaction solution was stirred at an external temperature of 45°C for 3 hours, and filtered to obtain a filtrate. Water (1000 mL) was added, and the resulting mixture was extracted three times with ethyl acetate (500 mL). The organic layers were combined, dried over anhydrous sodium sulfate, and concentrated under vacuum to obtain a crude product, which was purified by column chromatography (eluent: petroleum ether/ethyl acetate = 1/0 - 1/1) to obtain compound **C48-4** and compound **C48-4a** (6.8 g in total) as a white solid. LC-MS: m/z = 265.0 (M+H)⁺.

### Step 4: Synthesis of compound C48-5 (2-(4-bromophenyl)-4-(difluoromethyl)-1-methyl-1H-imidazole)

Compound **C48-4** and compound **C48-4a** (6.7 g, 25.38 mmol) were dissolved in anhydrous dichloromethane (200 mL), and diethylaminosulfur trifluoride (41 g, 253.8 mmol, 10 eq) was slowly added dropwise at 0°C. After the addition was completed, the resulting mixture was stirred at room temperature (25°C) for 5 hours. Then, the reaction solution was slowly added to saturated aqueous sodium bicarbonate solution, and extracted three times with dichloromethane (500 mL). The organic layers were combined, dried over anhydrous sodium sulfate, and concentrated under vacuum to obtain a crude product, which was purified by column chromatography (eluent: petroleum ether/ethyl acetate = 1/0 - 1/1) to obtain compounds **C48-5** and **C48-5a** (4.1 g) as a white solid. LC-MS: m/z = 286.9 (M+H)⁺.

### Step 5: Synthesis of compound C48-6 (ethyl 4-(4-(difluoromethyl)-1-methyl-1H-imidazol-2-yl)benzoate)

Compounds **C48-5** and **C48-5a** (800 mg, 2.8 mmol), [1,1'-bis(diphenylphosphino)ferrocene]palladium dichloride dichloromethane complex (1.36 g, 1.68 mmol, 0.6 eq), 4,5-bis(diphenylphosphino)-9,9-dimethylxanthene (730 mg, 1.26 mmol, 0.45 eq) and triethylamine (1.42 g, 14 mmol, 5 eq) were added to anhydrous ethanol (32 mL) successively, and the resulting mixture was stirred at an external temperature of 85°C for 20 hours under carbon monoxide atmosphere (double-layer balloon, 25 psi). Then, EA (100 mL) was added, the resulting mixture was stirred for 10 minutes, and filtered to obtain a filtrate. The filtrate was mixed with silica gel, and purified by column chromatography (eluent: petroleum ether/ethyl acetate = 1/0 - 1/1) to obtain compound **C48-6** and compound **C48-6a** (630 mg). LC-MS: m/z = 281.1 (M+H)⁺.

### Step 6: Synthesis of compound C48-7 ((4-(4-(difluoromethyl)-1-methyl-1H-imidazol-2-yl)phenyl)methanol)

Compound **C48-6** and compound **C48-6a** (630 mg) were dissolved in anhydrous THF (7 mL), and a solution of lithium aluminum hydride in THF (2.5 M, 2 mL) was added dropwise at 0°C. After the addition was completed, the resulting mixture was self-heated to room temperature (25°C) and stirred for 2 hours. Water (10 mL) was added at 0°C to quench the reaction, and the pH was adjusted to about 5 with 1 M aqueous hydrochloric acid solution. The resulting mixture was extracted three times with dichloromethane (50 mL). The organic layers were combined, dried over anhydrous sodium sulfate, and concentrated under vacuum to obtain crude compound **C48-7** and compound **C48-7a** (500 mg). LC-MS: m/z = 239.1 (M+H)⁺.

### Step 7: Synthesis of intermediate C48

Compound **C48-7** and compound **C48-7a** (500 mg) were dissolved in anhydrous dichloromethane (5 mL), and thionyl chloride (1.5 mL) was added and the resulting mixture was stirred at room temperature of 25°C for 1.5 hours. Then, the reaction solution was directly concentrated under vacuum to obtain a crude product, which was purified by column chromatography (eluent: petroleum ether/ethyl acetate = 1/0 - 0/1) to obtain compound **C48** (130 mg). LC-MS: m/z = 257.1 (M+H)⁺.

### Synthesis of general intermediate C49 (2-(chloromethyl)-5-(1-methyl-4-(trifluoromethyl)-1H-imidazol-2-yl)pyridine)

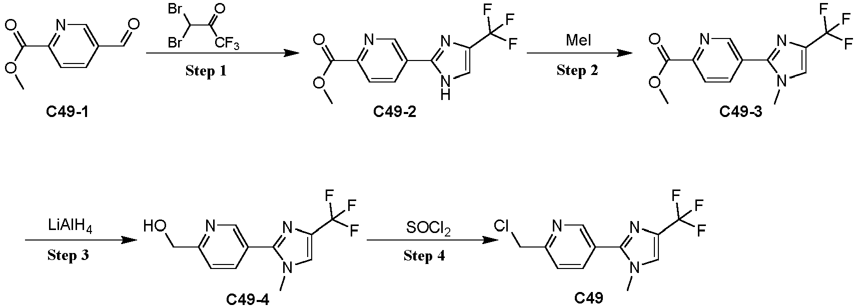

In accordance with the same steps of intermediate **C5,** intermediate **C49** (2.0 g) was obtained using methyl 5-formylpyridine-2-carboxylate (compound **C49-1)** as starting material. LC-MS: m/z = 276.0 (M+H)⁺.

### Synthesis of general intermediate C57 (2-(4-(chloromethyl)phenyl)-1-cyclopropyl-4-(trifluoromethyl)-1H-imidazole)

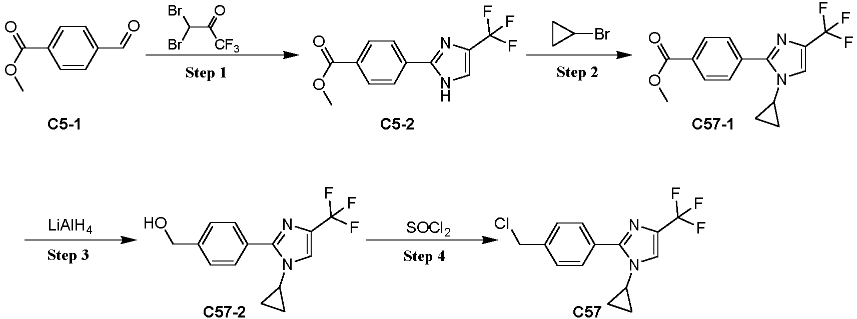

In accordance with the same steps of intermediate **C5,** intermediate **C57** (1.83 g) was obtained using methyl p-formylbenzoate (compound **C5-1)** as starting material. LC-MS: m/z = 301.2 (M+H)⁺.

### Synthesis of general intermediate C58 (2-(4-(chloromethyl)phenyl)-1-isopropyl-4-(trifluoromethyl)-1H-imidazole)

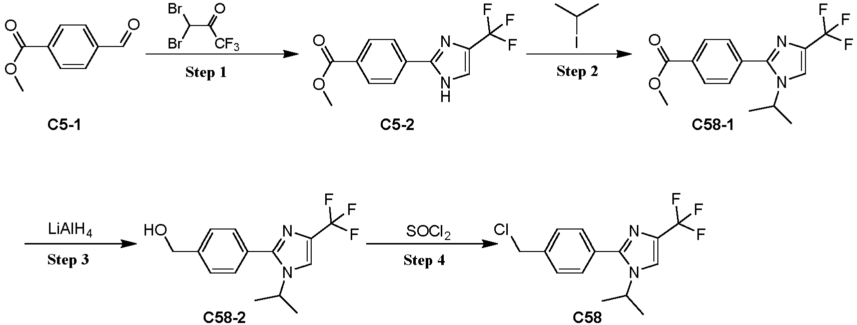

In accordance with the same steps of intermediate **C5,** intermediate **C58** (0.6 g) was obtained using methyl p-formylbenzoate (compound **C5-1)** as starting material. ¹H NMR (400 MHz, DMSO-*d*₆) δ 8.22 (s, 1H), 7.61-7.64 (m, 4H), 4.88 (d, *J =* 2.4 Hz, 2H), 4.50-4.55 (m, 1H), 1.44 (d, *J =* 6.6 Hz, 6H); LC-MS: m/z = 303.1 (M+H)⁺.

### Synthesis of general intermediate C59 (2-(4-(chloromethyl)phenyl)-1-(fluoromethyl)-4-(trifluoromethyl)-1H-imidazole)

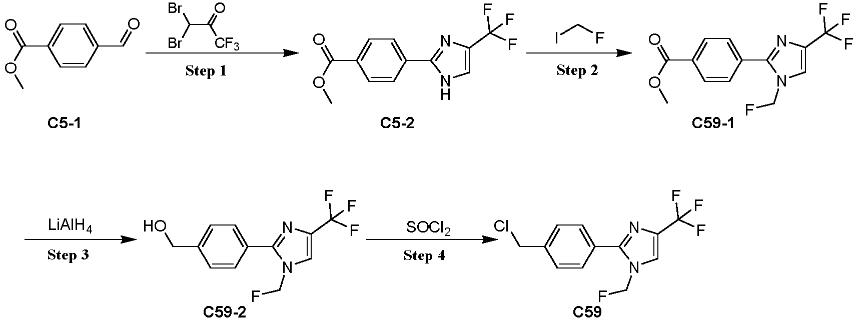

In accordance with the same steps of intermediate **C5,** intermediate **C59** (0.6 g) was obtained using methyl *p*-formylbenzoate (compound **C5-1)** as starting material. LC-MS: m/z = 293.1 (M+H)⁺.

### Synthesis of general intermediate D5 ((4-(1-methyl-4-(trifluoromethyl)-1H-imidazol-2-yl)phenyl)methanamine)

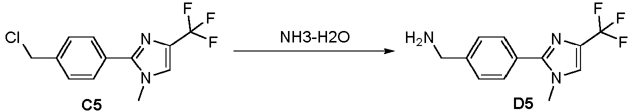

In accordance with the same steps of intermediate **BB13,** intermediate **D5** (0.16 g, 0.63 mmol, 85.9% yield) was obtained using compound **C5** (0.2 g, 0.73 mmol, 1.00 *eq)* as starting material. ¹H NMR (400 MHz, DMSO-d6) δ 7.92 (t, J = 1.4 Hz, 1H), 7.75 - 7.61 (m, 2H), 7.50 (d, J = 8.0 Hz, 2H), 3.83 (s, 2H), 3.80 (s, 3H); LC-MS: m/z = 256.0 (M+H)⁺.

### Synthesis of general intermediate D11 ((4-(5-methyl-3-(trifluoromethyl)-1H-pyrazol-1-yl)phenyl)methanamine)

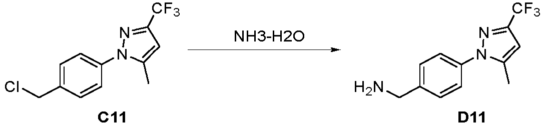

In accordance with the same steps of intermediate **BB13,** intermediate **D11** (1.59 g, 6.23 mmol, 77.6% yield) was obtained using compound **C11** (2.2 g, 8.03 mmol, 1.00 *eq)* as starting material. ¹H NMR (400 MHz, DMSO-d6) δ 7.60 - 7.47 (m, 4H), 6.76 (s, 1H), 3.84 (s, 2H), 2.35 (s, 3H); LC-MS: m/z = 256.1 (M+H)⁺.

### Synthesis of general intermediate D58 ((4-(1-isopropyl-4-(trifluoromethyl)-1H-imidazol-2-yl)phenyl)methanamine)

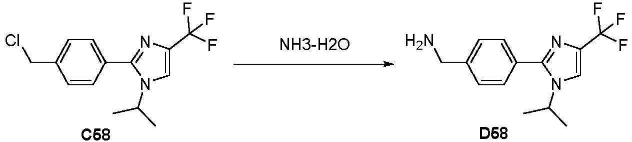

In accordance with the same steps of intermediate **BB13,** intermediate **D58** (0.98 g, 3.46 mmol, 69.9% yield) was obtained using compound **C58** (1.5 g, 4.95 mmol, 1.00 *eq)* as starting material. LC-MS: m/z = 284.3 (M+H)⁺.

### Synthesis of intermediate BB1C2 (2-chloro-5-methoxy-N-methyl-N-((4-(pyridin-2-yl)cyclohexyl)methyl)pyrimidin-4-amine)

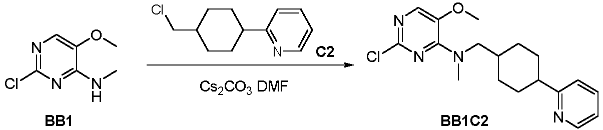

Compound **BB1** (500.0 mg, 2.89 mmol, 1.00 eq), compound **C2** (634.4 mg, 3.04 mmol, 1.05 eq) and cesium carbonate (3.77 g, 11.56 mmol, 4.0 eq) were added to N,N-dimethylformamide (10.0 mL) successively, and the resulting mixture was stirred at 80°C for 2 hours. The reaction solution was cooled to 25°C. Ethyl acetate (100.0 mL) was added, the resulting mixture was stirred for 5 minutes, and filtered to obtain a filtrate. The filtrate was washed three times with saturated brine (100.0 mL), dried over anhydrous sodium sulfate, and concentrated under vacuum to obtain a crude product, which was purified by column chromatography (eluent: petroleum ether/ethyl acetate = 1/0 - 1/2) to obtain intermediate **BB1C2** (910.3 mg, 2.63 mmol, 91.0% yield) as a light yellow solid. LC-MS: m/z = 347.2 (M+H)⁺.

### Synthesis of intermediate BB1C3 (2-chloro-5-methoxy-N-methyl-N-(4-(pyridin-2-yl)benzyl)pyrimidin-4-amine)

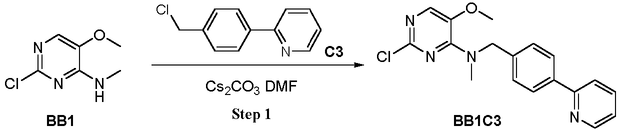

In accordance with the same steps of intermediate **BB1C2,** intermediate **BB1C3** (689.9 mg, 2.03 mmol, 87.5% yield, a light yellow solid) was obtained using intermediate **BB1** and compound **C3** as starting materials. LC-MS: m/z = 341.2 (M+H)⁺.

### Synthesis of intermediate BB1C4 (2-chloro-5-methoxy-N-methyl-N-((1-(1-methyl-4-(trifluoromethyl)-1H-imidazol-2-yl)piperidin-4-yl)methyl)pyrimidin-4-amine)

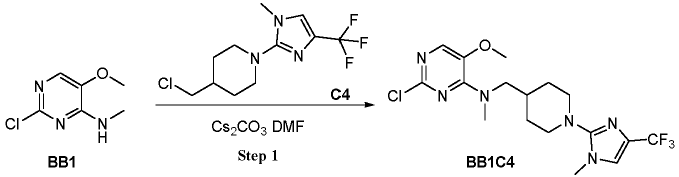

In accordance with the same steps of intermediate **BB1C2,** intermediate **BB1C4** (352.5 mg, 0.843 mmol, 69.5% yield, a light yellow solid) was obtained using intermediate **BB1** and compound **C4** as starting materials. LC-MS: m/z = 419.2 (M+H)⁺.

### Synthesis of intermediate BB1C5 (2-chloro-5-methoxy-N-methyl-N-(4-(1-methyl-4-(trifluoromethyl)-1H-imidazol-2-yl)benzyl)pyrimidin-4-amine)

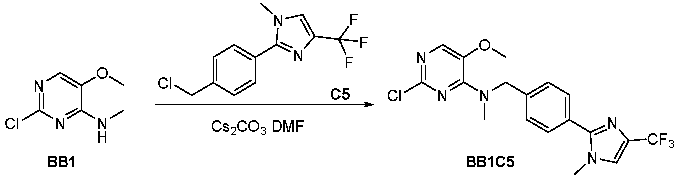

In accordance with the same steps of intermediate **BB1C2,** intermediate **BB1C5** (2.39 g, 5.81 mmol, 81.6% yield, a light yellow solid) was obtained using intermediate **BB1** and compound **C5** as starting materials. ¹H NMR (400 MHz, DMSO-*d*₆) δ 8.03 - 7.90 (m, 2H), 7.80 - 7.72 (m, 2H), 7.41 (d, *J =* 8.3 Hz, 2H), 4.98 (s, 2H), 3.18 (s, 3H), 2.92 (s, 3H), 2.76 (d, *J =* 0.6 Hz, 3H); LC-MS: m/z = 412.1 (M+H)⁺.

### Synthesis of intermediate BB1C6 (2-chloro-5-methoxy-N-methyl-N-(1-(pyridin-2-yl)piperidin-4-yl)methyl)pyrimidin-4-amine)

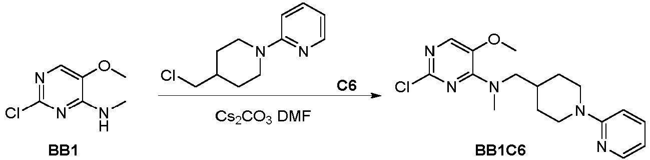

In accordance with the same steps of intermediate **BB1C2,** intermediate **BB1C6** (169.7 mg, 0.49 mmol, 59.9% yield, a light yellow solid) was obtained using intermediate **BB1** and compound **C6** as starting materials. LC-MS: m/z = 348.2 (M+H)⁺.

### Synthesis of intermediate BB1C11 (2-chloro-5-methoxy-N-methyl-N-(4-(5-methyl-3-(trifluoromethyl)-1H-pyrazol-1-yl)benzyl)pyrimidin-4-amine)

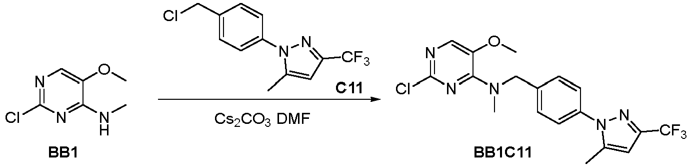

In accordance with the same steps of intermediate **BB1C2,** intermediate **BB1C11** (3.31g, 8.04 mmol, 73.2% yield, an off-white solid) was obtained using intermediate **BB1** and compound **C11** as starting materials. LC-MS: m/z = 412.8 (M+H)⁺.

### Synthesis of intermediate BB2C2 (2-chloro-5-methoxy-N-((4-(pyridin-2-yl)cyclohexyl)methyl)pyrimidin-4-amine)

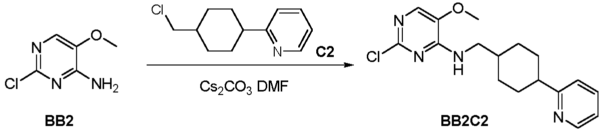

In accordance with the same steps of intermediate **BB1C2,** intermediate **BB2C2** (134.1 mg, 0.40 mmol, 46.6% yield, a light yellow oil) was obtained using intermediate **BB2** and compound **C2** as starting materials. LC-MS: m/z = 333.1 (M+H)⁺.

### Synthesis of intermediate BB2C3 (2-chloro-5-methoxy-N-(4-(pyridin-2-yl)benzyl)pyrimidin-4-amine)

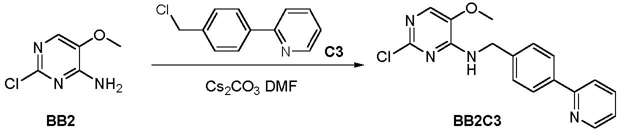

In accordance with the same steps of intermediate **BB1C2,** intermediate **BB2C3** (255.5 mg, 0.78 mmol, 87.7% yield, a light yellow solid) was obtained using intermediate **BB2** and compound **C3** as starting materials. LC-MS: m/z = 327.0 (M+H)⁺.

### Synthesis of intermediate BB2C4 (2-chloro-5-methoxy-N-((1-(1-methyl-4-(trifluoromethyl)-1H-imidazol-2-yl)piperidin-4-yl)methyl)pyrimidin-4-amine)

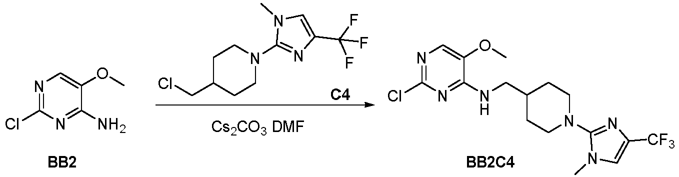

In accordance with the same steps of intermediate **BB1C2,** intermediate **BB2C4** (98.8 mg, 0.24 mmol, 39.2% yield, a light yellow solid) was obtained using intermediate **BB2** and compound **C4** as starting materials. LC-MS: m/z = 405.1 (M+H)⁺.

### Synthesis of intermediate BB2C5 (2-chloro-5-methoxy-N-((4-(1-methyl-4-(trifluoromethyl)-1H-imidazol-2-yl)benzyl)pyrimidin-4-amine)

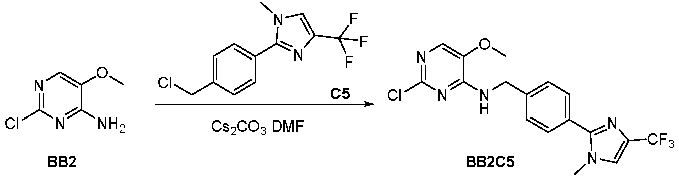

In accordance with the same steps of intermediate **BB1C2,** intermediate **BB2C5** (18.9 g, 47.60 mmol, 69.8% yield, a light yellow solid) was obtained using intermediate **BB2** and compound **C5** as starting materials. ¹H NMR (400 MHz, CDCl₃) δ 7.60 - 7.62 (m, 2H), 7.57 (s, 1H), 7.42 - 7.44 (m, 2H), 7.27 - 7.31 (m, 1H), 5.82 (s, 1H), 4.73 - 4.74 (m, 2H), 3.87 (s, 3H), 3.76 (s, 3H); LC-MS: m/z = 398.6 (M+H)⁺.

### Synthesis of intermediate BB2C6 (2-chloro-5-methoxy-N-((1-(pyridin-2-yl)piperidin-4-yl)methyl)pyrimidin-4-amine)

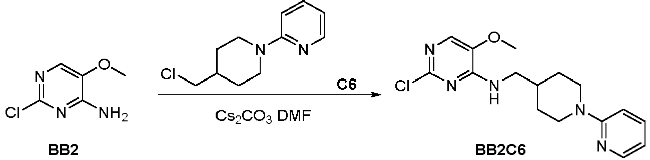

In accordance with the same steps of intermediate **BB1C2,** intermediate **BB2C6** (100.6 mg, 0.30 mmol, 35.3% yield, a light yellow solid) was obtained using intermediate **BB2** and compound **C6** as starting materials. LC-MS: m/z = 344.1 (M+H)⁺.

### Synthesis of intermediate BB2C9 (2-chloro-5-methoxy-N-(((1R,4R)-4-(1-methyl-4-(trifluoromethyl)-1H-imidazol-2-yl)cyclohexyl)methyl)pyrimidin-4-amine)

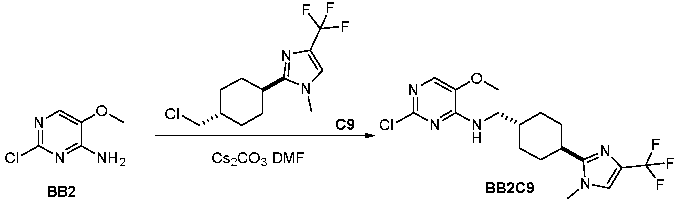

In accordance with the same steps of intermediate **BB1C2,** intermediate **BB2C9** (31.1 mg, 0.077 mmol, 13.2% yield, a light yellow solid) was obtained using intermediate **BB2** and compound **C9** as starting materials. LC-MS: m/z = 404.2 (M+H)⁺.

### Synthesis of intermediate BB2C11 (2-chloro-5-methoxy-N-(4-(5-methyl-3-(trifluoromethyl)-1H-pyrazol-1-yl)benzyl)pyrimidin-4-amine)

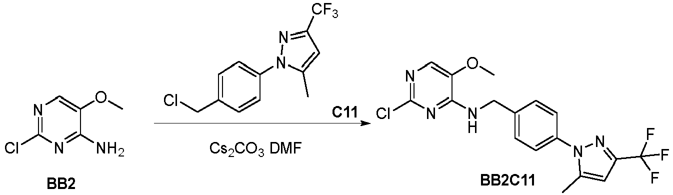

In accordance with the same steps of intermediate **BB1C2,** intermediate **BB2C11** (5.6 g, 14.10 mmol, 77.8% yield, a white solid) was obtained using intermediate **BB2** and compound **C11** as starting materials. ¹H NMR (400 MHz, CDCl₃) δ 7.49 (s, 1H), 7.43 - 7.47 (m, 4H), 6.47 - 6.49 (m, 1H), 5.76 (s, 1H), 4.75 - 4.82 (m, 2H), 3.90 (s, 3H), 2.36 (s, 3H); LC-MS: m/z = 398.0 (M+H)⁺.

### Synthesis of intermediate BB2C12 (2-chloro-N-(4-(imidazo[1,2-a]pyrazin-8-yl)benzyl)-5-methoxypyrimidin-4-amine)

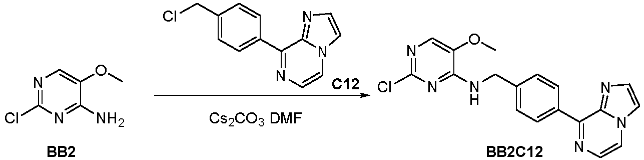

In accordance with the same steps of intermediate **BB1C2,** intermediate **BB2C12** (130.3 mg, 0.36 mmol, 88.2% yield, a light yellow solid) was obtained using intermediate **BB2** and compound **C12** as starting materials. ¹H NMR (400 MHz, DMSO) δ 8.71 (d, *J* = 8.4 Hz, 2H), 8.60 (d, *J =* 4.4 Hz, 1H), 8.19 - 8.21 (m, 2H), 7.99 (d, *J =* 4.4 Hz, 1H), 7.87 (s, 1H), 7.72 (s, 1H), 7.46 (d, *J = 8.4* Hz, 2H), 4.61 (d, *J=* 4.4 Hz, 2H), 3.88 (s, 3H); LC-MS: m/z = 367.0 (M+H)⁺.

### Synthesis of intermediate BB2C13 (2-chloro-5-methoxy-N-(4-(5-(trifluoromethyl)pyridin-2-yl)benzyl)pyrimidin-4-amine)

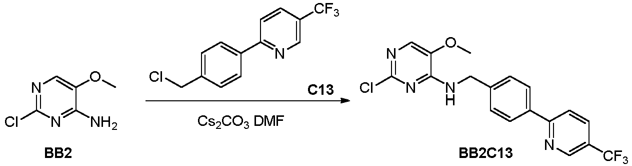

In accordance with the same steps of intermediate **BB1C2,** intermediate **BB2C13** (455.1 mg, 1.16 mmol, 69.9% yield, a light yellow solid) was obtained using intermediate **BB2** and compound C13 as starting materials. LC-MS: m/z = 395.0 (M+H)⁺.

### Synthesis of intermediate BB2C14 (2-chloro-5-methoxy-N-(4-(5-methylpyridin-2-yl)benzyl)pyrimidin-4-amine)

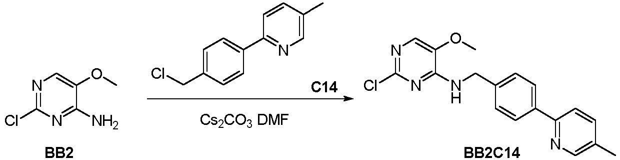

In accordance with the same steps of intermediate **BB1C2,** intermediate **BB2C14** (173.45 mg, 0.51 mmol, 90.3% yield, a light yellow solid) was obtained using intermediate **BB2** and compound **C14** as starting materials. LC-MS: m/z = 341.1 (M+H)⁺.

### Synthesis of intermediate BB2C19 (2-chloro-5-methoxy-N-((4-(5-(trifluoromethyl)pyridin-2-yl)bicyclo [2.2.2] octan-1-yl)methyl)pyrimidin-4-amine)

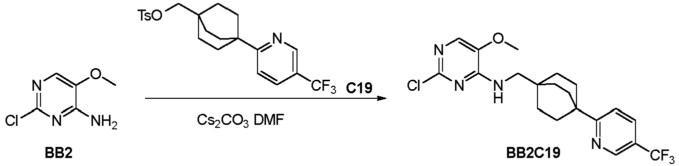

In accordance with the same steps of intermediate **BB1C2,** intermediate **BB2C19** (281.3 mg, 0.66 mmol, 35.6% yield, a light yellow solid) was obtained using intermediate **BB2** and compound **C19** as starting materials. LC-MS: m/z = 427.3 (M+H)⁺.

### Synthesis of intermediate BB2C26 (6-((2-chloro-5-methoxypyrimidin-4-yl)amino)methyl)-2-isopropyl-3,4-dihydroisoquinolin-1(2H)-one)

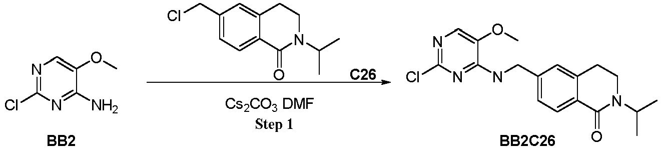

In accordance with the same steps of intermediate **BB1C2,** intermediate **BB2C26** (379.2 mg, 1.05 mmol, 82.0% yield, a light yellow solid) was obtained using intermediate **BB2** and compound **C26** as starting materials. LC-MS: m/z = 361.1 (M+H)⁺.

### Synthesis of intermediate BB2C27 (2-chloro-5-methoxy-N-(4-(pyridin-2-ylmethoxy)benzyl)pyrimidin-4-amine)

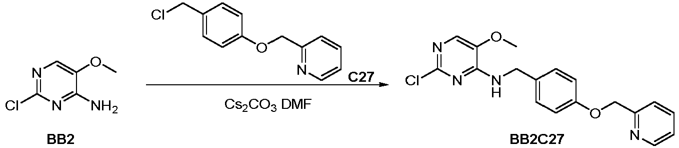

In accordance with the same steps of intermediate **BB1C2,** intermediate **BB2C27** (268.5 mg, 0.61 mmol, 93.6% yield, a light yellow solid) was obtained using intermediate **BB2** and compound **C27** as starting materials. ¹H NMR (400 MHz, CDCl₃) δ 8.62 (d, *J* = 4.8Hz, 1H), 7.72 - 7.76 (m, 1H), 7.72 - 7.75 (m, 2H), 7.29 (s, 1H), 7.24 - 7.25 (m, 1H), 6.99 (d, *J =* 8.4Hz, 2H), 5.61 (s, 1H), 5.23 (s, 2H), 4.60 (d, *J =* 5.6Hz, 2H), 3.85 (s, 3H); LC-MS: m/z = 357.0 (M+H)⁺.

### Synthesis of intermediate BB2C28 (2-chloro-N-(4-(2-ethoxyethoxy)benzyl)-5-methoxypyrimidin-4-amine)

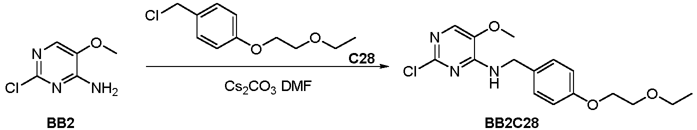

In accordance with the same steps of intermediate **BB1C2,** intermediate **BB2C28** (316.9 mg, 0.94 mmol, 78.8% yield, a white solid) was obtained using intermediate **BB2** and compound **C28** as starting materials. ¹H NMR (400 MHz, CDCl₃) δ 7.53 (s, 1H), 7.24 - 7.29 (m, 2H), 6.89 - 6.94 (m, 2H), 5.61 (s, 1H), 4.59 (d, *J =* 5.6 Hz, 2H), 4.10 - 4.14 (m, 2H), 3.84 (s, 3H), 3.76 - 3.81 (m, 2H), 3.60 - 3.62 (m, 2H), 1.25 (t, *J =* 6.8 Hz, 3H); LC-MS: m/z = 338.1 (M+H)⁺.

### Synthesis of intermediate BB2C30 (2-chloro-5-methoxy-N-(2-methoxy-4-(1-methyl-4-(trifluoromethyl)-1H-imidazol-2-yl)benzyl)pyrimidin-4-amine)

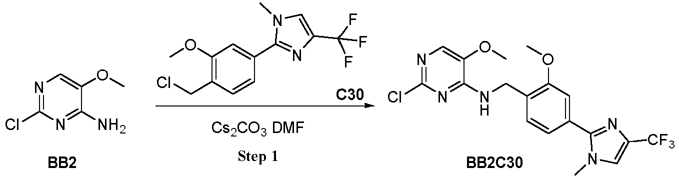

In accordance with the same steps of intermediate **BB1C2,** intermediate **BB2C30** (662.0 mg, 1.55 mmol, 86.7% yield, a light yellow solid) was obtained using intermediate **BB2** and compound C30 as starting materials. LC-MS: m/z = 428.0 (M+H)⁺.

### Synthesis of intermediate BB2C31 (2-chloro-5-methoxy-N-(3-methoxy-4-(1-methyl-4-(trifluoromethyl)-1H-imidazol-2-yl)benzyl)pyrimidin-4-amine)

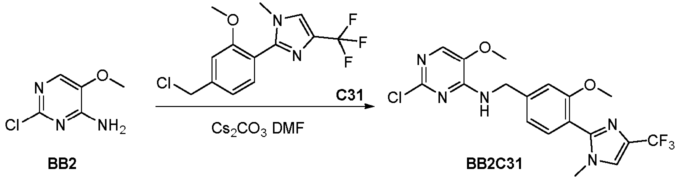

In accordance with the same steps of intermediate **BB1C2,** intermediate **BB2C31** (337.4 mg, 0.79 mmol, 93.9% yield, a white solid) was obtained using intermediate **BB2** and compound **C31** as starting materials. LC-MS: m/z = 428.1 (M+H)⁺.

### Synthesis of intermediate BB2C33 (2-chloro-5-methoxy-N-(3-methoxy-4-(5-methyl-3-(trifluoromethyl)-1H-pyrazol-1-yl)benzyl)pyrimidin-4-amine)

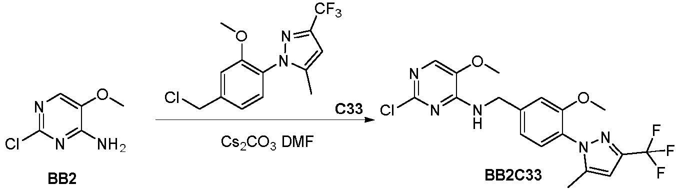

In accordance with the same steps of intermediate **BB1C2,** intermediate **BB2C33** (3.02 g, 2.66 mmol, 88.3% yield, a white solid) was obtained using intermediate **BB2** and compound **C33** as starting materials. LC-MS: m/z = 428.0 (M+H)⁺.

### Synthesis of intermediate BB2C35 (2-chloro-5-methoxy-N-((4-(1-methyl-4-(trifluoromethyl)-1H-imidazol-2-yl)bicyclo [2.2.2] octan-1-yl)methyl)pyrimidin-4-amine)

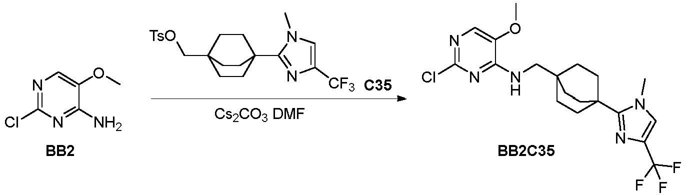

Compound **BB2** (1.0 g, 6.29 mmol, 1.00 eq), compound **C35** (2.78 g, 6.29 mmol, 1.0 eq) and cesium carbonate (10.2 g, 31.45 mmol, 5.0 eq) were added to N,N-dimethylformamide (20.0 mL) successively, and the resulting mixture was stirred at 130°C for 36 hours. The reaction solution was cooled to 25°C. Dichloromethane (100.0 mL) was added, the resulting mixture was stirred for 5 minutes, and filtered to obtain a filtrate. The filtrate was washed three times with saturated brine (50.0 mL), dried over anhydrous sodium sulfate, and concentrated under vacuum to obtain a crude product, which was purified by column chromatography (eluent: petroleum ether/dichloromethane = 1/0 - 0/1) to obtain intermediate **BB2C35** (1.03 g, 2.41 mmol, 38.3% yield) as a white solid. LC-MS: m/z = 430.1 (M+H)⁺.

### Synthesis of intermediate BB2C48 (2-chloro-N-(4-(4-(difluoromethyl)-1-methyl-1H-imidazol-2-yl)benzyl)-5-methoxypyrimidin-4-amine)

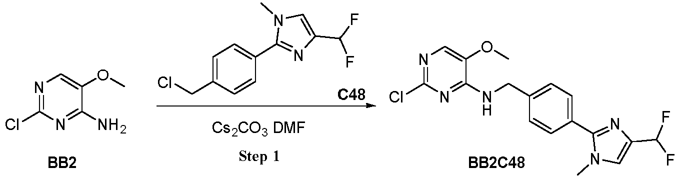

In accordance with the same steps of intermediate **BB1C2,** intermediate **BB2C48** (371.5 mg, 0.98 mmol, 77.5% yield, a white solid) was obtained using intermediate **BB2** and compound **C48** as starting materials. LC-MS: m/z = 380.3 (M+H)⁺.

### Synthesis of intermediate BB2C58 (2-chloro-N-(4-(1-isopropyl-4-(trifluoromethyl)-1H-imidazol-2-yl)benzyl)-5-methoxypyrimidin-4-amine)

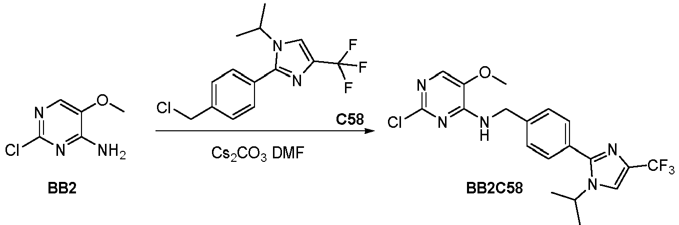

In accordance with the same steps of intermediate **BB1C2,** intermediate **BB2C58** (357.1 mg, 0.84 mmol, 97.0% yield, a light yellow solid) was obtained using intermediate **BB2** and compound **C58** as starting materials. ¹H NMR (400 MHz, DMSO-*d*₆) δ 8.31 - 8.15 (m, 2H), 7.75 (s, 1H), 7.54 (d, *J = 8.1* Hz, 2H), 7.45 (d, *J = 8.1* Hz, 2H), 4.63 (d, *J =* 6.3 Hz, 2H), 4.56 - 4.38 (m, 1H), 3.90 (s, 3H), 1.42 (d, *J = 6.6* Hz, 6H); LC-MS: m/z = 426.1 (M+H)⁺.

### Synthesis of intermediate BB2C59 (2-chloro-N-(4-(1-(fluoromethyl)-4-(trifluoromethyl)-1H-imidazol-2-yl)benzyl)-5-methoxypyrimidin-4-amine)

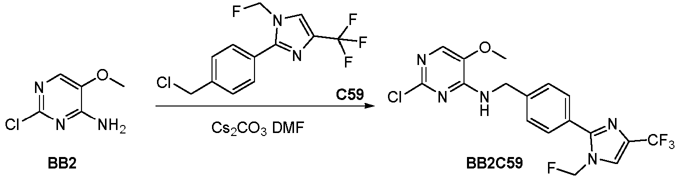

In accordance with the same steps of intermediate **BB1C2,** intermediate **BB2C59** (91.3 mg, 0.22 mmol, 78.8% yield, a white solid) was obtained using intermediate **BB2** and compound **C59** as starting materials. LC-MS: m/z = 426.1 (M+H)⁺.

### Synthesis of intermediate BB3C4 (2-chloro-5-isopropoxy-N-methyl-N-((1-(1-methyl-4-(trifluoromethyl)-1H-imidazol-2-yl)piperidin-4-yl)methyl)pyrimidin-4-amine)

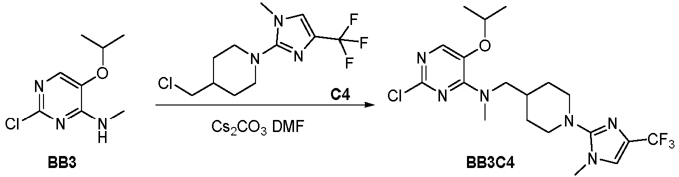

In accordance with the same steps of intermediate **BB2C35,** intermediate **BB3C4** (178.5 mg, 0.40 mmol, 28.7% yield, a white solid) was obtained using intermediate **BB3** and compound **C4** as starting materials. LC-MS: m/z = 447.3 (M+H)⁺.

### Synthesis of intermediate BB3C5 (2-chloro-5-isopropoxy-N-methyl-N-(4-(1-methyl-4-(trifluoromethyl)-1H-imidazol-2-yl)benzyl)pyrimidin-4-amine)

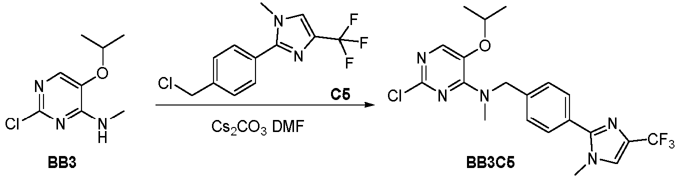

In accordance with the same steps of intermediate **BB1C2,** intermediate **BB3C5** (2.1 g, 4.78 mmol, 86.4% yield, a white solid) was obtained using intermediate **BB3** and compound **C5** as starting materials. LC-MS: m/z = 440.2 (M+H)⁺.

### Synthesis of intermediate BB4C4 (2-chloro-5-isopropoxy-N-((1-(1-methyl-4-(trifluoromethyl)-1H-imidazol-2-yl)piperidin-4-yl)methyl)pyrimidin-4-amine)

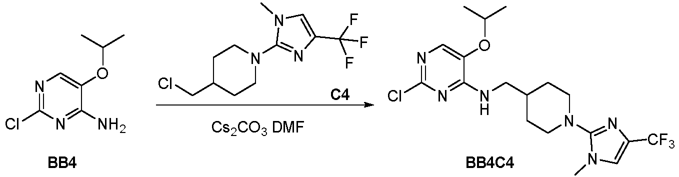

In accordance with the same steps of intermediate **BB2C35,** intermediate **BB4C4** (159.9 mg, 0.37 mmol, 39.6% yield, a white solid) was obtained using intermediate **BB4** and compound **C4** as starting materials. LC-MS: m/z = 433.2 (M+H)⁺.

### Synthesis of intermediate BB4C5 (2-chloro-5-isopropoxy-N-(4-(1-methyl-4-(trifluoromethyl)-1H-imidazol-2-yl)benzyl)pyrimidin-4-amine)

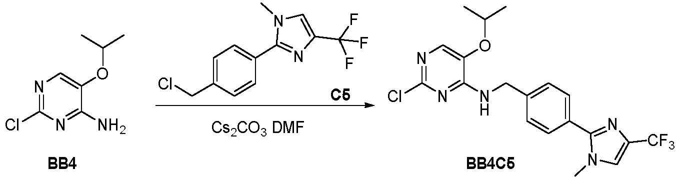

In accordance with the same steps of intermediate **BB1C2,** intermediate **BB4C5** (2.03 g, 4.78 mmol, 86.4% yield, a white solid) was obtained using intermediate **BB4** and compound **C5** as starting materials. LC-MS: m/z = 426.1 (M+H)⁺.

### Synthesis of intermediate BB4C19 (2-chloro-5-isopropoxy-N-((4-(5-(trifluoromethyl)pyridin-2-yl)bicyclo [2.2.2] octan-1-yl)methyl)pyrimidin-4-amine)

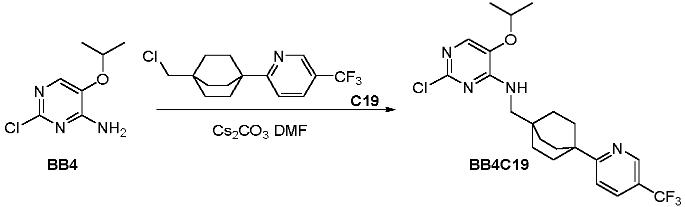

In accordance with the same steps of intermediate **BB1C2,** intermediate **BB4C19** (349.7 mg, 0.77 mmol, 83.2% yield, a white solid) was obtained using intermediate **BB4** and compound **C19** as starting materials. LC-MS: m/z = 455.2 (M+H)⁺.

### Synthesis of intermediate BB12C2 (2-chloro-N-((4-(pyridin-2-yl)cyclohexyl)methyl)-5-(trifluoromethyl)pyrimidin-4-amine)

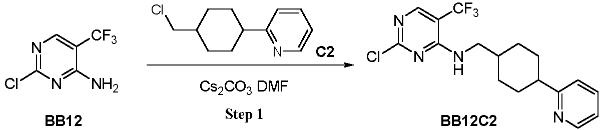

In accordance with the same steps of intermediate **BB2C35,** intermediate **BB12C2** (51.8 mg, 0.14 mmol, 35.4% yield, a white solid) was obtained using intermediate **BB12** and compound **C2** as starting materials. LC-MS: m/z = 371.1 (M+H)⁺.

### Synthesis of intermediate BB12C3 (2-chloro-N-(4-(pyridin-2-yl)benzyl)-5-(trifluoromethyl)pyrimidin-4-amine)

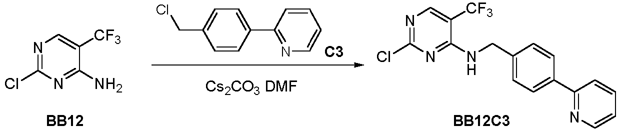

In accordance with the same steps of intermediate **BB1C2,** intermediate **BB12C3** (396.8 mg, 1.09 mmol, 76.6% yield, a light yellow solid) was obtained using intermediate **BB12** and compound **C3** as starting materials. LC-MS: m/z = 365.0 (M+H)⁺.

### Synthesis of intermediate BB12C4 (2-chloro-N-((1-(1-methyl-4-(trifluoromethyl)-1H-imidazol-2-yl)piperidin-4-yl)methyl)-5-(trifluoromethyl)pyrimidin-4-amine)

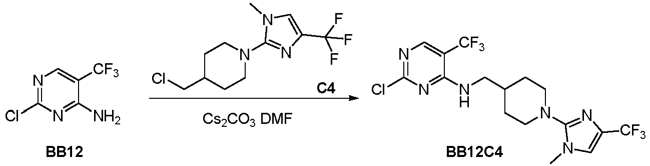

In accordance with the same steps of intermediate **BB2C35,** intermediate **BB12C4** (53.1 mg, 0.12 mmol, 21.1% yield, a light yellow oil) was obtained using intermediate **BB12** and **C4** as starting materials. LC-MS: m/z = 443.0 (M+H)⁺.

### Synthesis of intermediate BB12C5 (2-chloro-N-(4-(1-methyl-4-(trifluoromethyl)-1H-imidazol-2-yl)benzyl)-5-(trifluoromethyl)pyrimidin-4-amine)

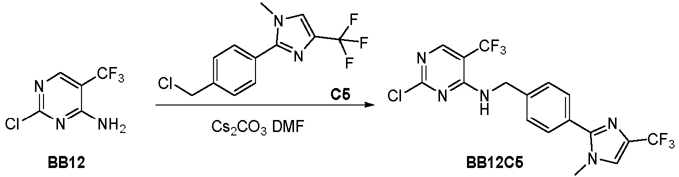

In accordance with the same steps of intermediate **BB1C2,** intermediate **BB12C5** (248.0 mg, 0.57 mmol, 88.8% yield, a light yellow solid) was obtained using intermediate **BB12** and compound **C5** as starting materials. LC-MS: m/z = 436.1 (M+H)⁺.

### Synthesis of intermediate BB12C6 (2-chloro-N-((1-(pyridin-2-yl)piperidin-4-yl)methyl)-5-(trifluoromethyl)pyrimidin-4-amine)

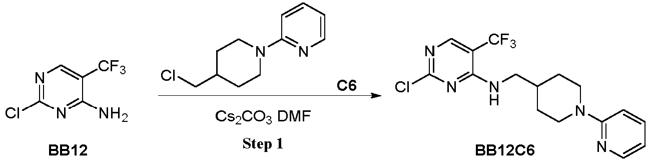

In accordance with the same steps of intermediate **BB2C35,** intermediate BB12C6 (29.7 mg, 0.08 mmol, 18.0% yield, a light yellow oil) was obtained using intermediate **BB12** and compound **C6** as starting materials. LC-MS: m/z = 372.3 (M+H)⁺.

### Synthesis of intermediate BB13C3 (2-chloro-N-(4-(pyridin-2-yl)benzyl)furo[3,2-d]pyrimidin-4-amine)

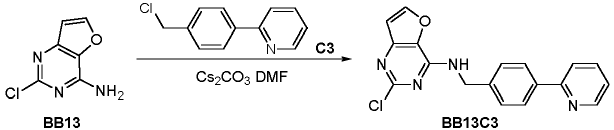

In accordance with the same steps of intermediate **BB1C2,** intermediate **BB13C3** (1.19 g, 3.55 mmol, 89.4% yield, a light yellow solid) was obtained using intermediate **BB13** and compound **C3** as starting materials. LC-MS: m/z = 337.1 (M+H)⁺.

### Synthesis of intermediate BB13C5 (2-chloro-N-(4-(1-methyl-4-(trifluoromethyl)-1H-imidazol-2-yl)benzyl)furo[3,2-d]pyrimidin-4-amine)

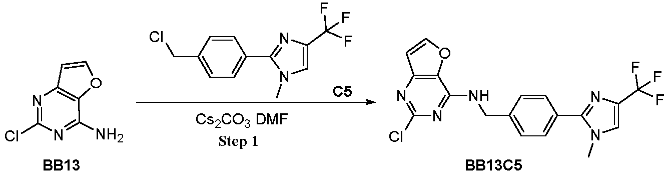

In accordance with the same steps of intermediate **BB1C2,** intermediate **BB13C5** (761.1 mg, 1.87 mmol, 76.7% yield, a light yellow solid) was obtained using intermediate **BB13** and compound **C5** as starting materials. ¹H NMR (400 MHz, DMSO) δ 9.00 (s, 1H), 8.29-8.33 (m, 1H), 7.90-7.94 (m, 1H), 7.67-7.72 (m, 2H), 7.46-7.52 (m, 2H), 6.96-7.00 (m, 1H), 4.74 (s, 2H), 3.77 (s, 3H); C-MS: m/z = 407.9 (M+H)⁺.

### Synthesis of intermediate BB13C7 (2-chloro-N-((5-(1-methyl-4-(trifluoromethyl)-1H-imidazol-2-yl)thiophen-2-yl)methyl)furo[3,2-d]pyrimidin-4-amine)

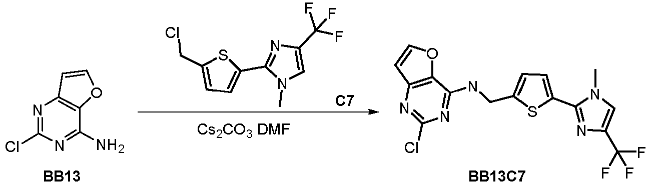

In accordance with the same steps of intermediate **BB1C2,** intermediate **BB13C7** (342.6 mg, 0.83 mmol, 56.9% yield, a white solid) was obtained using intermediate **BB13** and compound C7 as starting materials. LC-MS: m/z = 414.0 (M+H)⁺.

### Synthesis of intermediate BB13C11 (2-chloro-N-(4-(5-methyl-3-(trifluoromethyl)-1H-pyrazol-1-yl)benzyl)furo[3,2-d]pyrimidin-4-amine)

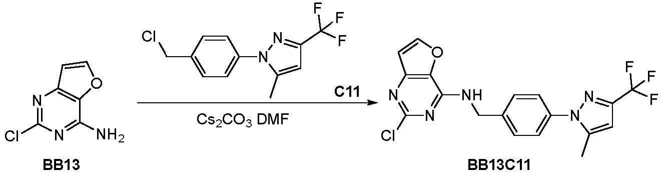

In accordance with the same steps of intermediate **BB1C2,** intermediate **BB13C11** (4.23 g, 10.39 mmol, 76.9% yield, a light yellow solid) was obtained using intermediate **BB13** and compound **C11** as starting materials. LC-MS: m/z = 408.0 (M+H)⁺.

### Synthesis of intermediate BB13C12 (2-chloro-N-(4-(imidazo[1,2-a]pyrazin-8-yl)benzyl)furo[3,2-d]pyrimidin-4-amine)

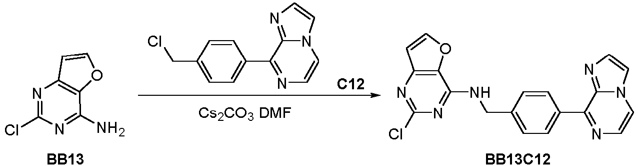

In accordance with the same steps of intermediate **BB1C2,** intermediate **BB13C12** (458.8 mg, 1.22 mmol, 88.7% yield, a white solid) was obtained using intermediate **BB13** and compound **C12** as starting materials. ¹H NMR (400 MHz, DMSO) δ 9.07 (s, 1H), 8.74 (d, *J =* 8.4Hz, 2H), 8.60 (d, *J =* 4.4Hz, 1H), 8.27 - 8.31 (m, 1H), 8.21 (s, 1H), 7.99 (d, *J =* 8.4Hz, 1H), 7.87 (s, 1H), 7.53 (d, *J =* 8.0Hz, 2H), 6.93-6.98 (m, 1H), 4.76 (s, 2H); LC-MS: m/z = 377.1 (M+H)⁺.

### Synthesis of intermediate BB13C19 (2-chloro-N-((4-(5-(trifluoromethyl)pyridin-2-yl)bicyclo [2.2.2] octan-1-yl)methyl)furo [3,2-d]pyrimidin-4-amine)

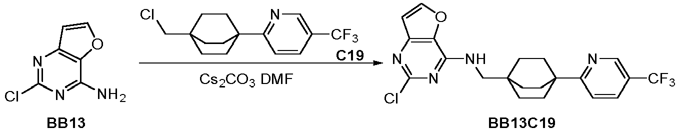

In accordance with the same steps of intermediate **BB2C35,** intermediate **BB13C19** (383.8 mg, 0.88 mmol, 22.3% yield, a light yellow oil) was obtained using intermediate **BB13** and compound **C19** as starting materials. LC-MS: m/z = 437.0 (M+H)⁺.

### Synthesis of intermediate BB13C25 (6-(((2-chlorofuro[3,2-d]pyrimidin-4-yl)amino)methyl)-2-methyl-3,4-dihydroisoquinolin-1(2H)-one)

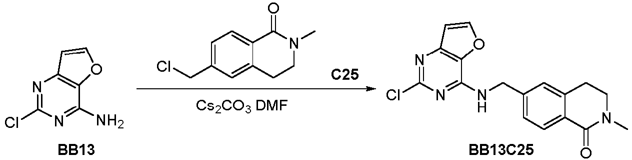

In accordance with the same steps of intermediate **BB1C2,** intermediate **BB13C25** (195.0 mg, 0.57 mmol, 90.4% yield, a white solid) was obtained using intermediate **BB13** and compound **C25** as starting materials. ¹H NMR (400 MHz, DMSO) δ 8.94 (s, 1H), 8.30 (s, 1H), 7.91 (d, *J*=8.0Hz, 1H), 7.29 (d, *J*=8.0Hz, 1H), 7.24 (s, 1H), 6.97 (d, *J*=2.0Hz, 1H), 4.69 (s, 2H),3.52 (t, *J₁*=6.4Hz, *J₂*=6.8Hz, 2H), 3.00 (s, 3H), 2.95 (t, *J₁*=6.4Hz, *J₂*=6.8Hz, 2H); LC-MS: m/z = 343.2 (M+H)⁺.

### Synthesis of intermediate BB13C26 (6-(((2-chlorofuro[3,2-d]pyrimidin-4-yl)amino)methyl)-2-isopropyl-3,4-dihydroisoquinolin-1(2H)-one)

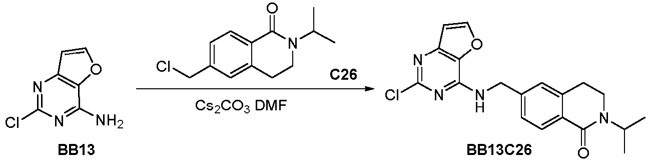

In accordance with the same steps of intermediate **BB1C2,** intermediate **BB13C26** (233.1 mg, 0.63 mmol, 82.6% yield, a light yellow solid) was obtained using intermediate **BB13** and compound **C26** as starting materials. LC-MS: m/z = 371.2 (M+H)⁺.

### Synthesis of intermediate BB13C27 (2-chloro-N-(4-(pyridin-2-ylmethoxy)benzyl)furo[3,2-d]pyrimidin-4-amine)

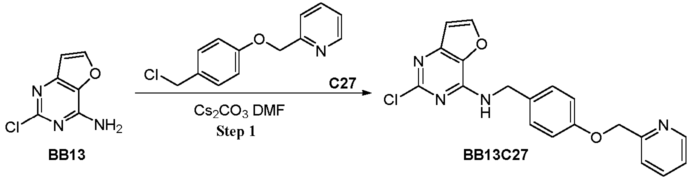

In accordance with the same steps of intermediate **BB1C2,** intermediate **BB13C27** (256.3 mg, 0.70 mmol, 66.8% yield, a light yellow solid) was obtained using intermediate **BB13** and compound **C27** as starting materials. LC-MS: m/z = 366.9 (M+H)⁺.

### Synthesis of intermediate BB13C28 (2-chloro-N-(4-(2-ethoxyethoxy)benzyl)furo[3,2-d]pyrimidin-4-amine)

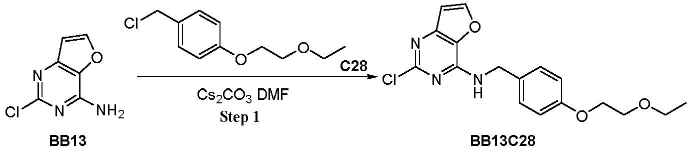

In accordance with the same steps of intermediate **BB1C2,** intermediate **BB13C28** (118.0 mg, 0.34 mmol, 84.5% yield, a light yellow solid) was obtained using intermediate **BB13** and **C28** as starting materials. LC-MS: m/z = 348.2 (M+H)⁺.

### Synthesis of intermediate BB13C29 (2-chloro-N-(4-(3-methoxycyclobutoxy)benzyl)furo[3,2-d]pyrimidin-4-amine)

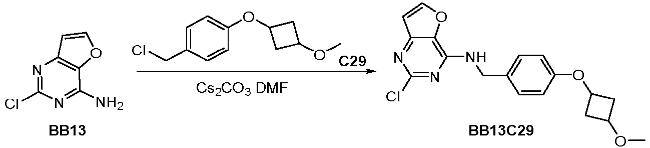

In accordance with the same steps of intermediate **BB1C2,** intermediate **BB13C29** (244.2 mg, 0.68 mmol, 93.5% yield, a light yellow solid) was obtained using intermediate **BB13** and compound **C29** as starting materials. LC-MS: m/z = 360.2 (M+H)⁺.

### Synthesis of intermediate BB13C30 (2-chloro-N-(2-methoxy-4-(1-methyl-4-(trifluoromethyl)-1H-imidazol-2-yl)benzyl)furo[3,2-d]pyrimidin-4-amine)

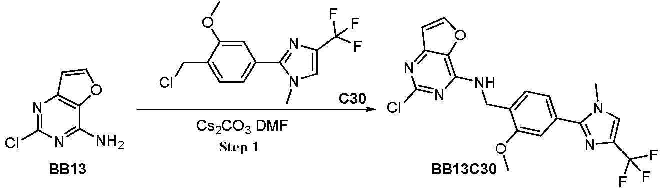

In accordance with the same steps of intermediate **BB1C2,** intermediate **BB13C30** (292.9 mg, 0.67 mmol, 78.8% yield, a white solid) was obtained using intermediate **BB13** and compound **C30** as starting materials. LC-MS: m/z = 438.1 (M+H)⁺.

### Synthesis of intermediate BB13C31 (2-chloro-N-(3-methoxy-4-(1-methyl-4-(trifluoromethyl)-1H-imidazol-2-yl)benzyl)furo[3,2-d]pyrimidin-4-amine)

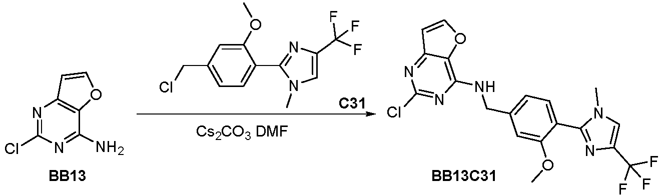

In accordance with the same steps of intermediate **BB1C2,** intermediate **BB13C31** (214.2 mg, 0.49 mmol, 73.9% yield, a white solid) was obtained using intermediate **BB13** and compound **C31** as starting materials. LC-MS: m/z = 438.0 (M+H)⁺.

### Synthesis of intermediate BB13C32 (2-chloro-N-(2-methoxy-4-(5-methyl-3-(trifluoromethyl)-1H-pyrazol-1-yl)benzyl)furo[3,2-d]pyrimidin-4-amine)

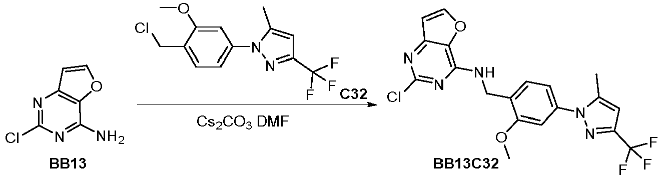

In accordance with the same steps of intermediate **BB1C2,** intermediate **BB13C32** (100.5 mg, 0.23 mmol, 70.0% yield, a white solid) was obtained using intermediate **BB13** and compound **C32** as starting materials. LC-MS: m/z = 438.0 (M+H)⁺.

### Synthesis of intermediate BB13C33 (2-chloro-N-(3-methoxy-4-(5-methyl-3-(trifluoromethyl)-1H-pyrazol-1-yl)benzyl)furo[3,2-d]pyrimidin-4-amine)

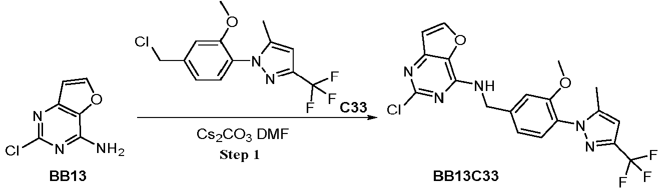

In accordance with the same steps of intermediate **BB1C2,** intermediate **BB13C33** (257.6 mg, 0.59 mmol, 84.2% yield, a light yellow solid) was obtained using intermediate **BB13** and compound **C33** as starting materials. LC-MS: m/z = 437.9 (M+H)⁺.

### Synthesis of intermediate BB13C35 (2-chloro-N-((4-(1-methyl-4-(trifluoromethyl)-1H-imidazol-2-yl)bicyclo [2.2.2] octan-1-yl)methyl)furo [3,2-d]pyrimidin-4-amine)

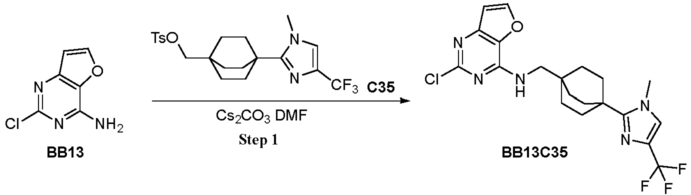

In accordance with the same steps of intermediate **BB2C35,** intermediate **BB13C35** (48.3 mg, 0.11 mmol, 28.8% yield, a white solid) was obtained using intermediate **BB13** and compound **C35** as starting materials. LC-MS: m/z = 440.2 (M+H)⁺.

### Synthesis of intermediate BB13C48 (2-chloro-N-(4-(4-(difluoromethyl)-1-methyl-1H-imidazol-2-yl)benzyl)furo[3,2-d]pyrimidin-4-amine)

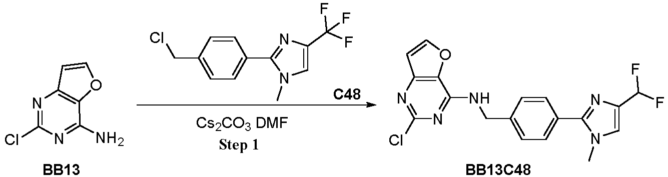

In accordance with the same steps of intermediate **BB1C2,** intermediate **BB13C48** (886.2 mg, 2.28 mmol, 77.9% yield, a white solid) was obtained using intermediate **BB13** and **C48** as starting materials. LC-MS: m/z = 390.1 (M+H)⁺.

### Synthesis of intermediate BB14C5 (2-chloro-N-(4-(1-methyl-4-(trifluoromethyl)-1H-imidazol-2-yl)benzyl)pyrido[3,2-d]pyrimidin-4-amine)

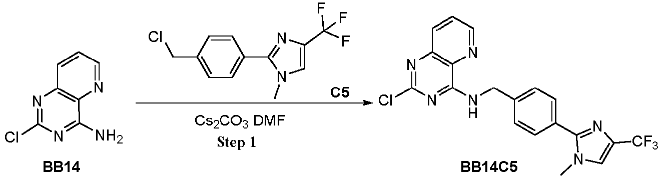

In accordance with the same steps of intermediate **BB1C2,** intermediate **BB14C5** (1.22 g, 2.92 mmol, 93.3% yield, a light yellow solid) was obtained using intermediate **BB14** and compound **C5** as starting materials. LC-MS: m/z = 419.1 (M+H)⁺.

### Synthesis of intermediate BB15C5 (2,5-dichloro-N-(4-(1-methyl-4-(trifluoromethyl)-1H-imidazol-2-yl)benzyl)pyrimidin-4-amine)

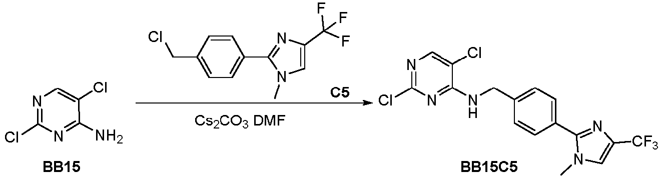

In accordance with the same steps of intermediate **BB1C2,** intermediate **BB15C5** (398.7 mg, 0.99 mmol, 80.4% yield, a white solid) was obtained using intermediate **BB15** and compound **C5** as starting materials. ¹H NMR (400 MHz, DMSO-d6) δ 8.60 (s, 1H), 7.96 (d, J = 1.3 Hz, 1H), 7.87 - 7.68 (m, 2H), 7.45 (d, J = 8.3 Hz, 2H), 5.00 (s, 2H), 3.21 (s, 3H); LC-MS: m/z = 402.0 (M+H)⁺.

### Synthesis of intermediate BB15C59 (2,5-dichloro-N-(4-(1-(fluoromethyl)-4-(trifluoromethyl)-1H-imidazol-2-yl)benzyl)pyrimidin-4-amine)

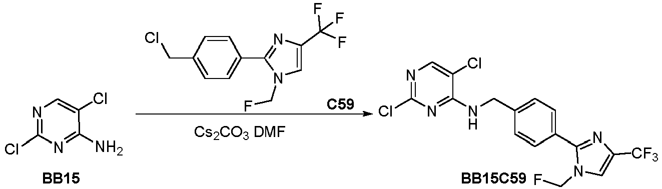

In accordance with the same steps of intermediate **BB1C2,** intermediate **BB15C59** (124.0 mg, 0.30 mmol, 58.9% yield, a white solid) was obtained using intermediate **BB15** and compound **C59** as starting materials. LC-MS: m/z = 420.0 (M+H)⁺.

### Synthesis of intermediate BB16C58 (2-chloro-5-fluoro-N-(4-(1-isopropyl-4-(trifluoromethyl)-1H-imidazol-2-yl)benzyl)-N-methylpyrimidin-4-amine)

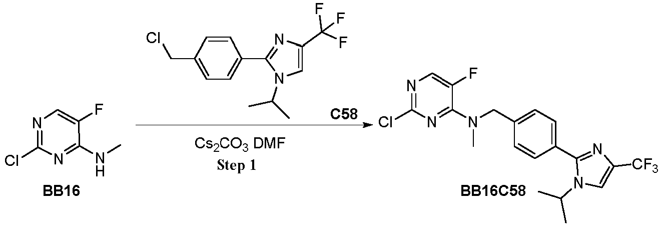

In accordance with the same steps of intermediate **BB1C2,** intermediate **BB16C58** (6.66 g, 16.69 mmol, 93.3% yield, a light yellow solid) was obtained using intermediate **BB16** and compound **C58** as starting materials. LC-MS: m/z = 428.2 (M+H)⁺.

### Synthesis of intermediate BB16-D3C57 (2-chloro-N-(4-(1-cyclopropyl-4-(trifluoromethyl)-1H-imidazol-2-yl)benzyl)-5-fluoro-N-(methyl-d3)pyrimidin-4-amine)

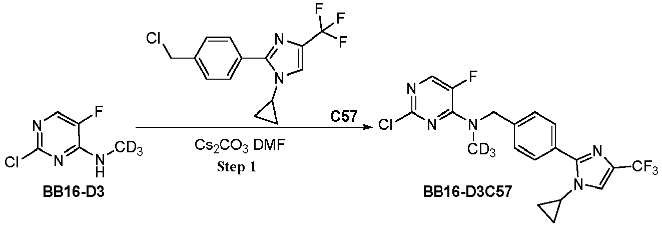

In accordance with the same steps of intermediate **BB1C2,** intermediate **BB16-D3C57** (1.33 g, 3.10 mmol, 89.8% yield, an off-white solid) was obtained using intermediate **BB16-D3** and compound **C57** as starting materials. LC-MS: m/z = 429.3 (M+H)⁺.

### Synthesis of intermediate BB16-D3C58 (2-chloro-5-fluoro-N-(4-(1-isopropyl-4-(trifluoromethyl)-1H-imidazol-2-yl)benzyl)-N-(methyl-d3)pyrimidin-4-amine)

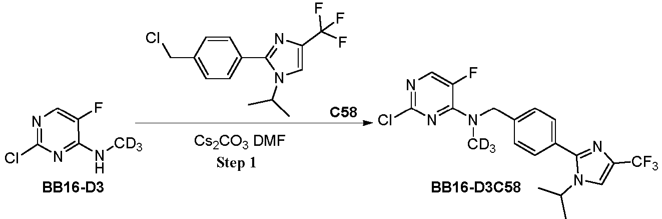

In accordance with the same steps of intermediate **BB1C2,** intermediate **BB16-D3C58** (1.54 g, 3.57 mmol, 79.6% yield, an off-white solid) was obtained using intermediate **BB16-D3** and compound **C58** as starting materials. LC-MS: m/z = 431.1 (M+H)⁺.

### Synthesis of intermediate BB16-D3C59 (2-chloro-5-fluoro-N-(4-(1-(fluoromethyl)-4-(trifluoromethyl)-1H-imidazol-2-yl)benzyl)-N-(methyl-D3)pyrimidin-4-amine)

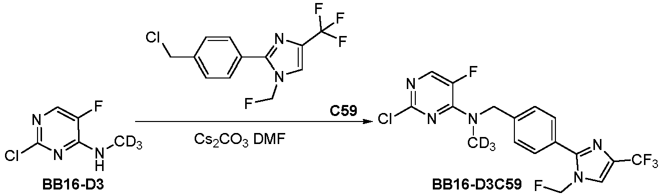

In accordance with the same steps of intermediate **BB1C2,** intermediate **BB16-D3C59** (874.2 mg, 2.08 mmol, 84.1% yield, a yellow solid) was obtained using intermediate **BB16-D3** and compound **C59** as starting materials. LC-MS: m/z = 421.1 (M+H)⁺.

### Synthesis of intermediate BB17C5 (2-chloro-4-((4-(1-methyl-4-(trifluoromethyl)-1H-imidazol-2-yl)benzyl)amino)pyrimidine-5-carbonitrile)

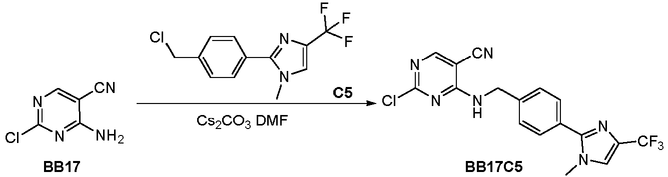

In accordance with the same steps of intermediate **BB1C2,** intermediate **BB17C5** (149.0 mg, 0.38 mmol, 48.7% yield, an off-white solid) was obtained using intermediate **BB17** and compound **C5** as starting materials. LC-MS: m/z = 393.0 (M+H)⁺.

### Synthesis of intermediate BB19C4 (2-chloro-5-(difluoromethoxy)-N-((1-(1-methyl-4-(trifluoromethyl)-1H-imidazol-2-yl)piperidin-4-yl)methyl)pyrimidin-4-amine)

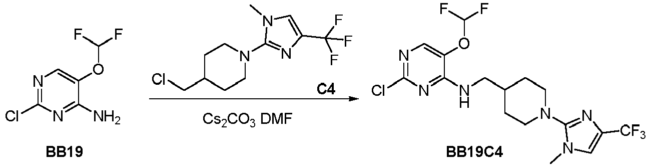

In accordance with the same steps of intermediate **BB2C35,** intermediate **BB19C4** (44.0 mg, 0.10 mmol, 17.7% yield, a colorless transparent oil) was obtained using intermediate BB19 and compound **C4** as starting materials. LC-MS: m/z = 441.0 (M+H)⁺.

### Synthesis of intermediate BB19C5 (2-chloro-5-(difluoromethoxy)-N-(4-(1-methyl-4-(trifluoromethyl)-1H-imidazol-2-yl)benzyl)pyrimidin-4-amine)

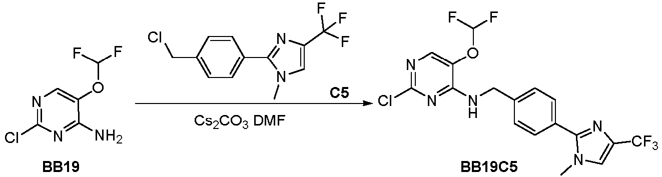

In accordance with the same steps of intermediate **BB1C2,** intermediate **BB19C5** (1.03 g, 2.33 mmol, 82.2% yield, a white solid) was obtained using intermediate **BB19** and compound **C5** as starting materials. LC-MS: m/z = 434.0 (M+H)⁺.

### Synthesis of intermediate BB19C11 (2-chloro-5-(difluoromethoxy)-N-(4-(5-methyl-3-(trifluoromethyl)-1H-pyrazol-1-yl)benzyl)pyrimidin-4-amine)

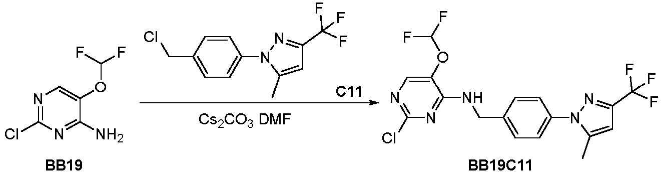

In accordance with the same steps of intermediate **BB1C2,** intermediate **BB19C11** (545.7 mg, 1.26 mmol, 77.3% yield, a white solid) was obtained using intermediate **BB19** and compound **C11** as starting materials. LC-MS: m/z = 434.2 (M+H)⁺.

### Synthesis of intermediate BB19C30 (2-chloro-5-(difluoromethoxy)-N-(2-methoxy-4-(1-methyl-4-(trifluoromethyl)-1H-imidazol-2-yl)benzyl)pyrimidin-4-amine)

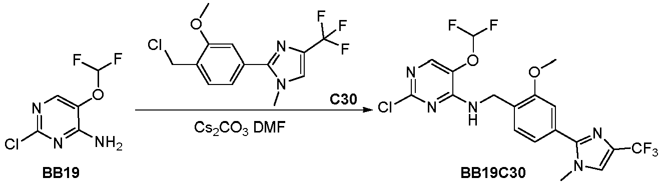

In accordance with the same steps of intermediate **BB1C2,** intermediate **BB19C30** (546.4 mg, 1.18 mmol, 66.5% yield, an off-white solid) was obtained using intermediate **BB19** and compound **C30** as starting materials. LC-MS: m/z = 464.0 (M+H)⁺.

### Synthesis of intermediate BB36C58 (2-chloro-N-(4-(1-isopropyl-4-(trifluoromethyl)-1H-imidazol-2-yl)benzyl)-5-(methoxymethyl)-N-methylpyrimidin-4-amine)

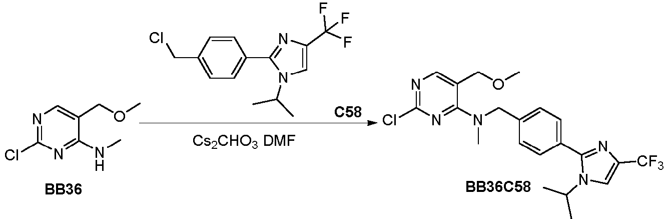

In accordance with the same steps of intermediate **BB1C2,** intermediate **BB36C58** (382.0 mg, 0.84 mmol, 69.3% yield, a colorless transparent oil) was obtained using intermediate **BB36** and compound **C58** as starting materials. LC-MS: m/z = 454.0 (M+H)⁺.

### Synthesis of intermediate BB42C5 (2-chloro-5-methoxy-N-(4-(1-methyl-4-(trifluoromethyl)-1H-imidazol-2-yl)benzyl)-N-(prop-2-yn-1-yl)pyrimidin-4-amine)

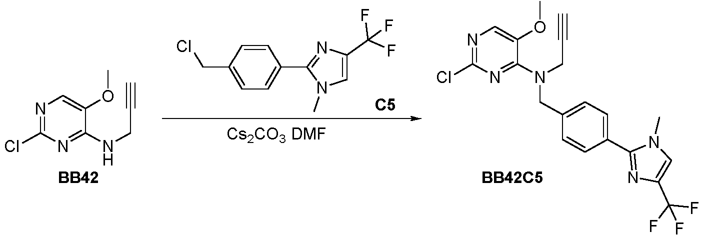

In accordance with the same steps of intermediate **BB1C2,** intermediate **BB42C5** (332.6 mg, 0.76 mmol, 44.5% yield, an off-white solid) was obtained using intermediate **BB42** and compound **C5** as starting materials. LC-MS: m/z = 436.2 (M+H)⁺.

### Synthesis of intermediate BB43C5 (2-chloro-5-methoxy-N-(4-(1-methyl-4-(trifluoromethyl)-1H-imidazol-2-yl)benzyl)-N-(tetrahydrofuran-2-yl)pyrimidin-4-amine)

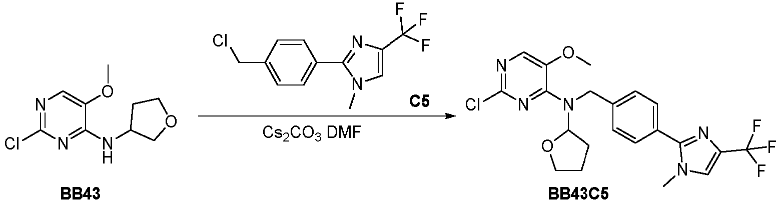

In accordance with the same steps of intermediate **BB1C2,** intermediate **BB43C5** (101.1 mg, 0.22 mmol, 37.3% yield, a white solid) was obtained using intermediate **BB43** and compound C5 as starting materials. LC-MS: m/z = 468.1 (M+H)⁺.

### Synthesis of intermediate BB44C5 (2-chloro-N-cyclopropyl-5-methoxy-N-(4-(1-methyl-4-(trifluoromethyl)-1H-imidazol-2-yl)benzyl)pyrimidin-4-amine)

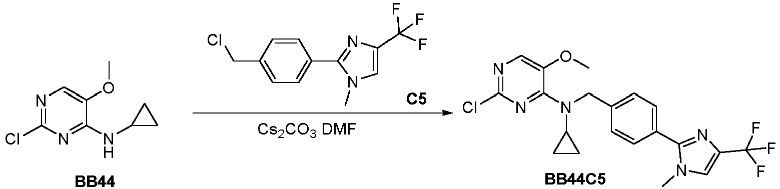

In accordance with the same steps of intermediate **BB1C2,** intermediate **BB44C5** (258.3 mg, 0.59 mol, 74.8% yield, a light yellow solid) was obtained using intermediate **BB44** and compound **C5** as starting materials. LC-MS: m/z = 438.1 (M+H)⁺.

### Synthesis of intermediate BB45C5 (N-(2-chloro-5-methoxypyrimidin-4-yl)-O-methyl-N-(4-(1-methyl-4-(trifluoromethyl)-1H-imidazol-2-yl)benzyl)hydroxylamine)

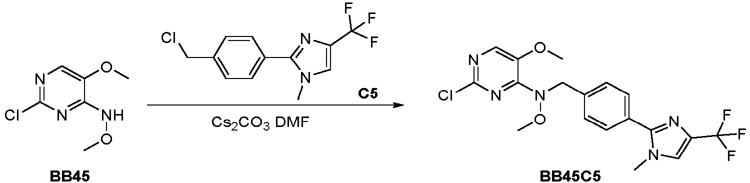

Compound **BB45** (250 mg, 1.3 mmol, 1.0 eq), compound **C5** (392 mg, 1.43 mmol, 1.1 eq) and potassium carbonate (548 mg, 4.0 mmol, 3.0 eq) were added to dimethyl sulfoxide (5.0 mL) successively, and the resulting mixture was stirred at 85°C for 1 hour. The reaction solution was cooled to 25°C. Ethyl acetate (20.0 mL) was added, the resulting mixture was stirred for 5 minutes, and filtered to obtain a filtrate. The filtrate was washed three times with saturated brine (10.0 mL), dried over anhydrous sodium sulfate, and concentrated under vacuum to obtain a crude product, which was purified by column chromatography (eluent: petroleum ether/ethyl acetate = 1/0 - 3/1) to obtain intermediate **BB45C5** (160.5 mg, 0.38 mmol, 28.9% yield) as a light yellow oil. ¹H NMR (400 MHz, DMSO-d6) δ 8.18 (s, 1H), 7.96 (d, J = 1.5 Hz, 1H), 7.77 - 7.71 (m, 2H), 7.51 (d, J = 8.2 Hz, 2H), 4.96 (s, 2H), 3.95 (s, 3H), 3.81 (s, 3H), 3.68 (s, 3H); LC-MS: m/z = 428.1 (M+H)⁺.

### Synthesis of intermediate BB18D5 (2-chloro-5-ethynyl-N-(4-(1-methyl-4-(trifluoromethyl)-1H-imidazol-2-yl)benzyl)pyrimidin-4-amine)

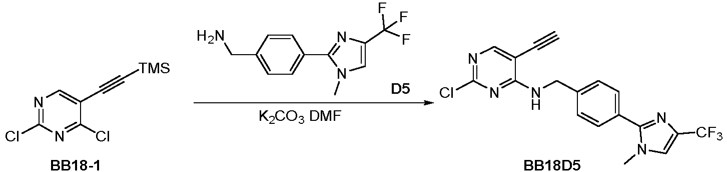

2-(2,4-Dichloropyrimidin-5-yl)ethynyl-trimethylsilane (compound **BB18-1,** 2.2 g, 8.16 mmol, 1.00 eq), compound **D5** (2.19 g, 8.57 mmol, 1.05 eq) and potassium carbonate (4.51 g, 32.64 mmol, 4.0 eq) were added to N,N-dimethylformamide (40.0 mL) successively, and the resulting mixture was stirred at 50°C for 2 hours. The reaction solution was cooled to 25°C. Ethyl acetate (100.0 mL) was added, the resulting mixture was stirred for 5 minutes, and filtered to obtain a filtrate. The filtrate was washed three times with saturated brine (100.0 mL), dried over anhydrous sodium sulfate, and concentrated under vacuum to obtain a crude product, which was purified by column chromatography (eluent: petroleum ether/ethyl acetate = 1/0 - 0/1) to obtain intermediate **BB18D5** (2.24 g, 5.73 mmol, 70.2% yield) as a white solid. LC-MS: m/z = 392.2 (M+H)⁺.

### Synthesis of intermediate BB21D5 (2-chloro-N-(4-(1-methyl-4-(trifluoromethyl)-1H-imidazol-2-yl)benzyl)-5-morpholinopyrimidin-4-amine)

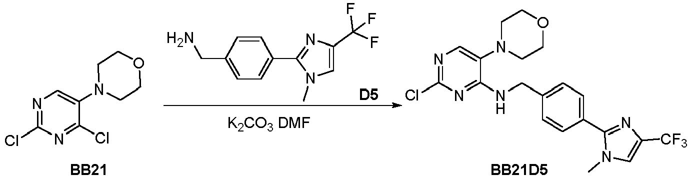

In accordance with the same steps of intermediate **BB18D5,** intermediate **BB21D5** (880.0 mg, 1.95 mmol, 88.6% yield, a white solid) was obtained using intermediate **BB21** and compound **D5** as starting materials. LC-MS: m/z = 453.1 (M+H)⁺.

### Synthesis of intermediate BB22D5 (2-chloro-N-(4-(1-methyl-4-(trifluoromethyl)-1H-imidazol-2-yl)benzyl)-5-(4-methylpiperazin-1-yl)pyrimidin-4-amine)

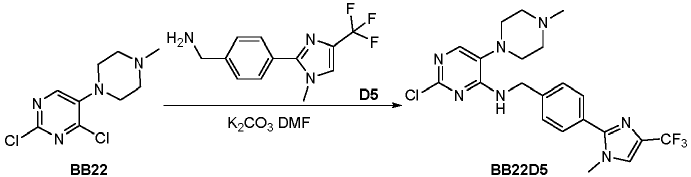

In accordance with the same steps of intermediate BB18D5, intermediate BB22D5 (441.9 mg, 0.95 mmol, 87.4% yield, an off-white solid) was obtained using intermediate **BB22** and compound **D5** as starting materials. LC-MS: m/z = 466.2 (M+H)⁺.

### Synthesis of intermediate BB24D5 (2-chloro-4-((4-(1-methyl-4-(trifluoromethyl)-1H-imidazol-2-yl)benzyl)amino)pyrimidin-5-ol)

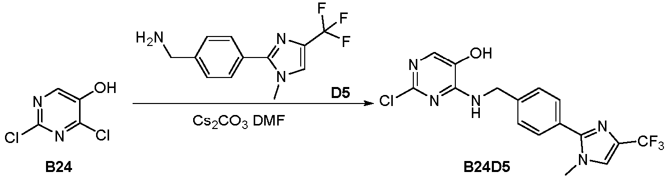

In accordance with the same steps of intermediate **BB18D5,** intermediate **BB24D5** (26.8 mg, 0.07 mmol, 22.8% yield, a light yellow solid) was obtained using intermediate **BB24** and compound **D5** as starting materials. LC-MS: m/z = 384.1 (M+H)⁺.

### Synthesis of intermediate BB24D11 (2-chloro-4-((4-(5-methyl-3-(trifluoromethyl)-1H-pyrazol-1-yl)benzyl)amino)pyrimidin-5-ol)

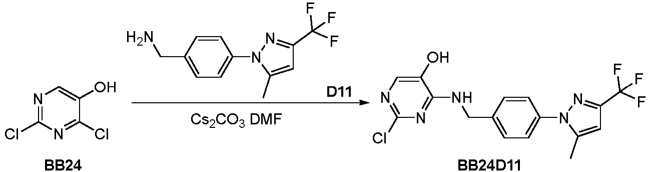

In accordance with the same steps of intermediate **BB18D5,** intermediate **BB24D11** (115.1 mg, 0.3 mmol, 83.0% yield, a white solid) was obtained using intermediate **BB24** and **D11** as starting materials. LC-MS: m/z = 384.2 (M+H)⁺.

### Synthesis of intermediate BB35D5 ((2-chloro-4-((4-(1-methyl-4-(trifluoromethyl)-1H-imidazol-2-yl)benzyl)amino)pyrimidin-5-yl)methanol)

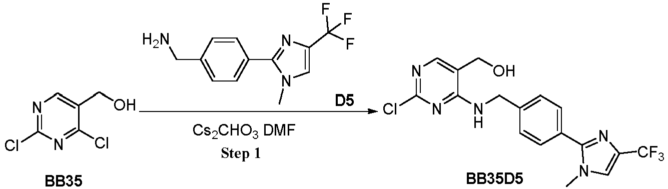

In accordance with the same steps of intermediate **BB18D5,** intermediate **BB35D5** (333.8 mg, 1.86 mmol, 55.6% yield, a colorless transparent oil) was obtained using intermediate **BB35** and compound **D5** as starting materials. LC-MS: m/z = 398.1 (M+H)⁺.

### Synthesis of intermediate BB41C5 (methyl 2-(2-chloro-4-((4-(1-methyl-4-(trifluoromethyl)-1H-imidazol-2-yl)benzyl)amino)pyrimidin-5-yl)acetate)

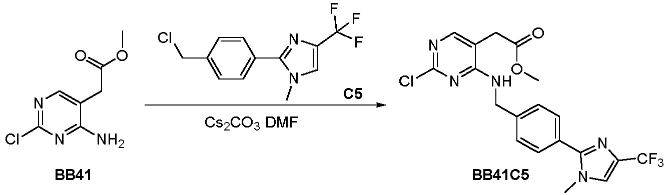

In accordance with the same steps of intermediate **BB18D5,** intermediate **BB41D5** (133.9 mg, 0.30 mmol, 69.9% yield, a light yellow solid) was obtained using intermediate **BB41** and compound **D5** as starting materials. LC-MS: m/z = 440.2 (M+H)⁺.

### Example 1: Synthesis of compound 1 (2-(2-isopropylphenyl)-5-methoxy-N-methyl-N-(4-(pyridin-2-yl)benzyl)pyrimidin-4-amine)

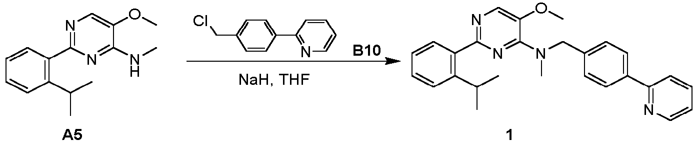

Compound **A5** (100 mg, 389 µmol, 1.00 eq) was dissolved in tetrahydrofuran (2.5 mL), and sodium hydride (62.2 mg, 1.55 mmol, 60.0% purity, 4.00 eq) was added in portions at 0°C. After the addition was completed, the resulting mixture was reacted at 0°C for 30 minutes. Then, compound **B10** (95.0 mg, 466 µmol, 1.20 eq) was added. After the addition was completed, the resulting mixture was heated up to 25°C and stirred for 12 hours. Then, ice water (2.0 mL) was added at 0°C to quench the reaction, and the resulting mixture was extracted three times with ethyl acetate (10.0 mL). The organic layers were combined, dried over anhydrous sodium sulfate, and filtered to obtain a filtrate. The filtrate was concentrated under vacuum to obtain a crude product, which was purified by preparative HPLC (column: 3_Phenomenex Luna C18 75×30mm×3µm; mobile phase: [water (HCl)-ACN]; B%: 15%-35%, 8 min) to obtain compound 1 (136 mg, 277 µmol, 71.3% yield, HCl) as a light yellow solid. ¹H NMR (400 MHz, CDCl₃) δ 8.91 (d, *J =* 4.40 Hz, 1H), 8.40 (s, 1H), 8.18-8.19 (m, 3H), 7.80 (s, 1H), 7.65 (s, 1H), 7.43-7.46 (m, 6H), 5.09-5.21 (m, 2H), 3.85-3.94 (m, 3H), 3.54 (s, 1H), 3.26-3.34 (m, 3H), 1.01-1.23 (m, 6H); LC-MS: m/z = 425.2 (M+H)⁺.

In accordance with the preparation method of compound **1,** the following compounds were obtained using different general intermediates A and B together with cesium carbonate or sodium hydride or potassium carbonate.

| **Example** | **Structure and name of compound** | **Reaction moiety** | **Analyzed data** |
|---|---|---|---|
| **2** | 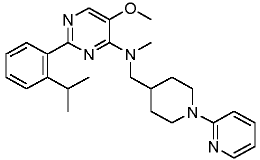 2-(2-Isopropylphenyl)-5-methoxy-N-methyl-N-((1-(pyridin-2-yl)piperidin-4-yl)methyl)pyrimidin-4-amine | **A5** | LC-MS: m/z =432.2 (M+H)⁺ |
| | | **B11** | |
| **3** | 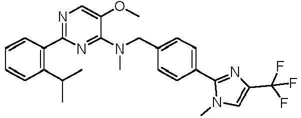 2-(2-Isopropylphenyl)-5-methoxy-N-methyl-N-(4-(1-methyl-4-(trifluoromethyl)-1H-imidazol-2-yl)benzyl)pyrimidin-4-amine | | LC-MS: m/z =496.3 (M+H)⁺; |
| | | **A5** | HNMR (400MHz, DMSO) δ 8.14 (s, 1H), 7.96 (s, 1H), 7.70-7.72 (m, 2H), 7.37-7.57 (m, 6H), 5.18 (s, 2H), 3.95 (s, 3H), 3.75 (s, 3H), 3.41-3.43 (m, 3H), 3.13 (s, 1H), 1.02-1.23 (m, 6H). |
| | | **B6** | |
| **4** | 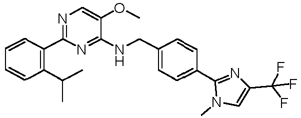 2-(2-Isopropylphenyl)-5-methoxy-N-(4-(1-methyl-4-(trifluoromethyl)-1H-imidazol-2-yl)benzyl)pyrimidin-4-amine | | LC-MS: m/z =482.3 (M+H)⁺; |
| | | **A1** | HNMR (400MHz, DMSO) δ 8.02 (s, 1H), 7.69-7.74 (m, 3H), 7.52-7.54 (m, 1H), 7.39-7.48 (m, 5H), 7.10 (s, 1H), 4.94 (d,*J =* 5.20 Hz, 2H), 4.05 (s, 3H), 3.82 (s, 3H), 3.33-3.36 (m, 1H), 1.21 (d, *J* = 6.80 Hz, 6H). |
| | | **B6** | |
| **5** | 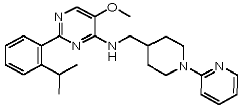 2-(2-Isopropylphenyl)-5-methoxy-N-((1-(pyridin-2-yl)piperidin-4-yl)methyl)pyrimidin-4-amine | **A1** | LC-MS: m/z =418.1 (M+H)⁺ |
| | | **B11** | |
| **6** | 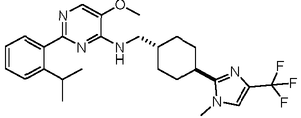 2-(2-Isopropylphenyl)-5-methoxy-N-(((1R,4R)-4-(1-methyl-4-(trifluoromethyl)-1H-imidazol-2-yl)cyclohexyl)methyl)pyrimidin-4-amine | **A1** | LC-MS: m/z =488.4 (M+H)⁺ |
| | | **B12** | |
| **7** | 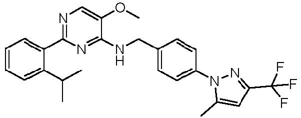 2-(2-Isopropylphenyl)-5-methoxy-N-(4-(5-methyl-3-(trifluoromethyl)-1H-pyrazol-1-yl)benzyl)pyrimidin-4-amine | **A1** | LC-MS: m/z =482.2 (M+H)⁺ |
| | | **B17** | |
| **8** | 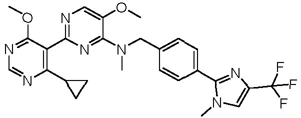 4'-Cyclopropyl-5,6'-dimethoxy-N-methyl-N-(4-(1-methyl-4-(trifluoromethyl)-1H-imidazol-2-yl)benzyl)-[2,5'-bipyrimidin]-4-amine | | LC-MS: m/z =526.2 (M+H)+; |
| | | **A2** | ¹H NMR (400 MHz, DMSO) δ 8.62 (s, 1H), 8.16 (s, 1H), 7.98 - 7.93 (m, 1H), 7.70 (d, *J* = 8.2 Hz, 2H), 7.42 (d, *J* = 8.1 Hz, 2H), 4.94 (s, 2H), 3.90 (s, 3H), 3.86 (s, 3H), 3.80 (s, 3H), 3.18 (s, 3H), 1.79-1.72 (m, 1H), 1.06 - 0.99 (m, 2H), 0.90-0.81 (m, 2H). |
| | | **B6** | |
| **9** | 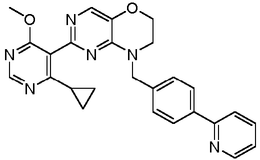 2-(4-Cyclopropyl-6-methoxypyrimidin-5-yl)-8-(4-(pyridin-2-yl)benzyl)-7,8-dihydro-6H-pyrimido[5,4-b][1,4]oxazine | | LC-MS: m/z =453.1 (M+H)+; |
| | | **A8** | ¹HNMR (400 MHz, DMSO) δ 8.65 (d, *J=* 4.4 Hz, 1H), 8.59 (s, 1H), 8.04 (d, *J =* 8.00 Hz, 2H), 7.94-7.95 (m, 2H), 7.85-7.89 (m, 1H), 7.36-7.42 (m, 2H), 7.33-7.36 (m, 1H), 4.85 (s, 2H), 4.28 (s, 2H), 3.85 (s, 3H), 3.59 (s, 2H), 1.74-1.76 (m, 1H), 0.97 (s, 2H), 0.81-0.83 (m, 2H). |
| | | **B10** | |
| **10** | 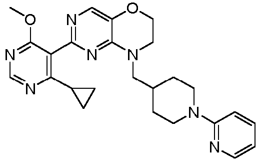 2-(4-Cyclopropyl-6-methoxypyrimidin-5-yl)-8-((1-(pyridin-2-yl)piperidin-4-yl)methyl)-7,8-dihydro-6H-pyrimido[5,4-b][1,4]oxazine | | LC-MS: m/z =460.2 (M+H)+; |
| | | **A8** | ¹HNMR (400 MHz, DMSO) δ 8.57 (s, 1H), 8.06-8.07 (m, 1H), 7.86 (s, 1H), 7.45-7.49 (m, 1H), 6.78 (d, *J =* 8.80 Hz, 1H), 6.54-6.57 (m, 1H), 4.23-4.29 (m, 4H), 3.81 (s, 3H), 3.61-3.63 (m, 2H), 3.46 (d, *J =* 7.60 Hz, 2H), 2.67-2.77 (m, 2H), 2.05-2.07 (m, 1H), 1.74-1.75 (m, 1H), 1.61 (d, *J =* 11.2 Hz, 2H), 1.16-1.17 (m, 2H), 0.96-0.99 (m, 2H), 0.88-0.90 (m, 2H). |
| | | **B11** | |
| **11** | 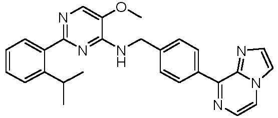 N-(4-(Imidazo[1,2-a]pyrazin-8-yl)benzyl)-2-(2-isopropylphenyl)-5-methoxypyrimidin-4-amine | **A1** | LC-MS: m/z =451.1 (M+H)+ |
| | | **B15** | |
| **12** | 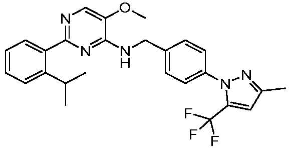 2-(2-Isopropylphenyl)-5-methoxy-N-(4-(3-methyl-5-(trifluoromethyl)-1H-pyrazol-1-yl)benzyl)pyrimidin-4-amine | **A1** | LC-MS: m/z =482.2 (M+H)+ |
| | | **B17** | |
| **13** | 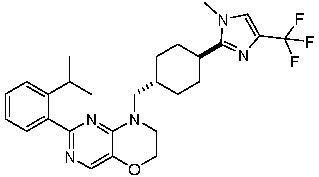 2-(2-Isopropylphenyl)-8-(((1R,4R)-4-(1-methyl-4-(trifluoromethyl)-1H-imidazol-2-yl)cyclohexyl)methyl)-7,8-dihydro-6H-pyrimido[5,4-b][1,4]oxazine | **A6** | LC-MS: m/z =500.4 (M+H)+ |
| | | **B12** | |
| **14** | 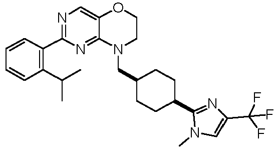 2-(2-Isopropylphenyl)-8-(((1s,4s)-4-(1-methyl-4-(trifluoromethyl)-1H-imidazol-2-yl)cyclohexyl)methyl)-7,8-dihydro-6H-pyrimido[5,4-b][1,4]oxazine | **A6** | |
| | | **B12** | |
| **15** | 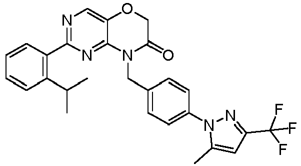 2-(2-Isopropylphenyl)-8-(4-(5-methyl-3-(trifluoromethyl)-1H-pyrazol-1-yl)benzyl)-6H-pyrimido[5,4-b][1,4]oxazin-7(8H)-one | **A7** | LC-MS: m/z = 508.3 (M+H)+ |
| | | **B17** | |
| **16** | 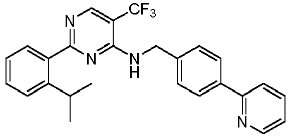 2-(2-Isopropylphenyl)-N-(4-(pyridin-2-yl)benzyl)-5-(trifluoromethyl)pyrimidin-4-amine | **A4** | LC-MS: m/z =449.1 (M+H)+ |
| | | **B10** | |
| **17** | 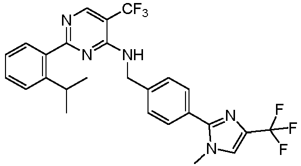 2-(2-Isopropylphenyl)-N-(4-(1-methyl-4-(trifluoromethyl)-1H-imidazol-2-yl)benzyl)-5-(trifluoromethyl)pyrimidin-4-amine | **A4** | LC-MS: m/z =520.1 (M+H)+ |
| | | **B6** | |
| **18** | 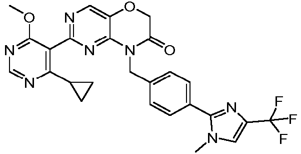 2-(4-Cyclopropyl-6-methoxypyrimidin-5-yl)-8-(4-(1-methyl-4-(trifluoromethyl)-1H-imidazol-2-yl)benzyl)-6H-pyrimido[5,4-b][1,4]oxazin-7(8H)-one | **A10** | LC-MS: m/z = 538.3 (M+H)+ |
| | | **B6** | |
| **19** | 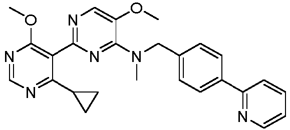 4'-Cyclopropyl-5,6'-dimethoxy-N-methyl-N-(4-(pyridin-2-yl)benzyl)-[2,5'-bipyrimidin]-4-amine | **A2** | LC-MS: m/z =455.4 (M+H)+ |
| | | **B10** | |
| **20** | 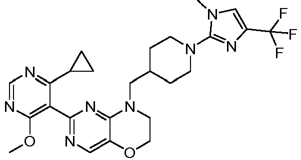 2-(4-Cyclopropyl-6-methoxypyrimidin-5-yl)-8-((1-(1-methyl-4-(trifluoromethyl)-1H-imidazol-2-yl)piperidin-4-yl)methyl)-7,8-dihydro-6H-pyrimido[5,4-b][1,4]oxazine | **A8** | LC-MS: m/z =531.2 (M+H)+ |
| | | **B13** | |
| **21** | 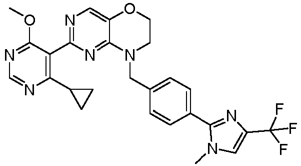 2-(4-Cyclopropyl-6-methoxypyrimidin-5-yl)-8-(4-(1-methyl-4-(trifluoromethyl)-1H-imidazol-2-yl)benzyl)-7,8-dihydro-6H-pyrimido[5,4-b][1,4]oxazine | | LC-MS:m/z=524.2 (M+H)+; |
| | | **A8** | 1HNMR (400 MHz, DMSO) δ8.60 (s, 1H), 7.95 (d, *J* = 7.20 Hz, 2H), 7.68 (d, *J =* 8.00 Hz, 2H), 7.44 (d, *J=* 8.00 Hz, 2H), 4.87 (s, 2H), 4.29 (s, 2H), 3.80 (d, *J* = 30.0 Hz, 6H), 3.59 (s, 2H), 1.76 (s, 1H), 0.98 (s, 2H), 0.82-0.84 (m, 2H). |
| | | **B6** | |
| **22** | 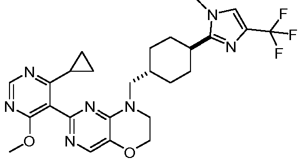 2-(4-Cyclopropyl-6-methoxypyrimidin-5-yl)-8-(((1r,4r)-4-(1-methyl-4-(trifluoromethyl)-1H-imidazol-2-yl)cyclohexyl)methyl)-7,8-dihydro-6H-pyrimido[5,4-b][1,4]oxazine | **A8** | LC-MS: m/z = 530.4 (M+H)+ |
| | | **B12** | |
| **23** | 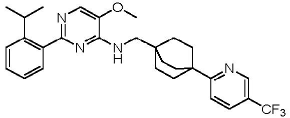 2-(2-Isopropylphenyl)-5-methoxy-N-((4-(5-(trifluoromethyl)pyridin-2-yl)bicyclo[2.2.2]octan-1-yl)methyl)pyrimidin-4-amine | **A1** | LC-MS: m/z =511.2 (M+H)⁺ |
| | | **B16** | |
| **24** | 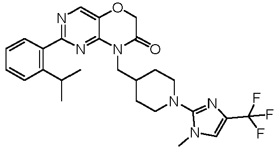 2-(2-Isopropylphenyl)-8-((1-(1-methyl-4-(trifluoromethyl)-1H-imidazol-2-yl)piperidin-4-yl)methyl)-6H-pyrimido[5,4-b][1,4]oxazin-7(8H)-one | **A7** | LC-MS: m/z =515.2 (M+H)⁺ |
| | | **B13** | |
| **25** | 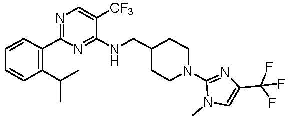 2-(2-Isopropylphenyl)-N-((1-(1-methyl-4-(trifluoromethyl)-1H-imidazol-2-yl)piperidin-4-yl)methyl)-5-(trifluoromethyl)pyrimidin-4-amine | **A4** | LC-MS: m/z =527.2 (M+H)⁺ |
| | | **B13** | |
| **26** | 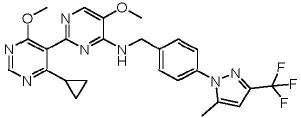 4'-Cyclopropyl-5,6'-dimethoxy-N-(4-(5-methyl-3-(trifluoromethyl)-1H-pyrazol-1-yl)benzyl)-[2,5'-bipyrimidin]-4-amine | | LC-MS: m/z = 512.3 (M+H)⁺; |
| | | **A9** | ¹H NMR (400 MHz, DMSO-*d*₆) δ 8.58 (s, 1H), 7.97 (s, 1H), 7.85 (t, *J =* 6.3 Hz, 1H), 7.47 (t, *J =* 8.3 Hz, 4H), 6.74 (s, 1H), 4.63 (d, *J =* 6.3 Hz, 2H), 3.94 (s, 3H), 3.80 (s, 3H), 2.31 (s, 3H), 1.67 (tt, *J =* 8.3, 4.7 Hz, 1H), 0.94 (dq, *J* = 6.3, 3.5 Hz, 2H), 0.76 (dt, *J =* 8.2, 3.3 Hz, 2H). |
| | | **B17** | |
| **27** | 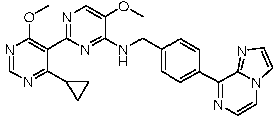 4'-Cyclopropyl-N-(4-(imidazo[1,2-a]pyrazin-8-yl)benzyl)-5,6'-dimethoxy-[2,5'-bipyrimidin]-4-amine | **A9** | LC-MS: m/z = 481.3 (M+H)+ |
| | | **B15** | |
| **28** | 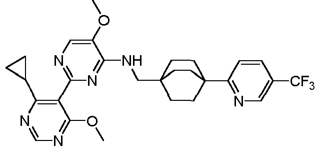 4'-Cyclopropyl-5,6'-dimethoxy-N-(4-(5-(trifluoromethyl)pyridin-2-yl)bicyclo[2.2.2]octan-1-yl)methyl)-[2,5'-bipyrimidin]-4-amine | **A9** | LC-MS: m/z = 541.3 (M+H)+ |
| | | **B16** | |
| **29** | 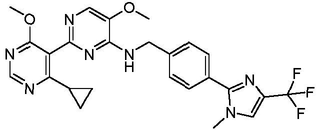 4'-Cyclopropyl-5,6'-dimethoxy-N-(4-(1-methyl-4-(trifluoromethyl)-1H-imidazol-2-yl)benzyl)-[2,5'-bipyrimidin]-4-amine | | LC-MS: m/z = 512.2 (M+H)⁺; |
| | | **A9** | ¹H NMR (400 MHz, DMSO-*d*₆) δ 8.60 (s, 1H), 7.96 (d, *J =* 16.5 Hz, 2H), 7.86 (t, *J* = 6.4 Hz, 1H), 7.65 (d, *J=* 8.0 Hz, 2H), 7.43 (d, *J* = 8.0 Hz, 2H), 4.64 (d, *J* = 6.3 Hz, 2H), 3.96 (s, 3H), 3.83 (s, 3H), 3.78 (s, 3H), 1.70 (tt, *J* = 8.5, 4.7 Hz, 1H), 0.97 (s, 2H), 0.78 (dd, *J =* 7.8, 3.3 Hz, 2H). |
| | | **B6** | |
| **30** | 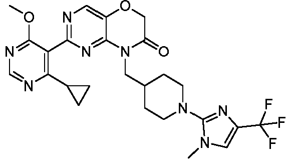 2-(4-Cyclopropyl-6-methoxypyrimidin-5-yl)-8-((1-(1-methyl-4-(trifluoromethyl)-1H-imidazol-2-yl)piperidin-4-yl)methyl)-6H-pyrimido[5,4-b][1,4]oxazin-7(8H)-one | **A10** | LC-MS: m/z = 545.3 (M+H)⁺ |
| | | **B13** | |
| **31** | 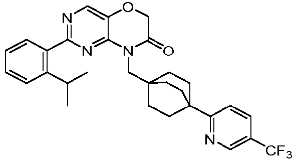 2-(2-Isopropylphenyl)-8-((4-(5-(trifluoromethyl)pyridin-2-yl)bicyclo[2.2.2]octan-1-yl)methyl)-6H-pyrimido[5,4-b][1,4]oxazin-7(8H)-one | **A7** | LC-MS: m/z = 537.3 (M+H)⁺ |
| | | **B16** | |

### Example 32: Synthesis of compound 32 (2-(2-isopropylphenyl)-N-(4-(pyridin-2-yl)benzyl)furo[3,2-d]pyrimidin-4-amine)

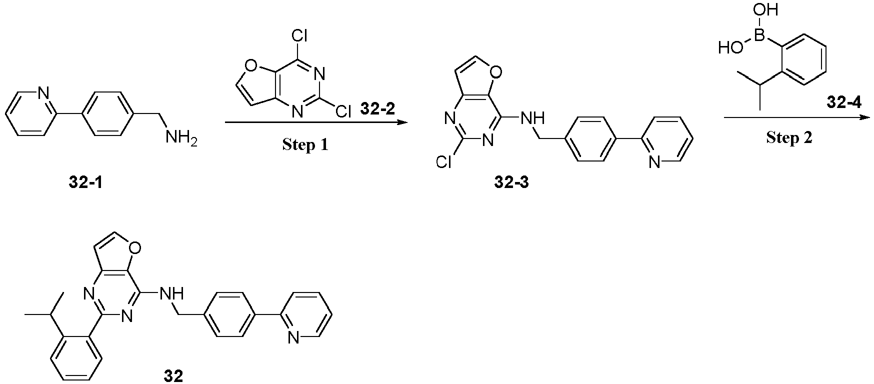

### Step 1: Synthesis of compound 32-3

Compound **32-1** (200.0 mg, 1.09 mmol, 1.00 *eq*), compound **32-2** (205.0 mg, 1.09 mmol, 1.00 *eq*) and cesium carbonate (1.1 g, 3.27 mmol, 3 *eq*) were added to N,N-dimethylformamide (4.0 mL) successively, and the resulting mixture was stirred at 60°C for 1 hour. Ethyl acetate (30.0 mL) was added, the resulting mixture was stirred for 5 minutes, and filtered to obtain a filtrate. The filtrate was concentrated under vacuum to obtain a crude product, which was purified by column chromatography (eluent: petroleum ether/ethyl acetate = 1/0 - 2/1) to obtain compound **32-3** (360.9 mg). LC-MS: m/z =337.0 (M+H)⁺.

### Step 2: Synthesis of compound 32

Compound **32-3** (100.0 mg, 0.3 mmol, 1.00 *eq),* compound **32-4** (97.7 mg, 0.6 mmol, *2.00 eq),* potassium phosphate (191.0 mg, 0.9 mmol, 3.00 *eq),* and tetrakis(triphenylphosphine)palladium (34.7 mg, 0.03 mmol, 0.10 *eq)* were added to dioxane (2.0 mL) and water (0.4 mL) successively. The resulting mixture was purged three times with nitrogen, and stirred at 90°C for 16 hours under nitrogen atmosphere. Water (10.0 mL) was added, and the resulting mixture was extracted three times with ethyl acetate (20.0 mL). The organic layers were combined, washed twice with saturated brine (10.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **32** (87.5 mg, 0.21 mmol, 69.4% yield, 98.6% purity) as a white solid. ¹H NMR (400 MHz, DMSO) δ 10.05 (s, 1H), 8.71 (d, *J =* 4.9 Hz, 1H), 8.59 (d, *J =* 2.2 Hz, 1H), 8.06 (d, *J =* 8.0 Hz, 2H), 8.03 - 7.95 (m, 2H), 7.58 - 7.48 (m, 5H), 7.47 - 7.42 (m, 1H), 7.40 - 7.33 (m, 1H), 7.20 (d, *J =* 2.2 Hz, 1H), 4.93 (d, *J =* 6.0 Hz, 2H), 3.29 - 3.22 (m, 1H), 1.07 (d, *J =* 6.8 Hz, 6H); LC-MS: m/z = 421.2 (M+H)⁺.

The following compounds were obtained in accordance with the preparation method of compound **32,** wherein the reaction condition of step 1 was cesium carbonate or sodium hydride or potassium carbonate, and the reaction condition of step 2 was Pd(PPh₃)₄/K₃PO₄ or XPhos-Pd-G2/XPhos/K₃PO₄.

| **Example** | **Structure and name of compound** | **Reaction moiety** | **Analyzed data** |
|---|---|---|---|
| **33** | 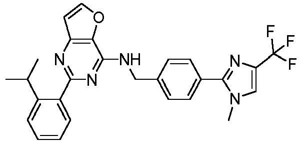 | 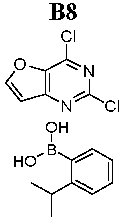 | LC-MS: m/z = 492.3 (M+H)⁺; |
| | | | ¹H NMR (400 MHz, DMSO) δ 9.59 (s, 1H), 8.50 (s, 1H), 7.92 (d, *J* = 1.4 Hz, 1H), 7.68 (d, *J* = 8.1 Hz, 2H), 7.46-7.51 (m, 5H), 7.29 - 7.31 (m, 1H), 7.14 (d, *J* = 2.2 Hz, 1H), 4.88 (d, *J =* 6.1 Hz, 2H), 3.75 (s, 3H), 3.32 - 3.25 (m, 1H), 1.05 (d, *J* = 6.8 Hz, 6H). |
| | 2-(2-Isopropylphenyl)-N-(4-(1-methyl-4-(trifluoromethyl)-1H-imidazol-2-yl)benzyl)furo[3,2-d]pyrimidin-4-amine | | |
| **34** | 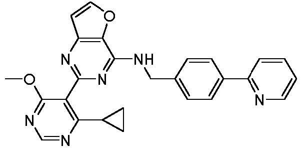 | 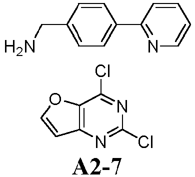 | LC-MS: m/z = 450.2 (M+H)⁺ |
| | 2-(4-Cyclopropyl-6-methoxypyrimidin-5-yl)-N-(4-(pyridin-2-yl)benzyl)furo[3,2-d]pyrimidin-4-amine | | |
| **35** | 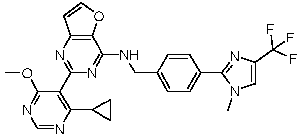 | 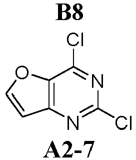 | LC-MS: m/z = 522.2 (M+H)⁺; |
| | | | ¹H NMR (400 MHz, DMSO) δ 9.29 (s, 1H), 8.69 (s, 1H), 8.46 (d, *J* = 2.2 Hz, 1H), 7.95 (d, *J* = 1.4 Hz, 1H), 7.69 (d, *J = 8.1* Hz, 2H), 7.50 (d, *J =* 7.9 Hz, 2H), 7.15 (d, *J* = 2.2 Hz, 1H), 4.83 (s, 2H), 3.87 (s, 3H), 3.78 (s, 3H), 1.91 - 1.77 (m, 1H), 1.02 (s, 2H), 0.83 (s, 2H). |
| | 2-(4-Cyclopropyl-6-methoxypyrimidin-5-yl)-N-(4-(1-methyl-4-(trifluoromethyl)-1H-imidazol-2-yl)benzyl)furo[3,2-d]pyrimidin-4-amine | | |
| **36** | 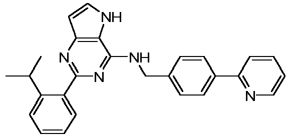 | 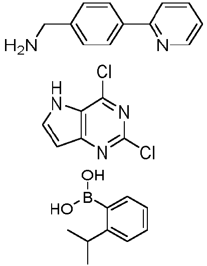 | LC-MS: m/z = 420.3 (M+H)⁺ |
| | 2-(2-Isopropylphenyl)-N-(4-(pyridin-2-yl)benzyl)-5H-pyrrolo[3,2-d]pyrimidin-4-amine | | |
| **37** | 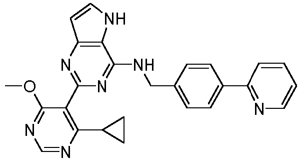 | 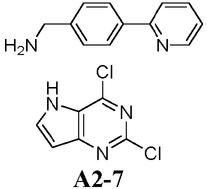 | LC-MS: m/z = 450.2 (M+H)⁺; |
| | | | ¹H NMR (400 MHz, DMSO) δ 12.11 (s, 1H), 9.61 (s, 1H), 8.80 (s, 1H), 8.69 (dd, *J =* 4.6, 1.6 Hz, 1H), 8.15 - 8.06 (m, 2H), 8.04 - 7.91 (m, 3H), 7.53 (d, *J =* 8.1 Hz, 2H), 7.44 - 7.36 (m, 1H), 6.73 - 6.64 (m, 1H), 4.96 (d, *J* = 5.7 Hz, 2H), 3.95 (s, 3H), 1.99 - 1.90 (m, 1H), 1.12 - 1.06 (m, 2H), 0.93 - 0.83 (m, 2H). |
| | 2-(4-Cyclopropyl-6-methoxypyrimidin-5-yl)-N-(4-(pyridin-2-yl)benzyl)-5H-pyrrolo[3,2-d]pyrimidin-4-amine | | |
| **38** | 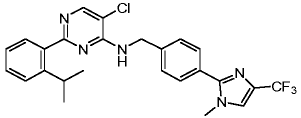 | 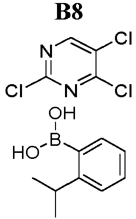 | LC-MS: m/z = 486.2 (M+H)⁺; |
| | | | ¹H NMR (400 MHz, DMSO) δ 8.4 (s, 1H), 8.15-8.19 (m, 1H), 7.9 (s, 1H), 7.65-7.67 (d, 2H), 7.42-7.44 (d, 1H), 7.39-7.41 (d, 2H), 7.35-7.36 (d, 2H), 7.17-7.21 (m,1H), 4.73-4.74 (d, 2H), 3.75(s, 3H), 3.44-3.47 (m, 1H), 0.99-1.01 (m, 6H). |
| | 5-Chloro-2-(2-isopropylphenyl)-N-(4-(1-methyl-4-(trifluoromethyl)-1H-imidazol-2-yl)benzyl)pyrimidin-4-amine | | |
| **39** | 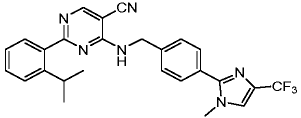 | 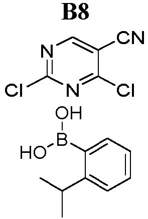 | LC-MS: m/z = 477.1 (M+H)⁺; |
| | | | ¹H NMR (400 MHz, DMSO) δ 8.79-8.81 (m, 2H),7.91 (s, 1H), 7.67 (d, 2H), 7.49-7.51 (d, 1H), 7.40-7.43 (m, 4H), 7.21-7.25 (m, 1H), 4.74-4.75 (d, 2H), 3.75 (s, 3H),3.48-3.58 (m, 1H) 1.01-1.02 (d,6H). |
| | 2-(2-Isopropylphenyl)-4-((4-(1-methyl-4-(trifluoromethyl)-1H-imidazol-2-yl)benzyl)amino)pyrimidine-5-carbonitrile | | |
| **40** | 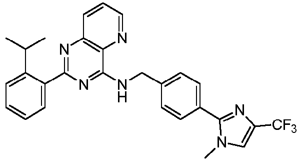 | 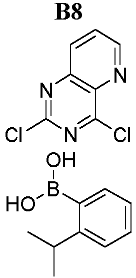 | LC-MS: m/z = 503.3 (M+H)⁺; |
| | | | ¹H NMR (400 MHz, DMSO) δ9.22-9.25 (m, 1H),8.85-8.86 (m, 1H), 8.15-8.16 (m, 1H), 7.86-7.90 (m, 2H),7.65-7.67 (d, 2H),7.53-7.55 (m, 1H),7.46-7.48 (m, 2H),7.38-7.39 (m, 2H), 7.23-7.25 (m, 1H), 4.86-4.88 (d, mH), 3.72 (s, 3H), 3.52-3.57 (m, 1H), 1.04-1.06 (m, 6H). |
| | 2-(2-Isopropylphenyl)-N-(4-(1-methyl-4-(trifluoromethyl)-1H-imidazol-2-yl)benzyl)pyrido[3,2-d]pyrimidin-4-amine | | |
| **41** | 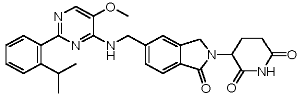 | 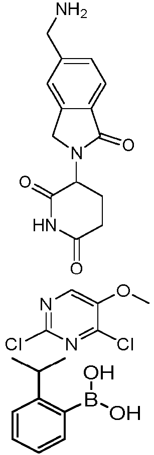 | LC-MS: m/z = 500.4 (M+H)⁺ |
| | 3-(5-(((2-(2-Isopropylphenyl)-5-methoxypyrimidin-4-yl)amino)methyl)-1-oxoisoindolin-2-yl)piperidine-2,6-dione | | |
| **42** | 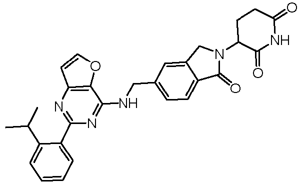 | 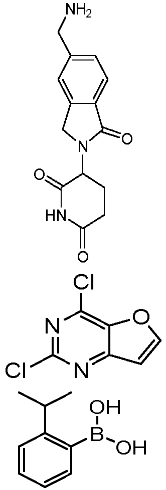 | LC-MS: m/z = 510.1 (M+H)⁺ |
| | 3-(5-(((2-(2-Isopropylphenyl)furo[3,2-d]pyrimidin-4-yl)amino)methyl)-1-oxoisoindolin-2-yl)piperidine-2,6-dione | | |
| **43** | 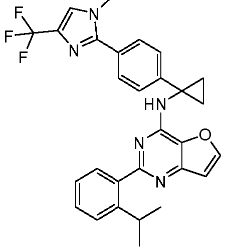 | 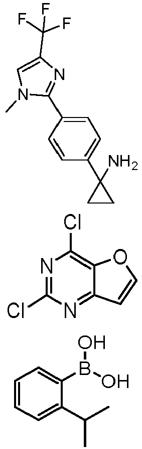 | LC-MS: m/z = 518.2 (M+H)⁺ |
| | 2-(2-Isopropylphenyl)-N-(1-(4-(1-methyl-4-(trifluoromethyl)-1H-imidazol-2-yl)phenyl)cyclopropyl)furo[3, 2-d]pyrimidin-4-amine | | |
| **44** | 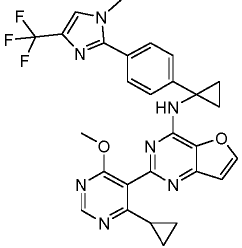 | 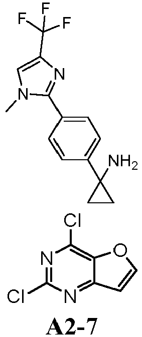 | LC-MS: m/z = 548.1 (M+H)⁺ |
| | 2-(4-Cyclopropyl-6-methoxypyrimidin-5-yl)-N-(1-(4-(1-methyl-4-(trifluoromethyl)-1H-imidazol-2-yl)phenyl)cyclopropyl)furo[3, 2-d]pyrimidin-4-amine | | |
| **50** | 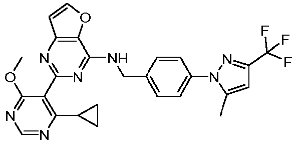 | 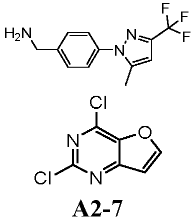 | LC-MS: m/z = 522.1 (M+H)⁺ |
| | | | ¹H NMR (400 MHz, DMSO) 8.60 (s, 1H), 8.32 (d, *J =* 2.0Hz, 1H), 7.50 - 7.54 (m, 4H), 6.98 - 7.04 (m, 1H), 6.74 (m, 1H), 4.75 (s, 2H), 3.81 (s, 3H), 2.29 (s, 3H), 1.64 - 1.66 (m, 1H), 0.95 - 0.98 (m, 2H), 0.76 - 0.79 (m, 2H). |
| | 2-(4-Cyclopropyl-6-methoxypyrimidin-5-yl)-N-(4-(5-methyl-3-(trifluoromethyl)-1H-pyrazol-1-yl)benzyl)furo[3,2-d]pyrimidin-4-amine | | |
| **57** | 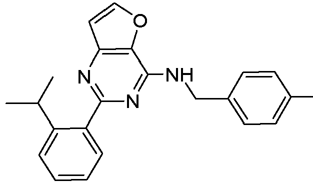 | 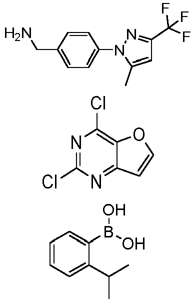 | LC-MS: m/z = 492.1 (M+H)⁺ |
| | 2-(2-Isopropylphenyl)-N-(4-(5-methyl-3-(trifluoromethyl)-1H-pyrazol-1-yl)benzyl)furo[3,2-d]pyrimidin-4-amine | | |

### Example 61: Synthesis of compound 61 (2-(2-isopropylphenyl)-5-methoxy-N-methyl-N-((4-(pyridin-2-yl)cyclohexyl)methyl)pyrimidin-4-amine)

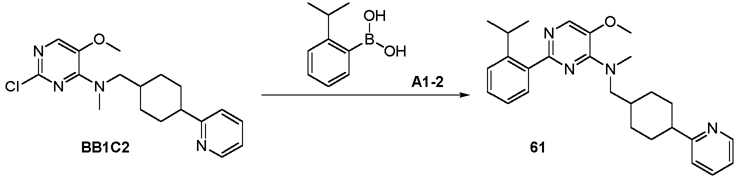

Compound **BB1C2** (125.0 mg, 0.36 mmol, 1.00 *eq*), compound **A1-2** (118.1 mg, 0.72 mmol, 2.00 *eq*), chloro(2-dicyclohexylphosphino-2',4',6'-triisopropyl-1,1'-biphenyl)[2-(2'-amino-1,1'-biphenyl)]palladium (II) (XPhos-Pd-G2, 56.58 mg, 0.072 mmol, 0.20 *eq*), 2-dicyclohexylphosphino-2',4',6'-triisopropylbiphenyl (XPhos, 68.65 mg, 0.144 mmol, 0.40 *eq*) and potassium phosphate (229.3 mg, 1.08 mmol, 3.00 *eq*) were added to dioxane (2.5 mL) and water (0.5 mL) successively. The resulting mixture was purged three times with nitrogen, and stirred at 95°C for 16 hours under nitrogen atmosphere. Water (15.0 mL) was added, and the resulting mixture was extracted three times with ethyl acetate (30.0 mL). The organic layers were combined, washed twice with saturated brine (20.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 0/1) to obtain compound **61** (93.5 mg, 0.22 mmol, 60.3% yield, 97.3% purity) as a white solid. LC-MS: m/z = 431.2 (M+H)⁺.

In accordance with the preparation method of compound 61, the following compounds were obtained using intermediates **A series, AA series, BBC series or A series, AA series, BBD series** as starting materials, XPhos-Pd-G2/XPhos/K₃PO₄ as reaction reagents, and dioxane and water as solvents.

| **Example** | **Structure and name of compound** | **Reaction moiety** | **Analyzed data** |
|---|---|---|---|
| **62** | 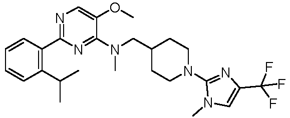 2-(2-Isopropylphenyl)-5-methoxy-N-methyl-N-((1-(1-methyl-4-(trifluoromethyl)-1H-imidazol-2-yl)piperidin-4-yl)methyl)pyrimidin-4-amine | **A1-2 and BB1C4** | LC-MS: m/z =503.2 (M+H)⁺ |
| **63** | 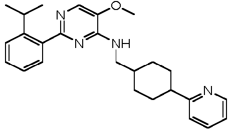 2-(2-Isopropylphenyl)-5-methoxy-N-((4-(pyridin-2-yl)cyclohexyl)methyl)pyrimidin-4-amine | **A1-2 and BB2C2** | LC-MS: m/z =417.2 (M+H)⁺ |
| **64** | 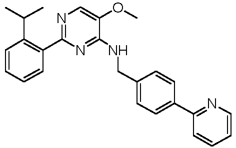 2-(2-Isopropylphenyl)-5-methoxy-N-(4-(pyridin-2-yl)benzyl)pyrimidin-4-amine | **A1-2 and BB2C3** | LC-MS: m/z =411.2 (M+H)⁺ |
| **65** | 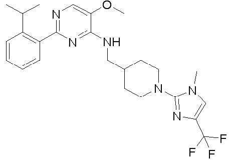 2-(2-Isopropylphenyl)-5-methoxy-N-((1-(1-methyl-4-(trifluoromethyl)-1H-imidazol-2-yl)piperidin-4-yl)methyl)pyrimidin-4-amine | **A1-2 and BB2C4** | LC-MS: m/z =489.2 (M+H)⁺ |
| **66** | 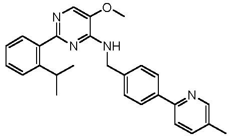 2-(2-Isopropylphenyl)-5-methoxy-N-(4-(5-methylpyridin-2-yl)benzyl)pyrimidin-4-amine | **A1-2 and BB2C14** | LC-MS: m/z =425.3 (M+H)⁺ |
| **67** | 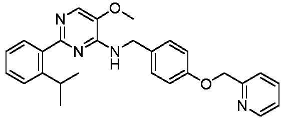 2-(2-Isopropylphenyl)-5-methoxy-N-(4-(pyridin-2-ylmethoxy)benzyl)pyrimidin-4-amine | **A1-2 and BB2C27** | LC-MS: m/z =441.1 (M+H)⁺ |
| **68** | 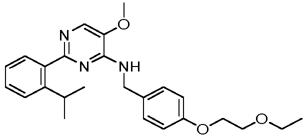 N-(4-(2-Ethoxyethoxy)benzyl)-2-(2-isopropylphenyl)-5-methoxypyrimidin-4-amine | **A1-2 and BB2C28** | LC-MS: m/z =422.2 (M+H)⁺; |
| | | | ¹H NMR (400 MHz, DMSO) δ 7.92 (s, 1H), 7.50 - 7.56 (m, 1H), 7.38-7.43 (m, 1H), 7.28 - 7.36 (m, 2H), 7.13 - 7.23 (m, 3H), 6.84 - 6.86 (m, 2H), 4.49 - 4.54 (m, 2H), 3.99 - 4.05 (m, 2H), 3.90 (s, 3H), 3.63 - 3.68 (m, 2H), 3.44 - 3.55 (m, 3H), 1.11 (t, *J =* 7.0 Hz, 3H), 1.04 (d, *J =* 6.8 Hz, 6H). |
| **69** | 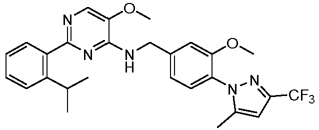 2-(2-Isopropylphenyl)-5-methoxy-N-(3-methoxy-4-(5-methyl-3-(trifluoromethyl)-1H-pyrazol-1-yl)benzyl)pyrimidin-4-amine | **A1-2 and BB2C33** | LC-MS: m/z =512.1 (M+H)⁺; |
| | | | ¹H NMR (400 MHz, CDCl3) δ 7.93 (s, 1H), 7.54 - 7.52 (m, 1H), 7.38 - 7.35 (m, 2H), 7.31 (s, 1H), 7.29 - 7.27 (m, 1H), 7.04 - 7.02 (m, 2H), 6.41 (s, 1H), 5.68 (s, 1H), 4.79 - 4.78 (m, 2H), 3.97 (s, 3H), 3.71 (s, 3H), 3.56 - 3.53 (m, 1H), 2.15 (s, 3H), 1.20 - 1.19 (m, 6H). |
| **70** | 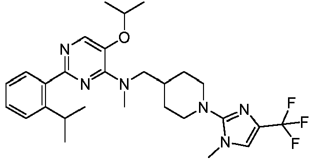 5-Isopropoxy-2-(2-isopropylphenyl)-N-methyl-N-((1-(1-methyl-4-(trifluoromethyl)-1H-imidazol-2-yl)piperidin-4-yl)methyl)pyrimidin-4-amine | **A1-2 and BB3C4** | LC-MS: m/z =531.3 (M+H)⁺ |
| **71** | 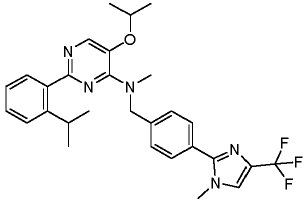 5-Isopropoxy-2-(2-isopropylphenyl)-N-methyl-N-(4-(1-methyl-4-(trifluoromethyl)-1H-imidazol-2-yl)benzyl)pyrimidin-4-amine | **A1-2 and BB3C5** | LC-MS: m/z =524.4 (M+H)⁺; |
| | | | ¹H NMR (400 MHz, CDCl3) δ 8.14 (s, 1H), 7.93 (s, 1H), 7.70 (d, *J* = 8.4 Hz, 2H), 7.49 - 7.47 (m, 1H), 7.39 - 7.32 (m, 4H), 7.21 - 7.17 (m, 1H), 4.97 (s, 2H), 4.72 - 4.66 (m, 1H), 3.77 (s, 3H), 3.63 - 3.56 (m, 1H), 3.17 (s, 3H), 1.23 (d, *J =* 6.0 Hz, 6H), 1.11 (d, *J* = 6.8 Hz, 6H). |
| **72** | 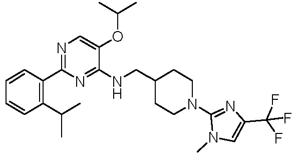 5-Isopropoxy-2-(2-isopropylphenyl)-N-((1-(1-methyl-4-(trifluoromethyl)-1H-imidazol-2-yl)piperidin-4-yl)methyl)pyrimidin-4-amine | **A1-2 and BB4C4** | LC-MS: m/z =517.2 (M+H)⁺ |
| **73** | 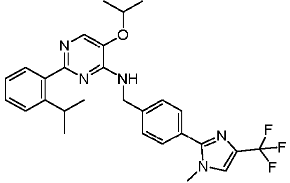 5-Isopropoxy-2-(2-isopropylphenyl)-N-(4-(1-methyl-4-(trifluoromethyl)-1H-imidazol-2-yl)benzyl)pyrimidin-4-amine | **A1-2 and BB4C5** | LC-MS: m/z =510.3 (M+H)⁺ |
| **74** | 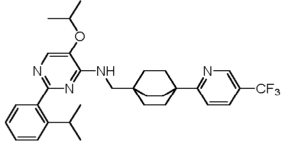 5-Isopropoxy-2-(2-isopropylphenyl)-N-((4-(5-(trifluoromethyl)pyridin-2-yl)bicyclo[2.2.2]octan-1-yl)methyl)pyrimidin-4-amine | **A1-2 and BB4C19** | LC-MS: m/z =539.4 (M+H)⁺ |
| **75** | 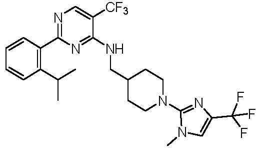 2-(2-Isopropylphenyl)-N-((1-(1-methyl-4-(trifluoromethyl)-1H-imidazol-2-yl)piperidin-4-yl)methyl)-5-(trifluoromethyl)pyrimidin-4-amine | **A1-2 and BB12C4** | LC-MS: m/z =527.2 (M+H)⁺ |
| **76** | 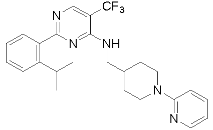 2-(2-Isopropylphenyl)-N-((1-(pyridin-2-yl)piperidin-4-yl)methyl)-5-(trifluoromethyl)pyrimidin-4-amine | **A1-2 and BB12C6** | LC-MS: m/z =456.5 (M+H)+ |
| **77** | 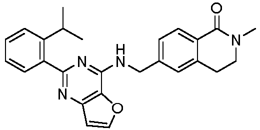 6-(((2-(2-Isopropylphenyl)furo[3,2-d]pyrimidin-4-yl)amino)methyl)-2-methyl-3,4-dihydroisoquinolin-1(2H)-one | **A1-2 and BB13C25** | LC-MS: m/z =427.2 (M+H)+ |
| **78** | 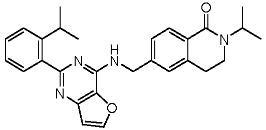 2-Isopropyl-6-(((2-(2-isopropylphenyl)furo[3,2-d]pyrimidin-4-yl)amino)methyl)-3,4-dihydroisoquinolin-1(2H)-one | **A1-2 and BB13C26** | LC-MS: m/z =455.1 (M+H)+ |
| **79** | 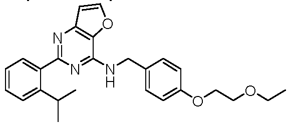 N-(4-(2-Ethoxyethoxy)benzyl)-2-(2-isopropylphenyl)furo[3,2-d]pyrimidin-4-amine | **A1-2 and BB13C28** | LC-MS: m/z =432.1 (M+H)+ |
| **80** | 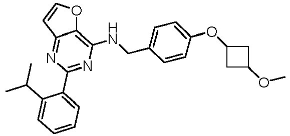 2-(2-Isopropylphenyl)-N-(4-(3-methoxycyclobutoxy)benzyl)furo[3, 2-d]pyrimidin-4-amine | **A1-2 and BB13C29** | LC-MS: m/z =444.2 (M+H)+ |
| **81** | 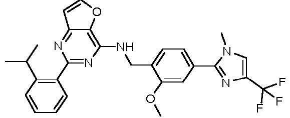 2-(2-Isopropylphenyl)-N-(2-methoxy-4-(1-methyl-4-(trifluoromethyl)-1H-imidazol-2-yl)benzyl)furo[3,2-d]pyrimidin-4-amine | **A1-2 and BB13C30** | LC-MS: m/z =522.2 (M+H)+ |
| **82** | 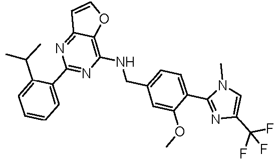 2-(2-Isopropylphenyl)-N-(3-methoxy-4-(1-methyl-4-(trifluoromethyl)-1H-imidazol-2-yl)benzyl)furo[3,2-d]pyrimidin-4-amine | **A1-2 and BB13C31** | LC-MS: m/z =522.0 (M+H)+ |
| **83** | 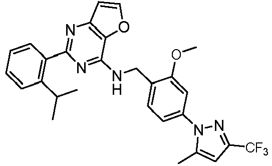 2-(2-Isopropylphenyl)-N-(2-methoxy-4-(5-methyl-3-(trifluoromethyl)-1H-pyrazol-1-yl)benzyl)furo[3,2-d]pyrimidin-4-amine | **A1-2 and BB13C32** | LC-MS: m/z =522.2 (M+H)+ |
| **84** | 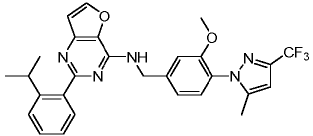 2-(2-Isopropylphenyl)-N-(3-methoxy-4-(5-methyl-3-(trifluoromethyl)-1H-pyrazol-1-yl)benzyl)furo[3,2-d]pyrimidin-4-amine | **A1-2 and BB13C33** | LC-MS: m/z =522.1 (M+H)+; |
| | | | ¹H NMR (400 MHz, CDCl3) δ 7.80 (s, 1H), 7.79-7.55 (m, 1H), 7.41-7.40 (m, 2H), 7.38 (s, 1H), 7.33-7.27 (m, 1H), 7.08 (s, 2H), 6.94-6.93 (m, 1H), 6.42 (s, 1H), 5.60-5.57 (m, 1H), 4.95-4.94 (m, 2H), 3.71 (s, 3H), 3.52-3.49 (m, 1H), 2.15 (s, 3H), 1.22-1.19 (m, 6H). |
| **85** | 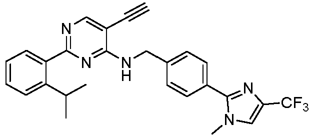 5-Ethynyl-2-(2-isopropylphenyl)-N-(4-(1-methyl-4-(trifluoromethyl)-1H-imidazol-2-yl)benzyl)pyrimidin-4-amine | **A1-2 and BB18D5** | LC-MS: m/z =476.1 (M+H)+; |
| | | | ¹H NMR (400 MHz, DMSO-d₆) δ 9.14 (s, 1H), 7.90 (s, 1H), 7.80 (d, J = 3.6 Hz, 1H), 7.67 (d, J = 8.3 Hz, 2H), 7.61 - 7.56 (m, 1H), 7.43 (dd, J = 14.1, 7.4 Hz, 2H), 7.36 (d, J = 8.3 Hz, 2H), 7.31 - 7.24 (m, 1H), 6.74 (d, J = 3.5 Hz, 1H), 5.58 (s, 2H), 3.73 (s, 3H), 3.55 - 3.49 (m, 1H), 1.12 (d, J = 6.9 Hz, 6H). |
| **86** | 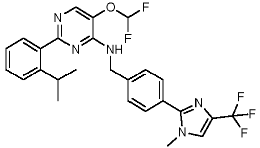 5-(Difluoromethoxy)-2-(2-isopropylphenyl)-N-(4-(1-methyl-4-(trifluoromethyl)-1H-imidazol-2-yl)benzyl)pyrimidin-4-amine | **A1-2 and BB19C5** | LC-MS: m/z =518.0 (M+H)+ |
| **87** | 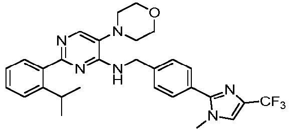 2-(2-Isopropylphenyl)-N-(4-(1-methyl-4-(trifluoromethyl)-1H-imidazol-2-yl)benzyl)-5-morpholinopyrimidin-4-amine | **A1-2 and BB21D5** | LC-MS: m/z =537.4 (M+H)+; |
| | | | ¹H NMR (400 MHz, DMSO-*d*₆) δ 8.07 (s, 1H), 7.90 (s, 1H), 7.65 (d, *J* = 8.2 Hz, 2H), 7.41 (d, *J =* 6.9 Hz, 2H), 7.38 (d, *J =* 8.2 Hz, 2H), 7.32 (d, *J =* 1.4 Hz, 1H), 7.18 - 7.12 (m, 1H), 4.73 (d, *J =* 6.3 Hz, 2H), 3.83 - 3.80 (m, 4H), 3.75 (s, 3H), 3.57 - 3.48 (m, 1H), 2.97 - 2.93 (m, 4H), 0.99 (d, *J* = 6.9 Hz, 6H). |
| **88** | 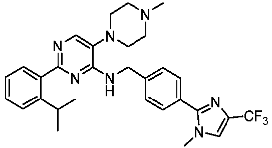 2-(2-Isopropylphenyl)-N-(4-(1-methyl-4-(trifluoromethyl)-1H-imidazol-2-yl)benzyl)-5-(4-methylpiperazin-1-yl)pyrimidin-4-amine | **A1-2 and BB22D5** | LC-MS: m/z =550.1 (M+H)⁺; |
| | | | ¹H NMR (400 MHz, DMSO-d₆) δ 8.06 (s, 1H), 7.90 (s, 1H), 7.65 (d, J = 8.2 Hz, 2H), 7.41 (d, J = 8.3 Hz, 1H), 7.38 (d, J = 8.2 Hz, 2H), 7.31 (d, J = 1.4 Hz, 1H), 7.22 (s, 1H), 7.18 - 7.11 (m, 1H), 4.72 (d, J = 6.2 Hz, 2H), 3.75 (s, 3H), 3.54 (d, J = 6.8 Hz, 1H), 2.96 (s, 4H), 2.56 (s, 4H), 2.26 (s, 3H), 1.00 (d, J = 6.9 Hz, 6H). |
| **89** | 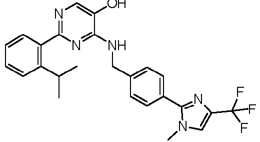 2-(2-Isopropylphenyl)-4-((4-(1-methyl-4-(trifluoromethyl)-1H-imidazol-2-yl)benzyl)amino)pyrimidin-5-ol | **A1-2 and BB24D5** | LC-MS: m/z =468.1 (M+H)+ |
| **90** | 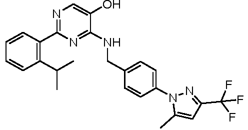 2-(2-Isopropylphenyl)-4-((4-(5-methyl-3-(trifluoromethyl)-1H-pyrazol-1-yl)benzyl)amino)pyrimidin-5-ol | **A1-2 and BB24D11** | LC-MS: m/z =468.3 M+H)+ |
| **91** | 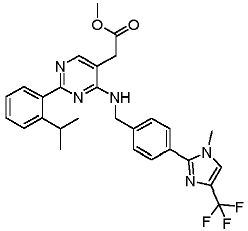 Methyl 2-(2-(2-isopropylphenyl)-4-((4-(1-methyl-4-(trifluoromethyl)-1H-imidazol-2-yl)benzyl)amino)pyrimidin-5-yl)acetate | **A1-2 and BB41D5** | LC-MS: m/z =524.2 (M+H)+ |
| **92** | 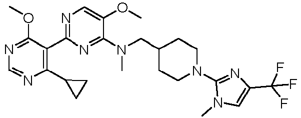 4'-Cyclopropyl-5,6'-dimethoxy-N-methyl-N-((1-(1-methyl-4-(trifluoromethyl)-1H-imidazol-2-yl)piperidin-4-yl)methyl)-[2,5'-bipyrimidin]-4-amine | **A2-7 and BB1C4** | LC-MS: m/z =533.4 (M+H)+ |
| **93** | 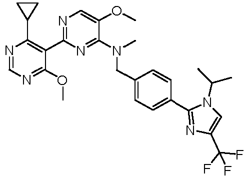 4'-Cyclopropyl-N-(4-(1-isopropyl-4-(trifluoromethyl)-1H-imidazol-2-yl)benzyl)-5,6'-dimethoxy-N-methyl-[2,5'-bipyrimidin]-4-amine | **A2-7 and BB1C58** | LC-MS: m/z =554.4 (M+H)+; |
| | | | ¹H NMR (400 MHz, DMSO-*d*₆) δ 8.62 (s, 1H), 8.20 (d, *J* = 1.4 Hz, 1H), 8.16 (s, 1H), 7.61 - 7.52 (m, 2H), 7.43 (d, *J* = 8.2 Hz, 2H), 4.95 (s, 2H), 4.61 - 4.35 (m, 1H), 3.90 (s, 3H), 3.85 (s, 3H), 3.19 (s, 3H), 1.77 (s, 1H), 1.43 (d, *J = 6.6* Hz, 6H), 1.00 (dt, *J* = 4.7, 3.0 Hz, 2H), 0.86 (dt, *J* = 8.1, 3.3 Hz, 2H). |
| **94** | 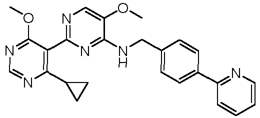 4'-Cyclopropyl-5,6'-dimethoxy-N-(4-(pyridin-2-yl)benzyl)-[2,5'-bipyrimidin]-4-amine | **A2-7 and BB2C3** | LC-MS: m/z =441.2 (M+H)+ |
| **95** | 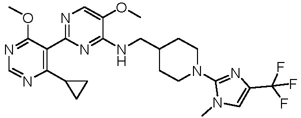 4'-Cyclopropyl-5,6'-dimethoxy-N-((1-(1-methyl-4-(trifluoromethyl)-1H-imidazol-2-yl)piperidin-4-yl)methyl)-[2,5'-bipyrimidin]-4-amine | **A2-7 and BB2C4** | LC-MS: m/z =519.3 (M+H)+ |
| **96** | 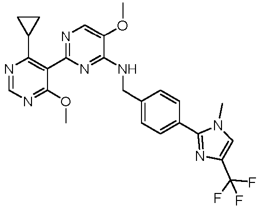 4'-Cyclopropyl-5,6'-dimethoxy-N-(4-(1-methyl-4-(trifluoromethyl)-1H-imidazol-2-yl)benzyl)-[2,5'-bipyrimidin]-4-amine | **A2-7 and BB2C5** | LC-MS: m/z =512.2 (M+H)+; |
| | | | ¹H NMR (400 MHz, DMSO-*d*₆) δ 8.60 (s, 1H), 7.96 (d, *J =* 16.5 Hz, 2H), 7.86 (t, *J =* 6.4 Hz, 1H), 7.65 (d, *J =* 8.0 Hz, 2H), 7.43 (d, *J* = 8.0 Hz, 2H), 4.64 (d, *J* = 6.3 Hz, 2H), 3.96 (s, 3H), 3.83 (s, 3H), 3.78 (s, 3H), 1.70 (tt, *J* = 8.5, 4.7 Hz, 1H), 0.97 (s, 2H), 0.78 (dd, *J* = 7.8, 3.3 Hz, 2H). |
| **97** | 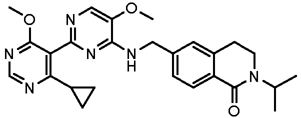 6-(((4'-Cyclopropyl-5,6'-dimethoxy-[2,5'-bipyrimidin]-4-yl)amino)methyl)-2-isopropyl-3,4-dihydroisoquinolin-1(2H)-one | **A2-7 and BB2C26** | LC-MS: m/z = 475.2 (M+H)+ |
| **98** | 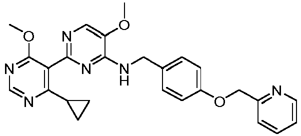 4'-Cyclopropyl-5,6'-dimethoxy-N-(4-(pyridin-2-ylmethoxy)benzyl)-[2,5'-bipyrimidin]-4-amine | **A2-7 and BB2C27** | LC-MS: m/z =471.1 (M+H)+ |
| **99** | 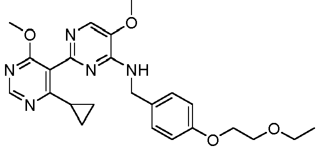 4'-Cyclopropyl-N-(4-(2-ethoxyethoxy)benzyl)-5,6'-dimethoxy-[2,5'-bipyrimidin]-4-amine | **A2-7 and BB2C28** | LC-MS: m/z =452.1 (M+H)+; |
| | | | ¹H NMR (400 MHz, DMSO) δ 8.58 (s, 1H), 7.91 (s, 1H), 7.63 (t, *J =* 6.0 Hz, 1H), 7.16-7.22 (m, 2H), 6.81-6.86 (m, 2H), 4.43-4.47 (m, 2H), 4.00-4.04 (m, 2H), 3.89 (s, 3H), 3.82 (s, 3H), 3.63-3.68 (m, 2H), 3.48 (q, *J* = 6.8 Hz, 2H), 1.62-1.72 (m, 1H), 1.11 (t, *J =* 6.8 Hz, 3H), 0.93-0.99 (m, 2H), 0.75-0.81 (m, 2H). |
| **100** | 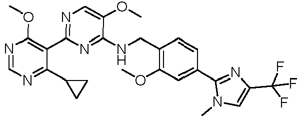 4'-Cyclopropyl-5,6'-dimethoxy-N-(2-methoxy-4-(1-methyl-4-(trifluoromethyl)-1H-imidazol-2-yl)benzyl)-[2,5'-bipyrimidin]-4-amine | **A2-7 and BB2C30** | LC-MS: m/z =542.1 (M+H)+ |
| **101** | 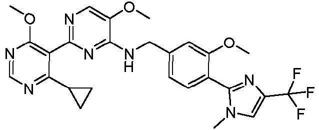 **4'-Cyclopropyl-5,6'-dimethoxy-N-(3-methoxy-4-(1-methyl-4-(trifluoromethyl)-1H-imidazol-2-yl)benzyl)-[2,5'-bipyrimidin]-4-amine** | **A2-7 and BB2C31** | LC-MS: m/z =542.0 (M+H)+ |
| **102** | 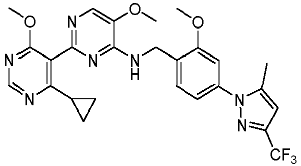 4'-Cyclopropyl-5,6'-dimethoxy-N-(2-methoxy-4-(5-methyl-3-(trifluoromethyl)-1H-pyrazol-1-yl)benzyl)-[2,5'-bipyrimidin]-4-amine | **A2-7 and BB2C32** | LC-MS: m/z =542.1 (M+H)+ |
| **103** | 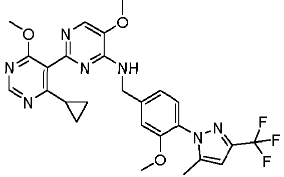 4'-Cyclopropyl-5,6'-dimethoxy-N-(3-methoxy-4-(5-methyl-3-(trifluoromethyl)-1H-pyrazol-1-yl)benzyl)-[2,5'-bipyrimidin]-4-amine | **A2-7 and BB2C33** | LC-MS: m/z =542.1 (M+H)+; |
| | | | ¹H NMR (400 MHz, CDCl3) δ 8.61 (s, 1H), 7.94 (s, 1H), 7.31 - 7.27 (m, 1H), 7.06 - 7.03 (m, 2H), 6.42 (s, 1H), 5.74 - 5.73 (m, 1H), 4.77 - 4.75 (m, 2H), 3.97 - 3.93 (m, 6H), 3.75 (s, 3H), 2.16 (m, 3H), 1.82 - 1.80 (m, 1H), 1.19 - 1.16 (m, 2H), 1.89 - 1.84 (m, 2H). |
| **104** | 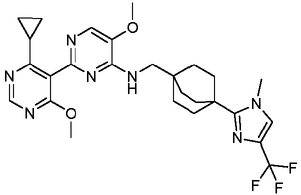 4'-Cyclopropyl-5,6'-dimethoxy-N-((4-(1-methyl-4-(trifluoromethyl)-1H-imidazol-2-yl)bicyclo[2.2.2]octan-1-yl)methyl)-[2,5'-bipyrimidin]-4-amine | **A2-7 and BB2C35** | LC-MS: m/z =544.3 (M+H)+; |
| | | | ¹H NMR (400 MHz, DMSO-*d*₆) δ 8.62 (s, 1H), 7.90 (s, 1H), 7.65 (d, *J* = 1.5 Hz, 1H), 6.99 (t, *J =* 6.5 Hz, 1H), 3.93 (s, 3H), 3.84 (s, 3H), 3.77 (s, 3H), 3.21 (d, *J =* 6.5 Hz, 2H), 1.90 (m, 6H), 1.74 (m, 1H), 1.48 (m, 6H), 1.06 - 1.01 (m, 2H), 0.92 - 0.85 (m, 2H). |
| **105** | 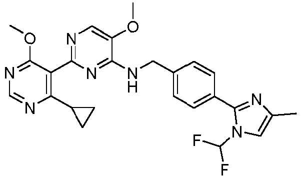 4'-Cyclopropyl-N-(4-(1-(difluoromethyl)-4-methyl-1H-imidazol-2-yl)benzyl)-5,6'-dimethoxy-[2,5'-bipyrimidin]-4-amine | **A2-7 and BB2C48** | LC-MS: m/z =494.2 (M+H)+; |
| | | | ¹H NMR (400 MHz, DMSO-*d*₆) δ 8.60 (s, 1H), 7.98 (s, 1H), 7.82 (t, *J =* 6.3 Hz, 1H), 7.67 - 7.62 (m, 3H), 7.42 (d, *J* = 8.1 Hz, 2H), 7.07 - 6.79 (m, 1H),4.64 (d, *J =* 6.3 Hz, 2H), 3.96 (s, 3H), 3.83 (s, 3H), 3.76 (s, 3H), 1.71 (dt, *J =* 8.1, 3.5 Hz, 1H), 1.02 - 0.93 (m, 2H), 0.86 - 0.71 (m, 2H). |
| **106** | 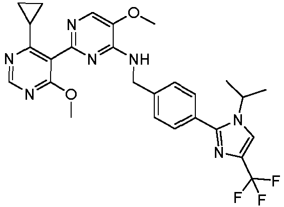 4'-Cyclopropyl-N-(4-(1-isopropyl-4-(trifluoromethyl)-1H-imidazol-2-yl)benzyl)-5,6'-dimethoxy-[2,5'-bipyrimidin]-4-amine | **A2-7 and BB2C58** | LC-MS: m/z =540.2 (M+H)+; |
| | | | ¹H NMR (400 MHz, DMSO-*d*₆) δ 8.59 (s, 1H), 8.19 (d, *J =* 1.4 Hz, 1H), 7.98 (s, 1H), 7.87 (t, *J =* 6.3 Hz, 1H), 7.49 (d, *J=* 8.0 Hz, 2H), 7.43 (d, *J* = 8.1 Hz, 2H), 4.64 (d, *J =* 6.3 Hz, 2H), 4.54 - 4.34 (m, 1H), 3.96 (s, 3H), 3.82 (s, 3H), 1.69 (s, 1H), 1.41 (d, *J= 6.6* Hz, 6H), 0.95 (t, *J =* 3.8 Hz, 2H), 0.80 - 0.69 (m, 2H). |
| **107** | 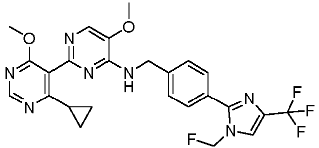 4'-Cyclopropyl-N-(4-(1-(fluoromethyl)-4-(trifluoromethyl)-1H-imidazol-2-yl)benzyl)-5,6'-dimethoxy-[2,5'-bipyrimidin]-4-amine | **A2-7 and BB2C59** | LC-MS: m/z =530.2 (M+H)⁺; |
| | | | ¹H NMR (400 MHz, CDCl₃) δ 8.58 (s, 1H), 7.91 (s, 1H), 7.67 (d, *J* = 8.1 Hz, 2H), 7.52 (s, 1H), 7.48 (d, *J = 8.0* Hz, 2H), 5.92 (s, 1H), 5.77 - 5.69 (m, 1H), 5.73 (s, 1H), 4.77 (d, *J* = 6.1 Hz, 2H), 3.94 (s, 3H), 3.91 (s, 3H), 1.83 - 1.73 (m, 1H), 1.17 - 1.12 (m, 2H), 0.85 - 0.78 (m, 2H). |
| **108** | 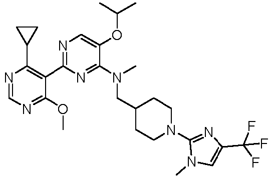 4'-Cyclopropyl-5-isopropoxy-6'-methoxy-N-methyl-N-((1-(1-methyl-4-(trifluoromethyl)-1H-imidazol-2-yl)piperidin-4-yl)methyl)-[2,5'-bipyrimidin]-4-amine | **A2-7 and BB3C4** | LC-MS: m/z =561.2 (M+H)+ |
| **109** | 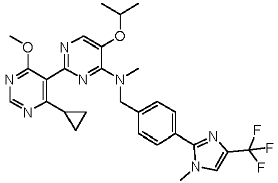 4'-Cyclopropyl-5-isopropoxy-6'-methoxy-N-methyl-N-(4-(1-methyl-4-(trifluoromethyl)-1H-imidazol-2-yl)benzyl)-[2,5'-bipyrimidin]-4-amine | **A2-7 and BB3C5** | LC-MS: m/z =554.3 (M+H)+ |
| **110** | 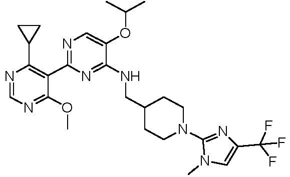 4'-Cyclopropyl-5-isopropoxy-6'-methoxy-N-((1-(1-methyl-4-(trifluoromethyl)-1H-imidazol-2-yl)piperidin-4-yl)methyl)-[2,5'-bipyrimidin]-4-amine | **A2-7 and BB4C4** | LC-MS: m/z =547.4 (M+H)+ |
| **111** | 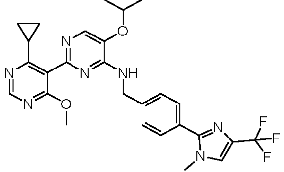 4'-Cyclopropyl-5-isopropoxy-6'-methoxy-N-(4-(1-methyl-4-(trifluoromethyl)-1H-imidazol-2-yl)benzyl)-[2,5'-bipyrimidin]-4-amine | **A2-7 and BB4C5** | LC-MS: m/z =540.2 (M+H)+ |
| **112** | 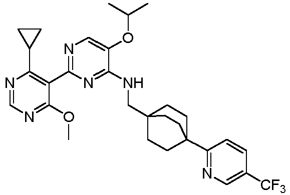 4'-Cyclopropyl-5-isopropoxy-6'-methoxy-N-((4-(5-(trifluoromethyl)pyridin-2-yl)bicyclo[2.2.2]octan-1-yl)methyl)-[2,5'-bipyrimidin]-4-amine | **A2-7 and BB4C19** | LC-MS: m/z =569.4 (M+H)+ |
| **113** | 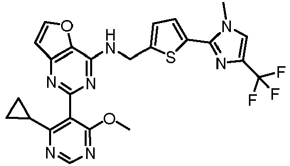 2-(4-Cyclopropyl-6-methoxypyrimidin-5-yl)-N-((5-(1-methyl-4-(trifluoromethyl)-1H-imidazol-2-yl)thiophen-2-yl)methyl)furo[3,2-d]pyrimidin-4-amine | **A2-7 and BB13C7** | LC-MS: m/z =528.3 (M+H)+ |
| **114** | 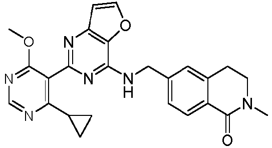 6-(((2-(4-Cyclopropyl-6-methoxypyrimidin-5-yl)furo[3,2-d]pyrimidin-4-yl)amino)methyl)-2-methyl-3,4-dihydroisoquinolin-1(2H)-one | **A2-7 and BB13C25** | LC-MS: m/z =457.1 (M+H)+ |
| **115** | 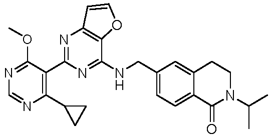 6-(((2-(4-Cyclopropyl-6-methoxypyrimidin-5-yl)furo[3,2-d]pyrimidin-4-yl)amino)methyl)-2-isopropyl-3,4-dihydroisoquinolin-1(2H)-one | **A2-7 and BB13C26** | LC-MS: m/z =485.2 (M+H)+ |
| **116** | 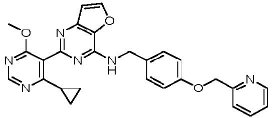 2-(4-Cyclopropyl-6-methoxypyrimidin-5-yl)-N-(4-(pyridin-2-ylmethoxy)benzyl)furo[3,2-d]pyrimidin-4-amine | **A2-7 and BB13C27** | LC-MS: m/z =481.1 (M+H)+ |
| **117** | 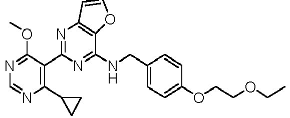 2-(4-Cyclopropyl-6-methoxypyrimidin-5-yl)-N-(4-(2-ethoxyethoxy)benzyl)furo[3,2-d]pyrimidin-4-amine | **A2-7 and BB13C28** | LC-MS: m/z =462.1 (M+H)+ |
| **118** | 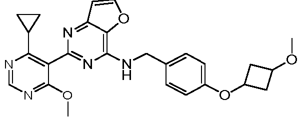 2-(4-Cyclopropyl-6-methoxypyrimidin-5-yl)-N-(4-(3-methoxycyclobutoxy)benzyl)furo[3, 2-d]pyrimidin-4-amine | **A2-7 and BB13C29** | LC-MS: m/z =474.2 (M+H)+ |
| **119** | 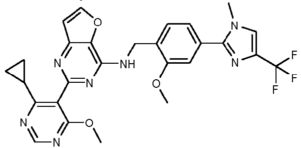 2-(4-Cyclopropyl-6-methoxypyrimidin-5-yl)-N-(2-methoxy-4-(1-methyl-4-(trifluoromethyl)-1H-imidazol-2-yl)benzyl)furo[3,2-d]pyrimidin-4-amine | **A2-7 and BB13C30** | LC-MS: m/z =552.0 (M+H)+ |
| **120** | 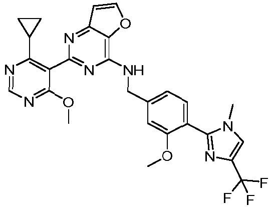 2-(4-Cyclopropyl-6-methoxypyrimidin-5-yl)-N-(3-methoxy-4-(1-methyl-4-(trifluoromethyl)-1H-imidazol-2-yl)benzyl)furo[3,2-d]pyrimidin-4-amine | **A2-7 and BB13C31** | LC-MS: m/z =552.0 (M+H)+ |
| **121** | 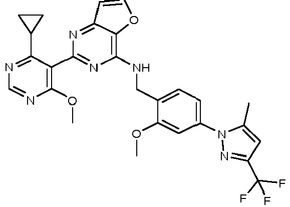 2-(4-Cyclopropyl-6-methoxypyrimidin-5-yl)-N-(2-methoxy-4-(5-methyl-3-(trifluoromethyl)-1H-pyrazol-1-yl)benzyl)furo[3,2-d]pyrimidin-4-amine | **A2-7 and BB13C32** | LC-MS: m/z =552.1 (M+H)+ |
| **122** | 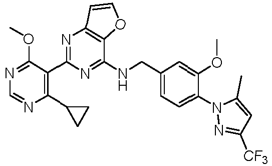 2-(4-Cyclopropyl-6-methoxypyrimidin-5-yl)-N-(3-methoxy-4-(5-methyl-3-(trifluoromethyl)-1H-pyrazol-1-yl)benzyl)furo[3,2-d]pyrimidin-4-amine | **A2-7 and BB13C33** | LC-MS: m/z =552.2 (M+H)+; |
| | | | ¹H NMR (400 MHz, CDCl3) δ 8.64 (s, 1H), 7.82 (s, 1H), 7.33 - 7.27 (m, 1H), 7.11 - 7.08 (m, 2H), 6.45 (s, 1H), 6.42 (s, 1H), 5.67 (s, 1H), 4.97-4.93 (m, 2H), 3.93 (s, 3H), 3.76 (s, 3H), 2.15 (s, 3H), 1.79 - 1.76 (m, 1H), 1.21 - 1.19 (m, 2H) , 0.88 - 0.86 (m, 2H). |
| **123** | 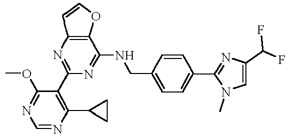 2-(4-Cyclopropyl-6-methoxypyrimidin-5-yl)-N-(4-(4-(difluoromethyl)-1-methyl-1H-imidazol-2-yl)benzyl)furo[3,2-d]pyrimidin-4-amine | **A2-7 and BB13C48** | LC-MS: m/z =504.2 (M+H)+; |
| | | | ¹H NMR (400 MHz, DMSO-*d*₆) δ 8.66 (s, 1H), 8.62 (s, 1H), 8.33 (d, *J =* 2.2 Hz, 1H), 7.63 (d, *J = 8.1* Hz, 2H), 7.48 (d, *J = 8.0* Hz, 2H), 7.42 - 7.34 (m, 1H), 7.12 - 7.07 (m, 1H), 7.05 (d, *J* = 2.2 Hz, 1H), 4.77 (d, *J* = 6.1 Hz, 2H), 3.83 (s, 3H), 3.73 (s, 3H), 1.68 (t, *J =* 7.8 Hz, 1H), 0.98 (d, *J* = 4.2 Hz, 2H), 0.78 (s, 2H). |
| **124** | 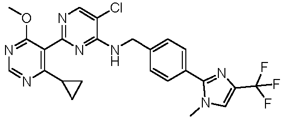 5-Chloro-4'-cyclopropyl-6'-methoxy-N-(4-(1-methyl-4-(trifluoromethyl)-1H-imidazol-2-yl)benzyl)-[2,5'-bipyrimidin]-4-amine | **A2-7 and BB15C5** | LC-MS: m/z =516.0 (M+H)+; |
| | | | ¹H NMR (400 MHz, DMSO-*d*₆) δ 8.61 (s, 1H), 8.42 (s, 1H), 8.29 (t, *J* = 6.2 Hz, 1H), 7.92 (d, *J* = 1.4 Hz, 1H), 7.76 - 7.54 (m, 2H), 7.41 (d, *J* = 8.2 Hz, 2H), 4.67 (d, *J* = 6.1 Hz, 2H), 3.81 (s, 3H), 3.75 (s, 3H), 1.77 - 1.59 (m, 1H), 0.95 (p, *J* = 3.7 Hz, 2H), 0.77 (dq, *J =* 7.0, 3.5 Hz, 2H). |
| **125** | 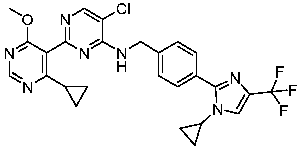 5-Chloro-4'-cyclopropyl-N-(4-(1-cyclopropyl-4-(trifluoromethyl)-1H-imidazol-2-yl)benzyl)-6'-methoxy-[2,5'-bipyrimidin]-4-amine | **A2-7 and BB15C57** | LC-MS: m/z =542.4 (M+H)⁺; |
| | | | ¹H NMR (400 MHz, MeOH-*d*₄) δ 8.53 (s, 1H), 8.29 (s, 1H), 7.74 (d, *J* = 8.2 Hz, 2H), 7.69 (s, 1H), 7.46 (d, *J* = 7.9 Hz, 2H), 4.78 (s, 2H), 3.88 (s, 3H), 3.66 - 3.56 (m, 1H), 1.74 - 1.63 (m, 1H), 1.07 - 1.02 (m, 2H), 1.01 - 0.96 (m, 2H), 0.85 - 0.77 (m, 4H). |
| **126** | 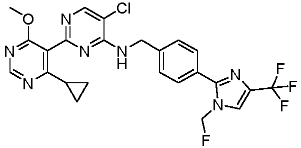 5-Chloro-4'-cyclopropyl-N-(4-(1-(fluoromethyl)-4-(trifluoromethyl)-1H-imidazol-2-yl)benzyl)-6'-methoxy-[2,5'-bipyrimidin]-4-amine | **A2-7 and BB15C59** | LC-MS: m/z =534.3 (M+H)⁺; |
| | | | ¹H NMR (400 MHz, DMSO-*d*₆) δ 8.57 (s, 1H), 8.39 (s, 1H), 8.33 - 8.22 (m, 2H), 7.60 (d, *J* = 8.2 Hz, 2H), 7.43 (d, *J* = 8.0 Hz, 2H), 6.05 (d, *J* = 51.7 Hz, 2H), 4.64 (d, *J* = 6.1 Hz, 2H), 3.77 (s, 3H), 1.71 - 1.58 (m, 1H), 0.96 - 0.88 (m, 2H), 0.78 - 0.69 (m, 2H). |
| **127** | 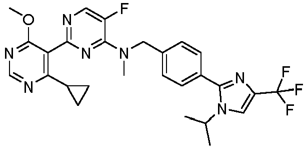 4'-Cyclopropyl-5-fluoro-N-(4-(1-isopropyl-4-(trifluoromethyl)-1H-imidazol-2-yl)benzyl)-6'-methoxy-N-methyl-[2,5'-bipyrimidin]-4-amine | **A2-7 and BB16C58** | LC-MS: m/z =542.0 (M+H)+; |
| | | | ¹H NMR (400 MHz, DMSO-*d*₆) δ 8.61 (s, 1H), 8.39 (d, *J* = 6.8 Hz, 1H), 8.18 (s, 1H), 7.54 (d, *J =* 8.0 Hz, 2H), 7.43 (d, *J* = 8.1 Hz, 2H), 4.90 (s, 2H), 4.50 - 4.40 (m, 1H), 3.84 (s, 3H), 3.27 - 3.23 (m, 3H), 1.78 - 1.70 (m, 1H), 1.40 (d, *J* = 6.6 Hz, 6H), 1.01 - 0.95 (m, 2H), 0.86 - 0.79 (m, 2H). |
| **128** | 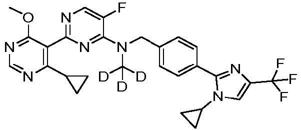 4'-Cyclopropyl-N-(4-(1-cyclopropyl-4-(trifluoromethyl)-1H-imidazol-2-yl)benzyl)-5-fluoro-6'-methoxy-N-(methyl-d3)-[2,5'-bipyrimidin]-4-amine | **A2-7 and BB16-D3C57** | LC-MS: m/z =543.2 (M+H)+; ¹H NMR (400 MHz, MeOH-*d*₄) δ 8.53 (s, 1H), 8.21 (d, *J =* 6.9 Hz, 1H), 7.79 (d, *J =* 8.3 Hz, 2H), 7.71 (s, 1H), 7.45 (d, *J =* 8.2 Hz, 2H), 4.97 (s, 2H), 3.90 (s, 3H), 3.69 - 3.56 (m, 1H), 1.81 - 1.71 (m, 1H), 1.10 - 1.05 (m, 2H), 1.04 - 0.98 (m, 2H), 0.89 - 0.83 (m, 4H). |
| **129** | 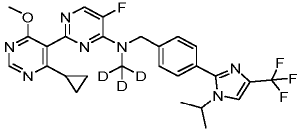 4'-Cyclopropyl-5-fluoro-N-(4-(1-isopropyl-4-(trifluoromethyl)-1H-imidazol-2-yl)benzyl)-6'-methoxy-N-(methyl-d3)-[2,5'-bipyrimidin]-4-amine | **A2-7 and BB16-D3C58** | LC-MS: m/z =545.4 (M+H)+; |
| | | | ¹H NMR (400 MHz, DMSO-*d*₆) δ 8.61 (s, 1H), 8.38 (d, *J* = 6.8 Hz, 1H), 8.18 (s, 1H), 7.54 (d, *J = 8.1* Hz, 2H), 7.43 (d, *J =* 7.9 Hz, 2H), 4.90 (s, 2H), 4.52 - 4.40 (m, 1H), 3.84 (s, 3H), 1.78 - 1.70 (m, 1H), 1.40 (d, *J* = 6.6 Hz, 6H), 1.00 - 0.96 (m, 2H), 0.85 - 0.81 (m, 2H). |
| **130** | 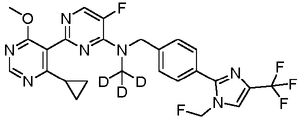 4'-Cyclopropyl-5-fluoro-N-(4-(1-(fluoromethyl)-4-(trifluoromethyl)-1H-imidazol-2-yl)benzyl)-6'-methoxy-N-(methyl-d3)-[2,5'-bipyrimidin]-4-amine | **A2-7 and BB16-D3C59** | LC-MS: m/z =535.1 (M+H)+; |
| | | | ¹H NMR (400 MHz, DMSO-*d*₆) δ 8.58 (s, 1H), 8.35 (d, *J =* 6.7 Hz, 1H), 8.27 (s, 1H), 7.64 (d, *J = 8.0* Hz, 2H), 7.45 (d, *J* = 8.0 Hz, 2H), 6.07 (d, *J =* 51.6 Hz, 2H), 4.87 (s, 2H), 3.80 (s, 3H), 1.77 - 1.67 (m, 1H), 0.99 - 0.93 (m, 2H), 0.85 - 0.77 (m, 2H). |
| **131** | 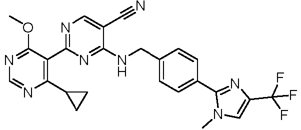 4'-Cyclopropyl-6'-methoxy-4-((4-(1-methyl-4-(trifluoromethyl)-1H-imidazol-2-yl)benzyl)amino)-[2,5'-bipyrimidine]-5-carbonitrile | **A2-7 and BB17C5** | LC-MS: m/z =507.3 (M+H)+ |
| **132** | 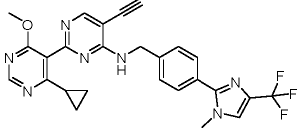 4'-Cyclopropyl-5-ethynyl-6'-methoxy-N-(4-(1-methyl-4-(trifluoromethyl)-1H-imidazol-2-yl)benzyl)-[2,5'-bipyrimidin]-4-amine | **A2-7 and BB18D5** | LC-MS: m/z =506.4 (M+H)+ |
| **133** | 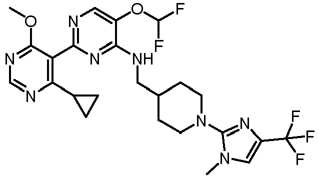 4'-Cyclopropyl-5-(difluoromethoxy)-6'-methoxy-N-((1-(1-methyl-4-(trifluoromethyl)-1H-imidazol-2-yl)piperidin-4-yl)methyl)-[2,5'-bipyrimidin]-4-amine | **A2-7 and BB19C4** | LC-MS: m/z =555.1 (M+H)+; |
| | | | ¹H NMR (400 MHz, CDCl3) δ 8.63 (s, 1H), 8.18 (s, 1H), 7.01 - 7.00 (m, 1H), 6.80 - 6.44 (m, 1H), 5.43 (s, 1H), 3.94 (s, 3H), 3.54 - 3.52 (m, 2H), 3.51 (s, 3H), 3.29 - 3.26 (m, 2H), 2.93 - 2.87 (m, 2H), 1.85 - 1.79 (m, 4H), 1.47 - 1.43 (m, 2H), 1.22 - 1.21 (m, 2H), 0.94 - 0.91 (m, 2H). |
| **134** | 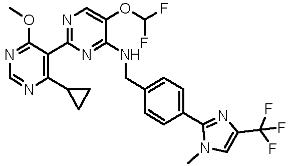 4'-Cyclopropyl-5-(difluoromethoxy)-6'-methoxy-N-(4-(1-methyl-4-(trifluoromethyl)-1H-imidazol-2-yl)benzyl)-[2,5'-bipyrimidin]-4-amine | A2-7 **and BB19C5** | LC-MS: m/z =548.4 (M+H)+ |
| **135** | 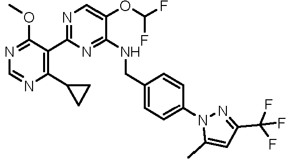 4'-Cyclopropyl-5-(difluoromethoxy)-6'-methoxy-N-(4-(5-methyl-3-(trifluoromethyl)-1H-pyrazol-1-yl)benzyl)-[2,5'-bipyrimidin]-4-amine | A2-7 **and BB19C11** | LC-MS: m/z =548.2 (M+H)+ |
| **136** | 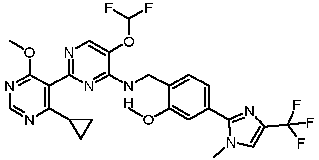 4'-Cyclopropyl-5-(difluoromethoxy)-6'-methoxy-N-(2-methoxy-4-(1-methyl-4-(trifluoromethyl)-1H-imidazol-2-yl)benzyl)-[2,5'-bipyrimidin]-4-amine | **A2-7 and BB19C30** | LC-MS: m/z =578.0 (M+H)+ |
| **137** | 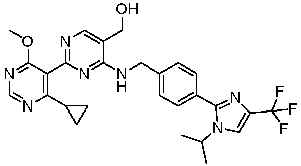 (4'-Cyclopropyl-4-((4-(1-isopropyl-4-(trifluoromethyl)-1H-imidazol-2-yl)benzyl)amino)-6'-methoxy-[2,5'-bipyrimidin]-5-yl)methanol | **A2-7 and BB35D58** | LC-MS: m/z =540.1 (M+H)+; |
| | | | ¹H NMR (400 MHz, CDCl3) δ 8.60 (s, 1H), 7.90 (s, 1H), 7.45 - 7.38 (m, 4H), 7.26 (s, 1H), 6.7 - 6.63 (m, 1H), 4.80 - 4.72 (m, 2H), 4.58 - 4.46 (m, 3H), 4.32 - 4.12 (m, 1H), 3.92 (s, 3H), 1.81 - 1.72 (m, 1H), 1.44 (d, *J = 6.6* Hz, 6H), 1.19 - 1.11 (m, 2H), 0.83 - 0.76 (m, 2H). |
| **138** | 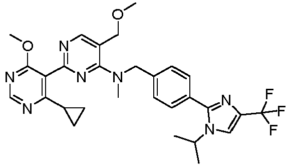 4'-Cyclopropyl-N-(4-(1-isopropyl-4-(trifluoromethyl)-1H-imidazol-2-yl)benzyl)-6'-methoxy-5-(methoxymethyl)-N-methyl-[2,5'-bipyrimidin]-4-amine | **A2-7 and BB36C58** | LC-MS: m/z =568.4 (M+H)⁺; |
| | | | ¹H NMR (400 MHz, DMSO-*d*₆) δ 8.60 (s, 1H), 8.31 (s, 1H), 8.17 (s, 1H), 7.53 (d, *J* = 7.9 Hz, 2H), 7.38 (d, *J =* 7.8 Hz, 2H), 4.95 (s, 2H), 4.49 - 4.43 (m, 1H), 4.42 (s, 2H), 3.82 (s, 3H), 3.31 (s, 3H), 3.27 (s, 3H), 1.76 - 1.68 (m, 1H), 1.40 (d, *J* = 6.6 Hz, 6H), 0.99 - 0.94 (m, 2H), 0.84 - 0.78 (m, 2H). |
| **139** | 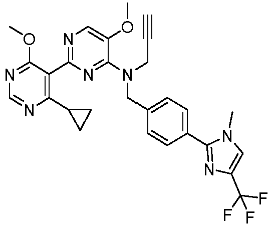 4'-Cyclopropyl-5,6'-dimethoxy-N-(4-(1-methyl-4-(trifluoromethyl)-1H-imidazol-2-yl)benzyl)-N-(prop-2-yn-1-yl)-[2,5'-bipyrimidin]-4-amine | **A2-7 and BB42C5** | LC-MS: m/z =550.2 (M+H)⁺; |
| | | | ¹H NMR (400 MHz, MeOH-*d*₄) δ 8.53 (s, 1H), 8.09 (s, 1H), 7.67 (s, 1H), 7.60 (d, *J* = 8.2 Hz, 2H), 7.50 (d, *J =* 8.2 Hz, 2H), 5.07 (s, 2H), 4.45 (d, *J* = 2.3 Hz, 2H), 3.92 (s, 3H), 3.89 (s, 3H), 3.76 (s, 3H), 2.66 - 2.62 (m, 1H), 1.83 - 1.75 (m, 1H), 1.12 - 1.02 (m, 2H), 0.95 - 0.82 (m, 2H). |
| **140** | 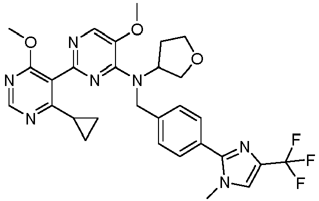 4'-Cyclopropyl-5,6'-dimethoxy-N-(4-(1-methyl-4-(trifluoromethyl)-1H-imidazol-2-yl)benzyl)-N-(tetrahydrofuran-3-yl)-[2,5'-bipyrimidin]-4-amine | **A2-7 and BB43C5** | LC-MS: m/z =582.2 (M+H)+ |
| **141** | 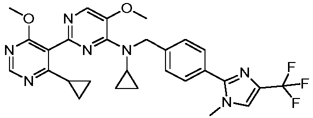 N,4'-Dicyclopropyl-5,6'-dimethoxy-N-(4-(1-methyl-4-(trifluoromethyl)-1H-imidazol-2-yl)benzyl)-[2,5'-bipyrimidin]-4-amine | **A2-7 and BB44C5** | LC-MS: m/z =552.3 (M+H)+ |
| **142** | 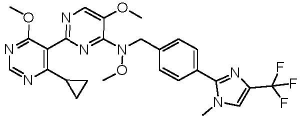 N-(4'-Cyclopropyl-5,6'-dimethoxy-[2,5'-bipyrimidin]-4-yl)-O-methyl-N-(4-(1-methyl-4-(trifluoromethyl)-1H-imidazol-2-yl)benzyl)hydroxylamine | **A2-7 and BB45C5** | LC-MS: m/z =542.2 (M+H)+; |
| | | | 1H NMR (400 MHz, DMSO-d6) δ 8.65 (s, 1H), 8.43 (s, 1H), 7.95 (d, J = 1.4 Hz, 1H), 7.79 - 7.60 (m, 2H), 7.58 - 7.43 (m, 2H), 4.92 (s, 2H), 4.02 (s, 3H), 3.84 (s, 3H), 3.80 (s, 3H), 3.67 (s, 3H), 1.62 (d, J = 4.4 Hz, 1H), 1.09 - 0.95 (m, 2H), 0.85 (dt, J = 8.3, 3.4 Hz, 2H). |
| **143** | 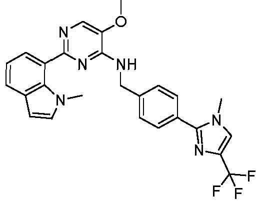 5-Methoxy-2-(1-methyl-1H-indol-7-yl)-N-(4-(1-methyl-4-(trifluoromethyl)-1H-imidazol-2-yl)benzyl)pyrimidin-4-amine | **AA3 and BB2C5** | LC-MS: m/z =493.1 (M+H)+ |
| **144** | 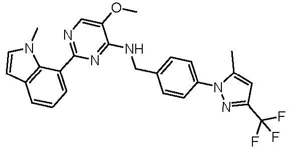 5-Methoxy-2-(1-methyl-1H-indol-7-yl)-N-(4-(5-methyl-3-(trifluoromethyl)-1H-pyrazol-1-yl)benzyl)pyrimidin-4-amine | **AA3 and BB2C11** | LC-MS: m/z =493.1 (M+H)+ |
| **145** | 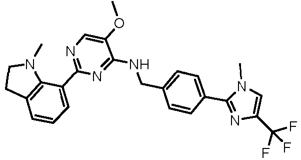 5-Methoxy-N-(4-(1-methyl-4-(trifluoromethyl)-1H-imidazol-2-yl)benzyl)-2-(1-methylindolin-7-yl)pyrimidin-4-amine | **AA4 and BB2C5** | LC-MS: m/z =495.2 (M+H)+ |
| **146** | 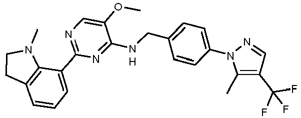 5-Methoxy-N-(4-(5-methyl-4-(trifluoromethyl)-1H-pyrazol-1-yl)benzyl)-2-(1-methylindolin-7-yl)pyrimidin-4-amine | **AA4 and BB2C11** | LC-MS: m/z =495.0 (M+H)+ |
| **147** | 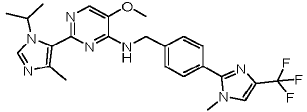 2-(1-Isopropyl-4-methyl-1H-imidazol-5-yl)-5-methoxy-N-(4-(1-methyl-4-(trifluoromethyl)-1H-imidazol-2-yl)benzyl)pyrimidin-4-amine | **AA5 and BB2C5** | LC-MS: m/z =486.3 (M+H)+ |
| **148** | 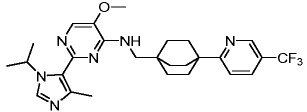 2-(1-Isopropyl-4-methyl-1H-imidazol-5-yl)-5-methoxy-N-((4-(5-(trifluoromethyl)pyridin-2-yl)bicyclo[2.2.2]octan-1-yl)methyl)pyrimidin-4-amine | **AA5 and BB2C19** | LC-MS: m/z =515.5 (M+H)+ |
| **149** | 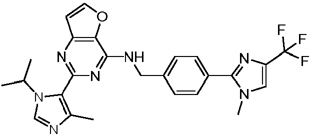 2-(1-Isopropyl-4-methyl-1H-imidazol-5-yl)-N-(4-(1-methyl-4-(trifluoromethyl)-1H-imidazol-2-yl)benzyl)furo[3,2-d]pyrimidin-4-amine | **AA5 and BB13C5** | LC-MS: m/z =496.2 (M+H)+ |
| **150** | 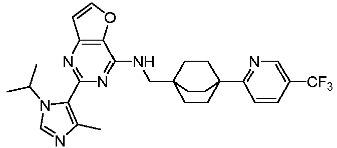 2-(1-Isopropyl-4-methyl-1H-imidazol-5-yl)-N-((4-(5-(trifluoromethyl)pyridin-2-yl)bicyclo[2.2.2]octan-1-yl)methyl)furo[3,2-d]pyrimidin-4-amine | **AA5 and BB13C19** | LC-MS: m/z =525.4 (M+H)+ |
| **151** | 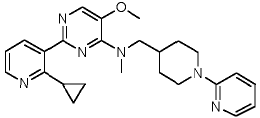 2-(2-Cyclopropylpyridin-3-yl)-5-methoxy-N-methyl-N-((1-(pyridin-2-yl)piperidin-4-yl)methyl)pyrimidin-4-amine | **AA6 and BB1C6** | LC-MS: m/z =431.2 (M+H)+ |
| **152** | 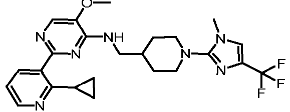 2-(2-Cyclopropylpyridin-3-yl)-5-methoxy-N-((1-(1-methyl-4-(trifluoromethyl)-1H-imidazol-2-yl)piperidin-4-yl)methyl)pyrimidin-4-amine | **AA6 and BB2C4** | LC-MS: m/z =488.1 (M+H)+ |
| **153** | 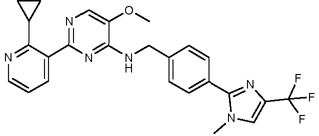 2-(2-Cyclopropylpyridin-3-yl)-5-methoxy-N-(4-(1-methyl-4-(trifluoromethyl)-1H-imidazol-2-yl)benzyl)pyrimidin-4-amine | **AA6 and BB2C5** | LC-MS: m/z =481.3 (M+H)+ |
| **154** | 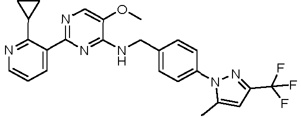 2-(2-Cyclopropylpyridin-3-yl)-5-methoxy-N-(4-(5-methyl-3-(trifluoromethyl)-1H-pyrazol-1-yl)benzyl)pyrimidin-4-amine | **AA6 and BB2C11** | LC-MS: m/z =481.4 (M+H)+ |
| ***155*** | 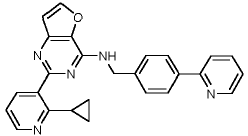 2-(2-Cyclopropylpyridin-3-yl)-N-(4-(pyridin-2-yl)benzyl)furo[3,2-d]pyrimidin-4-amine | **AA6 and BB13C3** | LC-MS: m/z =420.1 (M+H)+ |
| **156** | 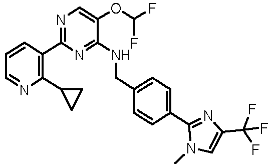 2-(2-Cyclopropylpyridin-3-yl)-5-(difluoromethoxy)-N-(4-(1-methyl-4-(trifluoromethyl)-1H-imidazol-2-yl)benzyl)pyrimidin-4-amine | **AA6 and BB19C5** | LC-MS: m/z =517.3 (M+H)+ |
| **157** | 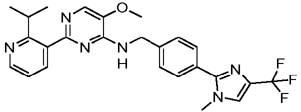 2-(2-Isopropylpyridin-3-yl)-5-methoxy-N-(4-(1-methyl-4-(trifluoromethyl)-1H-imidazol-2-yl)benzyl)pyrimidin-4-amine | **AA7 and BB2C5** | LC-MS: m/z =483.2 (M+H)+ |
| **158** | 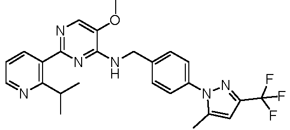 2-(2-Isopropylpyridin-3-yl)-5-methoxy-N-(4-(5-methyl-3-(trifluoromethyl)-1H-pyrazol-1-yl)benzyl)pyrimidin-4-amine | **AA7 and BB2C11** | LC-MS: m/z =483.0 (M+H)+ |
| **159** | 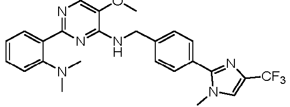 2-(2-(Dimethylamino)phenyl)-5-methoxy-N-(4-(1-methyl-4-(trifluoromethyl)-1H-imidazol-2-yl)benzyl)pyrimidin-4-amine | **AA8 and BB2C5** | LC-MS: m/z =483.4 (M+H)+ |
| **160** | 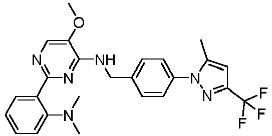 2-(2-(Dimethylamino)phenyl)-5-methoxy-N-(4-(5-methyl-3-(trifluoromethyl)-1H-pyrazol-1-yl)benzyl)pyrimidin-4-amine | **AA8 and BB2C11** | LC-MS: m/z =483.1 (M+H)+ |
| **161** | 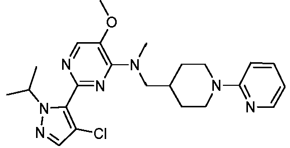 2-(4-Chloro-1-isopropyl-1H-pyrazol-5-yl)-5-methoxy-N-methyl-N-((1-(pyridin-2-yl)piperidin-4-yl)methyl)pyrimidin-4-amine | **AA9 and BB1C6** | LC-MS: m/z =456.2 (M+H)+ |
| **162** | 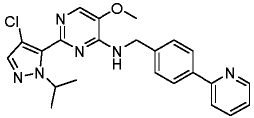 2-(4-Chloro-1-isopropyl-1H-pyrazol-5-yl)-5-methoxy-N-(4-(pyridin-2-yl)benzyl)pyrimidin-4-amine | **AA9 and BB2C3** | LC-MS: m/z =435.1 (M+H)+ |
| **163** | 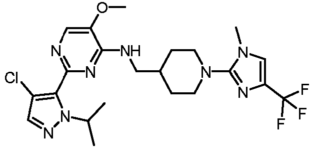 2-(4-Chloro-1-isopropyl-1H-pyrazol-5-yl)-5-methoxy-N-((1-(1-methyl-4-(trifluoromethyl)-1H-imidazol-2-yl)piperidin-4-yl)methyl)pyrimidin-4-amine | **AA9 and BB2C4** | LC-MS: m/z =513.3 (M+H)+ |
| **164** | 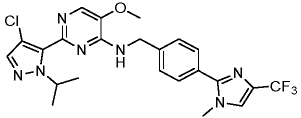 2-(4-Chloro-1-isopropyl-1H-pyrazol-5-yl)-5-methoxy-N-(4-(1-methyl-4-(trifluoromethyl)-1H-imidazol-2-yl)benzyl)pyrimidin-4-amine | **AA9 and BB2C5** | LC-MS: m/z =506.3 (M+H)+ |
| **165** | 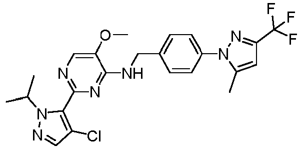 2-(4-Chloro-1-isopropyl-1H-pyrazol-5-yl)-5-methoxy-N-(4-(5-methyl-3-(trifluoromethyl)-1H-pyrazol-1-yl)benzyl)pyrimidin-4-amine | **AA9 and BB2C11** | LC-MS: m/z =506.1 (M+H)+ |
| **166** | 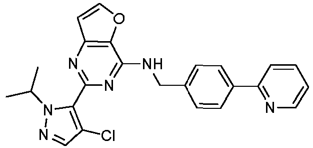 2-(4-Chloro-1-isopropyl-1H-pyrazol-5-yl)-N-(4-(pyridin-2-yl)benzyl)furo[3,2-d]pyrimidin-4-amine | **AA9 and BB13C3** | LC-MS: m/z =445.1 (M+H)+ |
| **167** | 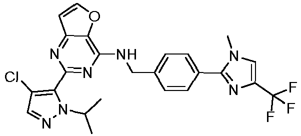 2-(4-Chloro-1-isopropyl-1H-pyrazol-5-yl)-N-(4-(1-methyl-4-(trifluoromethyl)-1H-imidazol-2-yl)benzyl)furo[3,2-d]pyrimidin-4-amine | **AA9 and BB13C5** | LC-MS: m/z =516.1 (M+H)+; |
| | | | ¹H NMR (400 MHz, DMSO) δ 8.81 (s, 1H), 8.36 (d, *J= 2.0* Hz, 1H), 7.89-7.93 (m, 1H), 7.64-7.70 (m, 2H), 7.59 (s, 1H), 7.44-7.50 (m, 2H), 7.11 (d, *J* = 2.4 Hz, 1H), 5.01-5.12 (m, 1H), 4.80-4.86 (m, 2H), 3.75 (s, 3H), 1.23 (d, *J* = 6.8 Hz, 6H). |
| **168** | 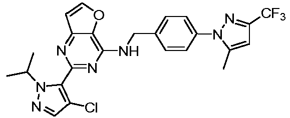 2-(4-Chloro-1-isopropyl-1H-pyrazol-5-yl)-N-(4-(5-methyl-3-(trifluoromethyl)-1H-pyrazol-1-yl)benzyl)furo[3,2-d]pyrimidin-4-amine | **AA9 and BB13C11** | LC-MS: m/z =516.2 (M+H)+ |
| **169** | 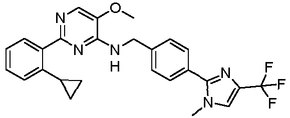 2-(2-Cyclopropylphenyl)-5-methoxy-N-(4-(1-methyl-4-(trifluoromethyl)-1H-imidazol-2-yl)benzyl)pyrimidin-4-amine | **AA10 and BB2C5** | LC-MS: m/z =480.4 (M+H)+ |
| **170** | 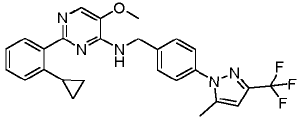 2-(2-Cyclopropylphenyl)-5-methoxy-N-(4-(5-methyl-3-(trifluoromethyl)-1H-pyrazol-1-yl)benzyl)pyrimidin-4-amine | **AA10 and BB2C11** | LC-MS: m/z =480.2 (M+H)+; |
| | | | ¹H NMR (400 MHz, CDCl3) δ 7.88 (s, 1H), 7.60 - 7.62 (m, 1H), 7.47 - 7.49 (m, 2H), 7.40 - 7.42 (m, 2H), 7.21 - 7.23 (m, 1H), 6.97 - 6.99 (m, 1H), 6.49 (s, 1H), 5.66 - 5.75 (m, 1H), 4.84 (d, *J =* 6.0Hz, 2H), 3.99 (s, 3H), 2.46 - 2.53 (m, 1H), 2.35 (s, 3H), 0.70 - 0.73 (m, 2H), 0.59 - 0.61 (m, 2H). |
| **171** | 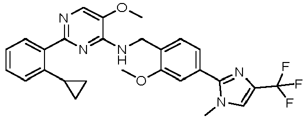 2-(2-Cyclopropylphenyl)-5-methoxy-N-(2-methoxy-4-(1-methyl-4-(trifluoromethyl)-1H-imidazol-2-yl)benzyl)pyrimidin-4-amine | **AA10 and BB2C30** | LC-MS: m/z =510.2 (M+H)+ |
| **172** | 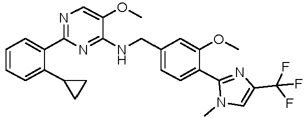 2-(2-Cyclopropylphenyl)-5-methoxy-N-(3-methoxy-4-(1-methyl-4-(trifluoromethyl)-1H-imidazol-2-yl)benzyl)pyrimidin-4-amine | **AA10 and BB2C31** | LC-MS: m/z =510.3 (M+H)+ |
| **173** | 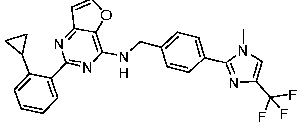 2-(2-Cyclopropylphenyl)-N-(4-(1-methyl-4-(trifluoromethyl)-1H-imidazol-2-yl)benzyl)furo[3,2-d]pyrimidin-4-amine | **AA10 and BB13C5** | LC-MS: m/z =490.4 (M+H)+ |
| **174** | 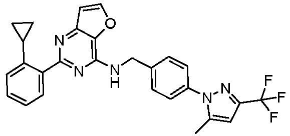 2-(2-Cyclopropylphenyl)-N-(4-(5-methyl-3-(trifluoromethyl)-1H-pyrazol-1-yl)benzyl)furo[3,2-d]pyrimidin-4-amine | **AA10 and BB13C11** | LC-MS: m/z =490.2 (M+H)+ |
| **175** | 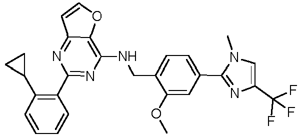 2-(2-Cyclopropylphenyl)-N-(2-methoxy-4-(1-methyl-4-(trifluoromethyl)-1H-imidazol-2-yl)benzyl)furo[3,2-d]pyrimidin-4-amine | **AA10 and BB13C30** | LC-MS: m/z =520.0 (M+H)+ |
| **176** | 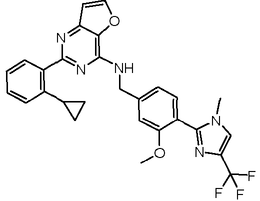 2-(2-Cyclopropylphenyl)-N-(3-methoxy-4-(1-methyl-4-(trifluoromethyl)-1H-imidazol-2-yl)benzyl)furo[3,2-d]pyrimidin-4-amine | **AA10 and BB13C31** | LC-MS: m/z =520.2 (M+H)+ |
| **177** | 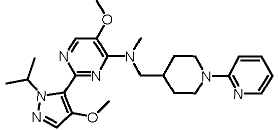 2-(1-Isopropyl-4-methoxy-1H-pyrazol-5-yl)-5-methoxy-N-methyl-N-((1-(pyridin-2-yl)piperidin-4-yl)methyl)pyrimidin-4-amine | **AA11 and BB1C6** | LC-MS: m/z =452.2 (M+H)+ |
| **178** | 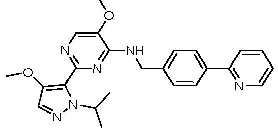 2-(1-Isopropyl-4-methoxy-1H-pyrazol-5-yl)-5-methoxy-N-(4-(pyridin-2-yl)benzyl)pyrimidin-4-amine | **AA11 and BB2C3** | LC-MS: m/z =431.4 (M+H)+ |
| **179** | 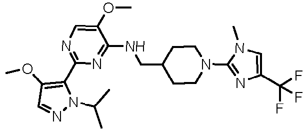 2-(1-Isopropyl-4-methoxy-1H-pyrazol-5-yl)-5-methoxy-N-((1-(1-methyl-4-(trifluoromethyl)-1H-imidazol-2-yl)piperidin-4-yl)methyl)pyrimidin-4-amine | **AA11 and BB2C4** | LC-MS: m/z =509.1 (M+H)+ |
| **180** | 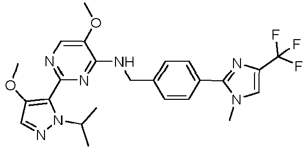 2-(1-Isopropyl-4-methoxy-1H-pyrazol-5-yl)-5-methoxy-N-(4-(1-methyl-4-(trifluoromethyl)-1H-imidazol-2-yl)benzyl)pyrimidin-4-amine | **AA11 and BB2C5** | LC-MS: m/z =502.1 (M+H)+ |
| **181** | 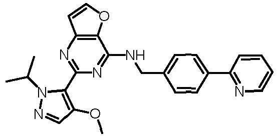 2-(1-Isopropyl-4-methoxy-1H-pyrazol-5-yl)-N-(4-(pyridin-2-yl)benzyl)furo[3,2-d]pyrimidin-4-amine | **AA11 and BB13C3** | LC-MS: m/z =441.4 (M+H)+ |
| **182** | 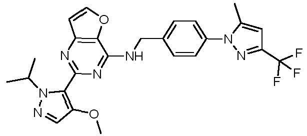 2-(1-Isopropyl-4-methoxy-1H-pyrazol-5-yl)-N-(4-(5-methyl-3-(trifluoromethyl)-1H-pyrazol-1-yl)benzyl)furo[3,2-d]pyrimidin-4-amine | **AA11 and BB13C11** | LC-MS: m/z =512.3 (M+H)+ |
| **183** | 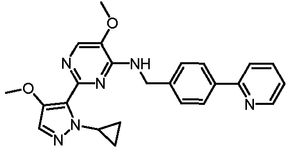 2-(1-Cyclopropyl-4-methoxy-1H-pyrazol-5-yl)-5-methoxy-N-(4-(pyridin-2-yl)benzyl)pyrimidin-4-amine | **AA12 and BB2C3** | LC-MS: m/z =429.2 (M+H)+ |
| **184** | 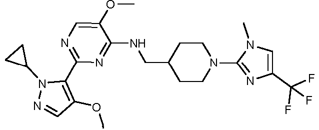 2-(1-Cyclopropyl-4-methoxy-1H-pyrazol-5-yl)-5-methoxy-N-((1-(1-methyl-4-(trifluoromethyl)-1H-imidazol-2-yl)piperidin-4-yl)methyl)pyrimidin-4-amine | **AA12 and BB2C4** | LC-MS: m/z =507.3 (M+H)+ |
| **185** | 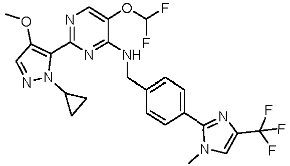 2-(1-Cyclopropyl-4-methoxy-1H-pyrazol-5-yl)-5-(difluoromethoxy)-N-(4-(1-methyl-4-(trifluoromethyl)-1H-imidazol-2-yl)benzyl)pyrimidin-4-amine | **AA12 and BB19C5** | LC-MS: m/z =536.3 (M+H)+ |
| **186** | 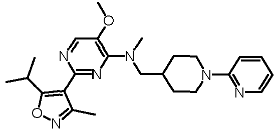 2-(5-Isopropyl-3-methylisoxazol-4-yl)-5-methoxy-N-methyl-N-((1-(pyridin-2-yl)piperidin-4-yl)methyl)pyrimidin-4-amine | **AA13 and BB1C6** | LC-MS: m/z =437.2 (M+H)+ |
| **187** | 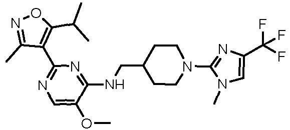 2-(5-Isopropyl-3-methylisoxazol-4-yl)-5-methoxy-N-((1-(1-methyl-4-(trifluoromethyl)-1H-imidazol-2-yl)piperidin-4-yl)methyl)pyrimidin-4-amine | **AA13 and BB2C4** | LC-MS: m/z =494.1 (M+H)+; |
| | | | ¹H NMR (400 MHz, CDCl3) δ 7.79 (s, 1H), 7.01 (s, 1H), 5.41 - 5.38 (m, 1H), 4.08 - 4.01 (m, 1H), 3.92 (s, 3H), 3.51 (s, 3H), 3.50 - 3.47 (m, 2H), 3.31 - 3.28 (m, 2H), 2.95 - 2.88 (m, 2H), 2.56 (s, 3H), 1.89 (s, 1H), 1.85 - 1.80 (m, 2H), 1.50 - 1.40 (m, 2H), 1.37 (s, 3H), 1.36 (s, 3H). |
| **188** | 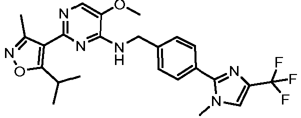 2-(5-Isopropyl-3-methylisoxazol-4-yl)-5-methoxy-N-(4-(1-methyl-4-(trifluoromethyl)-1H-imidazol-2-yl)benzyl)pyrimidin-4-amine | **AA13 and BB2C5** | LC-MS: m/z =487.0 (M+H)+ |
| **189** | 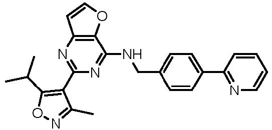 2-(5-Isopropyl-3-methylisoxazol-4-yl)-N-(4-(pyridin-2-yl)benzyl)furo[3,2-d]pyrimidin-4-amine | **AA13 and BB13C3** | LC-MS: m/z =426.1 (M+H)+ |
| **190** | 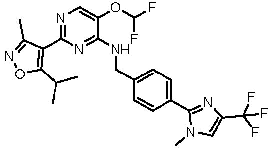 5-(Difluoromethoxy)-2-(5-isopropyl-3-methylisoxazol-4-yl)-N-(4-(1-methyl-4-(trifluoromethyl)-1H-imidazol-2-yl)benzyl)pyrimidin-4-amine | **AA13 and BB19C5** | LC-MS: m/z =523.5 (M+H)+ |
| **191** | 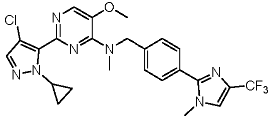 2-(4-Chloro-1-cyclopropyl-1H-pyrazol-5-yl)-5-methoxy-N-methyl-N-(4-(1-methyl-4-(trifluoromethyl)-1H-imidazol-2-yl)benzyl)pyrimidin-4-amine | **AA14 and BB1C5** | LC-MS: m/z =518.3 (M+H)+ |
| **192** | 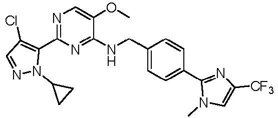 2-(4-Chloro-1-cyclopropyl-1H-pyrazol-5-yl)-5-methoxy-N-(4-(1-methyl-4-(trifluoromethyl)-1H-imidazol-2-yl)benzyl)pyrimidin-4-amine | **AA14 and BB2C5** | LC-MS: m/z =504.2 (M+H)+; |
| | | | ¹H NMR (400 MHz, DMSO-*d*₆) δ 8.06 (d, *J* = 4.9 Hz, 2H), 8.00 - 7.86 (m, 1H), 7.76 - 7.59 (m, 2H), 7.50 (s, 1H), 7.44 (d, *J* = 8.0 Hz, 2H), 4.71 (d, *J =* 6.2 Hz, 2H), 4.00 (s, 3H), 3.95 (tt, *J= 7.5*, 3.8 Hz, 1H), 3.77 (s, 3H), 0.97 - 0.77 (m, 2H), 0.71 - 0.50 (m, 2H). |
| **193** | 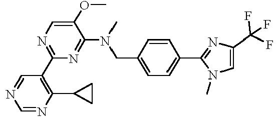 4'-Cyclopropyl-5-methoxy-N-methyl-N-(4-(1-methyl-4-(trifluoromethyl)-1H-imidazol-2-yl)benzyl)-[2,5'-bipyrimidin]-4-amine | **AA15 and BB1C5** | LC-MS: m/z =496.5 (M+H)+ |
| **194** | 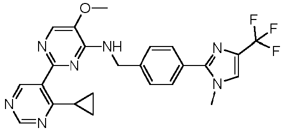 4'-Cyclopropyl-5-methoxy-N-(4-(1-methyl-4-(trifluoromethyl)-1H-imidazol-2-yl)benzyl)-[2,5'-bipyrimidin]-4-amine | | LC-MS: m/z =482.1 (M+H)+; |
| | | **AA15 and BB2C5** | ¹H NMR (400 MHz, DMSO-*d*₆) δ 8.93 (s, 1H), 8.88 (s, 1H), 8.08 (s, 1H), 8.01 (d, *J = 6.1* Hz, 1H), 7.93 (s, 1H), 7.68 (d, *J* = 8.0 Hz, 2H), 7.45 (d, *J* = 8.0 Hz, 2H), 4.73 (d, *J =* 6.3 Hz, 2H), 4.00 (s, 3H), 3.79 (s, 3H), 3.06 - 2.99 (m, 1H), 1.05 (s, 2H), 0.86 - 0.80 (m, 2H). |
| **195** | 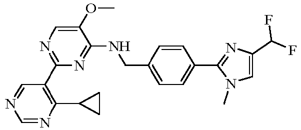 4'-Cyclopropyl-N-(4-(4-(difluoromethyl)-1-methyl-1H-imidazol-2-yl)benzyl)-5-methoxy-[2,5'-bipyrimidin]-4-amine | **AA15 and BB2C48** | LC-MS: m/z =464.4 (M+H)+; |
| | | | ¹H NMR (400 MHz, DMSO-*d*₆) δ 8.92 (s, 1H), 8.86 (s, 1H), 8.07 (s, 1H), 7.99 (t, *J =* 6.3 Hz, 1H), 7.69 - 7.59 (m, 3H), 7.42 (d, *J =* 8.1 Hz, 2H), 6.78 - 7.05 (m, 1H), 4.71 (d, *J =* 6.2 Hz, 2H), 3.98 (s, 3H), 3.75 (s, 3H), 3.00 (dt, *J =* 8.1, 3.9 Hz, 1H), 1.02 (d, *J =* 4.4 Hz, 2H), 0.79 (dt, *J =* 7.0, 3.4 Hz, 2H). |
| **196** | 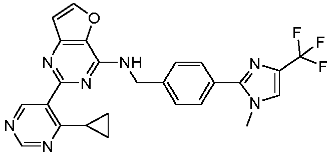 2-(4-Cyclopropylpyrimidin-5-yl)-N-(4-(1-methyl-4-(trifluoromethyl)-1H-imidazol-2-yl)benzyl)furo[3,2-d]pyrimidin-4-amine | **AA15 and BB13C5** | LC-MS: m/z =492.0 (M+H)+; |
| | | | ¹H NMR (400 MHz, DMSO-*d*₆) δ 8.94 (m, 2H), 8.81 (s, 1H), 8.38 (d, *J =* 2.2 Hz, 1H), 7.93 (d, *J =* 1.4 Hz, 1H), 7.70 (d, *J = 8.1* Hz, 2H), 7.51 (d, *J = 8.1* Hz, 2H), 7.14 (d, *J = 2.2* Hz, 1H), 4.86 (d, *J = 6.2* Hz, 2H), 3.79 (s, 2H), 3.07 - 2.97 (m, 1H), 1.32 - 1.25 (m, 1H), 1.11 - 1.04 (m, 2H), 0.86 (s, 2H). |
| **197** | 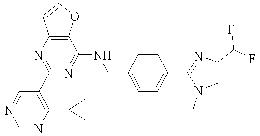 2-(4-Cyclopropylpyrimidin-5-yl)-N-(4-(4-(difluoromethyl)-1-methyl-1H-imidazol-2-yl)benzyl)furo[3,2-d]pyrimidin-4-amine | **AA15 and BB13C48** | LC-MS: m/z =474.1 (M+H)+; |
| | | | ¹H NMR (400 MHz, DMSO-*d*₆) δ 8.96 (s, 1H), 8.90 (s, 1H), 8.80 (s, 1H), 8.37 (d, *J =* 2.2 Hz, 1H), 7.65 (d, *J =* 8.2 Hz, 2H), 7.50 (d, *J =* 8.0 Hz, 2H), 7.39 - 7.3 5 (m, 1H), 7.28 - 7.12 (m, 1H), 6.75 - 7.03 (m, 1H), 4.85 (d, *J =* 6.2 Hz, 2H), 3.74 (s, 3H), 2.96 (dq, *J =* 8.6, 5.1, 4.5 Hz, 1H), 1.05 (m, 2H), 0.83 (m, 2H). |
| **198** | 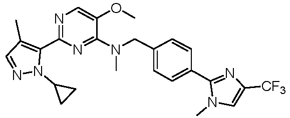 2-(1-Cyclopropyl-4-methyl-1H-pyrazol-5-yl)-5-methoxy-N-methyl-N-(4-(1-methyl-4-(trifluoromethyl)-1H-imidazol-2-yl)benzyl)pyrimidin-4-amine | **AA16 and BB1C5** | LC-MS: m/z =498.2 (M+H)+; |
| | | | ¹H NMR (400 MHz, DMSO-*d*₆) δ 8.21 (s, 1H), 7.95 (d, *J* = 1.5 Hz, 1H), 7.72 (d, *J =* 8.2 Hz, 2H), 7.41 (d, *J* = 8.1 Hz, 2H), 7.23 (s, 1H), 5.02 (s, 2H), 4.31 (dt, *J =* 7.4, 3.6 Hz, 1H), 3.91 (s, 3H), 3.80 (s, 3H), 3.24 (s, 3H), 2.15 (s, 3H), 1.06 - 0.87 (m, 2H), 0.87 - 0.62 (m, 2H). |
| **199** | 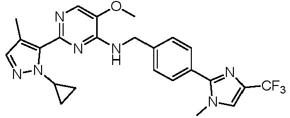 2-(1-Cyclopropyl-4-methyl-1H-pyrazol-5-yl)-5-methoxy-N-(4-(1-methyl-4-(trifluoromethyl)-1H-imidazol-2-yl)benzyl)pyrimidin-4-amine | **AA16 and BB2C5** | LC-MS: m/z =484.4 (M+H)+ |
| **200** | 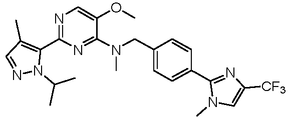 2-(1-Isopropyl-4-methyl-1H-pyrazol-5-yl)-5-methoxy-N-methyl-N-(4-(1-methyl-4-(trifluoromethyl)-1H-imidazol-2-yl)benzyl)pyrimidin-4-amine | **AA17 and BB1C5** | LC-MS: m/z =500.5 (M+H)+ |
| **201** | 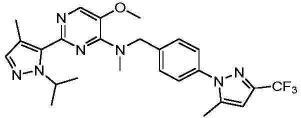 2-(1-Isopropyl-4-methyl-1H-pyrazol-5-yl)-5-methoxy-N-methyl-N-(4-(5-methyl-3-(trifluoromethyl)-1H-pyrazol-1-yl)benzyl)pyrimidin-4-amine | **AA17 and BB1C11** | LC-MS: m/z =500.1 (M+H)+ |
| **202** | 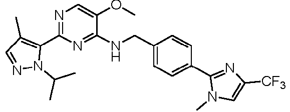 2-(1-Isopropyl-4-methyl-1H-pyrazol-5-yl)-5-methoxy-N-(4-(1-methyl-4-(trifluoromethyl)-1H-imidazol-2-yl)benzyl)pyrimidin-4-amine | **AA17 and BB2C5** | LC-MS: m/z =486.3 (M+H)+ |
| **203** | 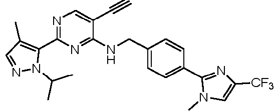 5-Ethynyl-2-(1-isopropyl-4-methyl-1H-pyrazol-5-yl)-N-(4-(1-methyl-4-(trifluoromethyl)-1H-imidazol-2-yl)benzyl)pyrimidin-4-amine | **AA17 and BB18D5** | LC-MS: m/z =480.2 (M+H)+ |

### Example 204: Synthesis of compound 204 (N-(4'-cyclopropyl-5,6'-dimethoxy-[2,5'-bipyrimidin]-4-yl)-N-(4-(1-methyl-4-(trifluoromethyl)-1H-imidazol-2-yl)benzyl)hydroxylamine)

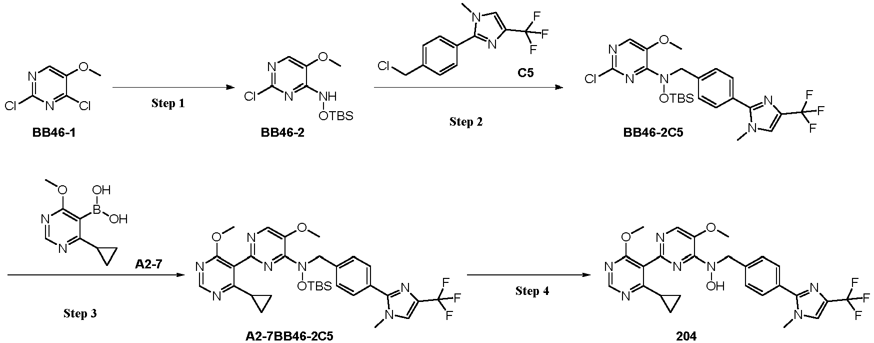

### Step 1: Synthesis of compound BB46-2 (O-(tert-butyldimethylsilyl)-N-(2-chloro-5-methoxypyrimidin-4-yl)hydroxylamine)

Compound 2,4-dichloro-5-fluoropyrimidine (compound **BB46-1,** 1.0 g, 5.6 mmol, 1.0 eq), O-(*tert*-butyldimethylsilyl)hydroxylamine (0.9 g, 6.2 mmol, 1.1 eq) and N,N-diisopropylethylamine (2.1 g, 16.8 mmol, 3.0 eq) were added to dioxane (20 mL) successively, and the resulting mixture was stirred at 80°C for 4 hours. The reaction solution was cooled to room temperature, and filtered to obtain a filtrate. The filtrate was concentrated under vacuum to obtain a crude product, which was purified by column chromatography (eluent: petroleum ether/ethyl acetate = 1/0 - 1/1) to obtain compound **BB46-2** (430.2 mg, 1.49 mmol, 26.6% yield) as a light yellow oil. LC-MS: m/z = 290.0 (M+H)⁺.

### Step 2: Synthesis of compound BB46-2C5 (O-(tert-butyldimethylsilyl)-N-(2-chloro-5-methoxypyrimidin-4-yl)-N-(4-(1-methyl-4-(trifluoromethyl)-1H-imidazol-2-yl)benzyl)hydroxylamine)

In accordance with the same steps of intermediate **BB1C2,** intermediate **BB46-2C5** (506.8 mg, 0.96 mmol, 64.1% yield, a light yellow solid) was obtained using intermediate **BB46-2** (430 mg,1.5 mmol,1.0 *eq)* and compound **C5** (452 mg, 1.65 mmol, 1.1 *eq)* as starting materials. LC-MS: m/z = 528.3 (M+H)⁺.

### Step 3: Synthesis of compound A2-7BB46-2C5 (O-(tert-butyldimethylsilyl)-N-(4'-cyclopropyl-5,6'-dimethoxy-[2,5'-bipyrimidin]-4-yl)-N-(4-(1-methyl-4-(trifluoromethyl)-1H-imidazol-2-yl)benzyl)hydroxylamine)

In accordance with the same steps of compound **A1-2BB1C2,** intermediate **A2-7BB46-2C5** (323.3 mg, 0.50 mmol, 56.0% yield, a yellow solid) was obtained using intermediate **BB46-2** (486.0 mg, 0.9 mmol,1.0 *eq*) and compound **A2-7** (262.1 mg, 1.35 mmo, 1.5 *eq*) as starting materials. LC-MS: m/z = 642.3 (M+H)⁺.

### Step 4: Synthesis of compound 204

Intermediate **A2-7BB46-2C5** (323.0 mg, 0.5 mmol, 1.0 *eq*) was dissolved in a 4 M solution of hydrochloric acid in methanol, and the mixture wa stirred at 25°C for 1 hour. Saturated aqueous sodium bicarbonate solution (30.0 mL) was added, and the resulting mixture was extracted three times with dichloromethane (20.0 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/2) to obtain compound **204** (109.9 mg, 0.21 mmol, 41.7% yield) as a white solid. ¹H NMR (400MHz, DMSO-*d*₆) δ 9.51 (s, 1H), 8.62 (s, 1H), 8.28 (s, 1H), 7.92 (d, *J =* 1.4 Hz, 1H), 7.75 - 7.57 (m, 2H), 7.56 - 7.41 (m, 2H), 4.87 (s, 2H), 3.94 (s, 3H), 3.82 (s, 3H), 3.77 (s, 3H), 1.62 (dt, *J =* 8.1, 3.5 Hz, 1H), 0.99 (dq, *J =* 5.9, 3.5 Hz, 2H), 0.84 (dq, *J =* 10.0, 3.4 Hz, 2H); LC-MS: m/z = 528.3 (M+H)⁺.

### Example 205: Synthesis of compound 205 (N-(2-(1-cyclopropyl-4-methyl-1H-pyrazol-5-yl)-5-methoxypyrimidin-4-yl)-N-(4-(1-methyl-4-(trifluoromethyl)-1H-imidazol-2-yl)benzyl)hydroxylamine)

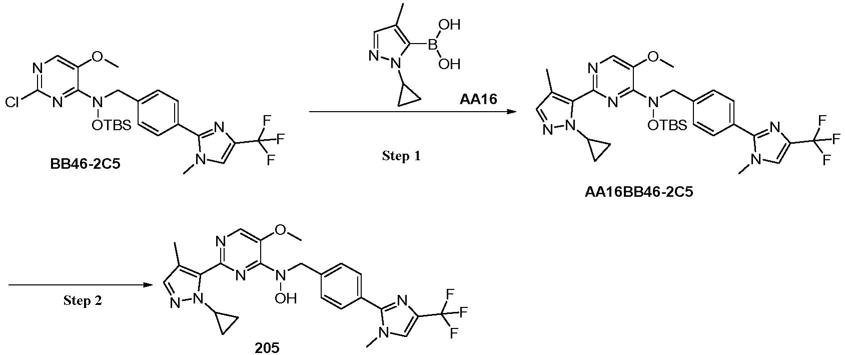

In accordance with the same steps of compound **204,** compound **205** (37.2 mg, 0.074 mmol, 66.9% yield, a white solid) was obtained using intermediate **BB46-2** and compound **AA16** as starting materials. LC-MS: m/z = 500.3 (M+H)⁺.

### Example 206: Synthesis of compound 206 (N-(4'-cyclopropyl-5,6'-dimethoxy-[2,5'-bipyrimidin]-4-yl)-N-(4-(1-methyl-4-(trifluoromethyl)-1H-imidazol-2-yl)benzyl)cyanamide)

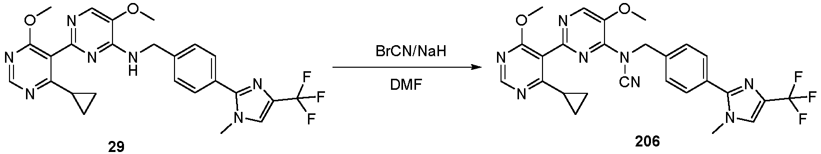

Compound **29** (50 mg, 0.092 mmol, 1.0 eq) was dissolved in N,N-dimethylformamide (1.0 mL), and sodium hydride (60% content, 36.8 mg, 0.92 mmol, 10 eq) was added in portions at 0°C. The reaction solution was naturally heated up to 25°C, and stirred for one hour. Cyanogen bromide (48.7 mg, 0.46 mmol, 5 eq) was added, and the resulting mixture was stirred at room temperature of 25°C for 2 hours. The reaction solution was concentrated under vacuum to obtain a crude product, which was purified by column chromatography (eluent: petroleum ether/ethyl acetate = 1/0 - 0/1) to obtain compound **206** (6.7 mg, 0.0125 mmol, 13.6% yield) as a pale white solid. ¹H NMR (400MHz, DMSO-*d*₆) δ ¹H NMR (400 MHz, DMSO-d6) δ 8.58 (s, 1H), 7.96 (s, 1H), 7.92 (s, 1H), 7.60 (d, *J =* 7.8 Hz, 2H), 7.40 (d, *J =* 7.8 Hz, 2H), 5.39 (d, *J =* 6.3 Hz, 2H), 3.93 (s, 3H), 3.81 (s, 3H), 3.70 (s, 3H), 1.68 (tt, J = 8.2, 4.8 Hz, 1H), 0.96 (s, 2H), 0.78-0.73 (m, 2H); LC-MS: m/z = 537.2 (M+H)⁺.

### Example 207: Synthesis of compound 207 (2-(4-cyclopropyl-6-methoxy-pyrimidin-5-yl)-5-methoxy-N-methyl-N-[[4-[1-methyl-4-(trifluoromethyl)imidazol-2-yl]cuban-1-yl]methyl]pyrimidin-4-amine)

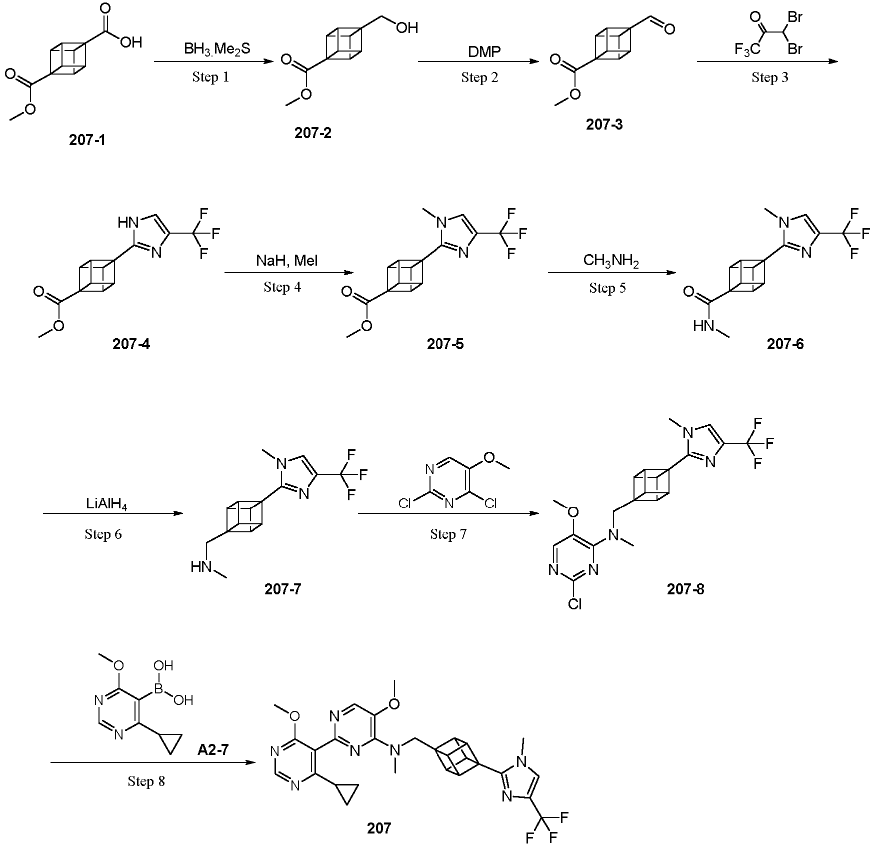

### Step 1: Synthesis of compound 207-2 (methyl (2R,3R,4S,5S)-4-(hydroxymethyl)cubane-1-carboxylate)

Compound (1S,2R,3R,8S)-4-(methoxycarbonyl)cubane-1-carboxylic acid (**207-1**, 10.0 g, 48.5 mmol, 1.00 eq) was dissolved in tetrahydrofuran (300 mL). A 10 M solution of borane dimethyl sulfide in tetrahydrofuran (5.82 mL, 1.20 eq) was added at 20°C, and the resulting mixture was stirred at 50°C for 13 hours. Methanol (300 mL) was slowly added at 0°C to quench the reaction, and the resulting mixture was filtered to obtain a filtrate, which was concentrated under vacuum to obtain compound **207-2** (11.0 g, crude) as a white solid. ¹H NMR (400MHz, CDCl3) δ 4.15 - 4.13 (m, 3H), 3.89 - 3.87 (m, 3H), 3.77 - 3.75 (m, 2H), 3.70 (s, 3H).

### Step 2: Synthesis of compound 207-3 (methyl (2R,3R,4S,5S)-4-formylcubane-1-carboxylate)

Compound **207-2** (11.0 g, 57.2 mmol, 1.00 eq) was dissolved in dichloromethane (150 mL), followed by the addition of Dess-Martin periodinane (DMP, 29.1 g, 68.6 mmol, 21.2 mL, 1.20 eq) at 0°C, and the resulting mixture was stirred at 0°C for 2 hours. Saturated aqueous sodium bicarbonate solution (100 mL) was added, and the resulting mixture was extracted three times with dichloromethane (150 mL). The organic layers were combined, washed twice with saturated brine (120 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 3/1) to obtain compound **207-3** (5.8 g, 30.5 mmol, 53.3% yield) as a white solid. ¹H NMR (400MHz, CDCl3) δ 9.75 (s, 1H), 4.38 - 4.36 (m, 3H), 4.27 - 4.25 (m, 3H), 3.72 (s, 3H).

### Step 3: Synthesis of compound 207-4 (methyl (2R,3R,4S,5S)-4-(4-(trifluoromethyl)-1H-imidazol-2-yl)cubane-1-carboxylate)

Sodium acetate (5.25 g, 64.0 mmol, 2.1 eq) was dissolved in water (25.0 mL), and 1,1-dibromo-3,3,3-trifluoroacetone (9.05 g, 33.5 mmol, 1.1 eq) was added at 20°C. The reaction solution was heated up to 100°C, and stirred for 1 hour. Compound **207-3** (5.8 g, 30.5 mmol, 1.0 eq) dissolved in methanol (65.0 mL) and ammonia water (25.0 mL) was added at 20°C. After the addition was completed, the resulting mixture was stirred for 11 hours at 20°C. The reaction solution was filtered to obtain a filtrate, which was concentrated under vacuum to obtain compound **207-4** (6.0 g, 18.5 mmol, 60.8% yield) as a white solid. LC-MS: m/z = 297.0 (M+H)⁺.

### Step 4: Synthesis of compound 207-5 (methyl (2R,3R,4S,5S)-4-(1-methyl-4-(trifluoromethyl)-1H-imidazol-2-yl)cubane-1-carboxylate)

Compound **207-4** (4.5 g, 15.2 mmol, 1.0 eq) was dissolved in tetrahydrofuran (40.0 mL), and sodium hydride (911 mg, 22.7 mmol, 60% content, 1.50 eq) was added in portions at 0°C. After the addition was completed, the resulting mixture was stirred at 20°C for 30 minutes. Iodomethane (2.16 g, 15.2 mmol, 945 µL, 1.0 eq) was added, and the resulting mixture was reacted at 20°C for 2 hours. Saturated aqueous ammonium chloride solution (50.0 mL) was added at 0°C to quench the reaction. Water (100 mL) was added, and the resulting mixture was extracted three times with ethyl acetate (70.0 mL). The organic layers were combined, washed twice with saturated brine (80.0 mL), dried over anhydrous sodium sulfate, and filtered to obtain a filtrate. The filtrate was concentrated under vacuum to obtain a crude product, which was purified by reverse phase chromatography HPLC (0.1% FA) to obtain compound **207-5** (1.6 g, 4.95 mmol, 32.5% yield) as a white solid. ¹H NMR (400MHz, CD₃OD) δ 7.54 (d, *J =* 1.2 Hz, 1H), 4.45 - 4.42 (m, 3H), 4.32 - 4.30 (m, 3H), 3.73 (s, 3H), 3.66 (s, 3H); LC-MS: m/z = 311.1 (M+H)⁺.

### Step 5: Synthesis of compound 207-6 ((2R,3R,4S,5S)-N-methyl-4-(1-methyl-4-(trifluoromethyl)-1H-imidazol-2-yl)cubane-1-carboxamide)

Compound **207-5** (120 mg, 386.76 mmol, 1 eq) was dissolved in ethanol (24.0 mL), and a solution of methylamine in ethanol (16.51 g, 159.48 mmol, 30% content, 412.35 eq) was added at 25°C. The resulting mixture was stirred at 70°C under 50 Psi pressure for 12 hours. The reaction solution was cooled to room temperature, and ice water (15.0 mL) was added. The resulting mixture was concentrated under vacuum to remove ethanol, and extracted three times with ethyl acetate (30.0 mL). The organic layers were combined, dried over anhydrous sodium sulfate, and filtered to obtain a filtrate, which was concentrated under vacuum to obtain compound **207-6** (0.11 g, crude) as a white solid. LC-MS: m/z = 310.1 (M+H)⁺.

### Step 6: Synthesis of compound 207-7 (N-methyl-1-((2R,3R,4S,5S)-4-(1-methyl-4-(trifluoromethyl)-1H-imidazol-2-yl)cuban-1-yl)methylamine)

Compound **207-6** (0.1 g, 323.33 mmol, 1 *eq*) was dissolved in tetrahydrofuran (10.0 mL), and a solution of lithium aluminum hydride in tetrahydrofuran (2.5 mmol, 387.99 µL, 3 *eq)* was added at 0°C. The resulting mixture was stirred at 0°C for 30 minutes, then heated up to 50°C and stirred for 3 hours. Sodium sulfate decahydrate (1.0 g) was added at 0°C, and the resulting mixture was stirred for 30 minutes, followed by the addition of tetrahydrofuran (5.0 mL), and stirred for 5 minutes. The resulting mixture was filtered to obtain a filtrate, which was concentrated under vacuum to obtain compound **207-7** (86 mg, crude) as a white solid. LC-MS: m/z = 296.3 (M+H)⁺.

### Step 7: Synthesis of compound 207-8 (2-chloro-5-methoxy-N-methyl-N-[[4-[1-methyl-4-(trifluoromethyl)imidazol-2-yl]cuban-1-yl]methyl]pyrimidin-4-amine)

Compound **207-7** (40 mg, 135.45 mmol, 1 *eq*), N,N-diisopropylethylamine (70.03 mg, 541.82 mmol, 94.37 µL, 4 *eq)* and 2,4-dichloro-5-methoxypyrimidine (38.80 mg, 216.73 mmol, 1.6 *eq*) were added to dioxane (2.0 mL) successively, and the resulting mixture was stirred at an external temperature of 60°C for 8 hours. The reaction solution was cooled to room temperature. Water (10.0 mL) was added, and the resulting mixture was extracted three times with ethyl acetate (10.0 mL). The organic layers were combined, washed twice with saturated brine (10.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 0/1) to obtain compound **207-8** (32.0 mg, 73.09 mmol, 53.96% yield) as a white solid. LC-MS: m/z = 438.2 (M+H)⁺.

### Step 8: Synthesis of compound 207 (4'-cyclopropyl-5,6'-dimethoxy-N-methyl-N-(4-(1-methyl-4-(trifluoromethyl)-1H-imidazol-2-yl)cuban-1-yl)methyl)-[2,5'-bipyrimidin]-4-amine)

In accordance with the same steps of compound **61,** compound **207** (2.66 mg, 4.32 mmol, 7.56% yield, a white solid) was obtained using compound **207-8** (25.0 mg, 57.10 mmol, 1 eq) and intermediate **A2-7** (22.15 mg, 114.20 mmol, 2.0 eq) as starting materials. LC-MS: m/z = 552.3 (M+H)⁺.

### Example 208: Synthesis of compound 208 (2-((4'-cyclopropyl-5,6'-dimethoxy-[2,5'-bipyrimidin]-4-yl)amino)-2-(4-(1-methyl-4-(trifluoromethyl)-1H-imidazol-2-yl)phenyl)acetonitrile)

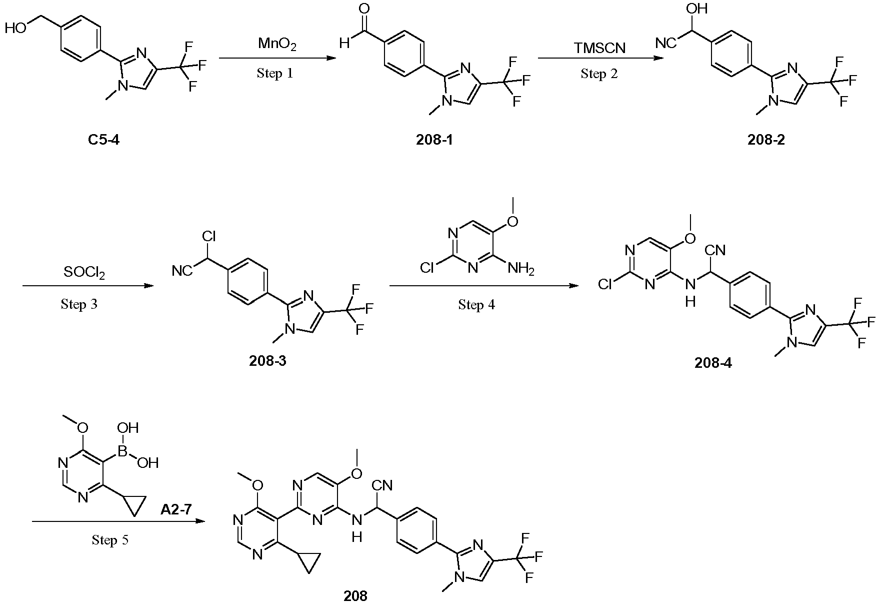

### Step 1: Synthesis of compound 208-1 (4-(1-methyl-4-(trifluoromethyl)-1H-imidazol-2-yl)benzaldehyde)

Compound **C5-4** (1.0 g, 3.91 mmol, 1.0 eq) and manganese dioxide (3.4 g, 39.1 mmol, 10.0 eq) were added to tetrahydrofuran (30.0 mL) successively, and the resulting mixture was stirred at an external temperature of 45°C for 16 hours. The reaction solution was filtered to obtain a filtrate. The filtrate was concentrated under vacuum to obtain a crude product, which was purified by column chromatography (eluent: petroleum ether/ethyl acetate = 1/0 - 1/1) to obtain compound **208-1** (823.1 mg, 3.24 mmol, 82.8% yield) as a white solid. LC-MS: m/z = 255.1 (M+H)⁺.

### Step 2: Synthesis of compound 208-2 (2-hydroxy-2-(4-(1-methyl-4-(trifluoromethyl)-1H-imidazol-2-yl)phenyl)acetonitrile)

Compound **208-1** (400.0 mg, 1.57 mmol, 1.0 eq), trimethylsilyl cyanide (203.0 mg, 2.05 mmol, 1.3 eq) and 1-octyl-3-methylimidazolium hexafluorophosphate (3.21 g, 9.42 mmol, 6.0 eq) were added to tetrahydrofuran (8.0 mL) successively, and the resulting mixture was stirred at an external temperature of 35°C for 16 hours. The reaction solution was concentrated under vacuum to obtain a crude product, which was purified by column chromatography (eluent: petroleum ether/ethyl acetate = 1/0 - 0/1) to obtain compound **208-2** (303.3 mg, 1.08 mmol, 68.7% yield) as a colorless transparent oil. LC-MS: m/z = 282.2 (M+H)⁺.

### Step 3: Synthesis of compound 208-3 (2-chloro-2-(4-(1-methyl-4-(trifluoromethyl)-1H-imidazol-2-yl)phenyl)acetonitrile)

Compound **208-2** (300.0 mg, 1.07 mmol, 1.00 eq) was dissolved in dichloromethane (6.0 mL), and thionyl chloride (635.0 mg, 5.33 mmol, 5.00 eq) was added at 0°C. After the addition was completed, the resulting mixture was heated up to room temperature (25°C) and stirred for 4 hours. The reaction solution was concentrated under vacuum to obtain compound **208-3** (288.8 mg, crude) as a colorless transparent oil. LC-MS: m/z = 300.2 (M+H)⁺.

### Step 4: Synthesis of compound 208-4 (2-((2-chloro-5-methoxypyrimidin-4-yl)amino)-2-(4-(1-methyl-4-(trifluoromethyl)-1H-imidazol-2-yl)phenyl)acetonitrile)

Compound **208-3** (260.0 mg, 0.87 mmol, 1.0 eq), 2-chloro-4-amino-5-methoxypyrimidine (415.3 mg, 2.61 mmol, 3.0 eq) and pyridine (206.4 mg, 2.61 mmol, 3.0 eq) were added to toluene (5.2 mL) successively, and the resulting mixture was reacted at 140°C under microwave for 1 hour. The reaction solution was cooled to room temperature, and concentrated under vacuum to obtain a crude product, which was purified by column chromatography (eluent: petroleum ether/ethyl acetate = 1/0 - 0/1) to obtain compound **208-4** (96.0 mg, 0.23 mmol, 26.1% yield) as a light yellow solid. LC-MS: m/z = 423.1 (M+H)⁺.

### Step 5: Synthesis of compound 208 (2-((4'-cyclopropyl-5,6'-dimethoxy-[2,5'-bipyrimidin]-4-yl)amino)-2-(4-(1-methyl-4-(trifluoromethyl)-1H-imidazol-2-yl)phenyl)acetonitrile)

Compound **208-4** (90.0 mg, 0.21 mmol, 1.00 *eq*), compound **A2-7** (83.4 mg, 0.43 mmol, 2.0 *eq*), chloro(2-dicyclohexylphosphino-2',4',6'-triisopropyl-1,1'-biphenyl)[2-(2'-amino-1,1'-biphenyl)]palladium (II) (XPhos-Pd-G2, 33.8 mg, 0.043 mmol, 0.20 *eq*), *2-*dicyclohexylphosphino-2',4',6'-triisopropylbiphenyl (XPhos, 41.0 mg, 0.086 mmol, 0.40 *eq)* and sodium bicarbonate (72.2 mg, 0.86 mmol, 4.0 *eq)* were added to dioxane (2.0 mL) and water (0.4 mL) successively. The resulting mixture was purged three times with nitrogen, and stirred at 95°C for 16 hours under nitrogen atmosphere. Water (10.0 mL) was added, and the resulting mixture was extracted three times with ethyl acetate (10.0 mL). The organic layers were combined, washed twice with saturated brine (10.0 mL), dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 0/1) to obtain compound **208** (38.2 mg, 0.071 mmol, 33.9% yield) as a white solid. ¹H NMR (400 MHz, DMSO-*d*₆) δ 8.70 (s, 1H), 8.60 (s, 1H), 8.01 (s, 1H), 7.92 - 7.89 (m, 2H), 7.86 - 7.81 (m, 2H), 7.53 (s, 1H),7.09 (s, 1H), 3.98 (s, 3H), 3.88 (s, 3H), 3.83 (s, 3H), 1.84-1.80 (m, 1H), 1.18 - 0.82 (m, 4H); LC-MS: m/z = 537.2 (M+H)⁺.

### Example 209: Synthesis of compound 209 (2-(4-chloro-1-isopropyl-1H-pyrazol-5-yl)-5-methoxy-N-((1-(1-methyl-4-(trifluoromethyl)-1H-imidazol-2-yl)-2-oxabicyclo[2.2.2]octan-4-yl)methyl)pyrimidin-4-amine)

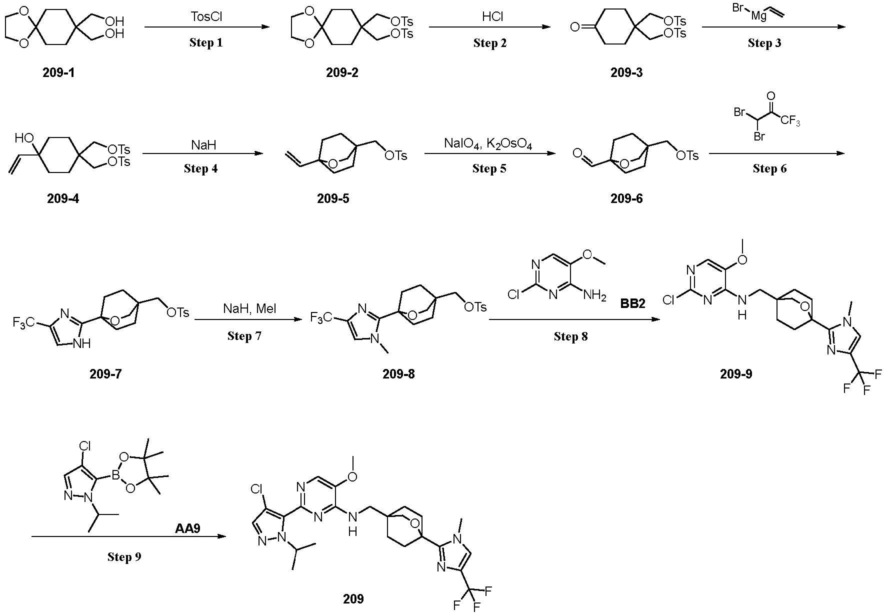

### Step 1: Synthesis of compound 209-2 ((1,4-dioxaspiro[4.5]decane-8,8-diyl)bis(methylene) bis(4-methylbenzenesulfonate))

Compound **209-1** ([8-(hydroxymethyl)-1,4-dioxaspiro[4.5]decane-8-yl]methanol, 50.0 g, 247 mmol, 1.00 eq) and *p*-toluenesulfonyl chloride (104 g, 544 mmol, 2.20 eq) were added to pyridine (250.0 mL) successively, and the resulting mixture was stirred at 25°C for 12 hours. Ethyl acetate (500.0 mL) was added, the resulting mixture was washed with 10% citric acid aqueous solution (500.0 mL) three times and saturated brine (500.0 mL) once, dried over anhydrous sodium sulfate, and filtered to obtain a filtrate, which was concentrated under vacuum to obtain a crude product. The crude product was triturated with ethanol (500.0 mL) for 1 hour, and filtered to obtain compound **209-2** (116.0 g, 227.0 mmol, 91.9% yield) as a white solid. ¹H NMR (400 MHz, CDCl3) δ 7.73 (d, *J =* 8.40 Hz, 4H), 7.36 (d, *J =* 8.00 Hz, 4H), 3.89 (s, 4H), 3.84 (s, 4H), 2.47 (s, 6H), 1.49 (s, 8H).

### Step 2: Synthesis of compound 209-3 ((4-oxocyclohexane-1,1-diyl)bis(methylene) bis(4-methylbenzenesulfonate))

Compound **209-2** (116 g, 227 mmol, 1.00 eq) was dissolved in tetrahydrofuran (1000 mL), followed by the addition of 1 M hydrochloric acid (579 mL, 2.55 eq), and the resulting mixture was stirred at 70°C for 12 hours. Saturated brine (200 mL) was added, and the resulting mixture was extracted three times with ethyl acetate (1000 mL). The organic layers were combined, washed twice with saturated brine (400 mL), dried over anhydrous sodium sulfate, and filtered to obtain a filtrate, which was concentrated under vacuum to obtain compound **209-3** (110 g, crude) as a white solid. ¹H NMR (400 MHz, DMSO-*d*₆) δ 7.76 (d, *J =* 8.00 Hz, 4H), 7.48 (d, *J =* 8.40 Hz, 4H), 3.98 (s, 4H), 2.46 (d, *J* = 27.6 Hz, 6H), 2.15 - 2.11 (t, *J =* 7.20 Hz, 4H), 1.62 - 1.54 (m, 4H); LC-MS: m/z = 467.1 (M+H)⁺.

### Step 3: Synthesis of compound 209-4 ((4-hydroxy-4-vinylcyclohexane-1,1-diyl)bis(methylene)bis(4-methylbenzenesulfonate))

Compound **209-3** (55.0 g, 118 mmol, 1.00 eq) was dissolved in tetrahydrofuran (500 mL), and a 1 M solution of vinylmagnesium bromide in tetrahydrofuran (236 mL, 2.00 eq) was slowly added dropwise at -70°C. After the addition was completed, the resulting mixture was stirred at -70°C for 2 hours. Saturated aqueous ammonium chloride solution (300 mL) was added at 0°C to quench the reaction. The resulting mixture was concentrated under vacuum to remove the solvent. Water (100 mL) was added, and the resulting mixture was extracted three times with ethyl acetate (300 mL). The organic layers were combined, dried over anhydrous sodium sulfate, and filtered to obtain a filtrate, which was concentrated under vacuum to obtain compound **209-4** (100 g, crude) as a yellow solid. ¹H NMR (400 MHz, CDCl3) δ 7.74 (dd, *J₁* = 8.0 Hz, *J₂* = 12.8 Hz, 4H), 7.36 (t, *J =* 8.0 Hz, 4H), 5.84 (dd, *J₁ =* 10.8 Hz, *J₂* = 17.2 Hz, 1H), 5.17 (d, *J=* 17.2 Hz, 1H), 5.04 (d, *J =* 10.8 Hz, 1H), 3.92 (s, 2H), 3.76 (s, 2H), 2.46 (d, *J =* 1.60 Hz, 6H), 1.56-1.26 (m, 8H).

### Step 4: Synthesis of compound 209-5 ((1-vinyl-2-oxabicyclo[2.2.2]octan-4-yl)methyl-4-methylbenzenesulfonate)

Compound **209-4** (30.0 g, 60.7 mmol, 1.00 eq) was dissolved in ethylene glycol dimethyl ether (500 mL), and sodium hydride (4.85 g, 121 mmol, 60% content, 2.00 eq) was added in portions at 0°C. After the addition was completed, the resulting mixture was stirred at 0°C for 30 minutes, heated up to 110°C and stirred for 16 hours. Saturated aqueous ammonium chloride solution (400 mL) was added at 0°C to quench the reaction, and water (500 mL) was added. The resulting mixture was concentrated under vacuum to remove the organic solvent, and extracted three times with ethyl acetate (600 mL). The organic layers were combined, washed twice with saturated brine (200 mL), dried over anhydrous sodium sulfate, and filtered to obtain a filtrate, which was concentrated under vacuum to obtain compound **209-5** (27.4 g, crude) as a light yellow solid. ¹H NMR (400 MHz, CDCl₃) δ 7.77 (d, *J =* 8.00 Hz, 2H), 7.36 (d, *J =* 8.00 Hz, 2H), 5.85 - 5.76 (m, 1H), 5.15- 5.11 (t, *J =* 1.60 Hz, 1H), 5.04 - 5.00 (d, *J₁ =* 1.60 Hz, *J₂* = 11.2 Hz, 1H), 3.69 (d, *J* = 6.80 Hz, 4H), 2.46 (s, 3H), 1.90 - 1.86 (m, 2H), 1.72 - 1.65 (m, 4H), 1.52 - 1.51 (m, 2H).

### Step 5: Synthesis of compound 209-6 ((1-formyl-2-oxabicyclo[2.2.2]octan-4-yl)methyl-4-methylbenzenesulfonate)

Compound **209-5** (4.00 g, 12.4 mmol, 1.00 *eq*) was dissolved in tetrahydrofuran (80.0 mL), and sodium periodate (7.96 g, 37.2 mmol, 2.06 mL, 3.00 *eq)* was added at 0°C. Potassium osmate (1.14 g, 3.10 mmol, 0.250 *eq*) dissolved in water (16.0 mL) was added, and the resulting mixture was stirred at 20°C for 12 hours, and filtered to obtain a filtrate. The filtrate was washed three times with saturated brine (100 mL), dried over anhydrous sodium sulfate, and filtered to obtain a filtrate, which was concentrated under vacuum to obtain **209-6** (4.0 g, crude) as a yellow oil. ¹H NMR (400 MHz, CDCl3) δ 9.53 (s, 1H), 7.77 - 7.74 (m, 2H), 7.37 - 7.34 (m, 2H), 3.74 - 3.67 (m, 4H), 2.46 (s, 3H),1.91 1.49 (m, 8H).

### Step 6: Synthesis of compound 209-7 ((1-(4-(trifluoromethyl)-1H-imidazol-2-yl)-2-oxabicyclo[2.2.2]octan-4-yl)methyl-4-methylbenzenesulfonate)

3,3-Dibromo-1,1,1-trifluoropropane-2-one (3.99 g, 14.8 mmol, 1.20 *eq*) and sodium acetate (2.12 g, 25.9 mmol, 2.10 *eq*) were added to water (8.0 mL) successively, and the resulting mixture was stirred at 100°C for 1 hour. Compound **209-6** (4.00 g, 12.3 mmol, 1.00 eq) dissolved in ammonia water (11.5 g, 98.7 mmol, 12.7 mL, 30.0% content, 8.0 eq) and methanol (40.0 mL) was added at 25°C. After the addition was completed, the resulting mixture was stirred at 25°C for 5 hours. Water (100 mL) was added, and the resulting mixture was extracted three times with ethyl acetate (180 mL). The organic layers were combined, dried over anhydrous sodium sulfate, and filtered to obtain a filtrate. The filtrate was concentrated under vacuum to obtain a crude product, which was purified by column chromatography (eluent: petroleum ether/ethyl acetate = 1/0 - 1/1) to obtain compound **209-7** (2.2 g, 5.05 mmol, 41.0% yield) as a white solid. LC-MS: m/z = 431.2 (M+H)⁺.

### Step 7: Synthesis of compound 209-8 ((1-(1-methyl-4-(trifluoromethyl)-1H-imidazol-2-yl)-2-oxabicyclo[2.2.2]octan-4-yl)methyl-4-methylbenzenesulfonate)

Compound **209-7** (2.20 g, 5.11 mmol, 1.00 *eq*) was dissolved in tetrahydrofuran (30.0 mL), and sodium hydride (307 mg, 7.67 mmol, 60.0% content, 1.50 *eq*) was added in portions at 0°C. After the addition was completed, the resulting mixture was stirred at 0°C for 30 minutes. Iodomethane (725 mg, 5.11 mmol, 1.0 *eq)* was added, and the resulting mixture was heated up to 25°C and reacted for 2 hours. Ice water (30.0 mL) was added at 0°C to quench the reaction. Water (20.0 mL) was added, and the resulting mixture was extracted with ethyl acetate (150 mL) three times. The organic layers were combined, washed twice with saturated brine (60.0 mL), dried over anhydrous sodium sulfate, and filtered to obtain a filtrate. The filtrate was concentrated under vacuum to obtain a crude product, which was purified by prep-HPLC (column: Phenomenex luna C18 150×40mm×15µm; mobile phase: [water (TFA)-ACN]; gradient: 45%-75%) to obtain compound **209-8** (1.5 g, 3.37 mmol, 66.0% yield) as a white solid. ¹H NMR (400 MHz, CDCl3) δ 7.78 (d, *J =* 8.4 Hz, 2H), 7.37 (d, *J =* 8.0 Hz, 2H), 7.11 (d, *J =* 1.2 Hz, 1H), 3.81 (s, 5H), 3.73 (s, 2H), 2.47 (s, 3H), 2.28 - 2.22 (m, 2H), 2.19 - 2.13 (m, 2H), 1.78 - 1.71 (m, 2H), 1.62 - 1.55 (m, 2H); LC-MS: m/z = 445.3 (M+H)⁺.

### Step 8: Synthesis of compound 209-9 (2-chloro-5-methoxy-N-((1-(1-methyl-4-(trifluoromethyl)-1H-imidazol-2-yl)-2-oxabicyclo[2.2.2]octan-4-yl)methyl)pyrimidin-4-amine)

Compound **209-8** (1.0 g, 2.25 mmol, 1.0 eq), compound **BB2** (716.0 mg, 4.50 mmol, 2.0 eq), sodium iodide (1.01 g, 6.75 mmol, 3.0 eq) and cesium carbonate (3.67 g, 11.25 mmol, 5.0 eq) were added to N,N-dimethylformamide (20.0 mL) successively, and the mixture was stirred at 130°C for 24 hours. The reaction solution was cooled to 25°C. Dichloromethane (100.0 mL) was added, the resulting mixture was stirred for 5 minutes, and filtered to obtain a filtrate. The filtrate was washed three times with saturated brine (50.0 mL), dried over anhydrous sodium sulfate, and concentrated under vacuum to obtain a crude product. The crude product was purified by column chromatography (eluent: petroleum ether/dichloromethane = 1/0 - 0/1) to obtain compound **209-9** (135.6 mg, 0.31 mmol, 14.0% yield) as a white solid. LC-MS: m/z = 432.3 (M+H)⁺.

### Step 9: Synthesis of compound 209 (2-(4-chloro-1-isopropyl-1H-pyrazol-5-yl)-5-methoxy-N-((1-(1-methyl-4-(trifluoromethyl)-1H-imidazol-2-yl)-2-oxabicyclo[2.2.2]octan-4-yl)methyl)pyrimidin-4-amine)

Compound **209-9** (40.0 mg, 92.6 µmol, 1.00 *eq),* compound **AA9** (75.2 mg, 277.8 µmol, 3.00 *eq*), and (SP-4-3)-[dicyclohexyl[2',4',6'-tri(isopropyl)[1,1'-biphenyl]-2-yl]phosphino](methanesulfonato)[2'-(methylamino)[1,1'-biphenyl]-2-yl]palladium (Xphos Pd G4, 16.0 mg, 18.52 µmol, 0.2 *eq*), 2-dicyclohexylphosphino-2',4',6'-triisopropylbiphenyl (Xphos, 17.7 mg, 37.04 µmol, 0.4 *eq)* and potassium phosphate (78.6 mg, 370.4 µmol, 4 *eq*) were added to dioxane (0.8 mL) and water (0.16 mL) successively, and the resulting mixture was stirred at an external temperature of 95°C for 16 hours. The reaction solution was concentrated under vacuum to obtain a crude product, which was purified by prep-HPLC (column: Phenomenex luna C18 150×25mm×10µm; mobile phase: [water (FA)-ACN]; gradient: 61%-91%) to obtain compound **209** (16.6 mg, 30.8 µmol, 33.3% yield) as a white solid. ¹H NMR (400 MHz, CDCl₃) δ 8.21 (s, 1H), 7.55 (s, 1H),7.13 (s, 1H), 6.01-5.88 (m, 1H), 5.32 - 5.26 (m, 1H), 4.27 (s, 2H), 4.01 (s, 2H), 4.00 (s, 3H), 3.87 (s, 3H), 2.39 - 2.31 (m, 2H), 2.29 - 2.21 (m, 2H), 1.97 - 1.91 (m, 2H), 1.83 - 1.76 (m, 2H), 1.51 (s, 3H), 1.49 (s, 3H); LC-MS: m/z =540.3 (M+H)⁺.

### Example 210: Synthesis of compound 210 (4'-cyclopropyl-5,6'-dimethoxy-N-(1-(1-methyl-4-(trifluoromethyl)-1H-imidazol-2-yl)-2-oxabicyclo[2.2.2]octan-4-yl)methyl)-[2,5'-bipyrimidin]-4-amine)

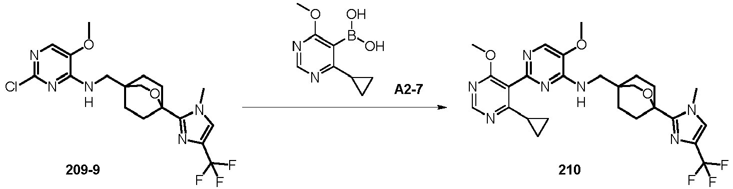

In accordance with the same steps of compound **209,** compound **210** (7.8 mg, 0.0143 mmol, 12.3% yield, a white solid) was obtained using compound **209-9** (50.0 mg, 0.116 mmol, 1 eq) and intermediate **A2-7** (67.4 mg, 0.348 mmol, 3.0 eq) as starting materials. ¹H NMR (400 MHz, CDCl₃) δ 8.63 (s, 1H), 7.91 (s, 1H),7.11 (s, 1H), 5.58 (s, 1H), 3.99 (s, 3H), 3.95 (s, 3H), 3.87 (s, 2H), 3.83 (s, 3H), 3.43 (d, *J* = 6.4 Hz, 2H), 2.29 - 2.21 (m, 4H), 1.82 - 1.71 (m, 5H), 1.23 - 1.21 (m, 2H), 0.94 - 0.91 (m, 2H); LC-MS: m/z = 546.3 (M+H)^{+.}

### Example 211: Synthesis of compound 211 (2-(4-chloro-1-isopropyl-1H-pyrazol-5-yl)-5-methoxy-N-methyl-N-((1-(1-methyl-4-(trifluoromethyl)-1H-imidazol-2-yl)-2-oxabicyclo[2.2.2]octan-4-yl)methyl)pyrimidin-4-amine)

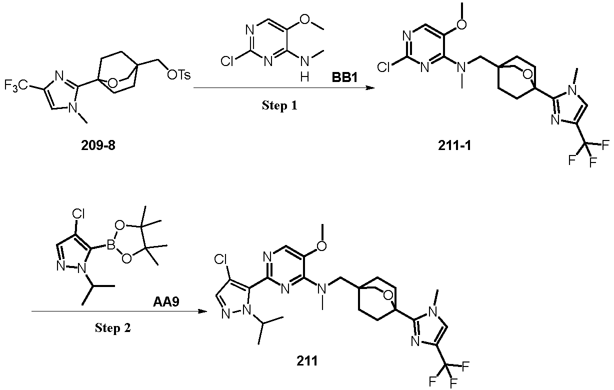

In accordance with the same steps of compound **209,** compound **211** (44.3 mg, 0.08 mmol, 18.9% yield, a light yellow solid) was obtained using compound **209-8** (300.0 mg, 0.675 mmol, 1 eq) and intermediate **BB-1** (351.5 mg, 2.02 mmol, 3.0 eq) as starting materials. LC-MS: m/z = 554.2 (M+H)⁺.

### Example 212: Synthesis of compound 212 (4'-cyclopropyl-5,6'-dimethoxy-N-methyl-N-((1-(1-methyl-4-(trifluoromethyl)-1H-imidazol-2-yl)-2-oxabicyclo[2.2.2]octan-4-yl)methyl)-[2,5'-bipyrimidin]-4-amine)

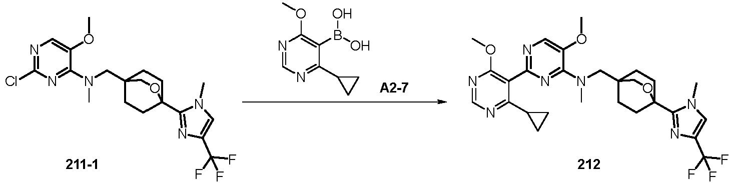

In accordance with the same steps of compound **209,** compound **212** (5.7 mg, 0.01 mmol, 15.2% yield, a white solid) was obtained using compound **211-1** (30.0 mg, 0.067 mmol, 1 eq) and intermediate **A2-7** (39.2 mg, 0.20 mmol, 3.0 eq) as starting materials. LC-MS: m/z = 560.3 (M+H)⁺.

### Example 213: Synthesis of compound 213 (2-(4-chloro-1-isopropyl-1H-pyrazol-5-yl)-N-((1-(1-cyclopropyl-4-(trifluoromethyl)-1H-imidazol-2-yl)-2-oxabicyclo[2.2.2]octan-4-yl)methyl)-5-methoxypyrimidin-4-amine)

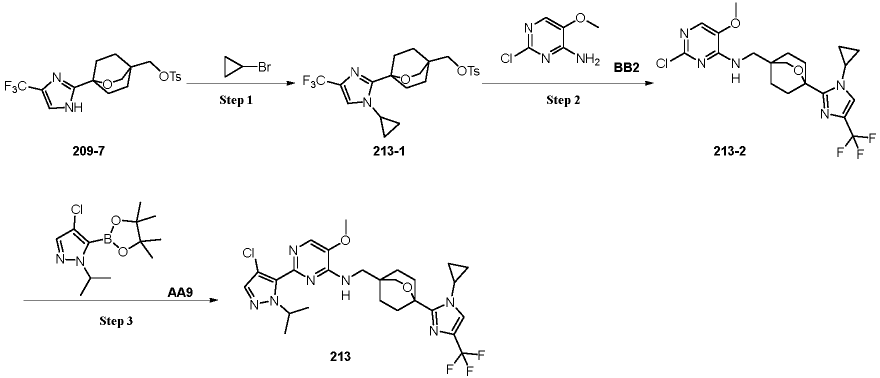

In accordance with the same steps of compound **209,** compound **213** (11.6 mg, 0.02 mmol, 17.2% yield, a white solid) was obtained using compound **209-7** (1.0 g, 2.32 mmol, 1 eq) and cyclopropyl bromide (843.2 mg, 6.97 mmol, 3.0 eq) as starting materials. LC-MS: m/z = 566.3 (M+H)⁺.

### Example 214: Synthesis of compound 214 (2-(4-chloro-1-isopropyl-1H-pyrazol-5-yl)-N-((1-(1-isopropyl-4-(trifluoromethyl)-1H-imidazol-2-yl)-2-oxabicyclo[2.2.2]octan-4-yl)methyl)-5-methoxypyrimidin-4-amine)

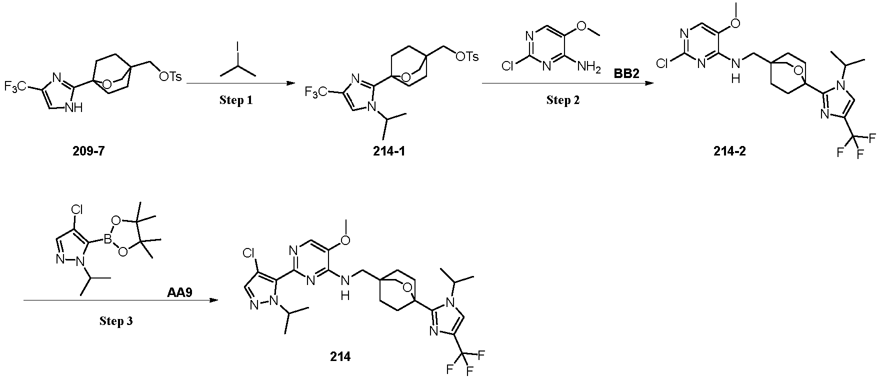

In accordance with the same steps of compound **209,** compound **214** (10.4 mg, 0.018 mmol, 20.4% yield, a white solid) was obtained using compound **209-7** (1.0 g, 2.32 mmol, 1 eq) and isopropyl iodide (1.18 g, 6.97 mmol, 3.0 eq) as starting materials. LC-MS: m/z = 568.4 (M+H)⁺.

### Example 215: Synthesis of compound 215 (4'-cyclopropyl-5,6'-dimethoxy-N-(4-(1-methyl-4-(trifluoromethyl)-1H-imidazol-2-yl)cuban-1-yl)methyl)-[2,5'-bipyrimidin]-4-amine)

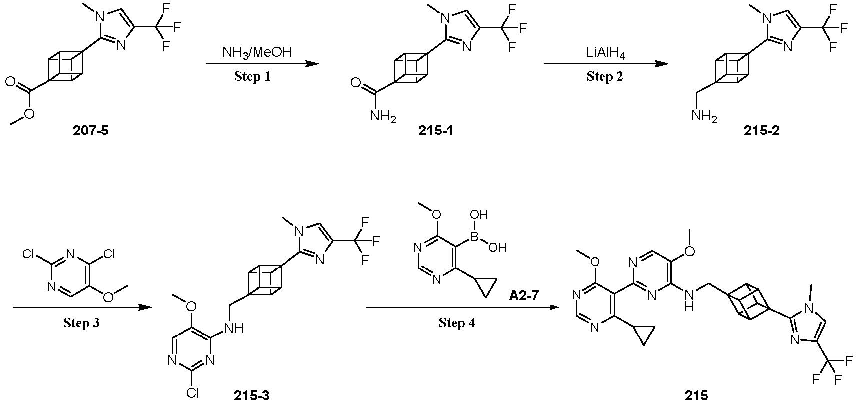

In accordance with the same steps of compound **207,** compound **215** (7.77 mg, 14.0 µM, 11.9% yield, a white solid) was obtained using compound **207-5** (500 mg, 1.61 mmol, 1.0 eq) and 7 M ammonia in methanol (40.0 mL, 173 eq) as starting materials. LC-MS: m/z = 538.3 (M+H)⁺.

### Example 216: Synthesis of compound 216 (4'-cyclopropyl-N-((4-(1-cyclopropyl-4-(trifluoromethyl)-1H-imidazol-2-yl)cuban-1-yl)methyl)-5,6'-dimethoxy-[2,5'-bipyrimidin]-4-amine)

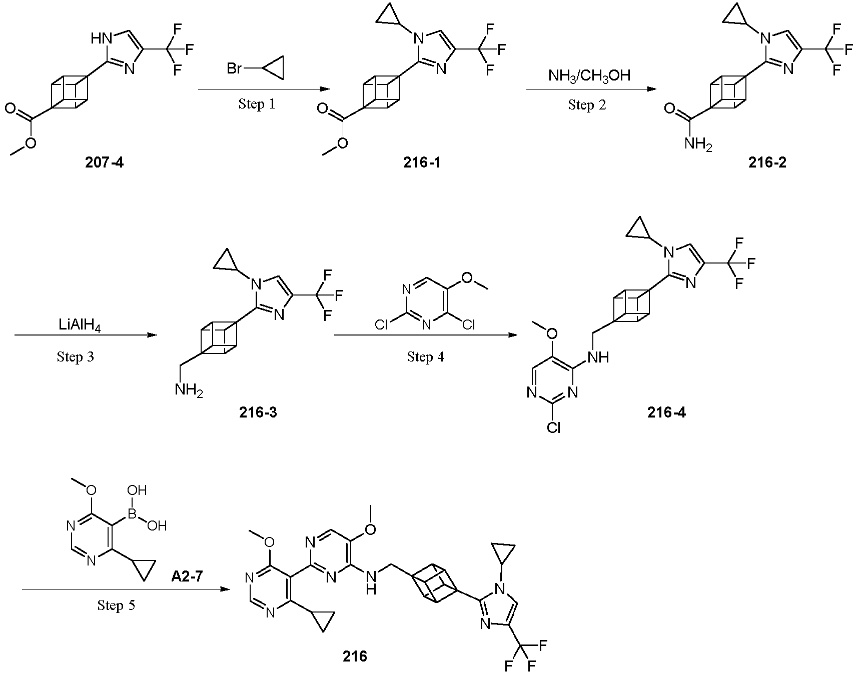

In accordance with the same steps of compound **207,** compound **216** (2.5 mg, 4.45 µmol, 10.5% yield, a white solid) was obtained using compound **207-4** (200 mg, 0.68 mmol, 1.0 eq) and cyclopropyl bromide (246.8 mg, 2.04 mmol, 3.0 eq) as starting materials. LC-MS: m/z = 564.2 (M+H)⁺.

### Example 217: Synthesis of compound 217 (4'-cyclopropyl-N-((4-(1-isopropyl-4-(trifluoromethyl)-1H-imidazol-2-yl)cuban-1-yl)methyl)-5,6'-dimethoxy-[2,5'-bipyrimidin]-4-amine)

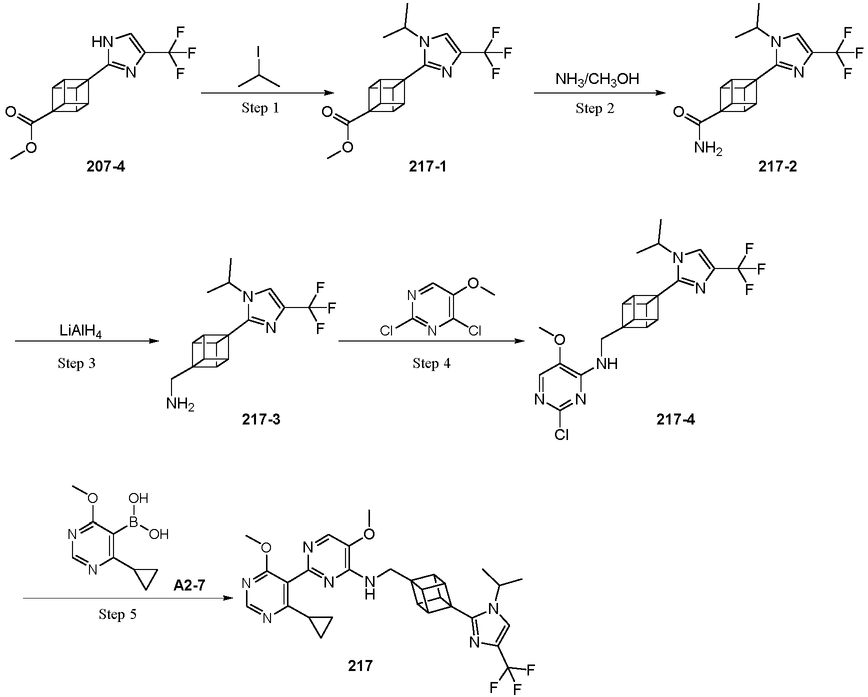

In accordance with the same steps of compound **207,** compound **217** (10.5 mg, 0.019 mmol, 13.8% yield, a white solid) was obtained using compound **207-4** (600 mg, 2.03 mmol, 1.0 eq) and isopropyl iodide (1.04 g, 6.09 mmol, 3.0 eq) as starting materials. LC-MS: m/z = 566.3 (M+H)⁺.

### Test Example 1: In vitro USP1 kinase inhibitory activity

**Experimental object:** The USP1 kinase inhibitory activity of the compounds was screened by fluorescence detection method using 384-well plates.

Experimental materials: Recombinant human His6-USP1 / His6-UAF1 complex protein (R&D, Catalog No.: E-568-050), ubiquitin rhodamine 110 protein (Ub-Rho) (R&D, Catalog No.: U-555-050), 384-well plate (Perkin Elmer, Catalog No.: 6007279); all compounds were dissolved in 100% DMSO to obtain a 50 mM stock solution.

### Experimental methods:

1) Preparation of 1× assay buffer: 1× assay buffer (modified Tris buffer) was prepared;
2) Compound dilution: The compound was transferred to the assay plate by Echo. The final concentration of DMSO is 1%;
3) Preparation of enzyme solution: Recombinant human His6-USP1 / His6-UAF1 complex protein was added to 1× assay buffer to obtain an enzyme solution;
4) Preparation of substrate solution: Ubiquitin rhodamine 110 protein CF (Ub-Rho) was added to 1× assay buffer to obtain a substrate solution;
5) 10 µL enzyme solution was transferred to the assay plate, and 10 µL 1× assay buffer was added to the control well;
6) The plate was incubated at room temperature for 1 hour;
7) 10 µL substrate solution was added to each well to start the reaction, and the plate was centrifuged for 30 seconds, and shaked for 30 seconds;
8) The plate was read with Envision after 30 minutes, and the parameters were excitation light 480 nm and emission light 540 nm;
9) The data was collected and summarized;
10) Curve fitting: The data was fitted in Excel,
Percent inhibition rate = (maximum value - signal value)/(maximum value - minimum value) ×100; the data was fitted in XL-Fit, Percent inhibition rate = minimum concentration + (maximum concentration - minimum concentration) / (1 + (half inhibition concentration / compound concentration) slope);

### Half inhibition concentration is referred to as IC₅₀ hereinafter.

### Test Example 2: CTG experiment on cell activity

1. Object: USP1 inhibitors were screened on 96-well plate by CTG assay.
2. Materials:
   The cells used were MDA-MB-436 (manufacturer: Nanjing Cobioer Biotechnology Co., Ltd.), and the culture medium was DMEM.
   CTG (CellTiter-Glo^{®} Luminescent Cell Viability Assay kit) (Promega, Catalog No.: G7572)
   96-well plate (Corning, Catalog No.: 3610)
3. Compounds:
   All compounds were dissolved in 100% DMSO to obtain 50 mM stock solutions.
4. Experimental methods:
   ① Plating:
      The cells to be tested were spread into a 96-well plate at a certain density at 200 µL per well, and 200 µL of cell culture medium was added to the blank well. The spread cell plate was placed in a 37°C incubator.
   ② Compound dilution:
      The compound was diluted to different concentrations with 100% DMSO, and the final concentration of DMSO was 4%.
   ③ Addition of compound:
      The compound formulated in ② was added to the cell plate of ① (10 µL per well). The cell plate with the compound added was placed in a 37°C incubator, and cultured for 7 days before assay.
   ④ Assay:
      The substrate and buffer of the CTG kit were mixed evenly. The cell plate with the compound added in ③ was taken out from the incubator, and placed at room temperature for 30 minutes. The compound-containing culture medium in the cell plate was removed, 100 µL of PBS and 50 µL of CTG reagent were added to each well. The mixture in each well was mixed and shaked for 3 minutes, and then incubated at room temperature for 10 minutes. The plate was read by BIotek microplate reader, and the parameter was 567 CPM.
5. Curve fitting

Same as enzyme assay.

The IC₅₀ value of USP1 kinase inhibitory activity and cellular IC₅₀ data of the compounds of the present invention are shown as follows:

| Example No. | USP1 IC₅₀ (nM) | MDA-MB-436 IC₅₀ (nM) |
|---|---|---|
| 1 | 26 | 1267.2 |
| 2 | 14 | 0.1 |
| 3 | 2.8 | 6.4 |
| 4 | 1.2 | 0.3 |
| 5 | 7.6 | 112.7 |
| 6 | 1.2 | 2 |
| 7 | 4.5 | 102.1 |
| 8 | 1.4 | 2.3 |
| 9 | 35 | / |
| 10 | 564 | / |
| 11 | 22 | 751 |
| 13 | 1.9 | / |
| 14 | 60 | / |
| 15 | 6.8 | 301 |
| 16 | 30.6 | / |
| 17 | 3.9 | 102.7 |
| 18 | 4 | 312.8 |
| 19 | 24 | /1.2 |
| 20 | 91.5 | 532.9 |
| 21 | 7.4 | 1.8 |
| 22 | 24.4 | 209 |
| 23 | 33 | 33.1 |
| 24 | 8.8 | / |
| 25 | 3.7 | / |
| 26 | 4.6 | 0.9 |
| 27 | 195 | / |
| 28 | 33 | 55.8 |
| 29 | 4.8 | / |
| 30 | 37 | / |
| 32 | 2.8 | 336 |
| 33 | 1.2 | 1.8 |
| 34 | 6.9 | 119 |
| 35 | 9.6 | 45 |
| 36 | 22.4 | 950.3 |
| 37 | 51 | >5000 |
| 38 | 1.1 | 13.7 |
| 39 | 2.8 | 56.9 |
| 40 | 2.3 | 21.4 |
| 41 | 1261 | / |
| 50 | 5.2 | 0.022 |
| 57 | 4.4 | 48.3 |
| 61 | 11 | 813 |
| 62 | 2.1 | 2.2 |
| 63 | 3.3 | 30 |
| 64 | 3.7 | 6.3 |
| 65 | 2.1 | 2.4 |
| 66 | 5.6 | 14.6 |
| 67 | 5.3 | 1169.4 |
| 68 | 3.2 | 266.9 |
| 69 | 9.2 | 709.1 |
| 70 | 19.4 | 599.8 |
| 71 | 9.9 | 274.3 |
| 72 | 6.1 | 181.17 |
| 73 | 3.6 | 112.3 |
| 74 | 872.8 | |
| 75 | 3.7 | 330.6 |
| 76 | 30.1 | |
| 77 | 28 | 1802.2 |
| 78 | 6.4 | >5000 |
| 79 | 4.5 | 1206.7 |
| 80 | 26 | >5000 |
| 81 | 3.7 | 66.1 |
| 82 | 1.7 | 278.3 |
| 83 | 4.4 | 99.1 |
| 84 | 9.0 | 704.4 |
| 85 | 0.47 | 22.5 |
| 86 | 1.1 | 29.4 |
| 87 | 6.2 | 2650.5 |
| 88 | 10 | 156.8 |
| 89 | 14.4 | 243.1 |
| 90 | 101.7 | |
| 91 | 1.3 | 479.4 |
| 92 | 3.3 | 27.3 |
| 93 | 1.5 | 3.5 |
| 94 | 6.5 | 275.4 |
| 95 | 8.4 | 75.1 |
| 96 | 4.8 | 12 |
| 97 | 263 | |
| 98 | 144.5 | 694.4 |
| 99 | 15.1 | 445.0 |
| 100 | 4.1 | 27.8 |
| 101 | 15 | 99.1 |
| 102 | 4.4 | 15.8 |
| 103 | 10 | 140.6 |
| 104 | 8.8 | 13.7 |
| 105 | 60 | 124.4 |
| 106 | 2.9 | 4.7 |
| 107 | 16 | 17.5 |
| 108 | 19.7 | 352.9 |
| 109 | 5.3 | 48.3 |
| 110 | 28.6 | 354.4 |
| 111 | 10.4 | 88.1 |
| 112 | 266.1 | >5000 |
| 113 | 23 | 87.2 |
| 114 | 648 | >5000 |
| 115 | 77 | 1605.3 |
| 116 | 19.4 | 535.7 |
| 117 | 13.8 | 278.3 |
| 118 | 58 | 1485.6 |
| 119 | 7.6 | 16.9 |
| 120 | 8.9 | 50.5 |
| 121 | 8.3 | 23.9 |
| 122 | 13 | 82.8 |
| 123 | 20 | 169.3 |
| 124 | 6.1 | 13.2 |
| 125 | 3.1 | 0.5 |
| 126 | 13 | 22.7 |
| 127 | 4.2 | 12.2 |
| 128 | 2.6 | 5.7 |
| 129 | 9 | 12.6 |
| 130 | 19 | 45.6 |
| 131 | 7.4 | 45.5 |
| 132 | 6.0 | 8.8 |
| 133 | 15 | 134.8 |
| 134 | 3.2 | 34.1 |
| 135 | 3.2 | 15.8 |
| 136 | 17 | 24.9 |
| 137 | 24 | 216.2 |
| 138 | 9.0 | 12.4 |
| 139 | 5.2 | 5.0 |
| 140 | 73 | |
| 141 | 9.5 | 76.7 |
| 142 | 25 | 50.0 |
| 143 | 31.0 | 1444.2 |
| 144 | 68.6 | 1669.5 |
| 145 | 19 | 730.9 |
| 146 | 18 | 1056.6 |
| 147 | 364 | |
| 148 | 1045.6 | |
| 149 | 69.2 | |
| 150 | 431.2 | |
| 151 | 72 | 58.4 |
| 152 | 7.5 | 64.4 |
| 153 | 3.4 | 25.4 |
| 154 | 6.2 | 7.3 |
| 155 | 12 | 85.7 |
| 156 | 2.2 | 59.5 |
| 157 | 2.9 | 23.3 |
| 158 | 2.1 | 12.3 |
| 159 | 1.7 | 39.4 |
| 160 | 3.6 | 121.3 |
| 161 | 6 | 537.1 |
| 162 | 3.9 | 77.5 |
| 163 | 2.3 | 13.5 |
| 164 | 1.1 | 0.8 |
| 165 | 1.4 | 7.1 |
| 166 | 2.1 | 41.4 |
| 167 | 0.45 | 2.9 |
| 168 | 1.2 | 5.1 |
| 169 | 0.8 | 0.4 |
| 170 | 2.9 | 14.5 |
| 171 | 2.3 | 84.7 |
| 172 | 12 | 954.5 |
| 173 | 1.0 | 23.6 |
| 174 | 5.1 | 43.7 |
| 175 | 3.6 | 87.1 |
| 176 | 4.0 | 186.6 |
| 177 | 75 | |
| 178 | 110 | |
| 179 | 56 | 196.7 |
| 180 | 15 | 122.7 |
| 181 | 27 | |
| 182 | 7.4 | 6.0 |
| 183 | 164 | |
| 184 | 94 | |
| 185 | 9.8 | 119.9 |
| 186 | 66 | |
| 187 | 23.5 | |
| 188 | 15 | |
| 189 | 16 | 307.2 |
| 190 | 33 | 339.1 |
| 191 | 4.3 | 10.7 |
| 192 | 3.8 | 20.4 |
| 193 | 87 | |
| 194 | 51 | 58.4 |
| 195 | 243 | |
| 196 | 12 | 82.1 |
| 197 | 133 | |
| 198 | 3.2 | 5.6 |
| 199 | 5.6 | 28.7 |
| 200 | 2.8 | 2.8 |
| 201 | 12 | 40.5 |
| 202 | 3 | 15.9 |
| 203 | 1.4 | 3.3 |
| 204 | 25 | 15.7 |
| 205 | 43 | |
| 206 | 3.5 | |
| 207 | 3.5 | 5.8 |
| 209 | 1.6 | 31.6 |
| 210 | 85 | 168.6 |
| 211 | 6.1 | |
| 212 | 19 | |
| 215 | 2.2 | 8.7 |
| 216 | 1.4 | 2.7 |

In addition, in some examples, the compounds of the present invention have good pharmacokinetic properties and good CYP (cytochrome P450), hERG, PPB (plasma protein binding), LM (liver microsomal metabolic stability) properties. Moreover, the compounds of the present invention show good anti-tumor activity in the MDA-MB-436 or MX-1 model in mice.

## Claims

1. A compound of formula (I'), 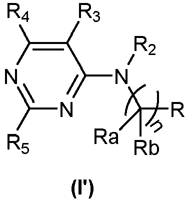 or a pharmaceutically acceptable salt, hydrate, solvate, isotope substitute or stereoisomer thereof,
wherein,
R is selected from the group consisting of C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, C₆₋₁₀ aryl, C₆₋₁₀ aryl fused with 5 to 10 membered heteroaryl, and 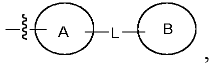 wherein the above groups are each independently optionally substituted by one or more R₁;
ring A and ring B are each independently selected from the group consisting of C₆₋₁₀ aryl, 5 to 10 membered heteroaryl, C₃₋₈ cycloalkyl and 3 to 8 membered heterocyclyl, wherein ring A and ring B are each independently optionally substituted by one or more R₁;
L is selected from the group consisting of a chemical bond, -O-, -S-, -C₁₋₆ alkylene-, -O-C₁₋₆ alkylene-, -C₁₋₆ alkylene-O-, -S-C₁₋₆ alkylene- and -C₁₋₆ alkylene-S-;
Rₐ and R_{b} are each independently selected from the group consisting of H atom, - CN, C₁₋₆ alkyl, -OH, halogen, C₂₋₆ alkenyl, C₂₋₆ alkynyl, C₁₋₆ hydroxyalkyl, C₁₋₆ haloalkyl, C₁₋₆ alkoxy and C₁₋₆ haloalkoxy; or Rₐ and R_{b} together form an oxo, C₃₋₈ cycloalkyl or 3 to 8 membered heterocyclyl;
R₂ is selected from the group consisting of H atom, -OH, -CN, C₁₋₆ alkyl, -C₁₋₆ alkyl-C₆₋₁₀ aryl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, C₆₋₁₀ aryl, 5 to 10 membered heteroaryl, C₃₋₈ cycloalkyl and 3 to 8 membered heterocyclyl, wherein the C₁₋₆ alkyl and -C₁₋₆ alkyl-C₆₋₁₀ aryl are each optionally substituted by one or more R₁;
R₃ is selected from the group consisting of H atom, -OH, -COOH, -NH₂, -CN, halogen, C₁₋₆ alkyl, -S-C₁₋₆ alkyl, -S(O)-C₁₋₆ alkyl, -S(O)₂-C₁₋₆ alkyl, phosphoryl, phosphonyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, C₆₋₁₀ aryl, 5 to 10 membered heteroaryl, C₃₋₈ cycloalkyl, 3 to 8 membered heterocyclyl and -C₁₋₆ alkylene-C(O)-O-C₁₋₆ alkyl, wherein the -NH₂, C₁₋₆ alkyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, C₆₋₁₀ aryl, 5 to 10 membered heteroaryl, C₃₋₈ cycloalkyl and 3 to 8 membered heterocyclyl are each independently optionally substituted by one or more R₁;
R₄ is selected from the group consisting of H atom, -OH, -COOH, -NH₂, -CN, halogen, C₁₋₆ alkyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, C₆₋₁₀ aryl, 5 to 10 membered heteroaryl, C₃₋₈ cycloalkyl and 3 to 8 membered heterocyclyl;
or, R₂ and R₃ together with the atoms to which they are attached form ring C, ring C is a 5 to 7 membered heteroaryl or 5 to 7 membered heterocyclyl, the heteroatom in the 5 to 7 membered heteroaryl or 5 to 7 membered heterocyclyl is O or N, further, the 5 to 7 membered heterocyclyl is selected from the group consisting of morpholinyl, 3-morpholinonyl, pyrrolidinyl, 2-oxazolidinonyl and 2-pyrrolidinonyl, and ring C is optionally substituted by one or more R₁;
or, R₃ and R₄ together with the atoms to which they are attached form ring D, ring D is a 5 to 7 membered heteroaryl or 5 to 7 membered heterocyclyl, and ring D is optionally substituted by one or more R₁;
R₅ is selected from the group consisting of C₁₋₆ alkyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, C₆₋₁₀ aryl, 5 to 10 membered heteroaryl, C₆₋₁₀ aryl fused with 5 to 10 membered heteroaryl, C₆₋₁₀ aryl fused with 3 to 8 membered heterocyclyl, C₃₋₈ cycloalkyl and 3 to 8 membered heterocyclyl, wherein the C₁₋₆ alkyl, C₂₋₆ alkenyl, C₂₋₆ alkynyl, C₁₋₆ alkoxy, C₁₋₆ haloalkyl, C₁₋₆ haloalkoxy, C₁₋₆ hydroxyalkyl, C₆₋₁₀ aryl, 5 to 10 membered heteroaryl, C₃₋₈ cycloalkyl and 3 to 8 membered heterocyclyl are each independently optionally substituted by one or more R₁;
R₁ at each occurrence is independently selected from the group consisting of D atom, -OH, -COOH, -NH₂, -CN, oxo, halogen, C₁₋₆ alkyl, C₂₋₆ alkynyl, C₁₋₆ haloalkyl, C₁₋₆ alkoxy, C₁₋₆ haloalkoxy, C₁₋₆ hydroxyalkyl, C₆₋₁₀ aryl, 5 to 10 membered heteroaryl, C₃₋₈ cycloalkyl, 3 to 8 membered heterocyclyl and -O-C₁₋₆ alkylene-O-C₁₋₆ alkyl, wherein the C₆₋₁₀ aryl, 5 to 10 membered heteroaryl, C₃₋₈ cycloalkyl and 3 to 8 membered heterocyclyl are each independently optionally substituted by one or more substituent(s) selected from the group consisting of D atom, -OH, -COOH, -NH₂, -CN, halogen, C₁₋₆ alkyl, C₁₋₆ haloalkyl, C₁₋₆ alkoxy, C₁₋₆ haloalkoxy and C₁₋₆ hydroxyalkyl; and
n is an integer from 0 to 8.

2. The compound according to claim 1, being a compound of formula (I), 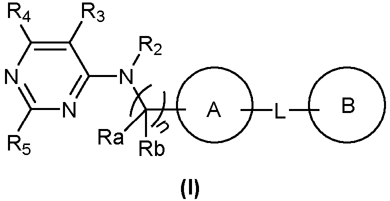 wherein ring A, ring B, L, Rₐ, R_{b}, R₂ to R₅ and n are as defined in claim 1.

3. The compound according to claim 1 or 2, wherein,
R₂ is selected from the group consisting of H atom, -OH, -CN, C₁₋₆ alkyl, C₂₋₆ alkynyl, -C₁₋₆ alkyl-C₆₋₁₀ aryl, C₁₋₆ alkoxy, C₁₋₆ haloalkyl, C₁₋₆ haloalkoxy, C₁₋₆ hydroxyalkyl, C₃₋₆ cycloalkyl and 5 to 7 membered heterocyclyl, wherein the C₁₋₆ alkyl and -C₁₋₆ alkyl-C₆₋₁₀ aryl are each optionally substituted by one or more R₁, R₁ is as defined in claim 1;
preferably, R₂ is selected from the group consisting of H atom, -CN, methyl, trideuteriomethyl, ethynyl, propynyl, tetrahydrofuranyl, cyclopropyl, methoxy and hydroxy;
R₃ is selected from the group consisting of H atom, -OH, -COOH, -NH₂, -CN, halogen, C₁₋₆ alkyl, C₂₋₆ alkynyl, C₁₋₆ alkoxy, C₁₋₆ haloalkyl, C₁₋₆ haloalkoxy, C₁₋₆ hydroxyalkyl, 5 to 7 membered heterocyclyl and -C₁₋₆ alkylene-C(O)-O-C₁₋₆ alkyl, wherein the C₁₋₆ hydroxyalkyl and 5 to 7 membered heterocyclyl are each independently optionally substituted by one or more C₁₋₆ alkyl;
preferably, R₃ is selected from the group consisting of H atom, methoxy, trifluoromethyl, Cl atom, -CN, isopropoxy, ethynyl, difluoromethoxy, morpholinyl, 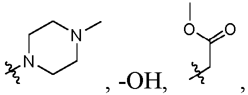 F atom, hydroxymethyl and 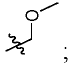 and
R₄ is selected from the group consisting of H atom, -OH, -COOH, -NH₂, -CN, halogen, C₁₋₆ alkyl, C₁₋₆ alkoxy, C₁₋₆ haloalkyl, C₁₋₆ haloalkoxy and C₁₋₆ hydroxyalkyl;
preferably, R₄ is H atom.

4. The compound according to claim 1 or 2, being a compound of formula (II), 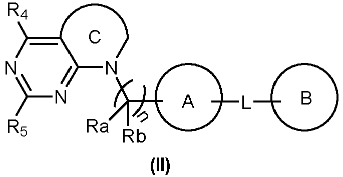
wherein ring C is selected from the group consisting of 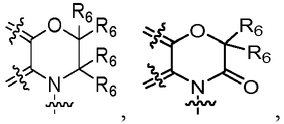 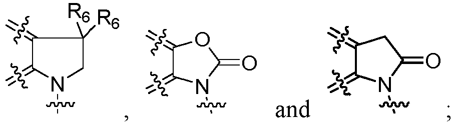
R₆ at each occurrence is independently H or C₁₋₆ alkyl, or two R₆ together with the atom to which they are attached form a C₃₋₈ cycloalkyl or 3 to 8 membered heterocyclyl; and
ring A, ring B, L, Rₐ, R_{b}, R₄, R₅ and n are as defined in claim 1;
particularly,
R₄ is selected from the group consisting of H atom, -OH, -COOH, -NH₂, -CN, halogen, C₁₋₆ alkyl, C₁₋₆ alkoxy, C₁₋₆ haloalkyl, C₁₋₆ haloalkoxy and C₁₋₆ hydroxyalkyl; and preferably, R₄ is H atom.

5. The compound according to claim 1, being a compound of formula (III), 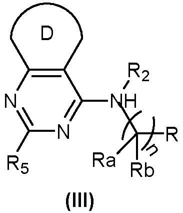
wherein ring D is selected from the group consisting of 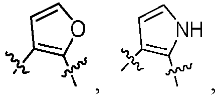 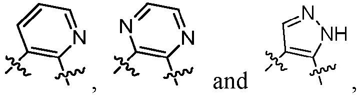 wherein the above groups are each independently optionally substituted by one or more R₁; and
R, Rₐ, R_{b}, R₁, R₂, R₅ and n are as defined in claim 1;
particularly, R₂ is selected from the group consisting of H atom, -OH, C₁₋₆ alkyl, C₂₋₆ alkynyl, -C₁₋₆ alkyl-C₆₋₁₀ aryl, C₁₋₆ alkoxy, C₁₋₆ haloalkyl, C₁₋₆ haloalkoxy and C₁₋₆ hydroxyalkyl, wherein the C₁₋₆ alkyl or -C₁₋₆ alkyl-C₆₋₁₀ aryl is each optionally substituted by one or more R₁, R₁ is as defined in claim 1;
preferably, R₂ is H atom or

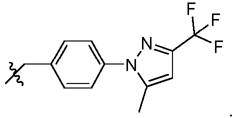

6. The compound according to any one of claims 1 to 5, wherein
ring A and ring B are each independently selected from the group consisting of phenyl, piperidinyl, cyclohexyl, cyclopropyl, cyclobutyl, pyridinyl, pyrimidinyl, imidazolyl, pyrazolyl, bicyclo[2.2.2]octanyl, 2-oxabicyclo[2.2.2]octanyl, pentacyclooctanyl, isoindolinonyl, imidazo[1,2-a]pyrazinyl, piperidine-2,6-dionyl, thienyl, furanyl, cyclopentyl, pyranyl, pyrrolidinyl, piperazinyl, morpholinyl, naphthyl, pyrrolyl, pyrazinyl, pyridazinyl, triazolyl, tetrazolyl, indolyl, isoindolyl, indolinyl, isoindolinyl, indolinonyl, pyrido[3,2-d]pyrimidinyl, pteridinyl, pyrazolo[4,3-c]pyridinyl, pyrazolo[3,4-d]pyrimidinyl and cubanyl, wherein ring A and ring B are each independently optionally substituted by one or more R₁, R₁ is as defined in claim 1;
L is selected from the group consisting of a chemical bond, -O-, -O-C₁₋₆ alkyleneand -C₁₋₆ alkylene-O-;
particularly, 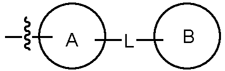
is selected from the group consisting of 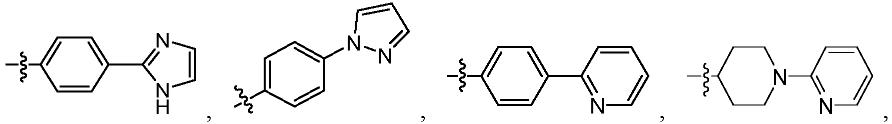 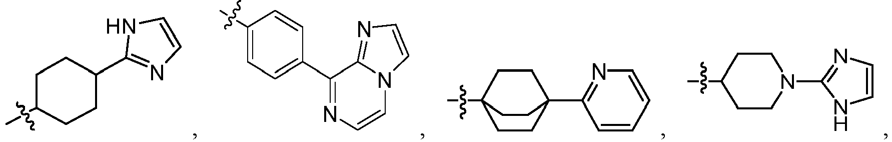 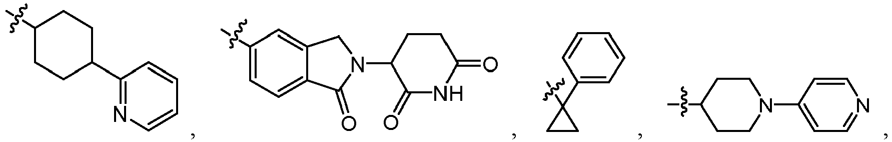 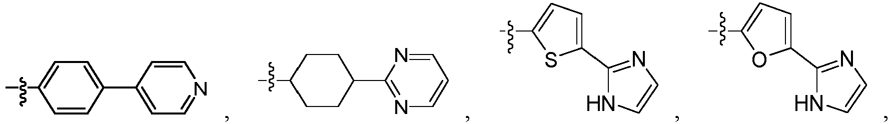 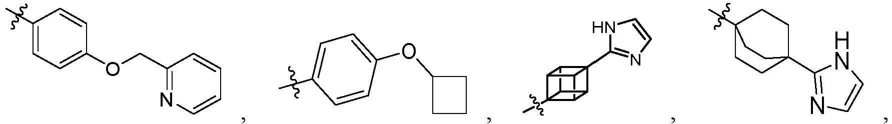 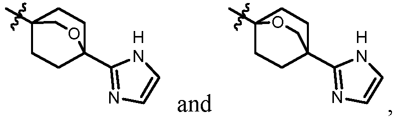 wherein ring A and ring B are each independently optionally substituted by one or more R₁, R₁ is as defined in claim 1;
more particularly, 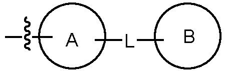
is selected from the group consisting of

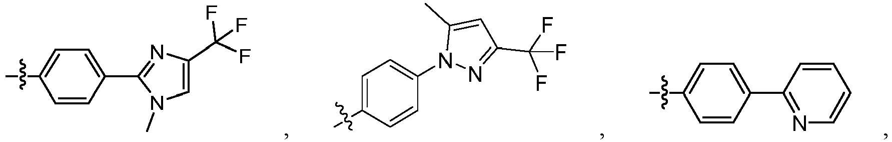

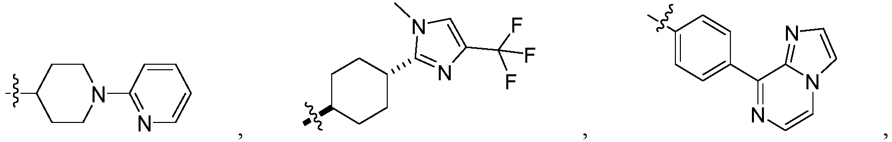

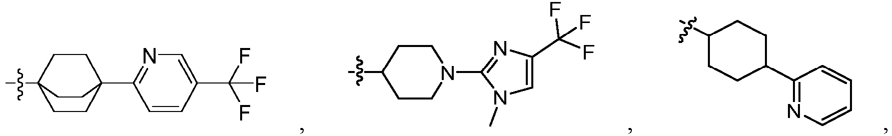

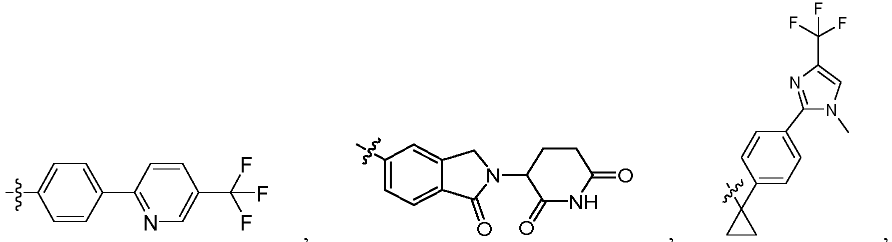

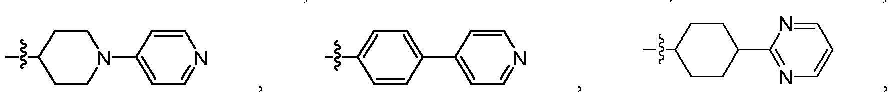

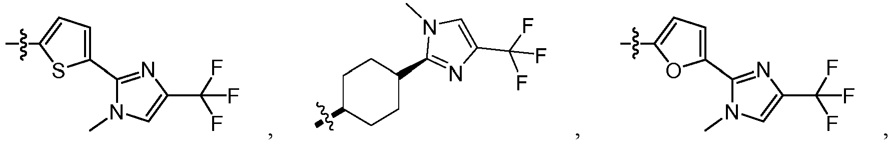

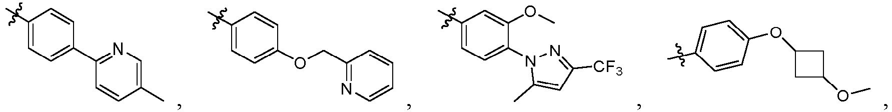

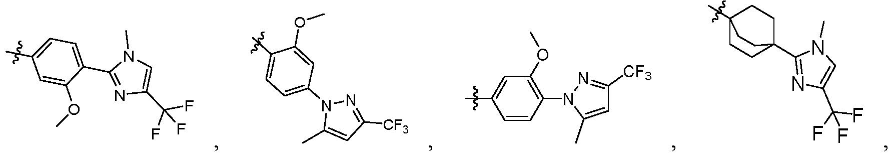

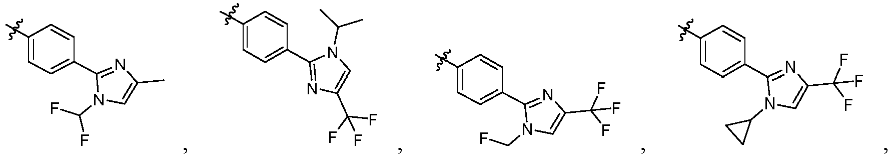

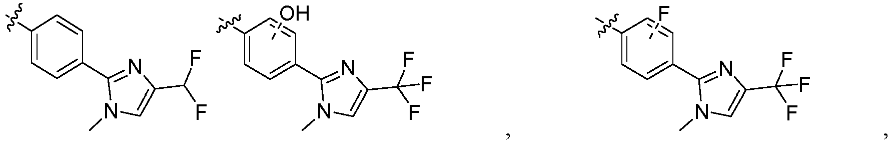

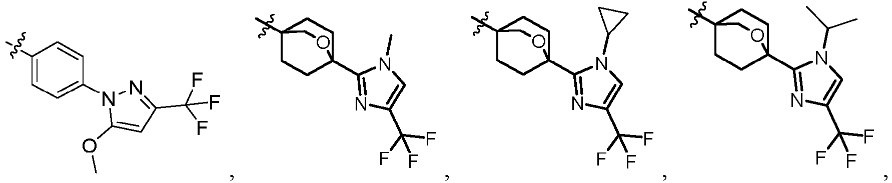

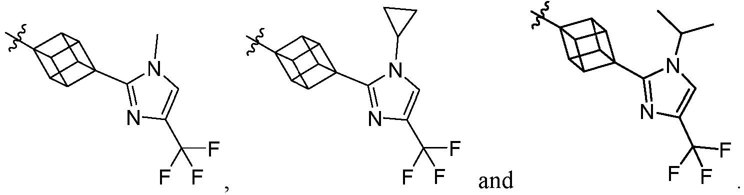

7. The compound according to claim 1, wherein R is a C₆₋₁₀ aryl or C₆₋₁₀ aryl fused with 5 to 6 membered heteroaryl, wherein the above groups are each independently optionally substituted by one or more substituent(s) selected from the group consisting of oxo, C₁₋₆ alkyl and -O-C₁₋₆ alkylene-O-C₁₋₆ alkyl;
particularly, R is 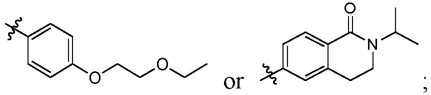
R₂ is selected from the group consisting of H atom, -OH, C₁₋₆ alkyl, C₂₋₆ alkynyl, - C₁₋₆ alkyl-C₆₋₁₀ aryl, C₁₋₆ alkoxy, C₁₋₆ haloalkyl, C₁₋₆ haloalkoxy and C₁₋₆ hydroxyalkyl; and preferably, R₂ is H atom;
R₃ is selected from H atom, -OH, -COOH, -NH₂, -CN, halogen, C₁₋₆ alkyl, C₂₋₆ alkynyl, C₁₋₆ alkoxy, C₁₋₆ haloalkyl, C₁₋₆ haloalkoxy and C₁₋₆ hydroxyalkyl; and preferably, R₃ is methoxy;
R₄ is selected from the group consisting of H atom, -OH, -COOH, -NH₂, -CN, halogen, C₁₋₆ alkyl, C₁₋₆ alkoxy, C₁₋₆ haloalkyl, C₁₋₆ haloalkoxy and C₁₋₆ hydroxyalkyl; and preferably, R₄ is H atom;
or, R₃ and R₄ together with the atom to which they are attached form ring D, ring D is a 5 to 7 membered heteroaryl or 5 to 7 membered heterocyclyl, and preferably furanyl.

8. The compound according to any one of claims 1 to 7, wherein R₅ is selected from the group consisting of C₆₋₁₀ aryl, 5 to 6 membered heteroaryl, C₆₋₁₀ aryl fused with 5 to 6 membered heterocyclyl, and C₆₋₁₀ aryl fused with 5 to 6 membered heteroaryl, and preferably selected from the group consisting of phenyl, pyridyl, pyrimidinyl, pyrazolyl, imidazolyl, thiazolyl, indolyl, indolinyl and isoxazolyl, wherein the C₆₋₁₀ aryl, 5 to 6 membered heteroaryl, C₆₋₁₀ aryl fused with 5 to 6 membered heterocyclyl, and C₆₋₁₀ aryl fused with 5 to 6 membered heteroaryl are each independently optionally substituted by one or more substituent(s) selected from the group consisting of -OH, -COOH, -NH₂, - CN, halogen, C₁₋₆ alkyl, C₁₋₆ haloalkyl, C₁₋₆ alkoxy, C₁₋₆ haloalkoxy, C₁₋₆ hydroxyalkyl and C₃₋₆ cycloalkyl;
particularly, R₅ is selected from the group consisting of

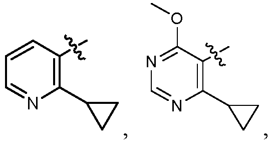

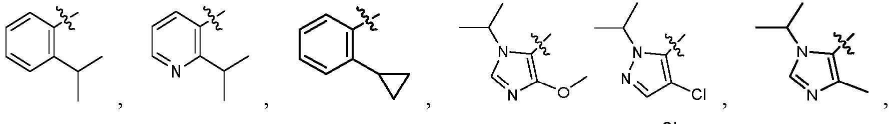

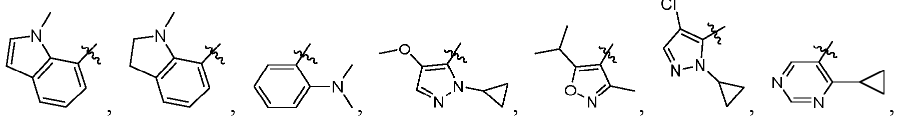

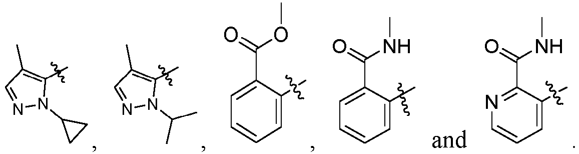

9. The compound according to any one of claims 1 to 8, wherein n is 0 or 1;
Rₐ and R_{b} are each independently selected from the group consisting of H atom, - CN, C₁₋₆ alkyl, -OH and halogen;
preferably, Rₐ and R_{b} are each independently H atom or -CN.

10. The compound according to any one of claims 1 to 9, being selected from the group consisting of

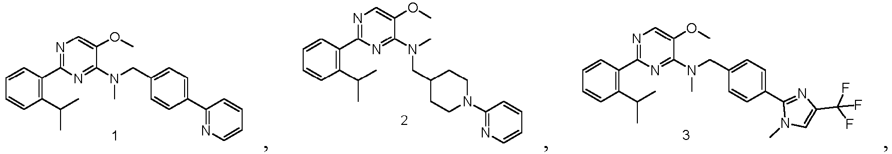

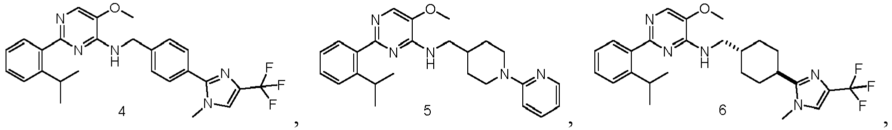

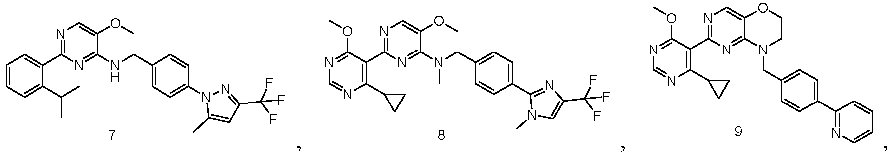

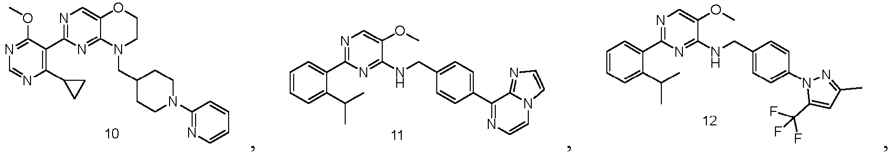

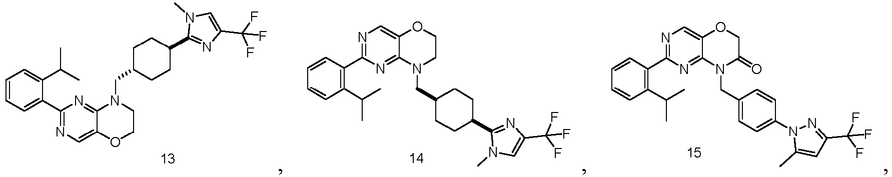

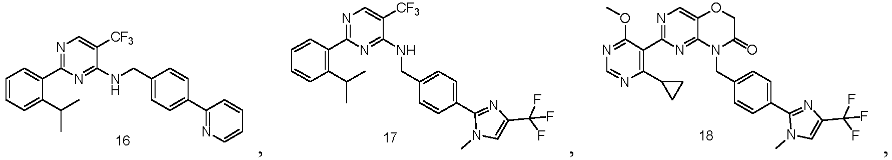

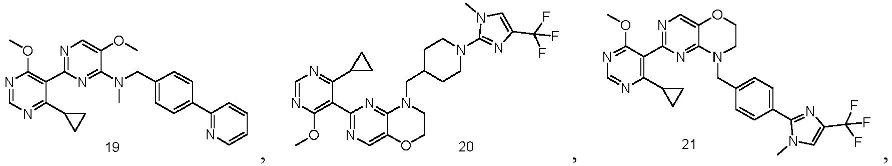

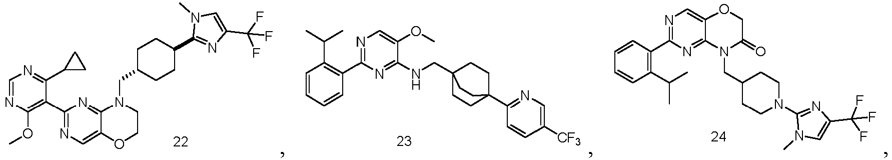

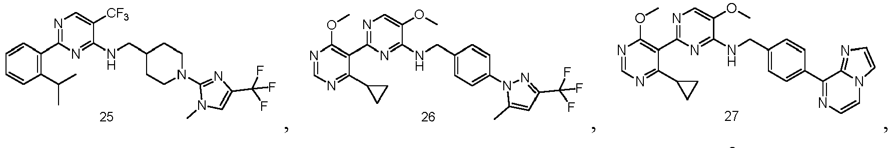

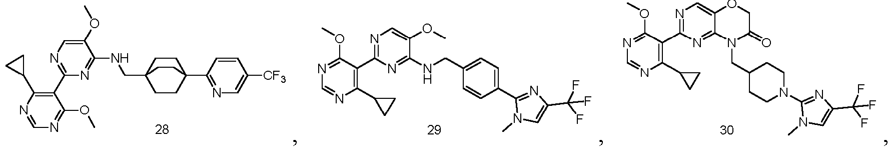

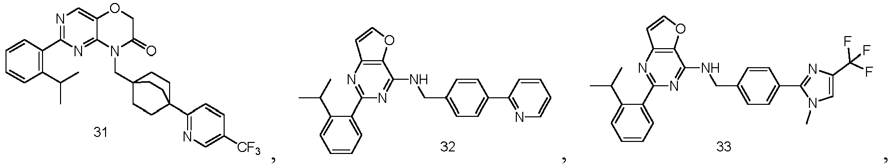

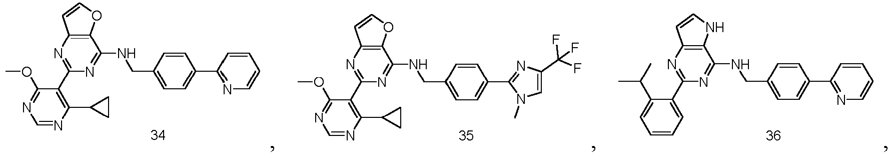

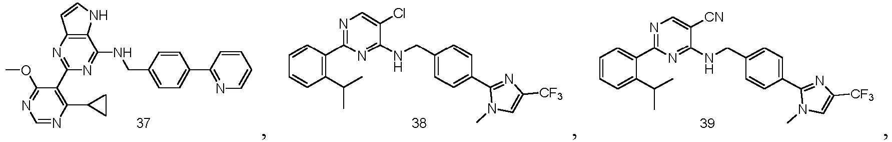

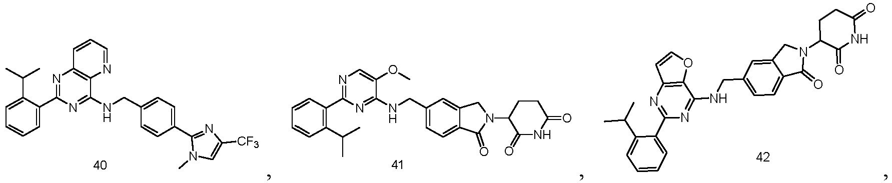

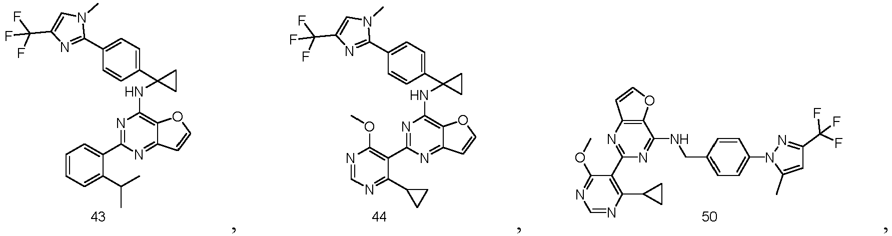

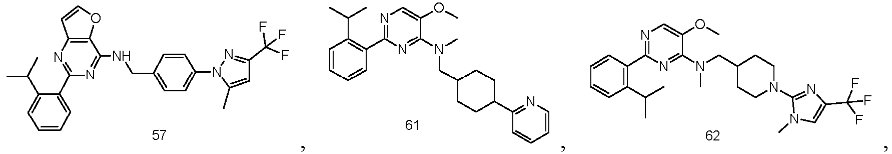

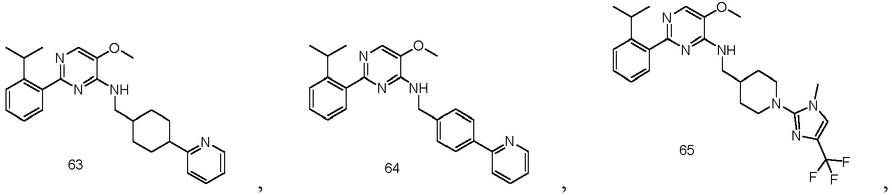

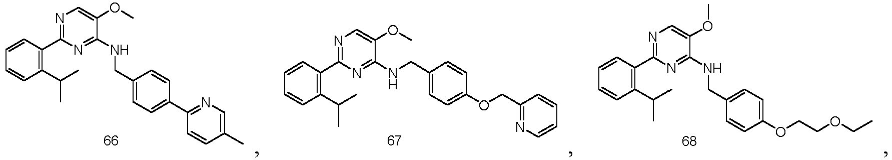

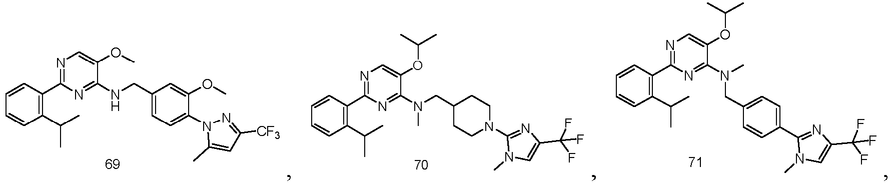

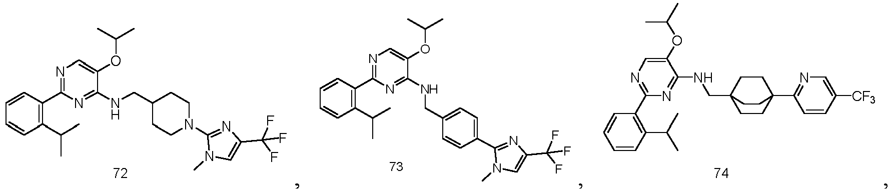

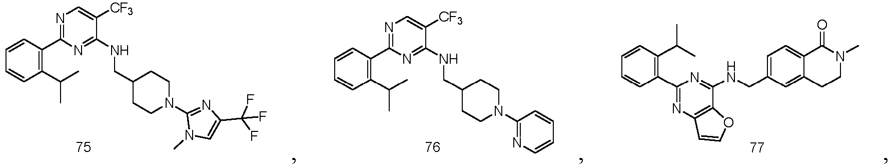

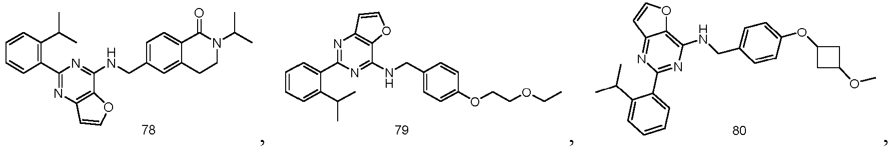

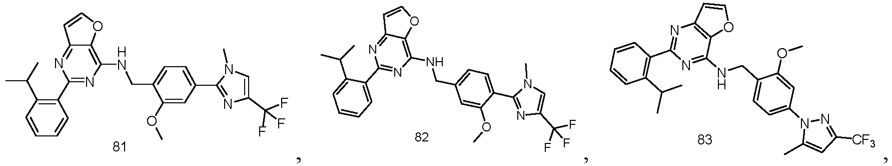

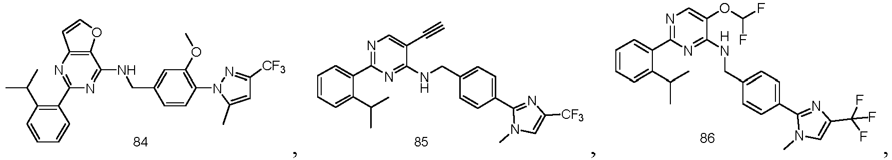

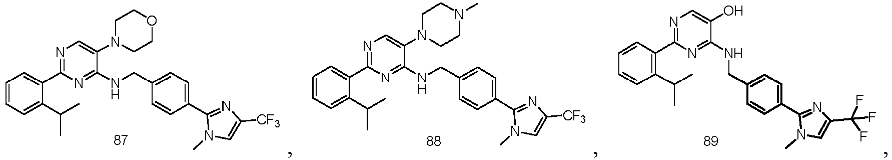

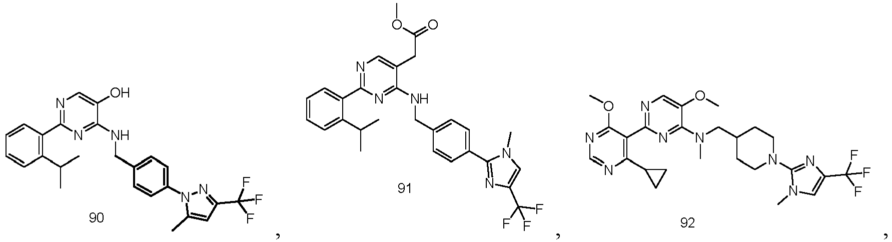

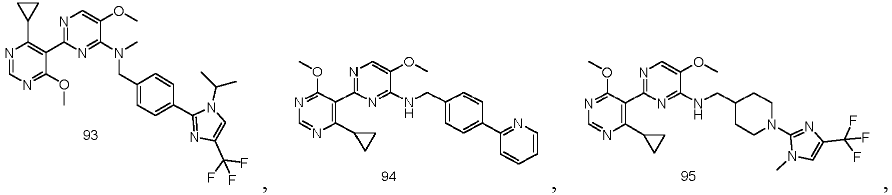

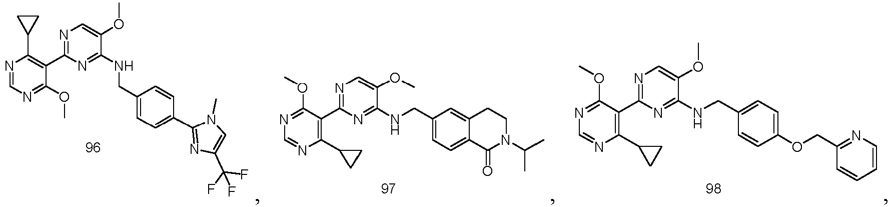

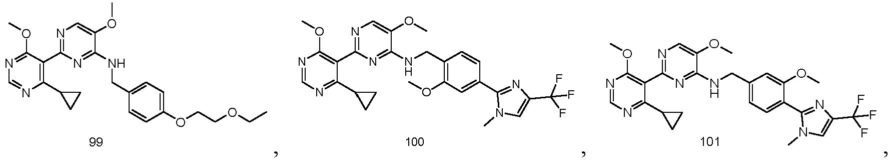

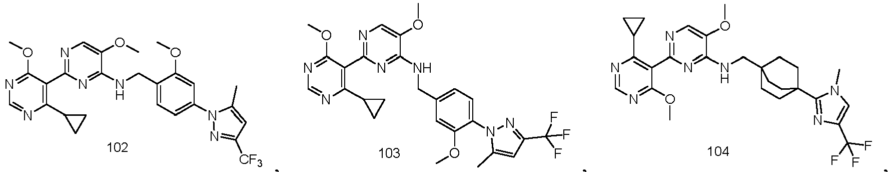

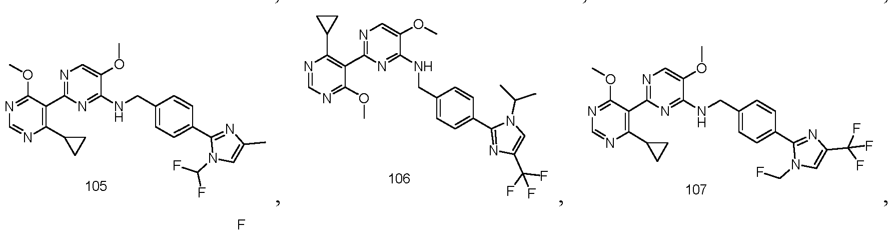

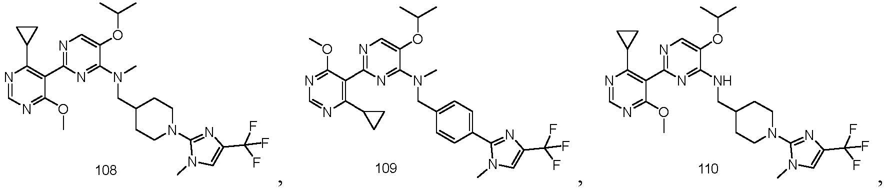

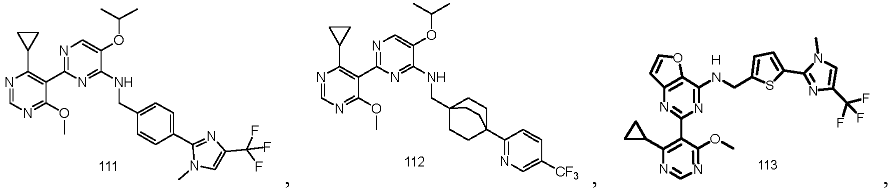

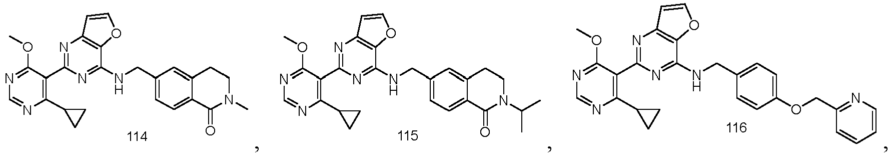

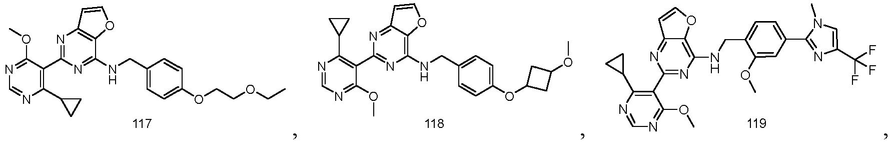

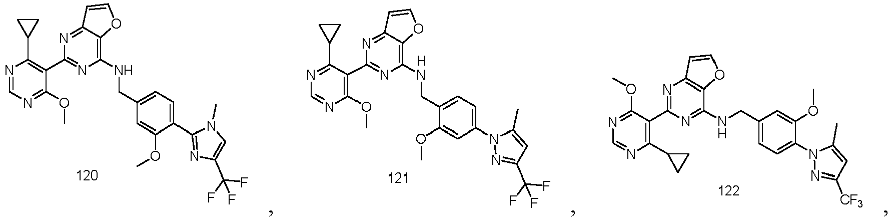

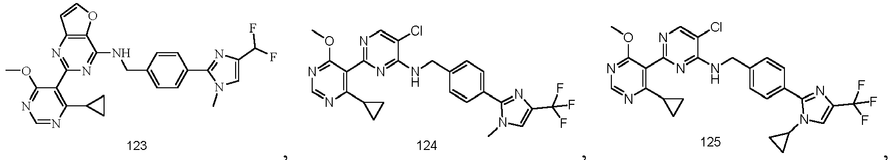

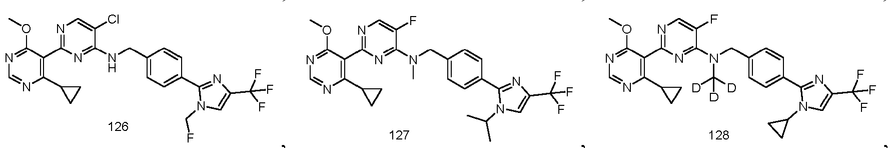

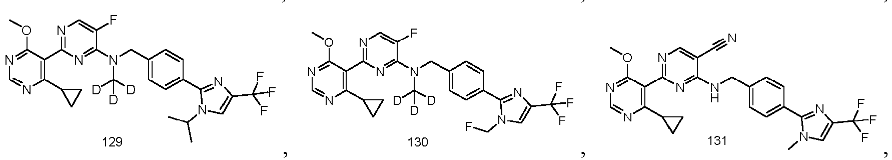

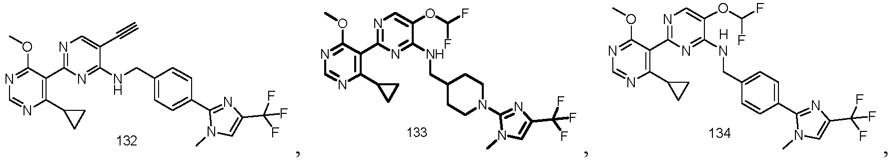

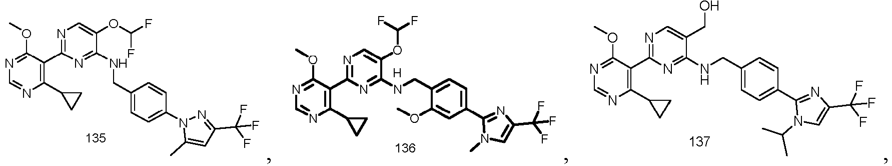

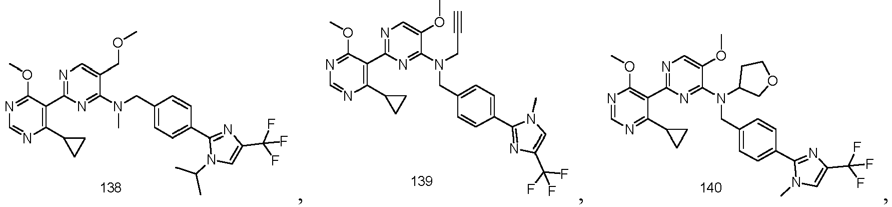

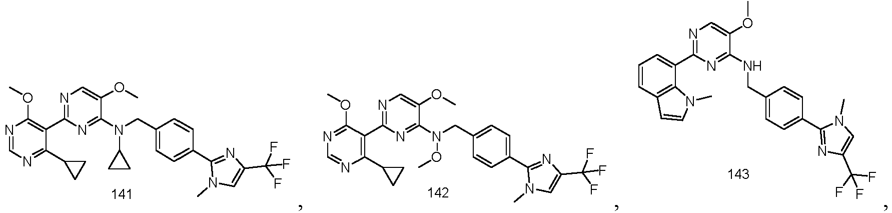

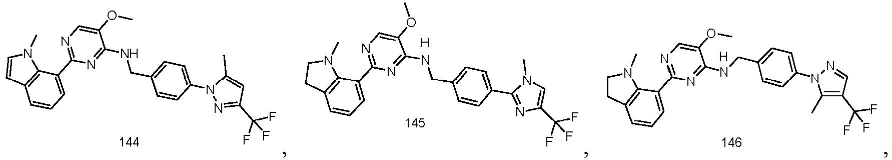

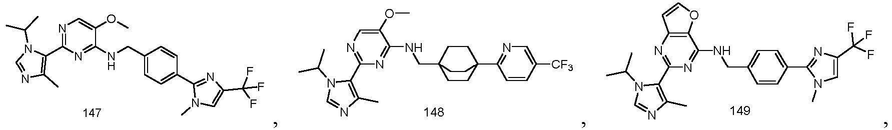

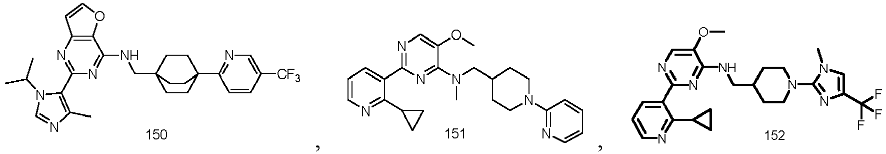

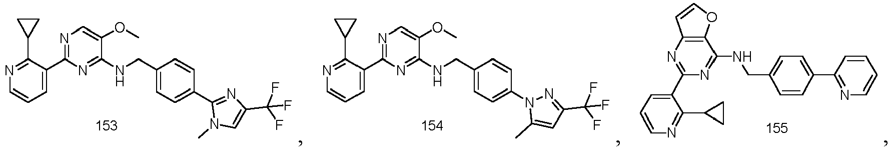

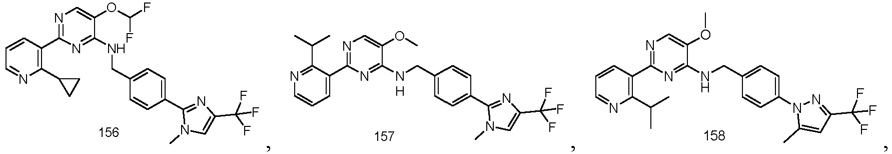

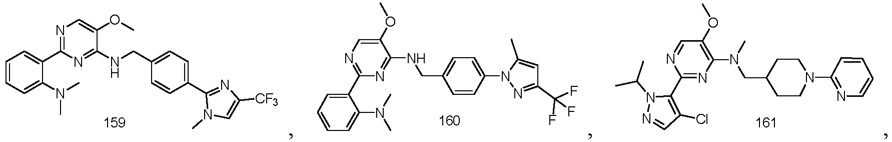

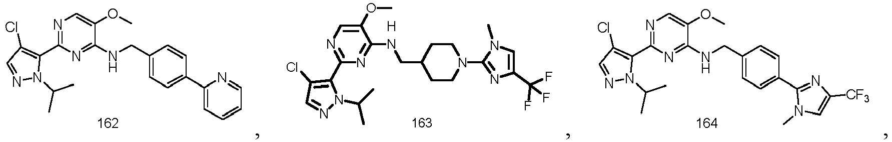

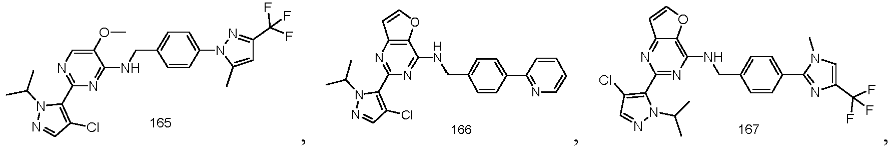

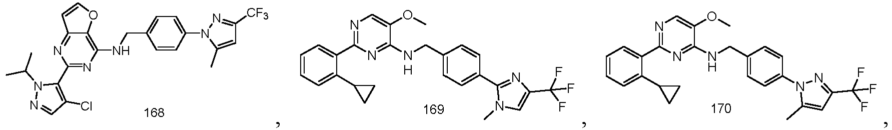

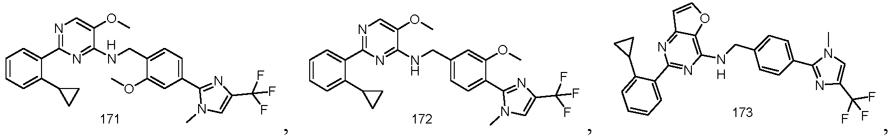

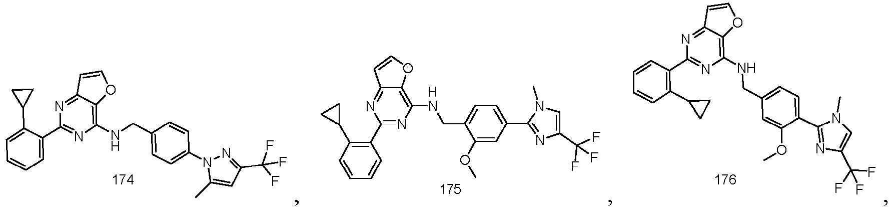

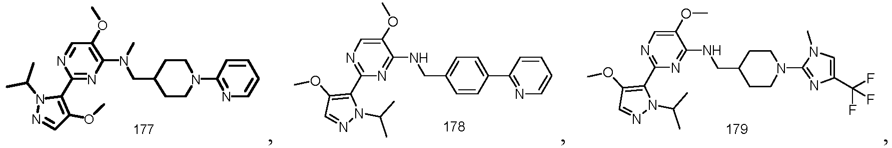

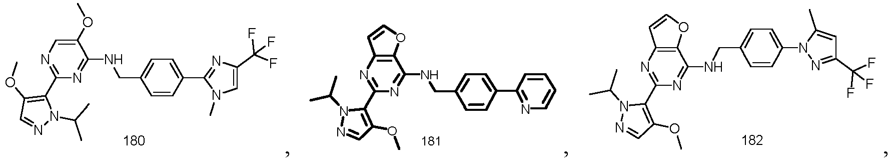

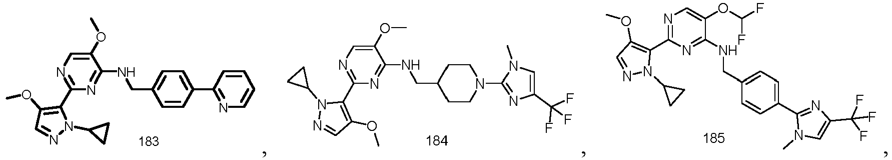

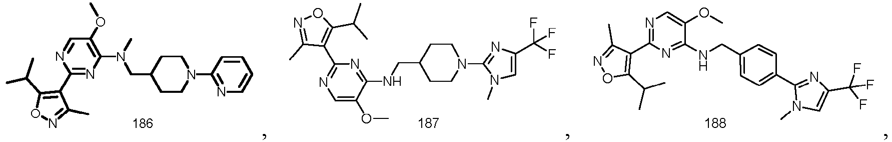

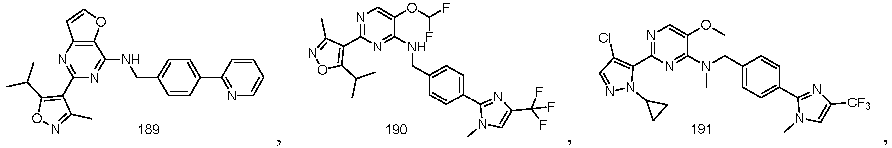

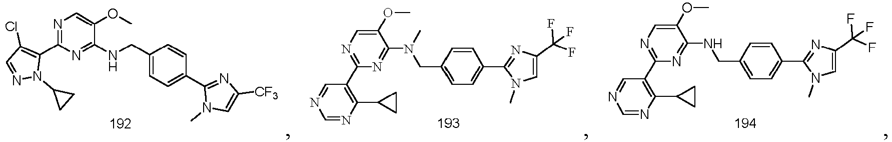

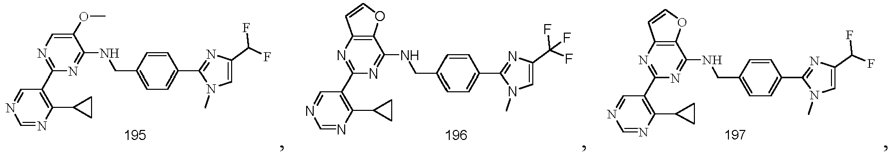

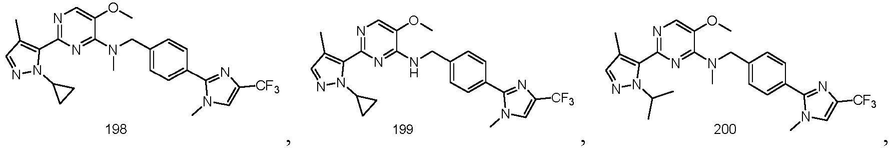

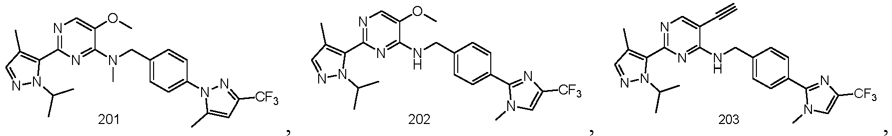

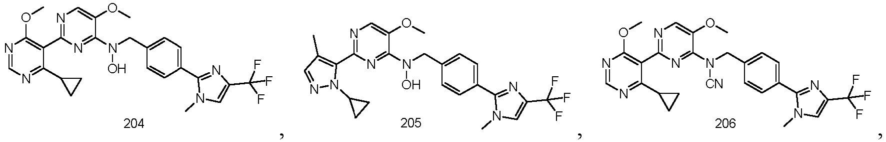

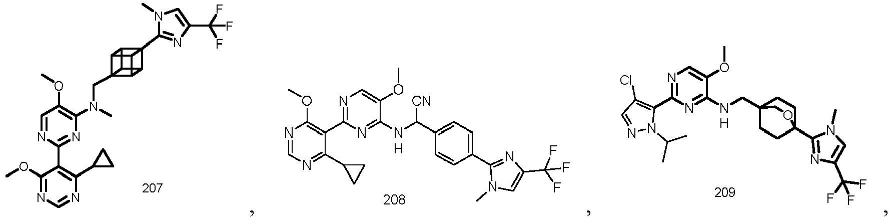

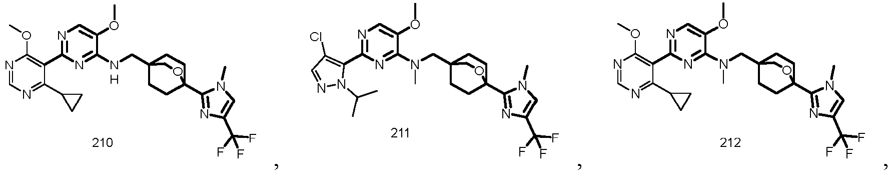

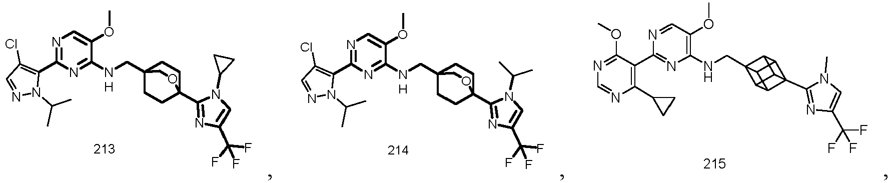

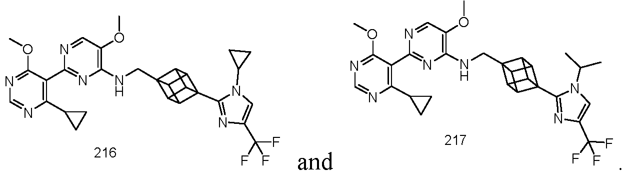

11. A method for preparing the compound according to claim 1, comprising the following step of: 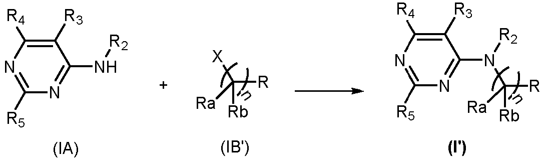
reacting a compound of formula (IA) with a compound of formula (IB') to obtain the compound of formula (I');
X is a leaving group selected from the group consisting of halogen, sulfonate, boronic acid and borate; and
R, Rₐ, R_{b}, R₂ to R₅ and n are as defined in claim 1;
or 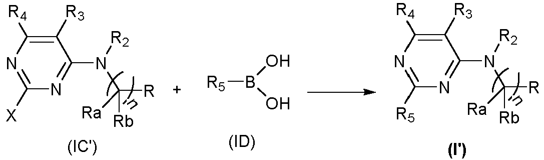
reacting a compound of formula (IC') with a compound of formula (ID) to obtain the compound of formula (I');
X is a leaving group selected from the group consisting of halogen, sulfonate, boronic acid and borate; and
R, Rₐ, R_{b}, R₂ to R₅ and n are as defined in claim 1;
or 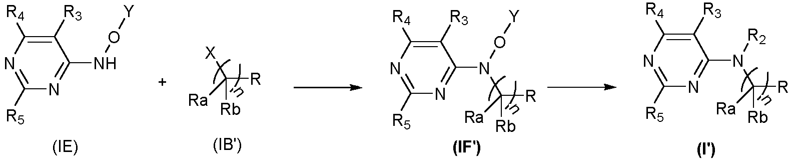
reacting a compound of formula (IE) with a compound of formula (IB') to obtain a compound of formula (IF'), and deprotecting the compound of formula (IF') to obtain the compound of formula (I');
X is a leaving group selected from the group consisting of halogen, sulfonate, boronic acid and borate;
Y is a hydroxy protecting group selected from the group consisting of *tert*butyldimethylsilyl, trimethylsilyl, triethylsilyl, triisopropylsilyl, tert-butyldiphenylsilyl, benzyl, methoxymethyl and ethoxyethyl;
R₂ is hydroxy;
R, Rₐ, R_{b}, R₃ to R₅ and n are as defined in claim 1;
or 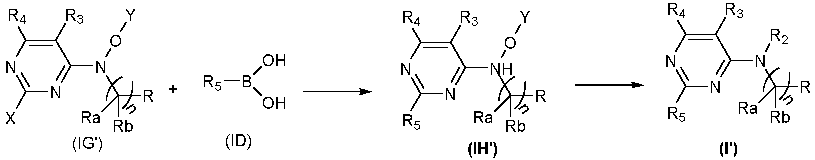
reacting a compound of formula (IG') with a compound of formula (ID) to obtain a compound of formula (IH'), and deprotecting the compound of formula (IH') to obtain the compound of formula (I');
X is a leaving group selected from the group consisting of halogen, sulfonate, boronic acid and borate;
Y is a hydroxy protecting group selected from the group consisting of *tert*butyldimethylsilyl, trimethylsilyl, triethylsilyl, triisopropylsilyl, tert-butyldiphenylsilyl, benzyl, methoxymethyl and ethoxyethyl;
R₂ is hydroxy;
R, Rₐ, R_{b}, R₃ to R₅ and n are as defined in claim 1;
or 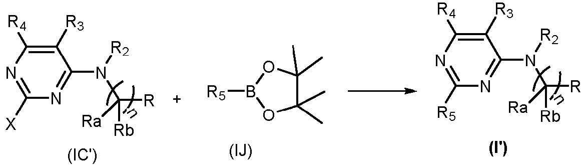
reacting a compound of formula (IC') with a compound of formula (IJ) to obtain the compound of formula (I');
X is a leaving group selected from the group consisting of halogen, sulfonate, boronic acid and borate; and
R, Rₐ, R_{b}, R₂ to R₅ and n are as defined in claim 1.

12. The method according to claim 11, wherein the compound of formula (I') is a compound of formula (I), the method comprises the following step of: 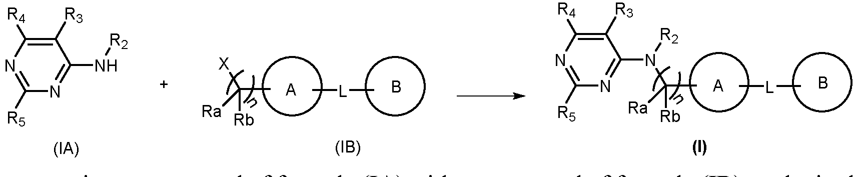
reacting a compound of formula (IA) with a compound of formula (IB) to obtain the compound of formula (I);
X is a leaving group selected from the group consisting of halogen, sulfonate, boronic acid and borate; and
ring A, ring B, L, Rₐ, R_{b}, R₂ to R₅ and n are as defined in claim 1;
or 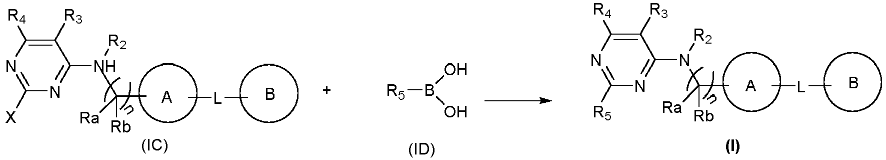
reacting a compound of formula (IC) with a compound of formula (ID) to obtain the compound of formula (I);
X is a leaving group selected from the group consisting of halogen, sulfonate, boronic acid and borate; and
ring A, ring B, L, Rₐ, R_{b}, R₂ to R₅ and n are as defined in claim 1;
or 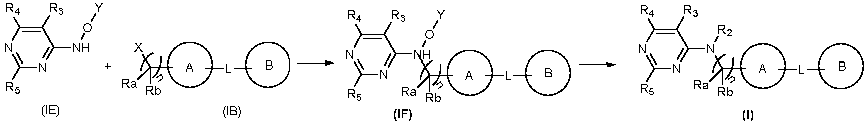
reacting a compound of formula (IE) with a compound of formula (IB) to obtain a compound of formula (IF), and deprotecting the compound of formula (IF) to obtain the compound of formula (I);
X is a leaving group selected from the group consisting of halogen, sulfonate, boronic acid and borate;
Y is a hydroxy protecting group selected from the group consisting of *tert*butyldimethylsilyl, trimethylsilyl, triethylsilyl, triisopropylsilyl, *tert*-butyldiphenylsilyl, benzyl, methoxymethyl and ethoxyethyl;
R₂ is a hydroxy;
ring A, ring B, L, Rₐ, R_{b}, R₃ to R₅ and n are as defined in claim 1;
or 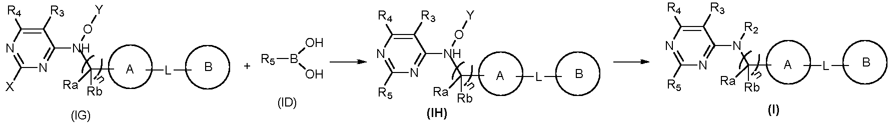
reacting a compound of formula (IG) with a compound of formula (ID) to obtain a compound of formula (IH), and deprotecting the compound of formula (IH) to obtain the compound of formula (I);
X is a leaving group selected from the group consisting of halogen, sulfonate, boronic acid and borate;
Y is a hydroxy protecting group selected from the group consisting of *tert*butyldimethylsilyl, trimethylsilyl, triethylsilyl, triisopropylsilyl, *tert*-butyldiphenylsilyl, benzyl, methoxymethyl and ethoxyethyl;
R₂ is a hydroxy;
ring A, ring B, L, Rₐ, R_{b}, R₃ to R₅ and n are as defined in claim 1;
or 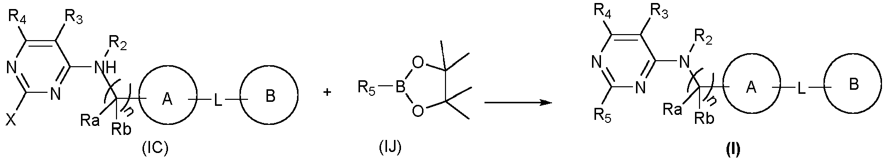
reacting a compound of formula (IC) with a compound of formula (IJ) to obtain the compound of formula (I);
X is a leaving group selected from the group consisting of halogen, sulfonate, boronic acid and borate; and
ring A, ring B, L, Rₐ, R_{b}, R₂ to R₅ and n are as defined in claim 1.

13. A pharmaceutical composition, comprising the compound according to any one of claims 1 to 10, and one or more pharmaceutically acceptable excipient(s).

14. Use of the compound according to any one of claims 1 to 10 or the pharmaceutical composition according to claim 13 in the preparation of a medicament for treating or preventing diseases or conditions associated with inhibition of ubiquitinspecific protease 1 (USP1).

15. Use of the compound according to any one of claims 1 to 10 or the pharmaceutical composition according to claim 13 in the preparation of a medicament for treating or preventing cancer,
particularly, the cancer is selected from the group consisting of lung cancer, nonsmall cell lung cancer (NSCLC), colon cancer, bladder cancer, osteosarcoma, ovarian cancer, skin cancer, and breast cancer.
